# EUROPÄISCHE PATENTSCHRIFT

(11) **EP 1 730 144 B1**
(45) Veröffentlichungstag und Bekanntmachung des Hinweises auf die Patenterteilung: **10.06.2009**
(21) Anmeldenummer: 05716300.8
(22) Anmeldetag: 22.03.2005
(51) Int. Cl.: C07D 471/04, A61K 31/437, A61P 25/24

(54) **SUBSTITUIERTE 1,4,8-TRIAZASPIRO¬4.5 DECAN-2-ON-VERBINDUNGEN**
SUBSTITUTED 1,4,8-TRIAZASPIRO¬4.5 DECAN-2-ONE COMPOUNDS
COMPOSES DE 1,4,8-TRIAZASPIRO¬4.5 DECAN-2-ONE SUBSTITUES

(30) Priorität: 22.03.2004 DE 102004014304
(43) Veröffentlichungstag der Anmeldung: 13.12.2006
(73) Patentinhaber: Grünenthal GmbH, 52078 Aachen (DE)
(72) Erfinder: SUNDERMANN, Corinna, 52074 Aachen (DE); SUNDERMANN, Bernd, 52074 Aachen (DE); OBERBÖRSCH, Stefan, 52074 Aachen (DE); ENGLBERGER, Werner, 52223 Stolberg (DE); HENNIES, Hagen-Heinrich, 52152 Simmerath (DE)
(74) Vertreter: Brosch, Oliver
(86) Internationale Anmeldenummer: PCT/EP2005/003051
(87) Internationale Veröffentlichungsnummer: WO 2005/095398

(56) Entgegenhaltungen:
- WO-A-94/10171
- WO-A-94/21619

## Beschreibung

Die vorliegende Erfindung betrifft substituierte 1,4,8-Triazaspiro[4.5]decan-2-on-Verbindungen, Verfahren zu deren Herstellung, Arzneimittel enthaltend diese Verbindung sowie die Verwendung dieser Verbindungen zur Herstellung von Arzneimitteln.

Depression ist eine Störung der Affektivität, bei der ein depressives Syndrom im Vordergrund steht, wobei depressiv mit Verstimmung verbunden bzw. traurig gestimmt heißt. Die zur Therapie verwendeten Antidepressiva sind auch wichtige Adjuvantien für die Schmerztherapie (wie beispielsweise in der Veröffentlichung von Tzschentke, NA and 5-HT Reuptake Inhibitors and α₂ agonists, in Analgesics: From Chemistry and Pharmacology to Clinical Application, S. 265-284, Wiley 2002 beschrieben), insbesondere bei chronischen Schmerauständen, da die andauernde Schmerzbelastung zu einer depressiven Verstimmung der Patienten führen kann. Dies ist besonders häufig bei Krebs-Schmerzpatienten der Fall (Berard, Int. Med. J. 1996, 3/4, 257-259). Die Behandlung von Depressionen hat daher in der Medizin eine große Bedeutung und es besteht ein weltweiter Bedarf an wirksamen antidepressiven Therapien. Grundlage für eine solche antidepressive Wirkung eines pharmakologischen Wirkstoffs ist dessen Hemmung der Wiederaufnahme von Serotonin. WO 9410171 und WO 94/21619 offenbaren Serotonin-Agonisten/Antagonisten basierend auf einem stickstoffhaltigen aromatischen bizyklischen System enthaltend einen 5-Ring mit 2 Stickstoffatomen.

Eine Aufgabe der vorliegenden Erfindung bestand daher darin, Verbindungen zur Verfügung zu stellen, die sich insbesondere als pharmazeutische Wirkstoffe in Arzneimitteln eignen, bevorzugt in Arzneimitteln zur Prophylaxe und/oder Behandlung von Depressionen.

Es wurde nun überraschenderweise gefunden, daß die substituierten 1,4,8-Triazaspiro[4.5]decan-2-on-Verbindungen der nachstehend angegebenen allgemeinen Formel I stich zur Regulation, vorzugsweise zur Inhibierung, der Serotonin-(5-Hydroxy-Tryptophan)-Wiederaufnahme eignen. Des weiteren wurde gefunden, daß sich die erfindungsgemäßen Verbindungen auch zur Regulation, vorzugsweise zur Inhibierung, der Noradrenalin-Wiederaufnahme eignen und ferner eine hohe Affinität für Batrachotoxin-(BTX)-Rezeptoren und/oder Cannabinoid-Rezeptoren CB2 (CB2-Rezeptoren) aufweisen und sich daher insbesondere als pharmazeutische Wirkstoffe in Arzneimitteln zur Prophylaxe und/oder Behandlung von mit diesen Rezeptoren bzw. Prozessen in Verbindung stehenden Erkrankungen eignen.

Ein Gegenstand der vorliegenden Erfindung sind daher substituierte 1,4,8-Triazaspiro[4.5]decan-2-on-Verbindungen der allgemeinen Formel I, 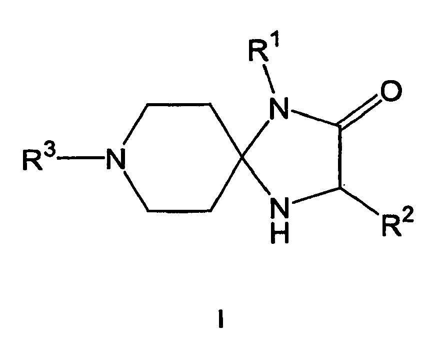 worin
R¹ für einen Wasserstoff-Rest, für einen linearen oder verzweigten, unsubstituierten oder wenigstens einfach substituierten, ggf. wenigstens ein Heteroatom als Kettenglied aufweisenden Alkyl-Rest, für einen linearen oder verzweigten, unsubstituierten oder wenigstens einfach substituierten, ggf. wenigstens ein Heteroatom als Kettenglied aufweisenden Alkenyl-Rest, für einen linearen oder verzweigten, unsubstituierten oder wenigstens einfach substituierten, ggf. wenigstens ein Heteroatom als Kettenglied aufweisenden Alkinyl-Rest, für einen unsubstituierten oder wenigstens einfach substituierten Aryl- oder Heteroaryl-Rest, der über eine lineare oder verzweigte, ggf. wenigstens ein Heteroatom als Kettenglied aufweisende Alkylen-Gruppe gebunden ist, oder für einen über eine lineare oder verzweigte Alkylen-Gruppe gebundenen -C(=O)-OR⁷-Rest steht,
R² für einen Wasserstoff-Rest, für einen linearen oder verzweigten, unsubstituierten oder wenigstens einfach substituierten, ggf. wenigstens ein Heteroatom als Kettenglied aufweisenden Alkyl-Rest, für einen linearen oder verzweigten, unsubstituierten oder wenigstens einfach substituierten, ggf. wenigstens ein Heteroatom als Kettenglied aufweisenden Alkenyl-Rest, für einen linearen oder verzweigten, unsubstituierten oder wenigstens einfach substituierten, ggf. wenigstens ein Heteroatom als Kettenglied aufweisenden Alkinyl-Rest, oder für einen unsubstituierten oder wenigstens einfach substituierten Aryl- oder Heteroaryl-Rest steht, der über eine lineare oder verzweigte, ggf. wenigstens ein Heteroatom als Kettenglied aufweisende Alkylen-Gruppe gebunden sein kann,
R³ für eine -S(=O)₂-R⁴-Gruppe, für eine -C(=S)-NH-R⁵-Gruppe oder für eine -C(=O)-NH-R⁶-Gruppe steht,
R⁴ für einen -NR¹⁰R¹¹-Rest, für einen linearen oder verzweigten, unsubstituierten oder wenigstens einfach substituierten, ggf. wenigstens ein Heteroatom als Kettenglied aufweisenden Alkyl-Rest, für einen linearen oder verzweigten, unsubstituierten oder wenigstens einfach substituierten, ggf. wenigstens ein Heteroatom als Kettenglied aufweisenden Alkenyl-Rest, für einen linearen oder verzweigten, unsubstituierten oder wenigstens einfach substituierten, ggf. wenigstens ein Heteroatom als Kettenglied aufweisenden Alkinyl-Rest, für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-Gruppe gebunden und/oder mit einem unsubstituierten oder wenigstens einfach substituierten monozyklischen Ringsystem kondensiert sein kann, oder für einen unsubstituierten oder wenigstens einfach substituierten, ggf. wenigstens ein Heteroatom als Ringglied aufweisenden, cycloaliphatischen Rest steht, der über eine lineare oder verzweigte, unsubstituierte oder wenigstens einfach substituierte, ggf. wenigstens ein Heteroatom als Kettenglied aufweisende Alkylen-Gruppe gebunden und/oder mit einer linearen oder verzweigten, unsubstituierten oder wenigstens einfach substituierten Alkylen-Gruppe überbrückt sein kann,
R⁵ für einen linearen oder verzweigten, unsubstituierten oder wenigstens einfach substituierten, ggf. wenigstens ein Heteroatom als Kettenglied aufweisenden Alkyl-Rest, für einen linearen oder verzweigten, unsubstituierten oder wenigstens einfach substituierten, ggf. wenigstens ein Heteroatom als Kettenglied aufweisenden Alkenyl-Rest, für einen linearen oder verzweigten, unsubstituierten oder wenigstens einfach substituierten, ggf. wenigstens ein Heteroatom als Kettenglied aufweisenden Alkinyl-Rest, für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-Gruppe gebunden sein kann, für einen unsubstituierten oder wenigstens einfach substituierten, ggf. wenigstens ein Heteroatom als Ringglied aufweisenden, cycloaliphatischen Rest steht, der über eine lineare oder verzweigte, unsubstituierte oder wenigstens einfach substituierte, ggf. wenigstens ein Heteroatom als Kettenglied aufweisende Alkylen-Gruppe gebunden sein kann, für einen -C(=O)-OR⁸-Rest oder für einen über eine lineare oder verzweigte Alkylen-Gruppe gebundenen -C(=O)-OR⁹-Rest steht,
R⁶ für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-Gruppe gebunden sein kann, oder für einen unsubstituierten oder wenigstens einfach substituierten, ggf. wenigstens ein Heteroatom als Ringglied aufweisenden, cycloaliphatischen Rest steht, der über eine lineare oder verzweigte, unsubstituierte oder wenigstens einfach substituierte, ggf. wenigstens ein Heteroatom als Kettenglied aufweisende Alkylen-Gruppe gebunden sein kann,
R⁷, R⁸, R⁹, R¹⁰, R¹¹, unabhängig voneinander, jeweils für einen linearen oder verzweigten Alkyl-Rest, einen linearen oder verzweigten Alkenyl-Rest oder einen linearen oder verzweigten Alkinyl-Rest stehen,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Sofern einer der Reste R¹ bis R¹⁸ für einen Alkyl-, Alkenyl- oder Alkinyl-Rest steht oder einen solchen Rest aufweist, kann dieser Rest - sofern nicht anders angegeben - unsubstituiert oder einfach oder mehrfach substituiert sein, vorzugsweise mit 1, 2, 3, 4 oder 5 Substituenten substituiert sein, wobei die Substituenten jeweils unabhängig voneinander bevorzugt ausgewählt werden können aus der Gruppe bestehend aus F, Cl, Br, C₁-₆Alkoxy, Hydroxy, SH, CN, CF₃, CHF₃, CH₂F, -NO₂, unsubstituiertem Phenyl und -NR^{a}R^{b}, worin R^{a} und R^{b} jeweils unabhängig voneinander ausgewählt werden können aus der Gruppe bestehend aus H, Methyl, Ethyl, n-Propyl, iso-Propyl und unsubstituiertem Phenyl. Alkenyl-Reste weisen wenigstens eine C-C-Doppelbindung und Alkinyl-Reste wenigstens eine C-C-Dreifachbindung auf.

Geeignete Alkyl-, Alkenyl- und Alkinyl-Reste, die einfach oder mehrfach substituiert sein können, vorzugsweise mit 1, 2, 3, 4 oder 5 Substituenten substituiert sein können, können beispielsweise ausgewählt werden aus der Gruppe bestehend aus Methyl, Ethyl, n-Propyl, Isopropyl, n-Butyl, Isobutyl, sec-Butyt, tert-Butyl, n-Pentyl, Isopentyl, neo-Pentyl, 2-Pentyl, 3-Pentyl, -(CH₂)-C(CH₃)₃, 2,2-Dimethyl-propyl, penta-1,3-dienyl, n-Hexyl, 2-Hexyl, 3-Hexyl, -(CH₂)-(CH₂-C(CH₃)₃, n-Heptyl, 3-Heptyl, 4-Heptyl, n-Octyl, -(CH₂)-(CH)(C₂H₅)-(CH₂)-(CH₂)-(CH₂)-(CH₃), n-Nonyl, n-Decyl, Vinyl, Ethinyl, Propenyl, 1-Propenyl, 2-Propenyl, Allyl, Propinyl, 1-Propinyl, 2-Propinyl, Butenyl, 1-Butenyl, 2-Butenyl, 3-Butenyl, Butinyl, 1-Butinyl, 2-Butinyl, 3-Butinyl, Pentenyl, Pentinyl, Hexenyl und Hexinyl.

Die erfindungsgemäß vorliegenden Alkyl-, Alkenyl- und Alkinyl-Reste können - sofern nicht anders angegeben - auch eines oder mehrere Heteroatome, vorzugsweise eines oder mehrere Sauerstoffatome und/oder eines oder mehrere Schwefelatome, besonders bevorzugt 1 oder 2 Sauerstoffatome und/oder Schwefelatome, als Kettenglied(er) aufweisen. Vorzugsweise befinden sich diese Heteroatome in einer nicht-endständigen Position des jeweiligen Restes. Beispielhaft seien Reste wie -CH₂-CH₂-S-CH₃ oder -CH₂-CH₂-O-CH₃ genannt.

Sofern einer der Reste R¹ bis R¹⁸ für einen cycloaliphatischen Rest steht oder einen cycloaliphatischen Rest aufweist, kann dieser Rest - sofern nicht anders angegebenunsubstituiert oder einfach oder mehrfach substituiert sein, vorzugsweise mit 1, 2, 3, 4 oder 5 Substituenten substituiert sein, wobei die Substituenten jeweils unabhängig voneinander vorzugsweise ausgewählt werden können aus der Gruppe bestehend aus F, Cl, Br, C₁₋₆-Alkoxy, C₁₋₆-Alkyl, Hydroxy, CN, CF₃, CHF₃, CH₂F, unsubstituiertem Phenyl, -NR^{a}R^{b}, worin R^{a} und R^{b} jeweils unabhängig voneinander ausgewählt werden können aus der Gruppe bestehend aus H, Methyl, Ethyl, n-Propyl, iso-Propyl und unsubstituiertem Phenyl, Oxo (=O), Thioxo (=S), I, -SF₅, -NO₂, -O-CF₃, -S-CF₃, -SH, -S-C₁₋₅-Alkyl, -C(=O)-H, -C(=O)-C₁₋₅-Alkyl, -C(=O)-_{C1-5}-Perflouralkyl, -C(=O)-OH, -C(=O)-O-C₁₋₅-Alkyl, -(CH₂)-C(=O)-OH, -(CH₂)-C(=O)-O-C₁₋₅-Alkyl, -(CH₂)-Benzo[b]furanyl, -O-Phenyl, -O-Benzyl, Phenyl, Benzyl, Naphthyl und -(CH₂)-Naphthyl, wobei jeweils der zyklische Teil der Reste -O-Phenyl, -O-Benzyl, Phenyl, -(CH₂)-Benzo[b]furanyl, Benzyl, Naphthyl und -(CH₂)-Naphthyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann.

Unter cycloaliphatischen Resten werden im Sinne der vorliegenden Erfindung sowohl gesättigte als auch ungesättigte Reste verstanden. Die cycloaliphatischen Reste können ggf. eines oder mehrere Heteroatome, vorzugsweise 1, 2, 3, 4 oder 5 Heteroatom(e), vorzugsweise unabhängig voneinander ausgewählt aus der Gruppe bestehend aus Stickstoff, Sauerstoff und Schwefel, als Ringglieder aufweisen.

Geeignete cycloaliphatische Reste, die einfach oder mehrfach substituiert sein können, können beispielsweise ausgewählt werden aus der Gruppe bestehend aus Cyclopropyl, Cyclobutyl, Cyclopentyl, Cyclohexyl, Cycloheptyl, Cyclooctyl, Cyclopentenyl, Cyclohexenyl, Cycloheptenyl, Cyclooctenyl, etrahydrofuryl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl.

Die cycloaliphatischen Reste können ferner wenigstens einfach mit einer linearen oder verzweigten Alkylen-Gruppe überbrückt sein, vorzugsweise mit ein oder zwei linearen oder verzweigten C₁₋₅-Alkylen-Gruppen überbrückt sein. Als Beispiele für derartige cycloaliphatischen Reste seien der 4,7,7-Dimethyl-2-oxo-bicyclo-[2.2.1]-heptyl- bzw. der Adamantyl-Rest genannt.

Sofern einer der Reste R¹ bis R¹⁸ für einen Aryl- oder Heteroaryl-Rest steht oder einen Aryl- oder Heteroaryl-Rest aufweist, kann dieser Aryl- oder Heteroaryl-Rest - sofern nicht anders angegeben - einfach oder mehrfach substituiert sein, vorzugsweise mit 1, 2, 3, 4 oder 5 Substituenten substiuiert sein, wobei die Substituenten jeweils unabhängig voneinander vorzugsweise ausgewählt werden können aus der Gruppe bestehend aus F, Cl, Br, CN, NO₂, C₁₋₅-Alkyl, C₁₋₅-Alkoxy, -S-C₁₋₅-Alkyl, OH, Phenoxy, CHO, -C(=O)-C₁₋₅-Alkyl, -C(=S)-C₁₋₅-Alkyl, -COOH, -C(=O)-NH₂, -C(=O)-NH-C₁₋₅-Alkyl, -C(=O)-N(C₁₋₅-Alkyl)₂, -C(=O)-O-C₁₋₅-Alkyl, -S(=O)-C₁₋₆-alkyl, -S(=O)₂-C₁₋₆-alkyl, -S(=O)₂-NH₂, -C(=O)-C₁₋₅-Perfluoroalkyl, -CF₃, -CHF₂, -CH₂F und -NR^{a}R^{b}, worin R^{a} und R^{b} jeweils unabhängig voneinander ausgewählt werden können aus der Gruppe bestehend aus H, Methyl, Ethyl, n-Propyl, iso-Propyl und unsubstituiertem Phenyl, I, -CF₃, -SF₅, -O-C₂₋₅-Alkenyl, -O-CF₃, -O-CHF₂, -O-CH₂F, -S-CF₃, -S-CHF₂, -S-CH₂F, -SH, -C₁₋₅-Alkyl, -O-C(=O)-C₁₋₅-Alkyl, -O-C(=O)-Phenyl, -(CH₂)-O-C(=O)-C₁₋₅-Alkyl, -(CH₂)-O-C(=O)-Phenyl, -NH-C(=O)-O-C₁₋₅-Alkyl, -NH-C(=O)-C₁₋₅-Alkyl, -C(=O)-C₁₋₅-Perflouralkyl, -S(=O)₂-NH₂, -S(=O)₂-NH-C₁₋₅-Alkyl, -S(=O)₂-NH-Phenyl, -S(=O)2-C₁₋₅-Alkyl, -(CH₂)-Benzo[b]furanyl, -O-Phenyl, -S-Phenyl, -S-Benzyl, -O-Benzyl, Phenyl und Benzyl, wobei jeweils der zyklische Teil der Reste -O-C(=O)-Phenyl, -(CH₂)-O-C(=O)-Phenyl, -S(=O)₂-NH-Phenyl, Phenoxy, Phenyl, -S-Phenyl, -S-Benzyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann.

Die Heteroatome des Heteroaryl-Restes können bevorzugt ausgewählt werden aus der Gruppe bestehend aus Sauerstoff, Stickstoff und Schwefel. Vorzugsweise weisen die Heteroaryl-Reste jeweils 1, 2, 3, 4 oder 5 Heteroatom(e) als Ringglied(er) auf, die unabhängig voneinander aus der Gruppe bestehend aus Sauerstoff, Stickstoff und Schwefel ausgewählt sind.

Geeignete Aryl-Reste können bevorzugt ausgewählt werden aus der Gruppe bestehend aus Phenyl-, 1-Naphthyl und 2-Naphthyl.

Geeignete Heteroaryl-Reste können bevorzugt ausgewählt werden aus der Gruppe bestehend aus Pyrrolyl, Indolyl, Furyl (Furanyl), Benzofuranyl, Thienyl (Thiophenyl), Benzothienyl, Pyrazolyl, Imidazolyl, Thiazolyl, Oxazolyl, Isoxazolyl, Pyridinyl, Pyridazinyl, Pyrimidinyl, Pyrazinyl, Indazolyl, Purinyl, Indolizinyl, Chinolinyl, Isochinolinyl, Chinazolinyl, Carbazolyl, Phenazinyl, Phenothiazinyl, Oxadiazolyl, Pyranyl, Triazolyl, Isoindolyl, Thiadiazolyl und Benzo[1,2,5]oxadiazolyl.

Unter einem monozyklischen Ringsystem wird im Sinne der vorliegenden Erfindung ein monozyklischer Kohlenwasserstoffrest verstanden, der gesättigt, ungesättigt oder aromatisch sein und ggf. ein oder mehrere Heteroatome als Ringglieder aufweisen kann. Ein solches monozyklisches Ringsystem kann beispielsweise mit einem ArylRest oder einem Heteroaryl-Rest kondensiert, d.h. anneliert bzw. verbunden sein. Die Heteroatome eines solchen monozyklischen Ringsystems können jeweils bevorzugt ausgewählt werden aus der Gruppe bestehend aus Sauerstoff, Stickstoff und Schwefel. Besonders bevorzugt weist der Ring der vorstehend genannten monozyklischen Ringsysteme jeweils 1, 2, 3, 4 oder 5 Heteroatom(e) als Ringglied(er) auf, die unabhängig voneinander aus der Gruppe bestehend aus Sauerstoff, Stickstoff und Schwefel ausgewählt sind. Vorzugsweise ist der Ring des Ringsystems 5-, 6- oder 7-gliedrig.

Das Ringsystem kann einfach oder mehrfach substituiert sein, vorzugsweise mit 1, 2, 3, 4 oder 5 Substituenten substituiert sein, wobei die Substituenten vorzugsweise ausgewählt werden können aus der Gruppe bestehend aus F, Cl, Br, CN, NO₂, C₁₋₅-Alkyl, C₁₋₅-Alkoxy, -C(=O)-C₁₋₅-Alkyl, -C(=O)-O-C₁₋₅-Alkyl, -S(=O)₂-C₁₋₆-alkyl, -C(=O)-C₁₋₅-Perfluoroalkyl, -CF₃, CHF₂, CH₂F und -NR^{a}R^{b}, worin R^{a} und R^{b} jeweils unabhängig voneinander ausgewählt werden können aus der Gruppe bestehend aus H, Methyl, Ethyl, n-Propyl, iso-Propyl und unsubstituiertem Phenyl, Oxo (=O), Thioxo (=S), I, -SF₅, -OH, -O-C₂₋₅-Alkenyl, -O-CF₃, -O-CHF₂, -O-CH₂F, -S-CF₃, -S-CHF₂, -S-CH₂F, -SH, -S-C₁₋₅-Alkyl, -C₁₋₅-Alkyl, -C(=O)-OH, -O-C(=O)-C₁₋₅-Alkyl, -O-C(=O)-Phenyl, -(CH₂)-O-C(=O)-C₁₋₅-Alkyl, -(CH₂)-O-C(=O)-Phenyl, -NH-C(=O)-O-C₁₋₅-Alkyl, -NH-C(=O)-C₁₋₅-Alkyl, -C(=O)-H, -C(=O)-NH₂, -C(=O)-NH-C₁₋₅-Alkyl, C(=O)-N-(C₁₋₅-Alkyl)₂, -S(=O)₂-NH₂, -S(=O)₂-NH-C₁₋₅-Alkyl, -S(=O)₂-NH-Phenyl, -(CH₂)-Benzo[b]furanyl, -O-Phenyl, -S-Phenyl, -S-Benzyl, -O-Benzyl, Phenyl und Benzyl, wobei jeweils der zyklische Teil der Reste -O-C(=O)-Phenyl, -(CH₂)-O-C(=O)-Phenyl, -S(=O)₂-NH-Phenyl, -O-Phenyl, -S-Phenyl, -S-Benzyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann.

Weist einer der Reste R¹ bis R¹⁸ eine lineare oder verzweigte Alkylen-Gruppe auf, so kann diese - sofern nicht anders angegeben - unsubstituiert oder einfach oder mehrfach substituiert sein, vorzugsweise mit 1, 2, 3, 4 oder 5 Substituenten substituiert sein, wobei die Substituenten unabhängig voneinander vorzugsweise ausgewählt werden können aus der Gruppe bestehend aus F, Cl, Br, C₁₋₆-Alkoxy, Hydroxy, CN, CF₃, CHF₃, CH₂F, -SH, -NO₂, unsubstituiertem Phenyl und -NR^{a}R^{b}, worin R^{a} und R^{b} jeweils unabhängig voneinander ausgewählt werden können aus der Gruppe bestehend aus H, C₁₋₃-Alkyl und unsubstituiertem Phenyl. Die Alkylen-Gruppe kann auch eines oder mehrere Heteroatome, vorzugsweise wenigstens ein Sauerstoffatom und/oder wenigstens ein Schwefelatom, als Kettenglied(er) aufweisen.

Die jeweiligen Alkylen-Gruppen können - sofern nicht anders angegeben - auch eines oder mehrere Heteroatome, vorzugsweise eines oder mehrere Sauerstoffatome und/oder eines oder mehrere Schwefelatome, besonders bevorzugt 1 oder 2 Sauerstoffatome und/oder Schwefelatome, als Kettenglied(er) aufweisen.

Beispielhaft seien Alkylen-Gruppen wie -(CH₂)-, -(CH(CH₃))-, -(CH₂)₂-, -(CH₂)₂-O-, -(CH₂)₃-, -(CH₂)₄-, -(CH₂)₅-, -(CH₂)₆-, -(CH₂)₇-, -(CH₂)₈-, -(CH₂)₉- oder -(CH₂)₁₀-genannt.

Bevorzugt sind substituierte 1,4,8-Triazaspiro[4.5]decan-2-on-Verbindungen der vorstehend angegebenen allgemeinen Formel I, in denen R¹ für einen Wasserstoff-Rest, für einen linearen oder verzweigten, unsubstituierten oder wenigstens einfach substituierten, ggf. wenigstens ein Heteroatom als Kettenglied aufweisenden C₁₋₁₀-Alkyl-Rest, für einen linearen oder verzweigten, unsubstituierten oder wenigstens einfach substituierten, ggf. wenigstens ein Heteroatom als Kettenglied aufweisenden C₂₋₁₀-Alkenyl-Rest, für einen linearen oder verzweigten, unsubstituierten oder wenigstens einfach substituierten, ggf. wenigstens ein Heteroatom als Kettenglied aufweisenden C₂₋₁₀-Alkinyl-Rest, für einen unsubstituierten oder wenigstens einfach substituierten, 5- bis 14-gliedrigen Aryl- oder Heteroaryl-Rest, der über eine lineare oder verzweigte, ggf. wenigstens ein Heteroatom als Kettenglied aufweisende C₁₋₅-Alkylen-Gruppe gebunden ist, oder für einen über eine lineare oder verzweigte C₁₋₅-Alkylen-Gruppe gebundenen -C(=O)-OR⁷-Rest steht,
vorzugsweise für einen Wasserstoff-Rest, für einen linearen oder verzweigten, unsubstituierten C₁₋₅-Alkyl-Rest, für einen linearen oder verzweigten, unsubstituierten C₂₋₅-Alkenyl-Rest, für einen linearen oder verzweigten, unsubstituierten C₂₋₅-Alkinyl-Rest, für einen unsubstituierten oder wenigstens einfach substituierten Phenyl- oder Naphthyl-Rest, der über eine lineare oder verzweigte, ggf. wenigstens ein Sauerstoffatom als Kettenglied aufweisende C₁₋₅-Alkylen-Gruppe gebunden ist, oder für einen über eine lineare oder verzweigte C₁₋₃-Alkylen-Gruppe gebundenen -C(=O)-OR⁷-Rest steht,
besonders bevorzugt für einen Wasserstoff-Rest, für einen linearen oder verzweigten, unsubstituierten C₁₋₄-Alkyl-Rest, für einen linearen oder verzweigten, unsubstituierten C₂₋₃-Alkenyl-Rest, für einen linearen oder verzweigten, unsubstituierten C₂₋₃-Alkinyl-Rest, für einen Phenyl- oder Naphthyl-Rest, der über eine -(CH₂)-, -(CH₂)₂-, -(CH₂)₃- oder -(CH₂)₂-O-Brücke gebunden und ggf. einfach oder mehrfach, gleich oder verschieden, mit einem Substituenten ausgewählt aus der Gruppe bestehend aus F, Cl, Br, CN, NO₂, C₁₋₅-Alkyl, C₁₋₅-Alkoxy, -C(=O)-C₁₋₅-Alkyl, -C(=O)-O-C₁₋₅-Alkyl, -S(=O)₂-C₁₋₆-alkyl, -C(=O)- C₁₋₅-Perfluoroalkyl, -CF₃, CHF₂ und CH₂F substituiert ist, oder für einen über eine -(CH₂)-Gruppe gebundenen -C(=O)-OR⁷-Rest steht, und jeweils die übrigen Reste R²-R¹¹ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind substituierte 1,4,8-Triazaspiro[4.5]decan-2-on-Verbindungen der vorstehend angegebenen allgemeinen Formel I, worin der Rest R² für einen Wasserstoff-Rest, für einen linearen oder verzweigten, unsubstituierten oder wenigstens einfach substituierten, ggf. wenigstens ein Heteroatom als Kettenglied aufweisenden C₁₋₁₀-Alkyl-Rest, für einen linearen oder verzweigten, unsubstituierten oder wenigstens einfach substituierten, ggf. wenigstens ein Heteroatom als Kettenglied aufweisenden C₂₋₁₀-Alkenyl-Rest, für einen linearen oder verzweigten, unsubstituierten oder wenigstens einfach substituierten, ggf. wenigstens ein Heteroatom als Kettenglied aufweisenden C₂₋₁₀-Alkinyl-Rest, oder für einen unsubstituierten oder wenigstens einfach substituierten, 5- bis 14-gliedrigen Aryl- oder Heteroaryl-Rest steht, der über eine lineare oder verzweigte, ggf. wenigstens ein Heteroatom als Kettenglied aufweisende C₁₋₅-Alkylen-Gruppe gebunden sein kann,
vorzugsweise für einen Wasserstoff-Rest, für einen linearen oder verzweigten, unsubstituierten ggf. wenigstens ein Sauerstoffatom und/oder wenigstens ein Schwefelatom als Kettenglied aufweisenden C₁₋₅-Alkyl-Rest oder für einen Phenyl- oder Naphthyl-Rest steht, der einfach oder mehrfach, gleich oder verschieden, mit einem Substituenten ausgewählt aus der Gruppe bestehend aus F, Cl, Br, CN, NO₂, C₁₋₅-Alkyl, C₁₋₅-Alkoxy, -C(=O)-C₁₋₅-Alkyl, -C(=O)-O-C₁₋₅-Alkyl, -S(=O)₂-C₁₋₆-alkyl, -C(=O)- C₁₋₅-Perfluoroalkyl, -CF₃, CHF₂ und CH₂F substituiert und/oder über eine -(CH₂)-, -(CH₂)₂-, -(CH₂)₃- oder -(CH₂)₂-O-Brücke gebunden sein kann,
besonders bevorzugt für einen Wasserstoff-Rest, für einen linearen oder verzweigten, unsubstituierten, ggf. wenigstens ein Schwefelatom als Kettenglied aufweisenden C₁₋₅-Alkyl-Rest oder für einen Phenyl-Rest steht, wobei der Phenyl-Rest einfach oder mehrfach, gleich oder verschieden, mit einem Substituenten ausgewählt aus der Gruppe bestehend aus F, Cl, Br, CN, NO₂, C₁₋₅-Alkyl, C₁₋₅-Alkoxy, -C(=O)-C₁₋₅-Alkyl, -C(=O)-O-C₁₋₅-Alkyl, -S(=O)₂-C₁₋₆-alkyl, -C(=O)- C₁-₅-Perfluoroalkyl, -CF₃, CHF₂ und CH₂F substituiert und/oder über eine -(CH₂)-Brücke gebunden sein kann, und jeweils die übrigen Reste R¹ und R³-R¹¹ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Bevorzugt sind auch substituierte 1,4,8-Triazaspiro[4.5]decan-2-on-Verbindungen der vorstehend angegebenen allgemeinen Formel I, in denen der Rest R⁴ für einen -NR¹⁰R¹¹-Rest, für einen linearen oder verzweigten, unsubstituierten oder wenigstens einfach substituierten, ggf. wenigstens ein Heteroatom als Kettenglied aufweisenden C₁₋₁₀-Alkyl-Rest, für einen linearen oder verzweigten, unsubstituierten oder wenigstens einfach substituierten, ggf. wenigstens ein Heteroatom als Kettenglied aufweisenden C₂₋₁₀-Alkenyl-Reet, einen linearen oder verzweigten, unsubstituierten oder wenigstens einfach substituierten, ggf. wenigstens ein Heteroatom als Kettenglied aufweisenden C₂₋₁₀-Alkinyl-Rest, für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte, ggf. wenigstens ein Heteroatom als Kettenglied aufweisende C₁₋₅-Alkylen-Gruppe gebunden und/oder mit einem 5- oder 6-gliedrigen monozyklischen Ringsystem kondensiert sein kann, oder für einen unsubstituierten oder wenigstens einfach substituierten, ggf. wenigstens ein Heteroatom als Ringglied aufweisenden, C₃₋₈-cycloaliphatischen Rest steht, der über eine lineare oder verzweigte, unsubstituierte oder wenigstens einfach substituierte, ggf. wenigstens ein Heteroatom als Kettenglied aufweisende C₁₋₅-Alkylen-Gruppe gebunden und/oder mit einer linearen oder verzweigten, unsubstituierten oder wenigstens einfach substituierten C₁₋₅-Alkylen-Gruppe überbrückt sein kann,
vorzugsweise für einen -NR¹⁰R¹¹-Rest, für einen linearen oder verzweigten, unsubstituierten, ggf. wenigstens ein Sauerstoffatom und/oder wenigstens ein Schwefelatom als Kettenglied aufweisenden C₁₋₄-Alkyl-Rest, für einen Furyl-(Furanyl-)-Rest, Thienyl-(Thiophenyl-)-Rest, Phenyl-Rest, Naphthyl-Rest oder 1,2,3,4-Tetrahydro-isochinolin-Rest steht, wobei der zyklische Rest jeweils einfach oder mehrfach, gleich oder verschieden, mit einem Substituenten ausgewählt aus der Gruppe bestehend aus F, Cl, Br, CN, NO₂, C₁₋₅-Alkyl, C₁₋₅-Alkoxy, -C(=O)-C₁₋₅-Alkyl, -C(=O)-O-_{C1-5-}Alkyl, -S(=O)₂-C₁₋₆-alkyl, -C(=O)- C₁₋₅-Perfluoroalkyl, -CF₃, CHF₂ und CH₂F substituiert und/oder über eine -(CH₂)-, -(CH₂)₂- oder -(CH₂)₃-Brücke gebunden sein kann, oder für einen unsubstituierten oder wenigstens einfach mit einer oxo-Gruppe substituierten, ggf. wenigstens ein Heteroatom als Ringglied aufweisenden, C₅- oder C₆- cycloaliphatischen Rest steht, der über eine -(CH₂)-, -(CH₂)₂- oder -(CH₂)₃-Brücke gebunden und/oder einer -(C(CH₃)₂)-Gruppe überbrückt sein kann,
besonders bevorzugt für einen -N(CH₃)₂-Rest, für einen linearen oder verzweigten, unsubstituierten C₁₋₄-Alkyl-Rest, für einen Thienyl-(Thiophenyl-)-, Phenyl-, oder 1,2,3,4-Tetrahydro-isochinolin-Rest steht, wobei der zyklische Rest jeweils einfach oder mehrfach, gleich oder verschieden, mit einem Substituenten ausgewählt aus der Gruppe bestehend aus F, Cl, Br, CN, NO₂, C₁₋₅-Alkyl, C₁₋₅-Alkoxy, -C(=O)-C₁₋₅-Alkyl, -C(=O)-O-C₁₋₅-Alkyl, -S(=O)₂-C₁₋₆-alkyl, -C(=O)- C₁₋₅-Perfluoroalkyl, -CF₃, CHF₂ und CH₂F substituiert und/oder über eine -(CH₂)-Brücke gebunden sein kann, oder für einen unsubstituierten oder wenigstens einfach mit einer oxo-Gruppe substituierten C₅- oder C₆- cycloaliphatischen Rest steht, der über eine -(CH₂)-Brücke gebunden und/oder mit einer -(C(CH₃)₂)-Gruppe überbrückt sein kann, und jeweils die Reste R¹-R³ und R⁵ bis R¹¹ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind substituierte 1,4,8-Triazaspiro[4.5]decan-2-on-Verbindungen der vorstehend angegebenen allgemeinen Formel I, worin der Rest R⁵ für einen linearen oder verzweigten, unsubstituierten oder wenigstens einfach substituierten, ggf. wenigstens ein Heteroatom als Kettenglied aufweisenden C₁₋₁₀-Alkyl-Rest, für einen linearen oder verzweigten, unsubstituierten oder wenigstens einfach substituierten, ggf. wenigstens ein Heteroatom als Kettenglied aufweisenden C₂₋₁₀-Alkenyl-Rest, für einen linearen oder verzweigten, unsubstituierten oder wenigstens einfach substituierten, ggf. wenigstens ein Heteroatom als Kettenglied aufweisenden C₂₋₁₀-Alkinyl-Rest, für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte, ggf. wenigstens ein Heteroatom als Kettenglied aufweisende C₁₋₅-Alkylen-Gruppe gebunden sein kann, für einen unsubstituierten oder wenigstens einfach substituierten, ggf. wenigstens ein Heteroatom als Ringglied aufweisenden, C₃₋₈-cycloaliphatischen Rest steht, der über eine lineare oder verzweigte, unsubstituierte oder wenigstens einfach substituierte, ggf. wenigstens ein Heteroatom als Kettenglied aufweisende C₁₋₅-Alkylen-Gruppe sein kann, für einen -C(=O)-OR⁸-Rest oder für einen über eine lineare oder verzweigte C₁₋₅-Alkylen-Gruppe gebundenen -C(=O)-OR⁹-Rest steht,
vorzugsweise für einen linearen oder verzweigten, ggf. wenigstens ein Sauerstoffatom und/oder wenigstens ein Schwefelatom als Kettenglied aufweisenden C₁₋₅-Alkyl-Rest, für einen linearen oder verzweigten, ggf. wenigstens ein Sauerstoffatom und/oder wenigstens ein Schwefelatom als Kettenglied aufweisenden C₂₋₅-Alkenyl-Rest, für einen linearen oder verzweigten, ggf. wenigstens ein Sauerstoffatom und/oder wenigstens ein Schwefelatom als Kettenglied aufweisenden C₂-₅-Alkinyl-Rest, für einen Furyl- (Furanyl-)-Rest, Thienyl-(Thiophenyl-)-Rest, Phenyl- oder Naphthyl-Rest steht, wobei der zyklische Rest jeweils einfach oder mehrfach, gleich oder verschieden, mit einem Substituenten ausgewählt aus der Gruppe bestehend aus F, Cl, Br, CN, NO₂, C₁₋₅-Alkyl, C₁₋₅-Alkoxy, -C(=O)-C₁₋₅-Alkyl, -C(=O)-O-C₁₋₅-Alkyl, -S(=O)₂-C₁-₆-alkyl. -C(=O)- C₁₋₅-Perfluoroalkyl, -CF₃, CHF₂ und CH₂F substituiert und/oder über eine -(CH₂)-, -(CH(CH₃))-, -(CH₂)₂- oder -(CH₂)₃-Brücke gebunden sein kann, für einen unsubstituierten, ggf. wenigstens ein Sauerstoffatom als Ringglied aufweisenden, C₄-, C₅- oder C₆-cycloaliphatischen Rest steht, der über eine -(CH₂), -(CH(CH₃))-, -(CH₂)₂- oder -(CH₂)₃-Brücke gebunden sein kann, für einen -C(=O)-O-R⁸-Rest oder für einen über eine -(CH₂)-, -(CH(CH₃))-, -(CH₂)₂- oder -(CH₂)₃-Brücke gebundenen -C(=O)-OR⁹-Rest steht,
besonders bevorzugt für einen linearen oder verzweigten, unsubstituierten, ggf. wenigstens ein Sauerstoffatom als Kettenglied aufweisenden C₁₋₃-Alkyl-Rest, für einen linearen oder verzweigten, unsubstituierten C₂₋₃-Alkenyl-Rest, für einen linearen oder verzweigten, unsubstituierten C₂₋₃-Alkinyl-Rest, für einen Phenyl-Rest steht, wobei der Phenyl-Rest einfach oder mehrfach, gleich oder verschieden, mit einem Substituenten ausgewählt aus der Gruppe bestehend aus F, Cl, Br, CN, NO₂, C₁₋₅-Alkyl, C₁₋₅-Alkoxy, -C(=O)-C₁₋₅-Alkyl, -C(=O)-O-C₁₋₅-Alkyl, -S(=O)₂-C₁₋₆-alkyl, -C(=O)- C₁₋₅-Perfluoroalkyl, -CF₃, CHF₂ und CH₂F substituiert und/oder über eine -(CH₂)-, -(CH(CH₃))-, -(CH₂)₂- oder -(CH₂)₃-Brücke gebunden sein kann, für einen unsubstituierten, ggf. wenigstens ein Sauerstoffatom als Ringglied aufweisenden, C₅- oder C₆-cycloaliphatischen Rest steht, der über eine -(CH₂)-, -(CH(CH₃))-, -(CH₂)₂- oder -(CH₂)₃-Brücke gebunden sein kann, für einen C(=O)-O-C₂H₅-Rest oder für einen über eine -(CH₂), -(CH(CH₃))-, -(CH₂)₂- oder -(CH₂)₃-Brücke gebundenen -C(=O)-O-R⁹-Rest steht, und jeweils die Reste R¹-R⁴ und R⁶-R¹¹ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Bevorzugt sind auch substituierte 1,4,8-Triazaspiro[4.5]decan-2-on-Verbindungen der vorstehend angegebenen allgemeinen Formel I, in denen der Rest R⁶ für einen unsubstituierten oder wenigstens einfach substituierten, 5- bis 14-gliedrigen Aryl- oder Heteroaryl-Rest, der über eine lineare oder verzweigte, unsubstituierte oder wenigstens einfach substituierte, ggf. wenigstens ein Heteroatom als Kettenglied aufweisende C₁₋₅-Alkylen-Gruppe gebunden sein kann, oder für einen unsubstituierten oder wenigstens einfach substituierten, ggf. wenigstens ein Heteroatom als Ringglied aufweisenden, C₃₋₈-cycloaliphatischen Rest steht, der über eine lineare oder verzweigte, unsubstituierte oder wenigstens einfach substituierte, ggf. wenigstens ein Heteroatom als Kettenglied aufweisende C₁₋₅-Alkylen-Gruppe sein kann,
vorzugsweise für einen Phenyl-Rest steht, wobei der Phenyl-Rest einfach oder mehrfach, gleich oder verschieden, mit einem Substituenten ausgewählt aus der Gruppe bestehend aus F, Cl, Br, CN, NO₂, C₁₋₅-Alkyl, C₁₋₅-Alkoxy, -C(=O)-C₁₋₅-Alkyl, -C(=O)-O-C₁₋₅-Alkyl, -S(=O)₂-C₁₋₆-alkyl, -C(=O)- C₁₋₅-Perfluoroalkyl, -CF₃, CHF₂ und CH₂F substituiert und/oder über eine -(CH₂)-, -(CH(CH₃))-, -(CH₂)₂- oder -(CH₂)₃-Brücke gebunden sein kann, oder für einen unsubstituierten C₅- oder C₆-cycloaliphatischen Rest steht, der über eine -(CH₂)-, -(CH(CH₃)), -(CH₂)₂- oder -(CH₂)₃-Brücke gebunden sein kann, besonders bevorzugt für einen Phenyl-Rest steht, wobei der Phenyl-Rest einfach oder mehrfach, gleich oder verschieden, mit einem Substituenten ausgewählt aus der Gruppe bestehend aus F, Cl, Br, CN, NO₂, C₁₋₅-Alkyl, C₁₋₅-Alkoxy, -C(=O)-C₁₋₅-Alkyl, -C(=O)-O-C₁₋₅-Alkyl, -S(=O)₂-C₁₋₆-alkyl, -C(=O)- C₁₋₅-Porfluoroalkyl, -CF₃, CHF₂ und CH₂F substituiert und/oder über eine -(CH₂)- oder (CH₂)₂-Brücke gebunden sein kann, oder für einen Cyclohexyl-Rest steht, der über eine -(CH₂)- oder -(CH₂)₂-Brücke gebunden sein kann, und jeweils die Reste R¹-R⁵ und R⁷-R¹¹ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin sind solche substituierte 1,4,8-Triazaspiro[4.5]decan-2-on-Verbindungen der vorstehend angegebenen allgemeinen Formel I Bevorzugt, in denen die Reste R⁷, R⁸, R⁹, R¹⁰, R¹¹, jeweils unabhängig voneinander, für einen linearen oder verzweigten C₁₋₅-Alkyl-Rest, einen linearen oder verzweigten C₂₋₅-Alkenyl-Rest oder einen linearen oder verzweigten C₂₋₅-Alkinyl-Rest, vorzugsweise für einen linearen oder ggf. verzweigten Methyl-, Ethyl-, n-Propyl oder iso-Propyl-Rest stehen, und jeweils die übrigen Reste R¹-R⁶ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Besonders bevorzugt sind substituierte 1,4,8-Triazaspiro[4.5]decan-2-on-Verbindungen der allgemeinen Formel I, 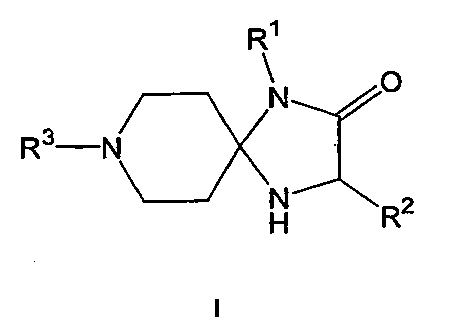 worin
R¹ für einen Wasserstoff-Rest, für einen Alkyl-Rest ausgewählt aus der Gruppe bestehend aus Methyl, Ethyl, n-Propyl, iso-Propyl, n-Butyl, Isobutyl, sec-Butyl, und tert-Butyl, für einen Alkenyl-Rest ausgewählt aus der Gruppe bestehend aus Vinyl und Allyl, für einen Propinyl-Rest, für einen Phenyl- 1-Naphthyl oder 2-Naphthyl-Rest, wobei der zyklische Rest über eine -(CH₂)-, -(CH₂)₂-, -(CH₂)₂- oder -(CH₂)₂-O-Brücke gebunden und ggf. einfach oder mehrfach, gleich oder verschieden, mit einem Substituenten ausgewählt aus der Gruppe bestehend aus F, Cl, Br, CN, NO₂, C₁₋₅-Alkyl, C₁₋₅-Alkoxy, -C(=O)-C₁₋₅-Alkyl, -C(=O)-O-C₁₋₅-Alkyl, -S(=O)₂-C₁₋₆-alkyl, -C(=O)- C₁₋₅-Perfluoroalkyl, -CF₃, CHF₂ und CH₂F substituiert ist, oder für einen über eine -(CH₂)-Gruppe gebundenen -C(=O)-OR⁷-Rest steht,
R² für einen Wasserstoff-Rest, für einen linearen oder verzweigten, unsubstituierten, ggf. wenigstens ein Schwefelatom als Kettenglied aufweisenden Alkyl-Rest ausgewählt aus der Gruppe bestehend aus Methyl, Ethyl, n-Propyl, iso-Propyl, n-Butyl, Isobutyl, sec-Butyl, tert-Butyl und 2-Methylsulfanylethyl oder für einen Phenyl- oder Benzyl-Rest steht, der jeweils unsubstituiert oder einfach oder mehrfach, gleich oder verschieden, mit einem Substituenten ausgewählt aus der Gruppe bestehend aus F, Cl, Br, CN, NO₂, C₁₋₅-Alkyl, C₁₋₅-Alkoxy, -C(=O)=C₁₋₅-Alkyl, -C(=O)-O-C₁₋₅-Alkyl, -S(=O)-C₁₋₆-alkyl, -C(=O)- C₁₋₅-Perfluoroalkyl, -CF₃, CHF₂ und CH₂F substituiert sein kann,
R³ für eine -S(=O)₂-R⁴-Gruppe, für eine -C(=S)-NH-R⁵-Gruppe oder für eine -C(=O)-NH-R⁶-Gruppe steht,
R⁴ für einen -N(CH₃)₂₋Rest, für einen Alkyl-Rest ausgewählt aus der Gruppe bestehend aus Methyl, Ethyl, n-Propyl, iso-Propyl, n-Butyl, Isobutyl, sec-Butyl, tert-Butyl, für einen Thienyl-(Thiophenyl-)-, Phenyl-, oder 1,2,3,4-Tetrahydro-isochinolin-Rest steht, wobei der zyklische Rest jeweils unsubstituiert oder einfach oder mehrfach, gleich oder verschieden, mit einem Substituenten ausgewählt aus der Gruppe bestehend aus F, Cl, Br, CN, NO₂, C₁₋₅-Alkyl, C₁₋₅-Alkoxy, -C(=O)C₁₋₅-Alkyl, -C(=O)-O-C₁₋₅-Alkyl, -S(=O)₂-C₁₋₆-alkyl, -C(=O)- C₁₋₅-Perfluoroalkyl, -CF₃, CHF₂ und CH₂F substituiert und/oder über eine -(CH₂)-Brücke gebunden sein kann, oder für einen 7,7-Dimethyl-2-oxo-bicyclo-[2.2.1]-hept-1-yl-methyl-Rest steht,
R⁵ für einen Alkyl-Rest ausgewählt aus der Gruppe bestehend aus Methyl, Ethyl, n-Propyl, iso-Propyl, n-Butyl, Isobutyl, sec-Butyl, tert-Butyl und 2-Methoxyethyl, für einen Alkenyl-Rest ausgewählt aus der Gruppe bestehend aus Vinyl und Allyl, für einen Propinyl-Rest, für einen Phenyl-Rest steht, wobei der Phenyl-Rest einfach oder mehrfach, gleich oder verschieden, mit einem Substituenten ausgewählt aus der Gruppe bestehend aus F, Cl, Br, CN, NO₂, C₁₋₅-Alkyl, C₁₋₅-Alkoxy, -C(=O)-C₁₋₅-Alkyl, -C(=O)-C₁₋₅-Alkyl, -S(=O)₂-C₁₋₆-alkyl, -C(=O)- C₁₋₅-Perfluoroalkyl, -CF₃, CHF₂ und CH₂F substituiert und/oder über eine -(CH₂)-, -(CH(CH₃))-, -(CH₂)₂- oder -(CH₂)₃-Brücke gebunden sein kann, für einen Cyclohexyl- oder Tetrahydrofuryl-Rest, der über eine -(CH₂)-Brücke gebunden sein kann, für einen C(=O)-O-C₂H₅-Rest oder für einen über eine -(CH₂)-, -(CH(CH₃)), -(CH₂)₂- oder-(CH₂)₃-Brücke gebundenen -C(=O)-O-C₂H₅-Rest steht,
R⁶ für einen Phenyl-Rest, der unsubstituiert oder einfach oder mehrfach, gleich oder verschieden, mit einem Substituenten ausgewählt aus der Gruppe bestehend aus F, Cl, Br, CN, NO₂, C₁₋₅-Alkyl, C₁₋₅-Alkoxy, -C(=O)-C₁₋₅-Alkyl, -C(=O)-O-C₁₋₅-Alkyl, -S(=O)₂-C₁₋₆-alkyl, -C(=O)- C₁₋₅-Perfluoroalkyl, -CF₃, CHF₂ und CH₂F substituiert und/oder über eine -(CH₂)- oder (CH₂)₂-Brücke gebunden sein kann, oder für einen Cyclohexyl-Rest steht, der über eine -(CH₂)- oder -(CH₂)₂-Brücke gebunden sein kann,
R⁷, R⁸, R⁹, R¹⁰, R¹¹, unabhängig voneinander, jeweils für einen linearen oder verzweigten C₁₋₅-Alkyl-Rest, einen linearen oder ggf. verzweigten C₂₋₅-Alkenyl-Rest oder einen linearen oder verzweigten C₂₋₅-Alkinyl-Rest stehen, vorzugsweise für einen linearen oder verzweigten Methyl-, Ethyl-, n-Propyl oder iso-Propyl-Rest stehen.
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ganz besonders bevorzugt sind substituierte 1,4,8-Triazaspiro[4.5]decan-2-on-Verbindungen der vorstehend angegebenen allgemeinen Formel I ausgewählt aus der Gruppe bestehend aus
[1] 3-Isopropyl-8-(4-nitro-benzolsulfonyl)-1,4,8-triaza-spiro[4.5]decan-2-on,
[2] 8-(2-Chlor-benzolsulfonyl)-3-isobutyl-1,4,8-tdaza-spiro[4.5]decan-2-on,
[3] 3-Benzyl-8-[2-(2,2,2-trifluor-acetyl)-1,2,3,4-tetrahydro-isoquinolin-7-sulfonyl]-1,4,8-triaza-spiro[4.5]decan-2-on,
[4] 3-Benzyl-8-(4-methoxy-2,3,6-trimethyl-benzolsulfonyl)-1,4,8-triaza-spiro[4.5]decan-2-on,
[5] 2-[8-(4-Butoxy-benzolsulfonyl)-2-oxo-1,4,8-triaza-spiro[4.5]dec-1-ylmethyl]-benzonitril,
[6] 2-{8-[4-(1,1-Dimethyl-propyl)-benzolsulfonyl]-2-oxo-1,4,8-triaza-spiro[4.5]dec-1-ylmethyl}-benzonitril,
[7] 3-Benzyl-8-(2-fluor-benzolsulfonyl)-1,4,8-triaza-spiro[4.5]decan-2-on,
[8] 8-(2-Chlor-benzolsulfonyl)-3-isopropyl-1,4,8-triaza-spiro[4.5]decan-2-on,
[9] 1-Benzyl-2-oxo-1,4,8-triaza-spiro[4.5]decan-8-sulfonsäuredimethylamid,
[10] 3-[8-(4-Acetyl-benzolsulfonyl)-3-(2-methylsulfanyl-ethyl)-2-oxo-1,4,8-triaza-spiro[4.5]dec-1-ylmethyl]-benzonitril,
[11] 1-(2-Fluor-benzyl)-3-isopropyl-8-(4-methoxy-2,3,6-trimethyl-benzolsulfonyl)-1,4,8-triaza-spiro[4.5]decan-2-on,
[12] 1-Butyl-8-(5-chlor-thiophen-2-sulfonyl)-3-(2-methylsulfanyl-ethyl)-1,4,8-triaza-spiro[4.5]decan-2-on,
[13] 8-(4-Acetyl-benzolsulfonyl)-1-butyl-3-(2-methylsulfanyl-ethyl)-1,4,8-triaza-spiro[4.5]decan-2-on,
[14] 1-Benzyl-3-isopropyl-8-(2,4,6-trimethyl-benzolsulfonyl)-1,4,8-triaza-spiro[4.5]decan-2-on,
[15] 2-(1-Butyl-3-isopropyl-2-oxo-1,4,8-triaza-spiro[4.5]decan-8-sulfonyl)-benzoesäuremethylester,
[16] 1,3-Dibenzyl-8-(thiophen-2-sulfonyl)-1,4,8-triaza-spiro[4.5]decan-2-on,
[17] 8-(3-Chlor-4-fluor-benzolsulfonyl)-3-Isopropyl-1-(2-phenoxy-ethyl)-1,4,8-triaza-spiro[4.5]decan-2-on,
[18] 1-Butyl-3-(2-methylsulfanyl-ethyl)-8-(toluol-3-sulfonyl)-1,4,8-triaza-spiro[4.5]decan-2-on,
[19] 1-Butyl-8-(2,4-difluor-benzolsulfonyl)-3-isopropyl-1,4,8-triaza-spiro[4.5]decan-2-on,
[20] 1,3-Dibenzyl-8-(2,5-dichlor-thiophen-3-sulfonyl)-1,4,8-triaza-spiro[4.5]decan-2-on,
[21] 3-Isopropyl-1-(2-phenoxy-ethyl)-8-(toluol-4-sulfonyl)-1,4,8-triaza-spiro[4.5]decan-2-on,
[22] 3-Benzyl-1-butyl-8-(2-trifluormethyl-benzolsulfonyl)-1,4,8-triaza-spiro[4.5]decan-2-on,
[23] 2-[1-(4-Fluor-benzyl)-3-(2-methylsulfanyl-ethyl)-2-oxo-1,4,8-triaza-spiro[4.5]decan-8-sulfonyl]-benzoesäuremethylester,
[24] 1,3-Dibenzyl-8-(2,3,5,6-tetramethyl-benzolsulfonyl)-1,4,8-triaza-spiro[4.5]decan-2-on,
[25] 1-(4-Fluor-benzyl)-3-(2-methylsulfanyl-ethyl)-8-(2-trifluormethyl-benzolsulfonyl)-1,4,8-triaza-spiro[4.5]decan-2-on,
[26] 8-(4-Chlor-2,5-dimethyl-benzolsulfonyl)-(4-fluor-benzyl)-3-(2-methylsulfanyl-ethyl)-1,4,8-triaza-spiro[4.5]decan-2-on,
[27] 1-Benzyl-8-(4-chlor-2,5-dimethyl-benzolsulfonyl)-3-isobutyl-1,4,8-triaza-spiro[4.5]decan-2-on,
[28] 3-Benzyl-1-butyl-8-[4-(1,1-dimethyl-propyl)-benzolsulfonyl]-1,4,8-triaza-spiro[4.5]decan-2-on,
[29] 1,3-Dibenzyl-8-(3,4-dimethoxy-benzolsulfonyl)-1,4,8-triaza-spiro[4.5]decan-2-on,
[30] 1-Butyl-8-(3,4-dimethoxy-benzolsulfonyl)-3-(2-methylsulfanyl-ethyl)-1,4,8-triaza-spiro[4.5]decan-2-on,
[31] 1,3-Dibenzyl-8-(4-methoxy-2,3,6-trimethyl-benzolsulfonyl)-1,4,8-triaza-spiro[4.5]decan-2-on,
[32] 1-Benzyl-8-ethanesulfonyl-3-(2-methylsulfanyl-ethyl)-1,4,8-triaza-spiro[4.5]decan-2-on,
[33] 3-Benzyl-1-butyl-8(4-ethyl-benzolsulfonyl)-1,4,8-triaza-spiro[4.5]decan-2-on,
[34] 3-(2-Methylsulfanyl-ethyl)-1-prop-2-ynyl-8-(3-trifluormethyl-benzolsulfonyl)-1,4,8-triaza-spiro[4.5]decan-2-on,
[35] 1,3-Dibenzyl-8-(4-ethyl-benzolsulfonyl)-1,4,8-triaza-spiro[4.5]decan-2-on,
[36] 3-Benzyl-1-butyl-8-(3-chlor-benzolsulfonyl)-1,4,8-triaza-spiro[4.5]decan-2-on,
[37] 1,3-Dibenzyl-8-(2-fluor-benzolsulfonyl)-1,4,8-triaza-spiro[4.5]decan-2-on,
[38] 2-[1-(2-Cyano-benzyl)-3-(2-methylsulfanyl-ethyl)-2-oxo-1,4,8-triazaspiro[4.5]decan-8-sulfonyl]-benzoesäuremethylester,
[39] 3-Benzyl-1-prop-2-ynyl-8-(2-trifluormethyl-benzolsulfonyl)-1,4,8-triaza-spiro[4.5]decan-2-on,
[40] 3-Benzyl-8-(3-chlor-benzolsulfonyl)-1-methyl-1,4,8-triaza-spiro[4.5]decan-2-on,
[41] 1-Benzyl-8-[4-(1,1-dimethyl-propyl)-benzolsulfonyl]-3-(2-methylsulfanyl-ethyl)-1,4,8-triaza-spiro[4.5]decan-2-on,
[42] 8-(2,4-Difluor-benzolsulfonyl)-1-(3,5-dimethyl-benzyl)-3-(2-methylsulfanyl-ethyl)-1,4,8-triaza-spiro[4.5]decan-2-on,
[43] 1,3-Dibenzyl-8-(4-nitro-benzolsulfonyl)-1,4,8-triaza-spiro[4.5]decan-2-on,
[44] 1-(4-Fluor-benzyl)-3-(2-methylsulfanyl-ethyl)-8-(toluol-4-sulfonyl)-1,4,8-triaza-spiro[4.5]decan-2-on,
[45] 1,3-Dibenzyl-8-(toluol-4-sulfonyl)-1,4,8-triaza-spiro[4.5]decan-2-on,
[46] [3-Benzyl-8-(2-methyl-5-nitro-benzolsulfonyl)-2-oxo-1,4,8-triaza-spiro[4.5]dec-1-yl]-essigsäureethylester,
[47] [3-Benzyl-8-(2-methansulfonyl-benzolsulfonyl)-2-oxo-1,4,8-triaza-spiro[4.5]dec-1-yl]-essigsäureethylester,
[48] [3-Benzyl-2-oxo-8-(thlophen-2-suffonyl)-1,4,8-triaza-spiro[4.5]dec-1-yl]-essigsäureethylester,
[49] 4-(8-Ethanesulfonyl-2-oxo-1,4,8-triaza-spiro[4.5]dec-1-ylmethyl)-benzoesäuremethylester,
[50] [3-Benzyl-8-(4-methoxy-benzolsulfonyl)-2-oxo-1,4,8-triaza-spiro[4.5]dec-1-yl]-essigsäureethylester,
[51] 4-(2-Oxo-8-benzolmethansulfonyl-1,4,8-triaza-spiro[4.5]dec-1-ylmethyl)-benzoesäuremethylester,
[52] 3-[3-Isopropyl-8-(4-nitro-benzolsulfonyl)-2-oxo-1,4,8-triaza-spiro[4.5]dec-1-ylmethyl]-benzonitril,
[53] 4-[8-(Butan-1-sulfonyl)-2-oxo-1,4,8-triaza-spiro[4.5]dec-1-ylmethyl]-benzoesäuremethylester,
[54] 1-Allyl-8-(2-methansulfonyl-benzolsulfonyl)-3-(2-methylsulfanyl-ethyl)-1,4,8-triaza-spiro[4.5]decan-2-on,
[55] 1-(2-Fluor-benzyl)-2-oxo-1,4,8-triaza-spiro[4.5]decan-8-sulfonsäure dimethylamid,
[56] 8-(7,7-Dimethyl-2-oxo-bicyclo[2.2.1]hept-1-ylmethansulfonyl)-1-(2-fluor-benzyl)-1,4,8-triaza-spiro[4.5]decan-2-on,
[57] [3-(2-Methylsulfanyl-ethyl)-2-oxo-8-benzolmethansulfonyl-1,4,8-triazaspiro[4.5]dec-1-yl]-essigsäureethylester,
[58] 1-Allyl-3-(2-methylsulfanyl-ethyl)-8-benzolmethansulfonyl-1,4,8-triazaspiro[4.5]decan-2-on,
[59] 3-[2-Oxo-8-(2,4,6-trimethyl-benzolsulfonyl)-1,4,8-triaza-spiro[4.5]dec-1-ylmethyl]-benzonitril,
[60] 4-[2-Oxo-8-(2,4,6-trimethyl-benzolsulfonyl)-1,4,8-triaza-spiro[4.5]dec-1-ylmethyl]-benzoesäuremethylester,
[61] [3-Benzyl-8-(5-chlor-thiophen-2-sulfonyl)-2-oxo-1,4,8-triaza-spiro[4.5]dec-1-yl]-essigsäureethylester,
[62] [3-Benzyl-8-(butan-1-sulfonyl)-2-oxo-1,4,8-triaza-spiro[4.5]dec-1-yl]-essigsäureethylester,
[63] [3-Benzyl-2-oxo-8-(toluol-3-sulfonyl)-1,4,8-triaza-spiro[4.5]dec-1-yl]-essigsäureethylester,
[64] 1-(2-Fluor-benzyl)-8-(4-methoxy-benzolsulfonyl)-1,4,8-triaza-spiro[4.5]decan-2-on,
[65] [3-Benzyl-2-oxo-8-(2-trifluormethyl-benzolsulfonyl)-1,4,8-triaza-spiro[4.5]dec-1-yl]-essigsäureethylester,
[66] (8-Benzolsulfonyl-3-benzyl-2-oxo-1,4,8-triaza-spiro[4.5]dec-1-yl)-essigsäureethylester,
[67] [3-Benzyl-2-oxo-8-(4-propyl-benzolsulfonyl)-1,4,8-triaza-spiro[4.5]dec-1-yl]-essigsäureethylester,
[68] (3-Benzyl-2-oxo-8-benzolmethansulfonyl-1,4,8-triaza-spiro[4.5]dec-1-yl)-essigsäureethylester,
[69] [3-Benzyl-8-(3-chlor-4-fluor-benzolsulfonyl)-2-oxo-1,4,8-triaza-spiro[4.5]dec-1-yl]-essigsäureethylester,
[70] 1-Allyl-3-isopropyl-8-(2,4,6-trimethyl-benzolsulfonyl)-1,4,8-triaza-spiro[4.5]decan-2-on,
[71] 8-Benzolsulfonyl-3-benzyl-1-(2-fluor-benzyl)-1,4,8-triaza-spiro[4.5]decan-2-on,
[72] [3-Benzyl-2-oxo-8-(2,4,6-trimethyl-benzolsulfonyl)-1,4,8-triaza-spiro[4.5]dec-1-yl]-essigsäureethylester,
[73] [3-Benzyl-8-(4-butoxy-benzolsulfonyl)-2-oxo-1,4,8-triaza-spiro[4.5]dec-1-yl]-essigsäureethylester,
[74] [3-Benzyl-8-(2,5-dimethoxy-benzolsulfonyl)-2-oxo-1,4,8-triaza-spiro[4.5]dec-1-yl]-essigsäureethylester,
[75] 3-(2-Methylsulfanyl-ethyl)-1-naphthalin-2-ylmethyl-2-oxo-1,4,8-triazaspiro[4.5]decan-8-sulfonsäure dimethylamid,
[76] 1-(3-Cyano-benzyl)-3-isopropyl-2-oxo-1,4,8-triaza-spiro[4.5]decan-8-sulfonsäure dimethylamid,
[77] 3-{3-Isopropyl-2-oxo-8-[2-(2,2,2-trifluor-acetyl)-1,2,3,4-tetrahydro-isoquinolin-7-sulfonyl]-1,4,8-triaza-spiro[4.5]dec-1-ylmethyl}-benzonitril,
[78] 1-Allyl-3-(2-methylsulfanyl-ethyl)-8-[2-(2,2,2-trifluor-acetyl)-1,2,3.4-tetrahydro-isoquinolin-7-sulfonyl]-1,4,8-triaza-spiro[4.5]decan-2-on,
[79] 1-Allyl-8-(4-methoxy-2,3,6-trimethyl-benzolsulfonyl)-3-(2-methylsulfanyl-ethyl)-1,4,8-triaza-spiro[4.5]decan-2-on,
[80] 2-(3-Benzyl-1-ethoxycarbonylmethyl-2-oxo-1,4,8-triaza-spiro[4.5]decan-8-sulfonyl)-benzoesäuremethylester,
[81] 3-Benzyl-1-(2-fluor-benzyl)-8-(2-trifluormethyl-benzolsulfonyl)-1,4,8-triaza-spiro[4.5]decan-2-on,
[82] 8-(2-Chlor-benzolsulfonyl)-3-(2-methylsulfanyl-ethyl)-1-(2-nitro-benzyl)-1,4,8-triaza-spiro[4.5]decan-2-on,
[83] 1-Allyl-3-(2-methylsulfanyl-ethyl)-8-(toluol-3-sulfonyl)-1,4,8-triaza-spiro[4.5]decan-2-on,
[84] [8-(2-Chlor-benzolsulfonyl)-3-(2-methylsulfanyl-ethyl)-2-oxo-1,4,8-triaza-spiro[4.5]dec-1-yl]-essigsäureethylester,
[85] 3-Benzyl-1-(2-fluor-benzyl)-8-(toluol-4-sulfonyl)-1,4,8-triaza-spiro[4.5]decan-2-on,
[86] 4-[8-(2,5-Dichlor-thiophen-3-sulfonyl)-2-oxo-1,4,8-triaza-spiro[4.5]dec-1-ylmethyl]-benzoesäuremethylester,
[87] 3-[8-(4-Fluor benzolsulfonyl)-3-isopropyl-2-oxo-1,4,8-triaza-spiro[4.5]dec-1-ylmethyl]-benzonitril,
[88] 8-(2,5-Dimethoxy-benzolsulfonyl)-3-(2-methylsulfanyl-ethyl)-1-(2-nitro-benzyl)-1,4,8-triaza-spiro[4.5]decan-2-on,
[89] 8-(3-Chlor-4-fluor-benzolsulfonyl)-1-(2-fluor-benzyl)-1,4,8-triazaspiro[4.5]decan-2-on,
[90] 1-(2-Fluor-benzyl)-8-(toluol-3-sulfonyl),1,4,8-triaza-spiro[4.5]decan-2-on,
[91] 1-(2,6-Dichlor-benzyl)-8-(4,5-dichlor-thiophen-2-sulfonyl)-3-isobutyl-1,4,8-triaza-spiro[4.5]decan-2-on,
[92] 8-(5-Chlor-thiophen-2-sulfonyl)-1-(2-fluor-benzyl)-1,4,8-triaza-spiro[4.5]decan-2-on,
[93] 3-Benzyl-1-(2-fluor-benzyl)-8-(4-propyl-benzolsulfonyl)-1,4,8-triaza-spiro[4.5]decan-2-on,
[94] 3-[8-(Butan-1-sulfonyl)-3-isopropyl-2-oxo-1,4,8-triaza-spiro[4.5]dec-1-ylmethyl]-benzonitril,
[95] 3-[8-(2-Methansulfonyl-benzolsulfonyl)-2-oxo-1,4,8-triaza-spiro[4.5]dec-1-ylmethyl]-benzonitril,
[96] 8-(2-Fluor-benzolsulfonyl)-1-(2-fluor-benzyl)-1,4,8-triaza-spiro[4.5]decan-2-on,
[97] 2-[1-(3-Cyano-benzyl)-3-isopropyl-2-oxo-1,4,8-triaza-spiro[4.5]decan-8-sulfonyl]-benzoesäuremethylester,
[98] 3-Benzyl-1-(2-fluor-benzyl)-8-(5-fluor-2-methyl-benzolsulfonyl)-1,4,8-triaza-spiro[4.5]decan-2-on,
[99] 1-Allyl-8-(4-methoxy-benzolsulfonyl)-3-(2-methylsulfanyl-ethyl)-1,4,8-triaza-spiro[4.5]decan-2-on,
[100] 1-(3,5-Dimethyl-benzyl)-3-isobutyl-8-(thiophen-2-sulfonyl)-1,4,8-triaza-spiro[4.5]decan-2-on,
[101] 3-(2-Methylsulfanyl-ethyl)-8-(4-nitro-benzolsulfonyl)-1-(2-nitro-benzyl)-1,4,8-triaza-spiro[4.5]decan-2-on,
[102]2-Isobutyl-3-oxo-1,4,8-triaza-spiro[4.5]decan-8-thiocarbonsäure-o-tolylamid,
[103]2-Benzyl-3-oxo-1,4,8-triaza-spiro[4.5]decan-8-thiocarbonsäure isopropylamid,
[104] 1-Butyl-3-isopropyl-2-oxo-1,4,8-triaza-spiro[4.5]decan-8-thiocarbonsäure (tetrahydro-furan-2-ylmethyl)-amid,
[105] 3-{[1-Methyl-3-(2-methylsulfanyl-ethyl)-2-oxo-1,4,8-triaza-spiro[4.5]decan-8-carbothioyl]-amino}-buttersäureethylester,
[106] 1-Allyl-3-isopmpyl-2-oxo-1,4,8-triaza-spim[4.5]decan-8-thiocarbonsäure isopropylamid,
[107] 1-Butyl-3-isopropyl-2-oxo-1,4,8-triaza-spiro[4.5]decan-8-thiocarbonsäure isopropylamid,
[108] 3-Benzyl-1-(2-fluor-benzyl)-2-oxo-1,4,8-triaza-spiro[4.5]decan-8-thiocarbonsäure (2-methoxy-benzol)-amid,
[109] [8-Isopropylthlocarbamoyl-3-(2-methylsulfanyl-ethyl)-2-oxo-1,4,8-triaza-spiro[4.5]dec-1-yl]-essigsäureethylester,
[110] 1-Methyl-3-(2-methylsulfanyl-ethyl)-2-oxo-1,4,8-triaza-spiro[4.5]decan-8-thiocarbonsäure m-tolylamid,
[111] 1-Methyl-3-(2-methylsulfanyl-ethyl)-2-oxo-1,4,8-triaza-spiro[4.5]decan-8-thiocarbonsäure 4-methoxy-benzylamid,
[112] 2-Isobutyl-3-oxo-1,4,8-triaza-spiro[4.5]decan-8-thiocarbonsäure (4-fluor-benzol)-amid,
[113] [8-Ethoxycarbonylaminocarbothioyl-3-(2-methylsulfanyl-ethyl)-2-oxo-1,4,8-triaza-spiro[4.5]dec-1-yl]-essigsäureethylester
[114] 3-{[1-Ethoxycarbonylmethyl-3-(2-methylsulfanyl-ethyl)-2-oxo-1,4,8-triazaspiro[4.5]decan-8-carbothioyl]-amino}-buttersäureethylester,
[115] 1-Allyl-3-isopropyl-2-oxo-1,4,8-triaza-spiro[4.5]decan-8-thiocarbonsäure (2-methoxy-benzol)-amid,
[116] 1-Butyl-3-isopropyl-2-oxo-1,4,8-triaza-spiro[4.5]decan-8-thiocarbonsäure benzolamid,
[117] 1-Butyl-3-isopropyl-2-oxo-1,4,8-triaza-spiro[4.5]decan-8-thiocarbonsäure (2,5-dichlor-benzol)-amid,
[118] 3-Benzyl-1-methyl-2-oxo-1,4,8-triaza-spiro[4.5]decan-8-thiocarbonsäure (2,5-difluor-benzol)-amid,
[119] 1-Methyl-3-(2-methylsulfanyl-ethyl)-2-oxo-1,4,8-triaza-spiro[4.5]decan-8-thiocarbonsäure (2-brom-benzol)-amid,
[120] 1-Butyl-3-(2-methylsulfanyl-ethyl)-2-oxo-1,4,8-triaza-spiro[4.5]decan-8-thiocarbonsäure (2,5-dichlor-benzol)-amid,
[121] 3-(2-Methylsulfanyl-ethyl)-1-naphthalin-2-ylmethyl-2-oxo-1,4,8-triaza-spiro[4.5]decan-8-thiocarbonsäure (2-methoxy-ethyl)-amid,
[122] 1-Butyl-3-isopropyl-2-oxo-1,4,8-triaza-spiro[4.5]decan-8-thiocarbonsäure (2-fluor-benzol)-amid,
[123] 3-Isobutyl-2-oxo-1-prop-2-ynyl-1,4,8-triaza-spiro[4.5]decan-8-thiocarbonsäure allylamid,
[124] 1-Butyl-3-isopropyl-2-oxo-1,4,8-triaza-spiro[4.5]decan-8-thiocarbonsäure m-tolylamid,
[125] 1-Allyl-3-isopropyl-2-oxo-1,4,8-triaza-spiro[4.5]decan-8-thiocarbonsäure (4-fluor-benzol)-amid,
[126] 4-(8-Allylthiocarbamoyl-3-isopropyl-2-oxo-1,4,8-triaza-spiro[4.5]dec-1-ylmethyl)-benzoesäuremethylester,
[127] 1-Butyl-3-(2-methylsulfanyl-ethyl)-2-oxo-1,4,8-triaza-spiro[4.5]decan-8-thiocarbonsäure (2-methoxy-benzol)-amid,
[128] 1-Allyl-3-isopropyl-2-oxo-1,4,8-triaza-spiro[4.5]decan-8-thiocarbonsäure (2,5-difluor-benzol)-amid,
[129] 1-(2-Cyano-benzyl)-3-(2-methylsulfanyl-ethyl)-2-oxo-1,4,8-triaza-spiro[4.5]decan-8-thiocarbonsäure allylamid,
[130] 1-(3-Cyano-benzyl)-3-(2-methylsulfanyl-ethyl)-2-oxo-1,4,8-triaza-spiro[4.5]decan-8-thiocarbonsäure allylamid,
[131] 3-[(3-Benzyl-1-methyl-2-oxo-1,4,8-triaza-spiro[4.5]decan-8-carbothioyl)-amino]-buttersäureethylester,
[132] 1-Butyl-3-(2-methylsulfanyl-ethyl)-2-oxo-1,4,8-triaza-spiro[4.5]decan-8-thiocarbonsäure cyclohexylmethyl-amid,
[133] 1-Methyl-3-(2-methylsulfanyl-ethyl)-2-oxo-1,4,8-triaza-spiro[4.5]decan-8-thiocarbonsäure (4-chlor-benzol)-amid,
[134] 1-Methyl-3-(2-methylsulfanyl-ethyl)-2-oxo-1,4,8-triaza-spiro[4.5]decan-8-thiocarbonsäure 4-fluor-benzylamid,
[135] 4-[8-(2,6-Dichlor-benzolthiocarbamoyl)-3-isopropyl-2-oxo-1,4,8-triaza-spiro[4.5]dec-1-ylmethyl]-benzoesäuremethylester,
[136] 1-Benzyl-3-isopropyl-2-oxo-1,4,8-triaza-spiro[4.5]decan-8-thiocarbonsäure (2,5-dichlor-benzol)-amid,
[137] 4-{3-Isopropyl-2-oxo-8-[(tetrahydro-furan-2-ylmethyl)-thiocarbamoyl]-1,4,8-triaza-spiro[4.5]dec-1-ylmethyl)-benzoesäuremethylester,
[138] 1-Methyl-3-(2-methylsulfanyl-ethyl)-2-oxo-1,4,8-triaza-spiro[4.5]decan-8-thiocarbonsäure (2,5-difluor-benzol)-amid,
[139] 1-Benzyl-3-(2-methylsulfanyl-ethyl)-2-oxo-1,4,8-triaza-spiro[4.5]decan-8-thiocarbonsäure (4-isopropyl-benzol)-amid,
[140] 1-Benzyl-3-(2-methylsulfanyl-ethyl)-2-oxo-1,4,8-triaza-spiro[4.5]decan-8-thiocarbonsäure (2,5-difluor-benzol)-amid,
[141]1-(2-Cyano-benzyl)-3-isobutyl-2-oxo-1,4,8-triaza-spiro[4.5]decan-8-thiocarbonsäure (3-acetyl-benzol)-amid,
[142] 1-Butyl-3-isopropyl-2-oxo-1,4,8-triaza-spiro[4.5]decan-8-thiocarbonsäure (3-chlor-benzol)-amid,
[143] 2-[(1-Butyl-3-isopropyl-2-oxo-1,4,8-triaza-spiro[4.5]decan-8-carbothioyl)-amino]-benzoesäuremethylester,
[144] (3-Benzyl-8-ethoxycarbonylaminocarbothioyl-2-oxo-1,4,8-triaza-spiro[4.5]dec-1-yl)-essigsäureethylester,
[145]2-[(3-Benzyl-1-ethoxycarbonylmethyl-2-oxo-1,4,8-triaza-spiro[4.5]decan-8-carbothioyl)-amino]-benzoesäuremethylester,
[146] [1-(2-Cyano-benzyl)-3-(2-methylsulfanyl-ethyl)-2-oxo-1,4,8-triaza-spiro[4.5]decan-8-carbothioyl]-carbamidsäureethylester,
[147]1-(2-Cyano-benzyl)-3-(2-methylsulfanyl-ethyl)-2-oxo-1,4,8-triaza-spiro[4.5]decan-8-thiocarbonsäure (2-chlor-5-trifluormethyl-benzol)-amid,
[148] 3-(2-Methylsulfanyl-ethyl)-1-naphthalin-2-ylmethyl-2-oxo-1,4,8-triaza-spiro[4.5]decan-8-thiocarbonsäure (1-benzol-ethyl)-amid,
[149] 1-Allyl-3-isopropyl-2-oxo-1,4,8-triaza-spiro[4.5]decan-8-thiocarbonsäure pentafluorbenzol-amid,
[150] 1-Butyl-3-isopropyl-2-oxo-1,4,8-triaza-spiro[4.5]decan-8-thiocarbonsäure 4-chlor-benzylamid,
[151] 3-(2-Methylsulfanyl-ethyl)-1-naphthalin-2-ylmethyl-2-oxo-1,4,8-triaza-spiro[4.5]decan-8-thlocarbonsäure o-tolylamid,
[152] 1-Butyl-3-isopropyl-2-oxo-1,4,8-triaza-spiro[4.5]decan-8-thiocarbonsäure pentafluorbenzol-amid,
[153]2-{[1-(2-Cyano-benzyl)-3-(2-methylsulfanyl-ethyl)-2-oxo-1,4,8-triaza-spiro[4.5]decan-8-carbothioyl]-amino}-benzoesäuremethylester,
[154] 1-Benzyl-3-isopropyl-2-oxo-1,4,8-triaza-spiro[4.5]decan-8-thiocarbonsäure pentafluorbenzol-amid,
[155] 1-Benzyl-3-isopropyl-2-oxo-1,4,8-triaza-spiro[4.5]decan-8-thiocarbonsäure (2-methoxy-benzol)-amid,
[156] 1-Benzyl-3-isopropyl-2-oxo-1,4,8-triaza-spiro[4.5]decan-8-thiocarbonsäure benzolamid,
[157] 1-Benzyl-3-isopropyl-2-oxo-1,4,8-triaza-spiro[4.5]decan-8-thiocarbonsäure (2,5-difluor-benzol)-amid,
[158] 1-Butyl-3-(2-methylsulfanyl-ethyl)-2-oxo-1,4,8-triaza-spiro[4.5]decan-8-thiocarbonsäure (4-ethoxy-benzol)-amid,
[159] [3-Benzyl-8-(cyclohexylmethyl-thiocarbamoyl)-2-oxo-1,4,8-triaza-spiro[4.5]dec-1-yl]-essigsäureethylester,
[160] 1-Butyl-3-(2-methylsulfanyl-ethyl)-2-oxo-1,4,8-triaza-spiro[4.5)decan-8-thiocarbonsäure benzylamid,
[161]1-(2-Cyano-benzyl)-3-(2-methylsulfanyl-ethyl)-2-oxo-1,4,8-triaza-spiro[4.5]decan-8-thiocarbonsäure m-tolylamid,
[162] 1-Allyl-3-isopropyl-2-oxo-1,4,8-triaza-spiro[4.5]decan-8-thiocarbonsäure 4-fluor-benzylamid,
[163] 3-Isobutyl-2-oxo-1-prop-2-ynyl-1,4,8-triaza-spiro[4.5]decan-8-thiocarbonsäure (3-acetyl-benzol)-amid,
[164]4-(3-Isopropyl-2-oxo-8-pentafluorbenzolthiocarbamoyl-1,4,8-triaza-spiro[4.5]dec-1-ylmethyl)-benzoesäuremethylester,
[165] 3-(2-Methylsulfanyl-ethyl)-1-naphthalin-2-ylmethyl-1-2-oxo-1,4,8-triaza-spiro[4.5]decan-8-thiocarbonsäure (3-acetyl-benzol)-amid,
[166]3-Benzyl-1-(2-fluor-benzyl)-2-oxo-1,4,8-triaza-spiro[4.5]decan-8-thiocarbonsäure m-tolylamid,
[167] 4-(8-Ethoxycarbonylaminocarbothioyl-3-isopropyl-2-oxo-1,4,8-triaza-spiro[4.5]dec-1-ylmethyl)-benzoesäuremethylester,
[168] 1-(4-Fluor-benzyl)-3-(2-methylsulfanyl-ethyl)-2-oxo-1,4,8-triaza-spiro[4.5]decan-8-thiocarbonsäure (tetrahydro-furan-2-ylmethyl)-amid,
[169] 1-(2-Cyano-benzyl)-3-isobutyl-2-oxo-1,4,8-triaza-spiro[4.5]decan-8-thiocarbonsäure (3,5-bis-trifluormethyl-benzol)-amid,
[170] 1-(2-Cyano-benzyl)-3-isobutyl-2-oxo-1,4,8-triaza-spiro[4.5]decan-8-thiocarbonsäure benzylamid,
[171] 3-Benzyl-1-(2-fluor-benzyl)-2-oxo-1,4,8-triaza-spiro[4.5]decan-8-thiocarbonsäure cyclohexylmethyl-amid,
[172] 1-(3-Cyano-benzyl)-3-(2-methylsulfanyl-ethyl)-2-oxo-1,4,8-triazaspiro[4.5]decan-8-thlocarbonsäure (4-chlor-benzol)-amid,
[173] 1-Butyl-3-(2-methylsulfanyl-ethyl)-2-oxo-1,4,8-triaza-spiro[4.5]decan-8-thiocarbonsäure pentafluorbenzol-amid,
[174] [3-Benzyl-1-(2-ftuor-benzyl)-2-oxo-1,4,8-triaza-spiro[4.5]decan-8-carbothioyl]-carbamidsäureethylester,
[175] 3-Benzyl-1-methyl-2-oxo-1,4,8-triaza-spiro[4.5]decan-8-thiocarbonsäure 4-chlor-benzylamid,
[176] 1-(4-Fluor benzyl)-3-(2-methylsulfanyl-ethyl)-2-oxo-1,4,8-triaza-spiro[4.5]decan-8-thiocarbonsäure isopropylamid,
[177] 1-Benzyl-3-isopropyl-2-oxo-1,4,8-triaza-spiro[4.5]decan-8-thiocarbonsäure (3,5-dichlor-benzol)-amid,
[178] [1-(3-Cyano-benzyl)-3-(2-methylsulfanyl-ethyl)-2-oxo-1,4,8-triaza-spiro[4.5]decan-8-carbothioyl]-carbamidsäureethylester,
[179] 1-Benzyl-3-isopropyl-2-oxo-1,4,8-triaza-spiro[4.5]decan-8-thiocarbonsäure 4-fluor-benzylamid,
[180] 1-Allyi-3-isopropyl-2-oxo-1,4,8-triaza-spiro[4.5]decan-8-thiocarbonsäure (3,5-dichlor-benzolfamid,
[181]1-(3-Cyano-benzyl)-3-(2-methylsulfanyl-ethyl)-2-oxo-1,4,8-triazaspiro[4.5]decan-8-thiocarbonsäure (1-benzol-ethyl)-amid,
[182] 1-(4-Fluor-benzyl)-3-(2-methylsulfanyl-ethyl)-2-oxo-1,4,8-triaza-spiro[4.5]decan-8-thiocarbonsäure 4-fluor-benzylamid,
[183]1-(2-Cyano-benzyl)-3-(2-methylsulfanyl-ethyl)-2-oxo-1,4,8-triaza-spiro[4.5]decan-8-thiocarbonsäure (2-methoxy-benzol)-amid,
[184] 1-(3-Cyano-benzyl)-3-(2-methylsulfanyl-ethyl)-2-oxo-1,4,8-triaza-spiro[4.5]decan-8-thiocarbonsäure (2-methoxy-benzol)-amid,
[185]1-(3-Cyano-benzyl)-3-(2-methylsulfanyl-ethyl)-2-oxo-1,4,8-triaza-spiro[4.5]decan-8-thiocarbonsäure 4-chlor-benzylamid,
[186] 1-(4-Fluor-benzyl)-3-(2-methylsulfanyl-ethyl)-2-oxo-1,4,8-triaza-spiro[4.5]decan-8-thiocarbonsäure o-tolylamid,
[187]3-Benzyl-1-(2-fluor-benzyl)-2-oxo-1,4,8-triaza-spiro[4.5]decan-8-thiocarbonsäure (2,6-dichlor-benzol)-amid,
[188]3-Benzyl-1-(2-fluor-benzyl)-2-oxo-1,4,8-triaza-spiro[4.5]decan-8-thiocarbonsäure (2-chlor-5-tdfluormethyl-benzol)-amid,
[189] 1-Butyl-3-(2-methylsulfanyl-ethyl)-2-oxo-1,4,8-triaza-spiro[4.5]decan-8-thiocarbonsäure (4-fluor-benzol)-amid,
[190] 3-Isobutyl-2-oxo-1-prop-2-ynyl-1,4,8-triaza-spiro[4.5]decan-8-thlocarbonsäure (2,6-dichlor-benzol)-amid,
[191] 4-[8-(4-Chlor-benzylthiocarbamoyl-3-isopropyl-2-oxo-1,4,8-triaza-spiro[4.5]dec-1-ylmethyl]-benzoesäuremethylester,
[192]1-(4-Fluor-benzyl)-3-(2-methylsulfanyl-ethyl)-2-oxo-1,4,8-triaza-spiro[4.5]decan-8-thiocarbonsäure (3-cyano-benzol)-amid,
[193] 1-(2-Cyano-benzyl)-3-(2-methylsulfanyl-ethyl)-2-oxo-1,4,8-triazaspiro[4.5]decan-8-thiocarbonsäure 4-chlor-benzylamid,
[194] 1-Methyl-3-(2-methylsulfanyl-ethyl)-2-oxo-1,4,8-triaza-spiro[4.5]decan-8-thiocarbonsäure (3-trifluormethyl-benzol)+amid,
[195] 3-Isobutyl-2-oxo-1-prop-2-ynyl-1,4,8-triaza-spiro[4.5]decan-8-thiocarbonsäure (3,5-dichlor-benzolfamid,
[196] 1-(4-Fluor-benzyl)-3-(2-methylsulfanyl-ethyl)-2-oxo-1,4,8-triaza-spiro[4.5]decan-8-thiocarbonsäure (1-benzol-ethyl)-amid,
[197] 3-Benzyl-1-(2-fluor-benzyl)-2-oxo-1,4,8-triaza-spiro[4.5]decan-8-thiocarbonsäure 4-chlor-benzylamid,
[198] 2-lsopropyl-3-oxo-1,4,8-triaza-spiro[4.5]decan-8-thiocarbonsäure (4-fluor-benzol)-amid,
[199] 1-Benzyl-3-isopropyl-2-oxo-1,4,8-triaza-spiro[4.5]decan-8-thiocarbonsäure benzylamid,
[200] 1-(4-Fluor-benzyl)-3-(2-methylsulfanyl-ethyl)-2-oxo-1,4,8-triaza-spiro[4.5]decan-8-thiocarbonsäure (4-ethoxy-benzol)-amid,
[201] 3-Benzyl-1-(2-fluor-benzyl)-2-oxo-1,4,8-triaza-spiro[4.5]decan-8-thiocarbonsäure (3-acetyl-benzol)-amid,
[202] 3-Benzyl-1-methyl-2-oxo-1,4,8-triaza-spiro[4.5]decan-8-carbonsäure cyclohexylamid,
[203][3-(2-Methylsulfanyl-ethyl)-2-oxo-8-benzolcarbamoyl-1,4,8-triaza-spiro[4.5]dec-1-yl]-essigsäureethylester,
[204] 1-Allyl-3-isopropyl-2-oxo-1,4,8-triaza-spiro[4.5]decan-8-carbonsäure (2,5-dimethoxy-benzol)-amid,
[205] 1-Methyl-3-(2-methylsulfanyl-ethyl)-2-oxo-1,4,8-triaza-spiro[4.5]decan-8-carbonsäure (3-fluor-benzol)-amid,
[206] 4-(8-Cyclohexylcarbamoyl-3-isopropyl-2-oxo-1,4,8-triaza-spiro[4.5]dec-1-ylmethyl)-benzoesäuremethylester,
[207] 1-Butyl-3-(2-methylsulfanyl-ethyl)-2-oxo-1,4,8-triaza-spiro[4.5]decan-8-carbonsäure benzolamid,
[208] 1-Benzyl-3-(2-methylsulfanyl-ethyl)-2-oxo-1,4,8-triaza-spiro[4.5]decan-8-carbonsäure (3-cyano-benzol)-amid,
[209] 1-Allyl-3-isopropyl-2-oxo-1,4,8-triaza-spiro[4.5]decan-8-carbonsäure (2,5-difluor-benzol)-amid,
[210] 1-Butyl-3-(2-methylsulfanyl-ethyl)-2-oxo-1,4,8-triaza-spiro[4.5]decan-8-carbonsäure (3,4,5-trimethoxy-benzol)-amid,
[211] 1-Benzyl-3-(2-methylsulfanyl-ethyl)-2-oxo-1,4,8-triaza-spiro[4.5]decan-8-carbonsäure (4-chlor-3-trifluormethyl-benzol)-amid,
[212] 1-Methyl-3-(2-methylsulfanyl-ethyl)-2-oxo-1,4,8-triaza-spiro[4.5]decan-8-carbonsäure (5-chlor-2-methoxy-benzol)-amid,
[213] 1-Benzyl-3-(2-methylsulfanyl-ethyl)-2-oxo-1,4,8-triaza-spiro[4.5]decan-8-carbonsäure (4-ethoxy-benzol)-amid,
[214] 1-Benzyl-3-(2-methylsulfanyl-ethyl)-2-oxo-1,4,8-triaza-spiro[4.5]decan-8-carbonsäure (3-acetyl-benzol)-amid,
[215] 3-[(1-Butyl-3-isopropyl-2-oxo-1,4,8-triaza-spiro[4.5]decan-8-carbonyl)-amino]-benzoesäureethylester,
[216] 1-Butyl-3-isopropyl-2-oxo-1,4,8-triaza-spiro[4.5]decan-8-carbonsäure (2,5-dimethoxy-benzol)-amid,
[217] 1-(2-Cyano-benzyl)-3-isobutyl-2-oxo-1,4,8-triaza-spiro[4.5]decan-8-carbonsäure 3,4-dichlor-benzylamid,
[218]1-(2-Cyano-benzyl)-3-isobutyl-2-oxo-1,4,8-triaza-spiro[4.5]decan-8-carbonsäure (2,5-difluor-benzol)-amid,
[219]1-Butyl-3-(2-methylsulfanyl-ethyl)-2-oxo-1,4,8-triaza-spiro[4.5]decan-8-carbonsäure 3,4-dichlor-benzylamid,
[220] 1-Allyl-3-isopropyl-2-oxo-1,4,8-triaza-spiro[4.5]decan-8-carbonsäure (4-trifluormethoxy-benzol)-amid,
[221]1-Butyl-3-(2-methylsulfanyl-ethyl)-2-oxo-1,4,8-triaza-spiro[4.5]decan-8-carbonsäure (4-ethoxy-benzol)-amid,
[222] 1-Methyl-3-(2-methylsulfanyl-ethyl)-2-oxo-1,4,8-triaza-spiro[4.5]decan-8-carbonsäure (4-trifluormethoxy-benzol)-amid,
[223] 4-[3-lsopropyl-8-(3-methoxy-benzolcarbamoyl)-2-oxo-1,4,8-triaza-spiro[4.5]dec-1-ylmethyl]-benzoesäuremethylester,
[224] 1-(2-Cyano-benzyl)-3-(2-methylsulfanyl-ethyl)-2-oxo-1,4,8-triaza-spiro[4.5]decan-8-carbonsäure benzolamid,
[225][3-Benzyl-8-(3,4-dichlor-benzylcarbamoyl)-2-oxo-1,4,8-triaza-spiro[4.5]dec-1-yl]-essigsäureethylester,
[226] 1-(2-Cyano-benzyl)-3-(2-methylsulfanyl-ethyl)-2-oxo-1,4,8-triazaspiro[4.5]decan-8-carbonsäure (3-fluor-benzol)-amid,
[227]1-Butyl-3-(2-methylsulfanyl-ethyl)-2-oxo-1,4,8-triaza-spiro[4.5]decan-8-carbonsäure (4-chlor-3-trifluormethyl-benzol)-amid,
[228] 1-(4-Fluor-benzyl)-3-(2-methylsulfanyl-ethyl)-2-oxo-1,4,8-triaza-spiro[4.5]decan-8-carbonsäure 3,4-dichlor-benzylamid,
[229] 1-(2-Cyano-benzyl)-3-(2-methylsulfanyl-ethyl)-2-oxo-1,4,8-triazaspiro[4.5]decan-8-carbonsäure 3,4-dichlor-benzylamid,
[230]3-Benzyl-1-(2-fluor-benzyl)-2-oxo-1,4,8-triaza-spiro[4.5]decan-8-carbonsäure (2,5-difluor-benzol)-amid,
[231]3-(2-Methylsulfanyl-ethylf)-naphthalin-2-ylmethyl-2-oxo-1,4,8-triazaspiro[4.5]decan-8-carbonsäure (4-ethoxy-benzol)-amid,
[232] 4-[8-(3,4-Dichlor-benzylcarbamoyl)-3-isopropyl-2-oxo-1,4,8-triaza-spiro[4.5]dec-1-ylmethyl]-benzoesäuremethylester,
[233]3-Benzyl-1-(2-fluor-benzyl)-2-oxo-1,4,8-triaza-spiro[4.5]decan-8-carbonsäure 3,4-dichlor-benzylamid,
[234] 1-(2-Cyano-benzyl)-3-(2-methylsutfanyl-ethyl)-2-oxo-1,4,8-triaza-spiro[4.5]decan-8-carbonsäure (3-acetyl-benzol)-amid,
[235]4-{[3-Benzyl-1-(2-fluor-benzyl)-2-oxo-1,4,8-triaza-spiro[4.5]decan-8-carbonyl]-amino}-benzoesäureethylester,
[236] 1-(3-Cyano-benzyl)-3-(2-methylsulfanyl-ethyl)-2-oxo-1,4,8-triaza-spiro[4.5]decan-8-carbonsäure (3-methoxy-benzol)-amid,
[237]1-(4-Fluor-benzyl)-3-(2-methylsulfanyl-ethyl)-2-oxo-1,4,8-triaza-spiro[4.5]decan-8-carbonsäure (4-trifluormethoxy-benzol)-amid,
[238] 3-Benzyl-1-(2-fluor-benzyl)-2-oxo-1,4,8-triaza-spiro[4.5]decan-8-carbonsäure cyclohexylamid,
[239]1-(4-Fluor benzyl)-3-(2-methylsulfanyl-ethyl)-2-oxo-1,4,8-triaza-spiro[4.5]decan-8-carbonsäure phenethyl-amid,
[240] 3-(2-Methylsulfanyl-ethyl)-1-naphthalin-2-ylmethyl-2-oxo-1,4,a-triazaspiro[4.5]decan-8-carbonsäure (3-fluor-benzol)-amid,
[241]4-[8-(4-Chlor-3-trifluormethyl-benzolcarbamoyl)-3-isopropyl-2-oxo-1,4,8-triazaspiro[4.5]dec-1-ylmethyl]-benzoesäuremethylester,
[242] 1-(3-Cyano-benzyl)-3-(2-methylsulfanyl-ethyl)-2-oxo-1,4,8-triazaspiro[4.5]decan-8-carbonsäure (2,5-dimethoxy-benzol)-amid,
[243] 1-(4-Fluor-benzyl)-3-(2-methylsulfanyl-ethyl)-2-oxo-1,4,8-triaza-spiro[4.5]decan-8-carbonsäure (4-ethoxy-benzol)-amid,
[244] 1-(4-Fluor-benzyl)-3-(2-methylsulfanyl-ethyl)-2-oxo-1,4,8-triaza-spiro[4.5]decan-8-carbonsäure (3-cyano-benzol)-amid und
[245] [3-Benzyl-8-(3-fluor-benzolcarbamoyl)-2-oxo-1,4,8-triaza-spiro[4.5]dec-1-yl]-essigsäureethylester,
[246] 1,3-Dibenzyl-8-methansulfonyl-1,4,8-triaza-spiro[4.5]decan-2-on,
[247] 8-Benzolsulfonyl-1,3-dibenzyl-1,4,8-triaza-spiro[4.5]decan-2-on,
[248] 1,3-Dibenzyl-8-(4-chlor benzolsulfonyl)-1,4,8-triaza-spiro[4.5]decan-2-on,
[249] 1,3-Dibenzyl-8-(4-methoxy-benzolsulfonyl)-1,4,8-triaza-spiro[4.5]decan-2-on,
[250] 1,3-Dibenzyl-2-oxo-1,4,8-triaza-spiro[4.5]decan-8-carbonsäurephenylamid,
[251] 1,3-Dibenzyl-2-oxo-1,4,8-triaza-spiro[4.5]decan-8-carbonsäure-(4-methoxyphenyl)-amid,
[252] 1,3-Dibenzyl-2-oxo-1,4,8-triaza-spiro[4.5]decan-8-thiocarbonsäurephenylamid,
[253] 1,3-Dibenzyl-2-oxo-1,4,8-triaza-spiro[4.5]decan-8-thiocarbonsäure-(2,4-difluorphenyl)-amid,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ein weiterer Gegenstand der vorliegenden Erfindung ist ein Verfahren zur Herstellung der erfindungsgemäßen substituierten 1,4,8-Triazaspiro[4.5]decan-2-on-Verbindungen der vorstehend angegebenen allgemeinen Formel I, gemäß dem ein geschütztes Piperidin-4-on der allgemeinen Formel II, 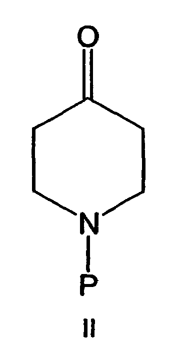 worin P für eine Schutzgruppe steht, durch Umsetzung mit wenigstens einer Aminosäureamid-Verbindung der allgemeinen Formel III, 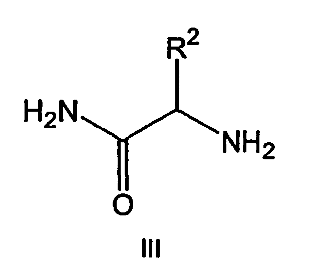 worin R² die vorstehend genannte Bedeutung hat, in wenigstens eine Verbindung der allgemeinen Formel IV, 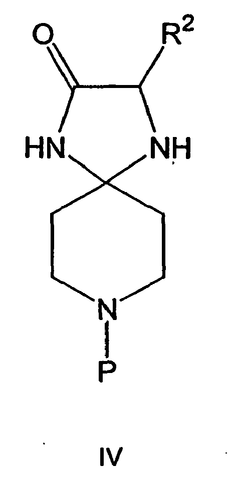 worin P und R² die vorstehend genannte Bedeutung haben, überführt wird, welche ggf. gereinigt und/oder ggf. isoliert wird, und gegebenenfalls durch Umsetzung mit wenigstens einer Verbindung der allgemeinen Formel R¹-X¹, worin R¹ die vorstehend genannte Bedeutung hat und X¹ für eine geeignete Austrittsgruppe, vorzugsweise für einen Halogen-Rest, steht, ggf. in Gegenwart wenigstens einer Base, in wenigstens eine Verbindung der allgemeinen Formel V, 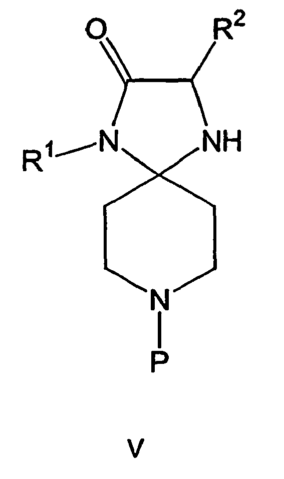 worin R¹, R² und P die vorstehend genannte Bedeutung haben, überführt wird, und diese ggf. gereinigt und/oder ggf. isoliert wird, und ggf. durch Abspaltung der Schutzgruppe P in wenigstens eine Verbindung der allgemeinen Formel VI, 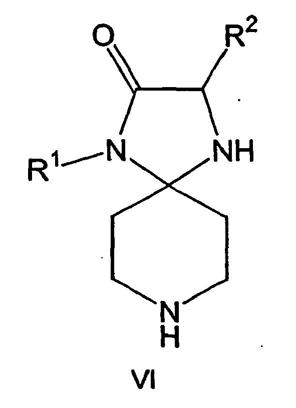 überführt wird, diese ggf. gereinigt und/oder ggf. isoliert wird, und wenigstens eine Verbindung der allgemeinen Formel IV, V oder VI durch Umsetzung mit einer Sulfonylverbindung der allgemeinen Formel R⁴-SO₂-X² ggf. in Gegenwart wenigstens einer Base, durch Umsetzung mit einer lsothiocyanatverbindung der allgemeinen Formel R⁵-NCS oder durch Umsetzung mit einer Isocyanätverbindung der allgemeinen Formel R⁶-NCO, worin die Reste R⁴, R⁵ und R⁶ jeweils die vorstehend genannte Bedeutung haben und und X² für eine geeignete Austrittsgruppe, vorzugsweise für einen Halogen-Rest, steht in wenigstens eine Verbindung der vorstehend angegebenen allgemeinen Formel I überführt wird, worin die Reste R¹-R³ jeweils die vorstehend genannte Bedeutung haben, und diese Verbindung ggf. gereinigt und/oder ggf. isoliert wird.

Die N-geschützten Piperidin-4-on-Verbindungen der allgemeinen Formel II sind käuflich am Markt erhältlich oder können nach üblichen, dem Fachmann bekannten Methoden hergestellt werden. Geeignete Schutzgruppen sind beispielsweise Trifluoracetamid, Methoxycarbonyl, Ethoxycarbonyl, tert-Butoxycarbonyl, Benzyloxycarbonyl, Allyloxycarbonyl oder 9-Fluorenylmethoxycarbonyl,

Die Aminosäureamide der allgemeinen Formel III, die in dem erfindungsgemäßen Verfahren auch in Form ihres entsprechenden Salzes eingesetzt werden können, sind ebenfalls käuflich am Markt erhältlich oder können nach üblichen, dem Fachmann bekannten Methoden hergestellt werden. Die jeweiligen Aminosäureamide können in dem erfindungsgemäßen Verfahren sowohl in enantiomerenreiner Form, d.h. in (*S*)- oder (*R*)-Konfiguration, oder in Form eines racemischen Gemisches mit (*S,R*)-Konfiguration eingesetzt werden.

Die Umsetzung von Verbindungen der allgemeinen Formel II mit Verbindungen der allgemeinen Formel III zu N-geschützten, ggf. in 3-Position substituierten 1,4,8-triaza-spiro[4.5]decan-2-on-Verbindungen der allgemeinen Formel IV kann unter üblichen, dem Fachmann bekannten Bedingungen erfolgen. Vorzugsweise erfolgt die Umsetzung in einem geeigneten Reaktionsmedium, beispielsweise in einem oder mehreren trockenen organischen Lösungsmitteln. Geeignete Lösungsmittel sind beispielsweise Alkohole wie Ethanol oder chlorierte Kohlenwasserstoffe wie Methylenchlorid oder Chloroform. Die Temperatur während der Vereingung und

Umsetzung der Reaktionskomponenten kann über einen weiten Bereich variieren.

Die Umsetzung einer N-geschützten, ggf. in 3-Position substituierten 1,4,8-triazaspiro[4.5]decan-2-on-Verbindung der allgemeinen Formel IV mit einer Verbindung der allgemeinen Formel R¹-X¹ zu Verbindungen der allgemeinen Formel V erfolgt bevorzugt in einem Reaktionsmedium in Gegenwart wenigstens einer organischen Base und/oder in Gegenwart wenigstens einer anorganischen Base unter üblichen, dem Fachmann bekannten Bedingungen. Die Umsetzung kann vorteilhafterweise auch in der Mikrowelle durchgeführt werden.

Geeignete anorganische Basen sind beispielsweise Metallalkoholate wie Natriummethanolat, Natriumethanolat, Kalium-tert-butylat, Lithium- oder Natriumbasen wie Lithiumdiisopropylamid, Buthyllithium, tert-Butyllithium, Natriummethylat oder Metallhydride wie Kaliumhydrid, Lithiumhydrid, Natriumhydrid. Als geeignete organische Basen kommen beispielsweise Diisopropylethylamin oder Triethylamin in Betracht. Geeignete Reaktionsmedien sind organische Lösungsmittel wie beispielsweise Tetrahydrofuran.

Die Abspaltung der Schutzgruppe (P) zur Herstellung von N-ungeschützten, ggf. in 1- und/oder 3-Position substituierten 1,4,8-triaza-spiro[4.5]decan-2-on-Verbindungen der allgemeinen Formel VI kann ebenfalls unter üblichen, dem Fachmann bekannten Bedingungen erfolgen, die jeweils in Abhängigkeit von der verwendeten Schutzgruppe variieren. Beispielhaft seien die Abspaltung in Gegenwart einer anorganischen Base, Säure oder Lewis-Säure, wie Kaliumcarbonat, Lithiumhydroxid, Kaliumhydroxid, Schwefelsäure, Bromwasserstoffsäure, Flusssäure, Salzsäure, Bortrifluoridetherat, Bortrichlorid, einer organischen Säure wie Trifluoressigsäure, Trifluormethansulfonsäure, Essigsäure, die Abspaltung in Gegenwart einer organischen Base, wie Morpholin, Triethylamin, Diethylamin, Diisopropylethylamin, Pyridin oder die Hydrierung genannt.

Die jeweilige Verbindungen der allgemeinen Formel IV, V oder VI, insbesondere die jeweilige Verbindung der allgemeinen Formel VI, kann anschließend durch Sulfonylierung mit einer Sulfonylverbindung der allgemeinen Formel R⁴-SO₂-X², vorzugsweise in Gegenwart wenigstens einer organischen Base und/oder wenigstens einer anorganischen Base wie beispielsweise Natriumhydrogencarbonat, Diisopropylethylamin, Triethylamin, Pyridin oder Diethylamin oder unter Thiohamstoffbildung mit Isothiocyanaten der allgemeinen Formel R⁵-NCS oder unter Harnstoffbildung mit Isocyanaten der allgemeinen Formel R⁵-NCO unter üblichen, dem Fachmann bekannten Methoden in die jeweilige erfindungsgemäße substituierte 1,4,8-triaza-spiro[4.5]decan-2-on-Verbindung der allgemeinen Formel I, einschließlich entsprechender Stereoisomeren übergeführt werden.

Die Umsetzung von Verbindungen der allgemeinen Formel IV, V oder VI, insbesondere von Verbindungen der allgemeinen Formel VI, zu einer erfindungsgemäßen substituierten 1,4,8-triaza-spiro[4.5]decan-2-on-Verbindung der allgemeinen Formel I erfolgt bevorzugt in einem geeigneten Reaktionsmedium, beispielsweise in einem oder mehreren trockenen organischen Lösungsmitteln. Geeignete Lösungsmittel sind beispielsweise ggf. chlorierte, ggf. aromatische Kohlenwasserstoffe wie Toluol, Methylenchlorid oder Chloroform. Die Temperatur während der Vereingung und Umsetzung der Reaktionskomponenten kann über einen weiten Bereich variieren.

Die Verbindungen der allgemeinen Formeln R¹-X¹, R⁴-SO₂-X², R⁵-NCS und R⁶-NCO sind jeweils am Markt käuflich erhältlich oder können nach üblichen, dem Fachmann bekannten Methoden hergestellt werden. X¹ und X² sind übliche, dem Fachmann bekannte Austrittsgruppen, vorzugsweise Halogen-Reste, besonders bevorzugt Chlor-Reste.

Die nach den vorstehend beschriebenen Umsetzungen erhaltenen Zwischen- und Endprodukte können jeweils, falls gewünscht und/oder erforderlich, nach üblichen, dem Fachmann bekannten Methoden gereinigt und/oder isoliert werden. Geeignete Reingungsverfahren sind beispielsweise Extraktionsverfahren und chromatographische Verfahren wie Säulenchromatographie oder präparative Chromatographie.

Die erfindungsgemäßen substituierten 1,4,8-triaza-spiro[4.5]decan-2-on-Verbindungen der vorstehenden allgemeinen Formeln I sowie ggf. jeweils entsprechende Stereoisomere können nach üblichen, dem Fachmann bekannten Verfahren in Form entsprechender Salze, insbesondere in Form entsprechender physiologisch verträglicher Salze erhalten werden, erhalten werden, wobei das erfindungsgemäße Arzneimittel eines oder mehrere Salze einer oder mehrerer dieser Verbindungen aufweisen kann.

Die jeweiligen Salze der erfindungsgemäßen substituierten 1,4,8-triazaspiro[4.5]decan-2-on-Verbindungen der vorstehenden allgemeinen Formel I können beispielsweise durch Umsetzung mit einer oder mehreren anorganischen Säuren und/oder einer oder mehreren organischen Säuren erhalten werden. Geeignete Säuren können bevorzugt ausgewählt werden aus der Gruppe bestehend aus Perchlorsäure, Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, Sacharinsäure, Cyclohexansulfamidsäure, Aspartam, Monomethylsebacinsäure, 5-Oxo-prolin, Hexan-1-sulfonsäure, Nicotinsäure, 2-Aminobenzoesäure, 3-Aminobenzoesäure oder 4-Aminobenzoesäure, 2,4,6-Trimethylbenzoesäure, α-Liponsäure, Acetylglycin, Hippursäure, Phosphorsäure, Maleinsäure, Malonsäure und Asparaginsäure.

Die erfindungsgemäßen substituierten 1,4,8-triaza-spiro[4.5]decan-2-on-Verbindungen der vorstehenden allgemeinen Formel I sowie ggf. entsprechende Stereoisomere und jeweils deren physiologisch verträgliche Salze können auch nach üblichen, dem Fachmann bekannten Verfahren in Form ihrer Solvate, insbesondere in Form ihrer Hydrate erhalten werden.

Sofern die erfindungsgemäßen substituierten 1,4,8-triaza-spiro[4.5]decan-2-on-Verbindungen der vorstehenden allgemeinen Formel I nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeits-chromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomere, z.B. mittels HPLC an chiraler Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Es wurde überraschenderweise gefunden, daß die erfindungsgemäßen substituierten 1,4,8-Triazaspiro[4.5]decan-2-on-Verbindungen der vorstehend angegebenen allgemeinen Formel I sich nicht nur zur Regulation, vorzugsweise zur Inhibierung, der 5-Hydroxy-Tryptophan-Wiederaufnahme (Serotonin-Wiederaufnahme) und/oder zur Regulation, vorzugsweise zur Inhibierung, der Noradrenalin-Wiederaufnahme eignen, sondern darüber hinaus auch eine hohe Affinität für Bätrachotoxin-(BTX)-Rezeptoren und/oder Cannabinoid-Rezeptoren CB2 (CB2-Rezeptoren) aufweisen und sich daher insbesondere als pharmazeutische Wirkstoffe in Arzneimitteln zur Prophylaxe und/oder Behandlung von mit diesen Rezeptoren bzw. Prozessen in Verbindung stehenden Erkrankungen eignen.

Die erfindungsgemäßen 1,4,8-Triazaspiro[4.5]decan-2-on-Verbindungen der vorstehend angegebenen allgemeinen Formel I und ggf. entsprechende Stereoisomere sowie jeweils die entsprechenden Salze und Solvate sind toxikologisch unbedenklich und eignen sich daher als pharmazeutische Wirkstoffe in Arzneimitteln.

Ein weiterer Gegenstand der vorliegenden Erfindung ist daher ein Arzneimittel enthaltend wenigstens eine substituierte 1,4,8-Triazaspiro[4.5]decan-2-on-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. einen oder mehrere pharmazeutisch verträgliche Hilfsstoffe.

Vorzugsweise eignet sich das erfindungsgemäße Arzneimittel zur Regulation, insbesondere zur Hemmung, der Noradrenalin-Wiederaufnahme (Noradrenalin-Uptake), zur Regulation, insbesondere zur Hemmung, der 5-Hydroxy-Tryptophan-Wiederaufnahme (5-HT-Uptake) und/oder zur Batrachotoxin-(BTX)-Rezeptor-Regulation und/oder zur CB2-Rezeptor-Regulation.

Das erfindungsgemäße Arzneimittel eignet sich insbesondere zur Prophylaxe und/oder Behandlung von Depressionen.

Ebenfalls bevorzugt eignet sich das erfindungsgemäße Arzneimittel zur Prophylaxe und/oder Behandlung von Schmerzen, vorzugsweise zur Behandlung und/oder Prophylaxe von akuten Schmerzen, chronischen Schmerzen, neuropathischen Schmerzen und/oder Clusterkopfschmerzen.

Besonders bevorzugt eignet sich das erfindungsgemäße Arzneimittel zur kombinierten Prophylaxe und/oder Behandlung von Depressionen und Schmerzen, vorzugsweise zur kombinierten Behandlung von Depressionen und Schmerzen ausgewählt aus der Gruppe bestehend aus akuten Schmerzen, chronischen Schmerzen, neuropathischen Schmerzen und Clusterkopfschmerzen, besonders bevorzugt zur kombinierten Behandlung von Depressionen und chronischen Schmerzen. Unter chronischen Schmerzen werden dabei im Sinne der vorliegenden Erfindung insbesondere auch solche Schmerzen verstanden, die im Zusammenhang mit Krebserkrankungen stehen.

Weiterhin eignet sich das erfindungsgemäße Arzneimittel zur Prophylaxe und/oder Behandlung von Akohol- und/oder Drogen- und/oder Medikamentenmißbrauch, zur Prophylaxe und/oder Behandlung von Akohol- und/oder Drogen- und/oder Medikamentenabhängigkeit, zur Prophylaxe und/oder Behandlung von Entzündungen, zur Prophylaxe und/oder Behandlung von Antriebslosigkeit, zur Prophylaxe und/oder Behandlung von Störungen der Nahrungsmittelaufnahme, vorzugsweise ausgewählt aus der Gruppe bestehend aus Bulimie, Anorexie, Fettsucht und Kachexie, zur Prophylaxe und/oder Behandlung von Katalepsie, zur Vigilanzsteigerung, zur Libidosteigerung, zur Anxiolyse, zur Prophylaxe und/oder Behandlung von neurodegenerativen Erkrankungen, vorzugsweise einer oder mehrerer neurodegenerativer Erkrankungen ausgewählt aus der Gruppe bestehend aus Morbus Parkinson, Morbus Huntington, Morbus Alzheimer und Multipler Sklerose, zur Prophylaxe und/oder Behandlung von Ischämie und/oder zur Lokalanästhesie.

Weiterhin eignet sich das erfindungsgemäße Arzneimittel zur Prophylaxe und/oder Behandlung von einer oder mehrerer Erkrankungen ausgewählt aus der Gruppe bestehend aus nicht-akuten allergischen Erkrankungen, vorzugsweise allergischer Dermatitis; Asthma; Rhinitis; Konjunktivitis; Erkrankungen die durch 2-arachidonglycerol und/oder entsprechender Ether wie Hematol vermittelt werden; Septikämie; Krebs, insbesondere Blutkrebs und/oder Himtumore; Kreislaufstörungen; grünem Star; Entzündungen, vorzugsweise immunologisch vermittelten Entzündungserkrankungen, besonders bevorzugt rheumatoide Arthritis, Lupus erythematodes, Psoriasis und Thyroiditis; Diabetes; Blutvergiftung; Epilepsie; Tourettes Syndrom; Osteoporose; Morbus Bechterew; Gicht; gichtartiger Arthritis; Osteoarthritis; Durchblutungsstörungen; Ischämien, vorzugsweise Nierenischämie und renale Ischämie, und/oder zur Regulation des Immunsystems, vorzugsweise zur Unterdrückung des Immunsystems.

Die erfindungsgemäßen Verbindungen sind ferner geeignet, eine Reduzierung der Spastizität im Mutiple Sklerose Modell an der Maus (bestimmt wie in Baker et al., Nature, 2000, 404, 84 beschrieben), eine Inhibierung des in-vivo Wachstums von Gliom-Tumoren (bestimmt wie in C. Sanchez et al., Cancer Res. 2001, 61, 5784 beschrieben), periphere Antinozizeption (bestimmt wie in Malan et al., Pain 2001, 93, 239 beschrieben) sowie eine Modulierung der antitumoralen Eigenschaften von ajulemischer Säure (bestimmt wie in Recht et al., Biochem. Pharmacol. 2001, 62, 755 beschrieben) zu bewirken. Die entsprechenden Literaturbeschreibungen werden hiermit als Referenz eingeführt und gelten als Teil der vorliegenden Offenbarung.

Ein weiterer Gegenstand der vorliegenden Erfindung ist ferner die Verwendung wenigstens einer substituierten 1,4,8-Triazaspiro[4.5]decan-2-on-Verbindung der vorstehend angegebenen allgemeinen Formel, I, ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, und ggf. wenigstens eines pharmazeutisch verträgliche Hilfsstoffes zur Herstellung eines Arzneimittels zur Regulation, insbesondere zur Hemmung, der Noradrenalin-Wiederaufnahme (Noradrenalin-Uptake), zur Regulation, insbesondere zur Hemmung, der 5-Hydroxy-Tryptophan-Wiederaufnahme (5-HT-Uptake) und/oder zur Batrachotoxin-(BTX)-Rezeptor-Regulation und/oder zur CB2-Rezeptor-Regulation.

Bevorzugt ist insbesondere die Verwendung wenigstens einer substituierten 1,4,8-Triazaspiro[4.5]decan-2-on-Verbindung der vorstehend angegebenen allgemeinen Formel 1, ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, und ggf. wenigstens eines pharmazeutisch verträgliche Hilfsstoffes zur Herstellung eines Arzneimittels zur Prophylaxe und/oder Behandlung von Depressionen.

Bevorzugt ist die Verwendung wenigstens einer substituierten 1,4,8-Triazaspiro[4.5]decan-2-on-Verbindung der vorstehend angegebenen allgemeinen Formel I, ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, und ggf. wenigstens eines pharmazeutisch verträglichen Hilfsstoffes zur Herstellung eines Arzneimittels zur Prophylaxe und/oder Behandlung von Schmerzen, vorzugsweise zur Behandlung und/oder Prophylaxe von akuten Schmerzen, chronischen Schmerzen, neuropathischen Schmerzen und/oder Clusterkopfschmerzen.

Besonders bevorzugt ist die Verwendung wenigstens einer substituierten 1,4,8-Triazaspiro[4.5]decan-2-on-Verbindung der vorstehend angegebenen allgemeinen Formel I, ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, und ggf. wenigstens eines pharmazeutisch verträgliche Hilfsstoffes zur Herstellung eines Arzneimittels zur kombinierten Prophylaxe und/oder Behandlung von Depressionen und Schmerzen, vorzugsweise zur kombinierten Behandlung von Depressionen und Schmerzen ausgewählt aus der Gruppe bestehend aus akuten Schmerzen, chronischen Schmerzen, neuropathischen Schmerzen und Clusterkopfschmerzen, besonders bevorzugt zur kombinierten Behandlung von Depressionen und chronischen Schmerzen.

Weiterhin bevorzugt ist die Verwendung wenigstens einer substituierten 1,4,8-Triazaspiro[4.5]decan-2-on-Verbindung der vorstehend angegebenen allgemeinen Formel I, ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, und ggf. wenigstens eines pharmazeutisch verträgliche Hilfsstoffes zur Herstellung eines Arzneimittels zur Prophylaxe und/oder Behandlung von Akohol- und/oder Drogen- und/oder Medikamentenmißbrauch, zur Prophylaxe und/oder Behandlung von Akohol- und/oder Drogen- und/oder Medikamentenabhängigkeit, zur Prophylaxe und/oder Behandlung von Entzündungen, zur Prophylaxe und/oder Behandlung von Antriebslosigkeit, zur Prophylaxe und/oder Behandlung von Störungen der Nahrungsmittelaufnahme, vorzugsweise ausgewählt aus der Gruppe bestehend aus Bulimie, Anorexie, Fettsucht und Kachexie, zur Prophylaxe und/oder Behandlung von Katalepsie, zur Vigilanzsteigerung, zur Libidosteigerung, zur Anxiolyse, zur Prophylaxe und/oder Behandlung von neurodegenerativen Erkrankungen, vorzugsweise einer oder mehrerer neurodegenerativer Erkrankungen ausgewählt aus der Gruppe bestehend aus Morbus Parkinson, Morbus Huntington, Morbus Alzheimer und Multipler Sklerose, zur Prophylaxe und/oder Behandlung von Ischämie und/oder zur Lokalanästhesie.

Weiterhin bevorzugt ist die Verwendung wenigstens einer substituierten 1,4,8-Triazaspiro[4.5]decan-2-on-Verbindung der vorstehend angegebenen allgemeinen Formel I, ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, und ggf. wenigstens eines pharmazeutisch verträgliche Hilfsstoffes zur Herstellung eines Arzneimittels zur Prophylaxe und/oder Behandlung einer oder mehrerer Erkrankungen ausgewählt aus der Gruppe bestehend aus nicht-akuten allergischen Erkrankungen, vorzugsweise allergischer Dermatitis; Asthma; Rhinitis; Konjunktivitis; Erkrankungen die durch 2-arachidonglycerol und/oder entsprechender Ether wie Hematol vermittelt werden; Septikämie; Krebs, insbesondere Blutkrebs und/oder Hirntumore; Kreislaufstörungen; grünem Star; Entzündungen, vorzugsweise immunologisch vermittelten Entzündungserkrankungen, besonders bevorzugt rheumatoide Arthritis, Lupus erythematodes, Psoriasis und Thyroiditis; Diabetes; Blutvergiftung; Epilepsie; Tourettes Syndrom; Osteoporose; Morbus Bechterew; Gicht; gichtartiger Arthritis; Osteoarthritis; Durchblutungsstörungen; Ischämien, vorzugsweise Nierenischämie und renale Ischämie, und/oder zur Regulation des Immunsystems, vorzugsweise zur Unterdrückung des Immunsystems.

Das erfindungsgemäße Arzneimittel eignet sich zur Verabreichung an Erwachsene und Kinder einschließlich Kleinkindern und Säuglingen.

Das erfindungsgemäße Arzneimittel kann als flüssige; halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben einer oder mehreren der in dem erfindungsgemäßen Arzneimittel zum Einsatz kommenden substituierten 1,4,8-Triazaspiro[4.5]decan-2-on-Verbindungen der vorstehenden allgemeinen Formel I, ggf. in Form ihres reinen Stereoisomeren, insbesondere Enantiomeren oder Diastereomeren, ihres Racemates oder in Form von Mischungen der Stereoisomeren, insbesondere der Enantiomeren oder Diastereomeren, in einem beliebigen Mischungsverhältnis, oder ggf. in Form eines entsprechenden Salzes oder jeweils in Form eines entsprechendes Solvates, enthält das erfindungsgemäße Arzneimittel üblicherweise weitere physiologisch verträgliche pharmazeutische Hilfsstoffe, die bevorzugt ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Die in dem erfindungsgemäßen Arzneimittel zum Einsatz kommenden substituierten 1,4,8-Triazaspiro[4.5]decan-2-on-Verbindungen in einem Depot in gelöster Form oder in einem Pflaster, gegebenenfalls unter Zusatz von die Hautpenetration fördernden Mitteln, sind geeignete perkutane Applikationszubereitungen.

Oral oder perkutan anwendbare Zubereitungsformen können die jeweiligen substituierten 1,4,8-Triazaspiro[4.5]decan-2-on-Verbindungen auch verzögert freisetzen.

Die Herstellung der erfindungsgemäßen Arzneimittel erfolgt mittels üblichen, aus dem Stande der Technik wohl bekannten Mitteln, Vorrichtungen, Methoden und Verfahren, wie sie beispielsweise in "Remington's Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind. Die entsprechende Beschreibung wird hiermit als Referenz eingeführt und gilt als Teil der Offenbarung.

Die an den Patienten zu verabreichende Menge der jeweiligen substituierten 1,4,8-Triazaspiro[4.5]decan-2-on-Verbindung kann variieren und ist beispielsweise abhängig vom Gewicht oder Alter des Patienten sowie von der Applikationsart, der Indikation und dem Schweregrad der Erkrankung. Üblicherweise werden 0,005 bis 500 mg/kg, vorzugsweise 0,05 bis 50 mg/kg Körpergewicht des Patienten wenigstens einer erfindungsgemäßen substituierten 1,4,8-Triazaspiro[4.5]decan-2-on-Verbindung appliziert.

### Pharmakologische Methoden:

### I. Methode zur Bestimmung der Noradrenalin- und der 5HT-Uptake-Inhiblerung:

Für in vitro Studien werden Synaptosomen aus Rattenhimarealen frisch isoliert, wie in der Veröffentlichung "The isolation of nerve endings from brain" von E.G. Gray und V.P. Whittaker, J. Anatomy 96, Seiten 79-88, 1962, beschrieben. Die entsprechende Literaturbeschreibung wird hiermit als Referenz eingeführt und gilt als Teil der Offenbarung.

Das Gewebe (Hypothalamus für die Bestimmung der Noradrenalin-Uptake-Inhibierung und Mark und Pons für die Bestimmung der 5HT-Uptake-Inhibierung) wird in eisgekühlter 0,32 M Sucrose (100 mg Gewebe / 1mL) in einem Glas-Homogenisierer mit Teflonstößel homogenisiert, indem fünf volle Auf und Abschläge bei 840 Umdrehungen /Minute benutzt werden.

Das Homogenat wird bei 4°C für 10 Minuten bei 1000 g zentrifugiert. Nach anschließender Zentrifugierung bei 17000 g für 55 Minuten erhält man die Synaptosomen (P₂-Fraktion), die in 0,32 M Glucose (0,5 mU 100 mg des ursprünglichen Gewichts) noch einmal suspendiert werden.

Der jeweilige Uptake wird in einer 96-well Mikrotiterplatte gemessen. Das Volumen beträgt 250 µl und die Inkubation erfolgt bei Raumtemperatur (ca. 20-25 °C) unter O₂ Atmosphäre.

Die Inkubationszeit beträgt 7,5 Minuten für [³H]-NA und 5 Minuten für [³H]-5-HT. Anschließend werden die 96 Proben durch eine Unifilter GF/B^{®} Mikrotiterplatte (Packard) filtriert und mit 200 mL inkubierten Puffer mit Hilfe eines "Brabdel Cell-Harvester MPXRI-96T" gewaschen. Die Unifilter GF/B Platte wird bei 55°C 1 Stunde getrocknet. Im Anschluß wird die Platte mit einem Back seal^{®} (Packard) verschlossen und mit 35 µl Szintilationsflüssigkeit pro Well (Ultima Gold^{®}, Packard) versetzt. Nach dem Verschließen mit einem top seal^{®} (Packard) wird, nach der Einstellung des Gleichgewichts (etwa 5 Stunden), die Radioaktivität in einem "Trilux 1450 Microbeta" (Wallac, Freiburg, Deutschland) bestimmt.

Die Menge des bei der vorstehenden Bestimmung eingesetzten Proteins entspricht den aus der Literatur bekannten Werten, wie z.B. in "Protein measurement with the folin phenol reagent", Lowry et al., J. Biol. Chem., 193,265-275, 1951 beschrieben.

Eine detaillierte Methodenbeschreibung kann ferner auch der Literatur, beispielsweise aus M.Ch. Frink, H.-H. Hennies, W. Engelberger, M. Haurand und B. Wilffert (1996) Arzneim.-Forsch./Drug Res. 46 (III), 11, 1029-1036, entnommen werden.

Die entsprechenden Literaturbeschreibungen werden hiermit jeweils als Referenz eingeführt und gelten als Teil der vorliegenden Offenbarung.

Folgende Kenndaten wurden für den NA- bzw. 5-HT-Transporter ermittelt:
NA-Uptake : Km = 0,32 ± 0,11 µM
5HT-Uptake : Km = 0,084 ± 0,011 µM

### II. Methode zur Bestimmung der Affinität zur Batrachotoxin-(BTX)-Bindungsstelle des Natriumkanals:

Die Bindungsstelle 2 des Natriumkanals ist die sogenannte Batrachotoxin-(BTX) Bindungsstelle. Als Ligand wird [³H]-Batrachotoxin A20 α-Benzoat (10 nM im Ansatz) eingesetzt. Die lonenkanal-Partikel (Synaptosomen) werden aus dem Ratten Cerebrocortex angereichert, wie in der Veröffentlichung von Gray und Whittaker, 1962, J. Anat. 76, 79-88 beschrieben. Die entsprechende Beschreibung wird hiermit als Referenz eingeführt und gilt als Teil der vorliegenden Offenbarung. Als unspezifische Bindung ist die Radioaktivität definiert, die in Gegenwart von Veratridin (3 x 10⁻⁴ M im Ansatz) gemessen wird.

Die Assaybedingungen werden entsprechend der Veröffentlichung von Pauwels, Leysen und Laduron durchgeführt, wie in Eur. J. Pharmacol. 124, 291-298 beschrieben. Die entsprechende Beschreibung wird hiermit als Referenz eingeführt und gilt als Teil der vorliegenden Offenbarung.

Abweichend von dieser Vorschrift wird der Gesamtansatz auf 250 µl verkleinert, so daß der Assay auf 96-well Mikrotiterplatten durchgeführt werden kann. Die Inkubationszeit in diesen Mikrotiterplatten beträgt zwei Stunden bei Raumtemperatur (ca. 20-25°C).

Folgende Kenndaten wurden für den K_{D}-Wert der Bindungsstelle ermittelt:
K_{D}: 24,63 ±1,56 nM.

### III. Bestimmung der Affinität zum Cannabinoidrezeptor CB2 (CB2-Rezeptor):

Zur Bestimmung der Affinität der erfindungsgemäßen Verbindungen zum Cannabinoidrezeptor werden Membranen aus humanen rekombinanten HEK-293EBNA-Zellen verwendet, die stabil mit dem menschlichen CB2-Rezeptor transfiziert wurden. Als Radioligand wurde mit Tritium markiertes 5-(1,1-Dimethylheptyl)-2-(5-hydroxypropyl)cyclohexyl)-1-alpha,2-beta,5-alpha)-phenol (^{[3]}H-CP 55,940 mit 103,4 Ci/mmol, 1 m Ci/ml) verwendet. Die Bestimmung erfolgte in einem Testpuffer aus 50 mM Tris-HCI, 2.5mM EDTA, 5mM MgCl₂ und 1.0 mg/ml Fettsäure-freiem BSA. Die Prüfsubstanzen wurden jeweils in DMSO gelöst.

Die Affinität der erfindungsgemäßen Verbindungen zum CB2-Rezeptor wird durch deren Fähigkeit, ^{[3]}H-CP 55,940 aus CB2-Rezeptoren in Membranen aus HEK-293EBNA-Zellen zu verdrängen, bestimmt. Dazu werden in Vertiefungen auf einer Mikrotiterplatte jeweils 8 µg der Membranen (20 µl einer Lösung aus Membranen in einer Konzentration von 400 µg/ml) mit einer 0.33 nM Lösung von ^{[3]}H-CP 55,949 (120 Ci/mmol) in einem Gesamtvolumen aus Testpuffer von 200 µl für 90 Minuten bei 30°C inkubiert.

Anschließend werden entweder die Prüfsubstanzen bzw. WIN 55212-2 zur Bestimmung der nicht-spezifischen Bindung jeweils gelöst in DMSO in die Vertiefungen gegeben, so dass jeweils eine Konzentration der entsprechenden Substanzen von 10 µM erhalten wird.

Es wird für weitere 40 Minuten bei 30°C inkubiert. Die Bindungs-Reaktion wurde durch rasche Filtration über GF/C-Filterpapier, das mit 0,05% PEI behandelt worden ist, unter Verwendung eines Brandel-Zell-Harvesters mit 96 Vertiefungen beendet. Die Filter werden neun Mal mit 0.5 ml eiskaltem Wasch-Puffer (50 nM Tris-HCI, 5 mM MgCl₂, 2.5mM EDTA, 2 % BSA, pH 7.4) gewaschen, luftgetrocknet, in Szintillationsflüssigkeit gesetzt, und die Radioaktivität mit Hilfe eines Szintillationszählers bestimmt.

Die prozentuale Verdrängung des radioaktiven Liganden ^{[3]}H-CP 55,940 aus seiner Bindung zum CB2-Rezeptor wird als Prozent Hemmung der spezifischen Bindung angegeben. Zur Bestimmung von Kᵢ, werden ausgehend von der prozentualen Verdrängung durch die zu prüfenden Verbindungen der allgemeinen Formel I bei verschiedenen Konzentrationen IC₅₀ Hemmkonzentrationen berechnet, die eine 50-prozentige Verdrängung des radioaktiven Liganden bewirken. Durch Umrechnung mittels der Cheng-Prusoff-Beziehung gemäß Biochem. Pharmacol. 1973, 22, 3099 werden Kᵢ-Werte für die Prüfsubstanzen erhalten.

Im folgenden wird die Erfindung anhand von Beispielen erläutert. Diese Erläuterungen sind lediglich beispielhaft und schränken den allgemeinen Erfindungsgedanken nicht ein.

### Beispiele:

Die Ausbeuten der hergestellten Verbindungen wurden nicht optimiert.

Alle Temperaturen sind unkorrigiert.

Die Angabe "EE" bedeutet Ethylacetat, "EtOH" Ethanol und die Angabe "THF" steht für Tetrahydrofuran. Die Angabe "Äquivalente" bedeutet Stoffmengenäquivalente, "RT" Raumtemperatur , "min" Minuten, "h" Stunden, "d" Tage, "Vol.%" Volumenprozent und "M" ist eine Konzentrationsangabe in mol/l.

Die eingesetzten Chemikalien und Lösungsmittel wurden kommerziell bei den herkömmlichen Anbietern (Acros, Acocado, Aldrich, Bachem, Fluka, Lancaster, Maybridge, Merck, Sigma, TCi etc.) erworben oder nach üblichen, dem Fachmann bekannten Methoden synthetisiert.

Als stationäre Phase für die Säulenchromathographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Laufmitteln für chromatographische Untersuchungen sind stets in Volumen/Volumen angegeben.

Die Analytik erfolgte über HPLC-MS, Vorstufen wurden über NMR-Spektroskopie bestätigt.

### Allgemeine Vorschrift zur Herstellung erfindungsgemäßer substituierter 1,4,8-Triazaspiro[4.5]decan-2-on-Verbindungen:

### Stufe 1:

### Allgemeine Vorschrift zur Herstellung von ggf. in 3-Position substituierten 2-Oxo-1,4,8-triaza-spiro[4.5]decan-8-carbonsäure tert-butylesterverbindungen:

Zu der Lösung von einem Äquivalent des jeweiligen Aminosäureamids (d.h. der jeweiligen Verbindung der allgemeinen Formel III), ggf. in Form eines entsprechenden Salzes, in einem trockenem Lösungsmittel wie beispielsweise Ethanol wurden ein Äquivalent N-tert-Butyloxycarbonyl-piperidon und ein Äquivalent Triethylamin gegeben und die so erhaltene Reaktionsmischung für 1 bis 5 h unter Rückfluß erhitzt. Das Lösungsmittel wurde ab destilliert und die so erhaltene, ggf. in 3-Position substituierte 2-Oxo-1,4,8-triaza-spiro[4.5]decan-8-carbonsäure tert-butylester-Verbindung (Verbindung der allgemeinen Formel IV, worin P für eine Carbonsäure-tert-butylester-(BOC)-Schutzgruppe steht) wurde üblicherweise ohne weitere Aufarbeitung in den nachfolgenden Umsetzungen verwendet.

Sofern erforderlich wurde die jeweilige ggf. in 3-Position substituierte 3-Oxo-1,4,8-triaza-spiro[4.5]decan-8-carbonsäure-tert-butylester-Verbindung wie folgt gereinigt:

Nach Zugabe von Wasser und einem geeigneten organischen Lösungsmittel wie beispielsweise CH₂Cl₂ wurde die organische Phase abgetrennt und die wäßrige Phase mit CH₂Cl₂ extrahiert. Die vereinigten organischen Phasen wurden über Na₂SO₄ getrocknet. Nach Filtration wurde das Lösungsmittel ab destilliert. Man erhielt die entsprechende ggf. in 3-Position substituierte 2-Oxo-1,4,8-triazaspiro[4.5]decan-8-carbonsäure tert-butylester-Verbindung.

### Stufe 2:

### Allgemeine Vorschrift zur Herstellung von in 1-Position und ggf. in 3-Position substituierten 2-Oxo-1,4,8-triaza-spiro[4.5]decan-8-carbonsäure tert-butylester-Verbindungen:

Zu der Suspension bzw. Lösung von 1 bis 2 Äquivalenten einer geeigneten Base wie beispielsweise NaH in einem geeigneten Lösungsmittel wie beispielsweise THF wurde bei einer Temperatur von 0°C bis RT die Lösung von 1 Äquivalent der jeweiligen ggf. substituierten 2-Oxo-1,4,8-triaza-spiro[4.5]decan-8-carbonsäure-tert-butylester-Verbindung gemäß Stufe 1 in einem geeigneten organischen Lösungsmittel wie beispielsweise THF getropft.

Alternativ wurde zu einer Lösung der jeweiligen ggf. substituierten 2-Oxo-1,4,8-triaza-spiro[4.5]decan-8-carbonsäure-tert-butylester-Verbindung gemäß Stufe 1 in einem geeigneten organischen Lösungsmittel wie beispielsweise THF bei einer Temperatur von 0°C bis RT die Suspension bzw. Lösung von 1 bis 2 Äquivalenten einer geeigneten Base wie beispielsweise NaH in einem geeigneten Lösungsmittel wie beispielsweise THF getropft.

Nach Rühren für 1 h bei 0°C wurden 1 bis 1,3 Äquivalente der jeweiligen Chlorid-Verbindung (d.h. einer Verbindung der allgemeinen Formel R¹-X¹, worin X¹ für einen CI-Rest steht) zu getropft, das so erhaltene Reaktionsgemisch auf RT erwärmen lassen und anschließend für 2 Stunden bis 3 Tage unter Rückfluß erhitzt. Sofern erforderlich, wurden weitere 0,5 bis 1 Äquivalente der jeweiligen Chlorid-Verbindung zugegeben und die Reaktionsmischung für weitere 10-25 h unter Rückfluß erhitzt.

Anschließend wurde die Reaktionsmischung mit wäßriger gesättigter NH₄Cl-Lösung versetzt, die organische Phase abgetrennt und die wässerige Phase mit EE extrahiert. Die vereinigte organische Phase wurde über Na₂SO₄ getrocknet, filtriert und das Lösungsmittel ab destilliert. Die Aufarbeitung erfolgte über Säulenchromathographie und ergab die jeweilige, in 1-Position und ggf. in 3-Position substituierte 2-Oxo-1,4,8-triaza-spiro[4.5]decan-8-carbonsäure tert-butylester-Verbindung (d.h. eine Verbindung der allgemeinen Formel V, worin P für eine BOC-Schutzgruppe steht).

### Stufe 3:

### Allgemeine Vorschrift zur Herstellung von ggf. in 1-Position und/oder in 3-Position substituierten 2-Oxo-1,4,8-triaza-spiro[4.5]decan-2-on-Verbindungen

Zu der Lösung von 1 Äquivalent der jeweiligen ggf. in 1-Position und/oder in 3-Position substituierten 2-Oxo-1,4,8-triaza-spiro[4.5]decan-8-carbonsäure tert-butylester-Verbindung in CH₂Cl₂ wurden 10 bis 25 Äquivalente Trifluoressigsäure bei 0°C zu getropft und 15 min bei dieser Temperatur gerührt. Nach Erwärmen auf RT wurde die Reaktionslösung für weitere 2.5 h gerührt. Anschließend wurde die Reaktionslösung mit wäßriger, gesättigter NaHCO₃-Lösung auf einen pH-Wert 7-8 eingestellt, die organische Phase abgetrennt und die wäßrige Phase mit CH₂Cl₂ (2 x 30 ml) extrahiert. Die vereinigte organische Phase wurde über Na₂SO₄ getrocknet, filtriert und das Lösungsmittel ab destilliert. Die Aufarbeitung erfolgte über Säulenchromathographie und ergab die jeweilige am N(8)-Stickstoff ungeschützte ggf. in 1-Position und/oder in 3-Position substituierte 2-Oxo-1,4,8-triazaspiro[4.5]decan-2-on-Verbindung (d.h. eine Verbindung der allgemeinen Formel VI).

### Stufe 4a:

### Allgemeine Vorschrift zur Herstellung von erfindungsgemäßen sulfonylierten 2-Oxo-1,4,8-Triazaspiro[4.5]decan-2-on-Verbindungen (Verbindungen der allgemeinen Formel I, in denen R³ für eine -S(=O)₂-R⁴-Gruppe steht):

Zu einer Lösung von einer ggf. in 1-Position und/oder in 3-Position substituierten 2-Oxo-1,4,8-triaza-spiro[4.5]decan-2-on-Verbindung in einem trockenen organischen Lösungsmittel wie beispielsweise CH₂Cl₂ und in Gegenwart einer organischen oder anorganischen Base wie beispielsweise Natriumhydrogencarbonat, Diisopropylethylamin, Triethylamin oder Diethylamin wurden bei einer Temperatur von 0°C bis RT 1-5 Äquivalente des jeweiligen Sulfonylchlorids (d.h. einer Verbindung der allgemeinen Formel R⁴-SO₂-X², worin X² für einen Cl-Rest steht), unverdünnt oder gelöst in einem organischen Lösungsmittel wie beispielsweise CH₂Cl₂ gegeben und bei einer Temperatur von 0°C, RT oder unter Rückfluß für 1-24h gerührt. Die so erhaltene Reaktionslösung wurde-durch Zugabe einer anorganischen Base in wässeriger Lösung beispielsweise einer wässrigen gesättigten KOH-Lösung, NaHCO₃-Lösung oder Natronlauge alkalisch gestellt, die organische Phase abgetrennt und die wässerige Phase mehrmals mit einem geeigneten Lösungsmittel wie beispielsweise CH₂Cl₂ extrahiert. Die vereinigten organischen Phasen wurden mit einem Trockenmittel wie beispielsweise Na₂SO₄ getrocknet. Nach Abdestillation des Lösungsmittels erfolgte die weitere Aufreinigung über Säulenchromathographie oder präparative Chromatographie und ergab die jeweilige sulfonylierte 2-Oxo-1,4,8-Triazaspiro[4.5]decan-2-on-Verbindung.

### Stufe 4b:

### Allgemeine Vorschrift zur Herstellung von erfindungsgemäßen 2-Oxo-1,4,8-Triazaspiro[4.5]decan-2-on-Verbindungen der allgemeinen Formel I, in denen R³ für eine -C(=S)-NH-R⁵-Gruppe oder für eine -C(=O)-NH-R⁶-Gruppe steht:

Zu der Lösung einer entsprechenden, ggf. in 1-Position und/oder in 3-Position substituierten 2-Oxo-1,4,8-triaza-spiro[4.5]decan-2-on-Verbindung in einem trockenen organischen Lösungsmittel wie beispielsweise CH₂Cl₂ oder Toluol wurden bei einer Temperatur zwischen 0°C und RT 1-5 Äquivalente der jeweiligen Isothiocyanat-Verbindung der allgemeinen Formel R⁵-NCS oder der jeweiligen Isocyanat-Verbindung der allgemeinen Formel R⁶-NCO, unverdünnt oder gelöst in einem organischen Lösungsmittel wie beispielsweise CH₂Cl₂ oder Toluol, gegeben und bei einer Temperatur von 0°C, RT oder unter Rückfluß für 1-24h gerührt. Die so erhaltene Reaktionslösung wurde mit wäßriger gesättigter NaCl-Lösung versetzt, die organische Phase abgetrennt und die wässerige Phase mehrmals mit einem geeigneten Lösungsmittel wie beispielsweise CH₂Cl₂ extrahiert oder die Reaktionslösung wird durch Zugabe beispielsweise einer wässerigen gesättigten NaHCO₃-Lösung alkalisch gestellt, die organische Phase abgetrennt und die wässerige Phase mehrmals mit einem geeigneten Lösungsmittel wie beispielsweise CH₂Cl₂ extrahiert. Die vereinigten organischen Phasen werden mit einem Trockenmittel wie beispielsweise Na₂SO₄ getrocknet. Nach Abdestillation des Lösungsmittels erfolgte die weitere Aufreinigung über Säulenchromathographie oder präparative Chromatographie und ergab die jeweilige 2-Oxo-1,4,8-Triazaspiro[4.5]decan-2-on-Verbindung der allgemeinen Formel **I,** worin R³ für eine - C(=S)-NH-R⁵-Gruppe oder für eine -C(=O)-NH-R⁶-Gruppe steht.

Im folgenden werden die vorstehend beschriebenen allgemeinen Synthesevorschriften der Stufen 1-3 anhand von detaillierten Beispielen weiter verdeutlicht:

### Stufe 1:

### Herstellung von 3-Benzyl-2-oxo-1,4,8-triaza.spiro[4.5]decan-8-carbonsäure tert-butylester

Zu der Lösung von Phenylalaninamid (4.9g, 29.8 mmol) in trockenem EtOH (20 ml) gab man N-tert-Butyloxycarbonyl-piperidon (6.0 g, 29.8 mmol) und erhitzte für 2.5 h unter Rückfluß. Das Lösungsmittel wurde ab destilliert und der 3-Benzyl-2-oxo-1,4,8-triaza-spiro[4.5]decan-8-carbonsäure tert-butylester (10.8 g) wurde ohne weitere Aufarbeitung in der nachfolgenden Umsetzung verwendet.

### Stufe 2:

### Herstellung von 1,3-Dibenzyl-2-oxo-1,4,8-triaza-spiro[4.5]decan-8-carbonsäure tert-butylester

Zu der Lösung von 3-Benzyl-2-oxo-1,4,8-triaza-spiro[4.5]decan-8-carbonsäure tert-butylester (12.1 g, 35 mmol) in THF (250 ml) gab man bei RT NaH (1,26 g, 52,5 mmol) hinzu. Nach Rühren für 1 h bei 0°C wurde Benzylchlorid (4.9 g, 38.5 mmol) zu getropft, ließ auf RT erwärmen und erhitzte für 60 h unter Rückfluß. Anschließend wurde erneut Benzylchlorid (2.4 g, 19 mmol) zugegeben und für weitere 16 h unter Rückfluß erhitzt. Die Reaktionsmischung wurde mit wässeriger gesättigter NH₄Cl-Lösung (20 ml) versetzt und mit EE (3 x 50 ml) extrahiert. Die vereinigte organische Phase wurde über Na₂SO₄ getrocknet, filtriert und das Lösungsmittel ab destilliert. Die Aufarbeitung erfolgte über Säulenchromathographie und ergab 1,3-Dibenzyl-2-oxo-1,4,8-triaza-spiro[4.5]decan-8-carbonsäure tert-butylester mit einer Ausbeute von 13.5 g (88 % der Theorie).

### Stufe 3:

### Herstellung von 1,3-Dibenzyl-1,4,8-triaza-spiro[4.5]decan-2-on

Zu einer Lösung von 1,3-Dibenzyl-2-oxo-1,4,8-triaza-spiro[4.5]decan-8-carbonsäure tert-butylester (13.5 g, 31.0 mmol) in CH₂Cl₂ (93 ml) wurde Trifluoressigsäure (68.5 g, 601 mmol) bei 0°C zu getropft und 15 min bei dieser Temperatur gerührt. Nach Erwärmen auf RT wurde die Reaktionslösung für weitere 2.5 h gerührt. Anschließend wurde die Reaktionslösung mit wässeriger gesättigter NaHCO₃-Lösung auf den pH-Wert 7-8 eingestellt, die organische Phase abgetrennt und die wässerige Phase mit CH₂Cl₂ (2 x 30 ml) extrahiert. Die vereinigte organische Phase wurde über Na₂SO₄ getrocknet, filtriert und das Lösungsmittel ab destilliert. Die Aufarbeitung erfolgte über Säulenchromathographie und ergab 1,3-Dibenzyl-1,4,8-triaza-spiro[4.5]decan-2-on mit einer Ausbeute von 7.8 g (75 % der Theorie).

### Beispiel 246:

### Herstellung von 1,3-Dibenzyl-8-methansulfonyl-1,4,8-triaza-spiro[4.5]decan-2-on

Zu einer Lösung von 1,3-Dibenzyl-1,4,8-triaza-spiro[4.5]decan-2-on (300 mg, 0.89 mmol) in CH₂Cl₂ (8.1 ml) wurde unter Rühren mit Stickstoff als Inertgas bei RT Triethylamin (0.15 ml, 1.78 mmol) und anschließend Methansulfonylchlorid (0.14 ml, 1.78 mmol) gegeben. Die Reaktionsmischung wurde. über Nacht gerührt. Die organische Phase wurde mit wäßriger gesättigter Natriumcarbonatlösung (20 ml) alkalisch gestellt und die wäßrige Phase mit CH₂Cl₂ (15 ml) extrahiert. Die vereinigten organischen Phasen wurden über Na₂SO₄ getrocknet. Nach Filtration, Entfernen des Lösungsmittels und Säulenchromatographie wurde das gewünschte Produkt 1,3-Dibenzyl-8-methansulfonyl-1,4,8-triaza-spiro[4.5]decan-2-on mit einer Ausbeute von 287 mg (77% der Theorie) erhalten.

### Beispiel 247:

### Herstellung von 8-Benzolsulfonyl-1,3-dibenzyl-1,4,8-triaza-spiro[4.5]decan-2-on

Zu einer Lösung von 1,3-Dibenzyl-1,4,8-triaza-spiro[4.5]decan-2-on (250 mg, 0.75 mmol) in CH₂Cl₂ (6.7 ml) wurde unter Rühren in einer Stickstoffatmosphäre bei RT Triethylamin (0.12 ml, 0.89 mmol) und anschließend Benzolsulfonylchlorid (0.19 ml, 0.26 mmol) gegeben. Die Reaktionsmischung wurde über Nacht gerührt. Die organische Phase wurde mit wäßriger gesättigter Natriumcarbonatlösung (20 ml) alkalisch gestellt und die wäßrige Phase mit CH₂Cl₂ (15 ml) extrahiert. Die vereinigten organischen Phasen wurden über Na₂SO₄ getrocknet. Nach Filtration, Entfernen des Lösungsmittels und Säulenchromatographie wurde das gewünschte Produkt 8-Benzolsulfonyl-1,3-dibenzyl-1,4,8-triaza-spiro[4.5]decan-2-on mit einer Ausbeute von 259 mg (73% der Theorie) erhalten.

### Beispiel 248:

### Herstellung von 1,3-Dibenzyl-8-(4-chlor-benzolsulfonyl)-1,4,8-triaza-spiro[4.5]decan-2-on

Zu einer Lösung von 1,3-Dibenzyl-1,4,8-triaza-spiro[4.5]decan-2-on (249 mg, 0.75 mmol) in CH₂Cl₂ (6.7 ml) wurde unter Rühren in einer Stickstoffatmosphäre bei RT Triethylamin (0.12 ml, 0.89 mmol) und anschließend 4-Chlorbenzolsulfonylchlorid (314 mg, 1.49 mmol) gegeben. Die Reaktionsmischung wurde über Nacht gerührt. Die organische Phase wurde mit wäßriger gesättigter Natriumcarbonatlösung (20 ml) alkalisch gestellt und die wäßrige Phase mit CH₂Cl₂ (15 ml) extrahiert. Die vereinigten organischen Phasen wurden über Na₂SO₄ getrocknet. Nach Filtration, Entfernen des Lösungsmittels und Säulenchromatographie wurde das gewünschte Produkt 1,3-Dibenzyl-8-(4-chlor benzolsulfonyl)-1,4,8-triaza-spiro[4.5]decan-2-on mit einer Ausbeute von 382 mg (100% der Theorie) erhalten.

### beispiel 249:

### Herstellung von 1,3-Dibenzyl-8-(4-methoxy-benzolsulfonyl)-1,4,8-triaza-spiro[4.5]decan-2-on

Zu einer Lösung von 1,3-Dibenzyl-1,4,8-triaza-spiro[4.5]decan-2-on (249 mg, 0.75 mmol) in CH₂Cl₂ (6.7 ml) wurde unter Rühren in einer Stickstoffatmosphäre bei RT Triethylamin (0.12 ml, 0.89 mmol) und anschließend 4-Methoxybenzolsulfonylchlorid (308 mg, 1.49 mmol) gegeben. Die Reaktionsmischung wurde über Nacht gerührt. Die organische Phase wurde mit wäßriger gesättigter Natriumcarbonatlösung (20 ml) alkalisch gestellt und die wäßrige Phase mit CH₂Cl₂ (15 ml) extrahiert. Die vereinigten organischen Phasen wurden über Na₂SO₄ getrocknet. Nach Filtration, Entfernen des Lösungsmittels und Säulenchromatographie wurde das gewünschte Produkt 1,3-Dibenzyl-8-(4-methoxy-benzolsulfonyl)-1,4,8-triaza-spiro[4.5]decan-2-on mit einer Ausbeute von 301 mg (79% der Theorie) erhalten.

### Beispiel 250:

### Herstellung von 1,3-Dibenzyl-2-oxo-1,4,8-triaza.spiro[4.5]decan-8-carbonsäurephenylamid

Zu einer Lösung von 1,3-Dibenzyl-1,4,8-triaza-spiro[4.5]decan-2-on (502 mg, 1.49 mmol) in Toluol (12.4 ml) wurde unter Rühren in einer Stickstoffatmosphäre bei RT Phenylisocyanat (0.16 ml, 1.49 mmol) gegeben. Die Reaktionsmischung wurde über Nacht gerührt. Nach Entfernen des Lösungsmittels wurde mittels präparative HPLC das gewünschte Produkt 1,3-Dibenzyl-2-oxo-1,4,8-triaza-spiro[4.5]decan-8-carbonsäurephenylamid erhalten.

### Beispiel 251:

### Herstellung von 1,3-Dibenzyl-2-oxo-1,4,8-triaza-spiro[4.5]decan-8-carbonsäure-(4-methoxy-phenyl)-amid

Zu einer Lösung von 1,3-Dibenzyl-1,4,8-triaza-spiro[4.5]decan-2-on (499 mg, 1.49 mmol) in Toluol (12.3 ml) wurde unter Rühren in einer Stickstoffatmosphäre bei RT 4-Methoxyphenylisocyanat (221 mg, 1.49 mmol) gegeben. Die Reaktionsmischung wurde 4 h bei 115°C gerührt. Nach Entfernen des Lösungsmittels wurde mittels präparativer HPLC das gewünschte Produkt 11,3-Dibenzyl-2-oxo-1,4,8-triazaspiro[4.5]decan-8-carbonsäure-(4-methoxy-phenyl)-amid erhalten.

### Beispiel 252:

### Herstellung von 1,3-Dibenzyl-2-oxo-1,4,8-triaza-spiro[4.5]decan-8-thiocarbonsäurephenylamid

Zu einer Lösung von 1,3-Dibenzyl-1,4,8-triaza-spiro[4.5]decan-2-on (505 mg, 1.50 mmol) in Toluol (14.3 ml) wurde unter Rühren in einer Stickstoffatmosphäre bei RT Phenylisothiocyanat (203 mg, 1.50 mmol) gegeben und die Reaktionsmischung 8 h bei 50°C gerührt. Nach Entfernen des Lösungsmittels wurde mittels präparativer HPLC das gewünschte Produkt 1,3-Dibenzyl-2-oxo-1,4,8-triaza-spiro[4.5]decan-8-thiocarbonsäurephenylamid.

### Beispiel 253:

### Herstellung von 1,3-Dibenzyl-2-oxo-1,4,8-triaza-spiro[4.5]decan-8-thiocarbonsäure-(2,4-difluor-phenyl)-amid

Zu einer Lösung von 1,3-Dibenzyl-1,4,8-triaza-spiro[4.5]decan-2-on (503 mg, 1.50 mmol) in Toluol (14.3 ml) wurde unter Rühren in einer Stickstoffatmosphäre bei RT 2,4-Difluorhenylisothiocyanat (256 mg, 1.50 mmol) gegeben und die Reaktionsmischung 8 h bei 50°C gerührt. Nach Entfernen des Lösungsmittels wurde mittels präparativer HPLC das gewünschte Produkt 1,3-Dibenzyl-2-oxo-1,4,8-triazaspiro[4.5]decan-8-thiocarbonsäure-(2,4-difluor-phenyl)-amid erhalten.

### Stufe 1:

### Herstellung von 3-(2-methylsulfanyl-ethyl)-2-oxo-1,4,8-triaza-spiro[4.5]decan-8-carbonsäure tert-butylester

Zu der Lösung von Methioninamid Hydrochlorid (5.5 g, 29.8 mmol) in trockenem EtOH (66 ml) gab man N-tert-Butyloxycarbonyl-piperidon (5.9 g, 29.8 mmol), Triethylamin (3.02 g, 29.8 mmol) und erhitzte für 2.5 h unter Rückfluß. Nach Zugabe von Wasser (50 ml) und CH₂Cl₂ (200 ml) wurde die organische Phase abgetrennt und die wässerige Phase mit CH₂Cl₂ (3 x 20 ml) extrahiert. Die vereinigten organischen Phasen wurden über Na₂SO₄ getrocknet. Nach Filtration wurde das Lösungsmittel ab destilliert. Man erhielt 3-(2-methylsulfanyl-ethyl)-2-oxo-1,4,8-triazaspiro[4.5]decan-8-carbonsäure tert-butylester mit einer Ausbeute von 8.0 g (82 % der Theorie).

### Stufe 2:

### Herstellung von 1-Benzyl-3-(2-methylsulfanyl-ethyl)-2-oxo-1,4,8-triaza-spiro[4.5]decan-8-carbonsäure tert-butylester

Zu der Suspension von NaH (385 mg, 16,0 mmol) in THF (60 ml) tropfte man bei 0°C eine Lösung von 3-(2-methylsulfanyl-ethyl)-2-oxo-1,4,8-triaza-spiro[4.5]decan-8-carbonsäure tert-butylester (4.4 g, 13.4 mmol) in THF (72 ml). Nach Rühren für 1 h bei 0°C ließ man Benzylchlorid (2.0 g, 16.0 mmol) zu tropfen, ließ auf RT erwärmen und erhitzte für 68 h unter Rückfluß. Die Reaktionsmischung wurde mit wässeriger gesättigter NH₄Cl-Lösung (20 ml) versetzt, die organische Phase abgetrennt und die wässerige Phase mit EE (3 x 50 ml) extrahiert. Die vereinigte organische Phase wurde über Na₂SO₄ getrocknet, filtriert und das Lösungsmittel ab destilliert. Die Aufarbeitung erfolgte über Säulenchromathographie und ergab 1-Benzyl-3-(2-methylsulfanyl-ethyl)-2-oxo-1,4,8-triaza-spiro[4.5]decan-8-carbonsäure tert-butylester mit einer Ausbeute von 1.6 g (30 % der Theorie).

### Stufe 3:

### Herstellung von 1-Benzyl-3-(2-methylsulfanyl-ethyl)-1,4,8-triaza-spiro[4.5]decan-2-on

Zu der Lösung von 1-Benzyl-3-(2-methylsulfanyl-ethyl)-2-oxo-1,4,8-triaza-spiro[4.5]decan-8-carbonsäure tert-butylester (1.5 g, 3.5 mmol) in CH₂Cl₂ (10 ml) wurde Trifluoressigsäure (7.8 g, 68.0 mmol) bei 0°C zu getropft und 15 min bei dieser Temperatur gerührt. Nach Erwärmen auf RT wurde die Reaktionslösung für weitere 2.5 h gerührt. Anschließend wurde die Reaktionslösung mit wässeriger gesättigter NaHCO₃-Lösung auf den pH-Wert 7-8 eingestellt, die organische Phase abgetrennt und die wässerige Phase mit CH₂Cl₂ (2 x 10 ml) extrahiert. Die vereinigte organische Phase wurde über Na₂SO₄ getrocknet, filtriert und das Lösungsmittel ab destilliert. Die Aufarbeitung erfolgte über Säulenchromathographie und ergab 1-Benzyl-3-(2-methylsulfanyl-ethyl)-1,4,8-triaza-spiro[4.5]decan-2-on mit einer Ausbeute von 655 mg (58 % der Theorie).

Die folgenden erfindungsgemäßen substituierten 1,4,8-Triazaspiro[4.5]decan-2-on-Verbindungen der allgemeinen Formel I wurden gemäß den vorstehend beschriebenen Herstellungsvorschriften erhalten. Der Fachmann erkennt anhand der jeweiligen Beispielverbindung und der vorstehend beschriebenen Methoden, welche Ausgangsverbindungen jeweils zur ihrer Herstellung eingesetzt wurden.

### Beispielverbindungen:

[1] 3-Isopropyl-8-(4-nitro-benzolsulfonyl)-1,4,8-triaza-spiro[4.5]decan-2-on,
[2] 8-(2-Chlor-benzolsulfonyl)-3-isobutyl-1,4,8-triaza-spiro[4.5]decan-2-on,
[3] 3-Benzyl-8-[2-(2,2,2-trifluor-acetyl)-1,2,3,4-tetrahydro-isoquinolin-7-sulfonyl]-1,4,8-triaza-spiro[4.5]decan-2-on,
[4] 3-Benzyl-8-(4-methoxy-2,3,6-trimethyl-benzolsulfonyl)-1,4,8-triaza-spiro[4.5]decan-2-on,
[5] 2-[8-(4-Butoxy-benzolsulfonylF2-oxo-1,4,8-triaza-spiro[4.5]dec-1-ylmethyl]-benzonitril,
[6] 2-{8-[4-(1,1-Dimethyl-propyl)-benzolsulfonyl]-2-oxo-1,4,8-triaza-spiro[4.5]dec-1-ylmethyl}-benzonitril,
[7] 3-Benzyl-8-(2-fluor-benzolsulfonyl)-1,4,8-triaza-spiro[4.5]decan-2-on,
[8] 8-(2-Chlor-benzolsulfonyl)-3-isopropyl-1,4,8-triaza-spiro[4.5]decan-2-on,
[9] 1-Benzyl-2-oxo-1,4,8-triaza-spiro[4.5]decan-8-sulfonsäuredimethylamid,
[10] 3-[8-(4-Acetyl-benzolsulfonyl)-3-(2-methylsulfanyl-ethyl)-2-oxo-1,4,8-triaza-spiro[4.5]dec-1-ylmethyl]-benzonitril,
[11] 1-(2-Fluor-benzyl)-3-isopropyl-8-(4-methoxy-2,3,6-trimethyl-benzolsulfonyl)-1,4,8-triaza-spiro[4.5]decan-2-on,
[12] 1-Butyl-8-(5-chlor-thlophen-2-sulfonyl)-3-(2-methylsulfonyl-ethyl)-1,4,8-triaza-spiro[4.5]decan-2-on,
[13] 8-(4-Acetyl-benzolsulfonyl)-1-butyl-3-(2-methylsulfanyl-ethyl)-1,4,8-triaza-spiro[4.5]decan-2-on,
[14] 1-Benzyl-3-Isopropyl-8-(2,4,6-trimethyl-benzolsulfonyl)-1,4,8-triaza-spiro[4.5]decan-2-on,
[15] 2-(1-Butyl-3-isopropyl-2-oxo-1,4,8-triaza-spiro[4.5]decan-8-sulfonyl)-benzoesäuremethylester,
[16] 1,3-Dibenzyl-8-(thiophen-2-sulfonyl)-1,4,8-triaza-spiro[4.5]decan-2-on,
[17] 8-(3-Chlor-4-fluor-benzolsulfonyl)-3-isopropyl-1-(2-phenoxy-ethyl)-1,4,8-triaza-spiro[4.5]decan-2-on,
[18] 1-Butyl-3-(2-methylsulfanyl-ethyl)-8-(toluol-3-sulfonyl)-1,4,8-triaza-spiro[4.5]decan-2-on,
[19] 1-Butyl-8-(2,4-difluor-benzolsulfonyl)-3-isopropyl-1,4,8-triaza-spiro[4.5]decan-2-on,
[20] 1,3-Dibenzyl-8-(2,5-dichlor-thiophen-3-sulfonyl)-1,4,8-triaza-spiro[4.5]decan-2-on,
[21] 3-Isopropyl-1-(2-phenoxy-ethyl)-8-(toluol-4-sulfonyl)-1,4,8-triaza-spiro[4.5]decan-2-on,
[22] 3-Benzyl-1-butyl-8-(2-trifluormethyl-benzolsulfonyl)-1,4,8-triaza-spiro[4.5]decan-2-on,
[23] 2-[1-(4-Fluor-benzyl)-3-(2-methylsulfanyl-ethyl)-2-oxo-1,4,8-triaza-spiro[4.5]decan-8-sulfonyl]-benzoesäuremethylester,
[24] 1,3-Dibenzyl-8(2,3,5,6-tetramethyl-benzolsulfonyl)-1,4,8-triaza-spiro[4.5]decan-2-on,
[25] 1-(4-Fluor-benzyl)-3-(2-methylsulfanyl-ethyl)-8-(2-trifluormethyl-benzolsulfonyl)-1,4,8-triaza-spiro[4.5]decan-2-on,
[26] 8-(4-Chlor-2,5-dimethyl-benzolsulfonyl)-1-(4-fluor-benzyl)-3-(2-methylsulfanylethyl)-1,4,8-triaza-spiro[4.5]decan-2-on,
[27] 1-Benzyl-8-(4-chlor-2,5-dimethyl-benzolsulfonyl)-3-isobutyl-1,4,8-triaza-spiro[4.5]decan-2-on,
[28] 3-Benzyl-1-butyl-8-[4-(1,1-dimethyl-propyl)-benzolsulfonyl]-1,4,8-triaza-spiro[4.5]decan-2-on,
[29] 1,3-Dibenzyl-8-(3,4-dimethoxy-benzolsulfonyl)-1,4,8-triaza-spiro[4.5]decan-2-on,
[30] 1-Butyl-8-(3,4-dimethoxy-benzolsulfonyl)-3-(2-methylsulfanyl-ethyl)-1,4,8-triaza-spiro[4.5]decan-2-on,
[31] 1,3-Dibenzyl-8-(4-methoxy-2,3,6-trimethyl-benzolsulfonyl)-1,4,8-triaza-spiro[4.5]decan-2-on,
[32] 1-Benzyl-8-ethanesulfonyl-3-(2-methylsulfanyl-ethyl)-1,4,8-triaza-spiro[4.5]decan-2-on,
[33] 3-Benzyl-1-butyl-8-(4-ethyl-benzolsulfonyl)-1,4,8-triaza-spiro[4.5]decan-2-on,
[34] 3-(2-Methylsulfanyl-ethyl)-1-prop-2-ynyl-8-(3-trifluormethyl-benzolsulfonyl)-1,4,8-triaza-spiro[4.5]decan-2-on,
[35] 1,3-Dibenzyl-8-(4-ethyl-benzolsulfonyl)-1,4,8-triaza-spiro[4.5]decan-2-on,
[36] 3-Benzyl-1-butyl-8-(3-chlor-benzolsulfonyl)-1,4,8-triaza-spiro[4.5]decan-2-on,
[37] 1,3-Dibenzyl-8-(2-fluor-benzolsulfonyl)-1,4,8-triaza-spiro[4.5]decan-2-on,
[38] 2-[1-(2-Cyano-benzyl)-3-(2-methylsulfanyl-ethyl)-2-oxo-1,4,8-triaza-spiro[4.5]decan-8-sulfonyl]-benzoesäuremethylester,
[39] 3-Benzyl-1-prop-2-ynyl-8-(2-trifluormethyl-benzolsulfonyl)-1,4,8-triaza-spiro[4.5]decan-2-on,
[40] 3-Benzyl-8-(3-chlor-benzolsulfonyl)-1-methyl-1,4,8-triaza-spiro[4.5]decan-2-on,
[41] 1-Benzyl-8-[4-(1,1-dimethyl-propyl)-benzolsulfonyl]-3-(2-methylsulfanyl-ethyl)-1,4,8-triaza-spiro[4.5]decan-2-on,
[42] 8-(2,4-Difluor-benzolsulfonyl)-(3,5-dimethyl-benzyl)-3-(2-methylsulfanyl-ethyl)-1,4,8-triaza-spiro[4.5]decan-2-on,
[43] 1,3-Dibenzyl-8-(4-nitro-benzolsulfonyl)-1,4,8-triaza-spiro[4.5]decan-2-on,
[44] 1-(4-Fluor-benzyl)-3-(2-methylsulfanyl-ethyl)-8-(toluol-4-sulfonyl)-1,4,8-triaza-spiro[4.5]decan-2-on,
[45] 1,3-Dibenzyl-8-(toluol-4-sulfonyl)-1,4,8-triaza-spiro[4.5]decan-2-on,
[46] [3-Benzyl-8-(2-methyl-5-nitro-benzolsulfonyl)-2-oxo-1,4,8-triaza-spiro[4.5]dec-1-yl]-essigsäureethylester,
[47] [3-Benzyl-8-(2-methansulfonyl-benzolsulfonyl)-2-oxo-1,4,8-triaza-spiro[4.5]dec-1-yl]-essigsäureethylester,
[48] [3-Benzyl-2-oxo-8-(thiophen-2-sulfonyl)-1,4,8-triaza-spiro[4.5]dec-1-yl]-essigsäureethylester,
[49] 4-(8-Ethanesulfonyl-2-oxo-1,4,8-triaza-spiro[4.5]dec-1-ylmethyl)-benzoesäuremethylester,
[50] [3-Benzyl-8-(4-methoxy-benzolsulfonyl)-2-oxo-1,4,8-triaza-spiro[4.5]dec-1-yl]-essigsäureethylester,
[51] 4-(2-Oxo-8-benzolmethansulfonyl-1,4,8-triaza-spiro[4.5]dec-1-ylmethyl)-benzoesäuremethylester,
[52] 3-[3-Isopropyl-8-(4-nitro-benzolsulfonyl)-2-oxo-1,4,8-triaza-spiro[4.5]dec-1-ylmethyl]-benzonitril,
[53] 4-[8-(Butan-1-sulfonyl)-2-oxo-1,4,8-triaza-spiro[4.5]dec-1-ylmethyl]-benzoesäuremethylester,
[54] 1-Allyl-8-(2-methansulfonyl-benzolsulfonyl)-3-(2-methylsulfanyl-ethyl)-1,4,8-triaza-spiro[4.5]decan-2-on,
[55] 1-(2-Fluor-benzyl)-2-oxo-1,4,8-triaza-spiro[4.5]decan-8-sulfonsäure dimethylamid,
[56] 8-(7,7-Dimethyl-2-oxo-bicyclo[2.2.1]hept-1-ylmethansulfonyl)-1-(2-fluor-benzyl)-1,4,8-triaza-spiro[4.5]decan-2-on,
[57] [3-(2-Methylsulfanyl-ethyl)-2-oxo-8-benzolmethansulfonyl-1,4,8-triaza-spiro[4.5]dec-1-yl]-essigsäureethylester,
[58] 1-Allyl-3-(2-methylsulfanyl-ethyl)-8-benzolmethansulfonyl-1,4,8-triaza-spiro[4.5]decan-2-on,
[59] 3-[2-Oxo-8-(2,4,6-trimethyl-benzolsulfonyl)-1,4,8-triaza-spim[4.5]dec-1-ylmethyl]-benzonitril,
[60] 4-[2-Oxo-8-(2,4,6-trimethyl-benzolsulfonyl)-1,4,8-triaza-spim[4.5]dec-1-ylmethyl]-benzoesäuremethylester,
[61] [3-Benzyl-8-(5-chlor-thlophen-2-sulfonyl)-2-oxo-1,4,8-triaza-spiro[4.5]dec-1-yl]-essigsäureethylester,
[62] [3-Benzyl-8-(butan-l-sulfonyl)-2-oxo-1,4,8-triaza-spiro[4.5]dec-1-yl]-essigsäureethylester,
[63] [3-Benzyl-2-oxo-8-(toluol-3-sulfonyl)-1,4,8-triaza-spiro[4.5]dec-1-yl]-essigsäureethylester,
[64] 1-(2-Fluor-benzyl)-8-(4-methoxy-benzolsulfonyl)-1,4,8-triaza-spiro[4.5]decan-2-on,
[65] [3-Benzyl-2-oxo-8-(2-trifluormethyl-benzolsulfonyl)-1,4,8-triaza-spiro[4.5]dec-1-yl]-essigsäureethylester,
[66] (8-Benzolsulfonyl-3-benzyl-2-oxo-1,4,8-triaza-spiro[4.5]dec-1-yl)-essigsäureethylester,
[67] [3-Benzyl-2-oxo-8-(4-propyl-benzolsulfonyl)-1,4,8-triaza-spiro[4.5]dec-1-yl]-essigsäureethylester,
[68] (3-Benzyl-2-oxo-8-benzolmethansulfonyl-1,4,8-triaza-spiro[4.5]dec-1-yl)-essigsäureethylester,
[69] [3-Benzyl-8-(3-chlor-4-fluor-benzolsulfonyl)-2-oxo-1,4,8-triaza-spiro[4.5]dec-1-yl]-essigsäureethylester,
[70] 1-Allyl-3-isopropyl-8-(2,4,6-trimethyl-benzolsulfonyl)-1,4,8-triaza-spiro[4.5]decan-2-on,
[71] 8-Benzolsulfonyl-3-benzyl-1-(2-fluor-benzyl)-1,4,8-triaza-spiro[4.5]decan-2-on,
[72] [3-Benzyl-2-oxo-8-(2,4,6-trimethyl-benzolsulfonyl)-1,4,8-triaza-spiro[4.5]dec-1-yl]-essigsäureethylester,
[73] [3-Benzyl-8-(4-butoxy-benzolsulfonyl)-2-oxo-1,4,8-triaza-spiro[4.5]dec-1-yl]-essigsäureethylester,
[74] [3-Benzyl-8-(2,5-dimethoxy-benzolsulfonyl)-2-oxo-1,4,8-triaza-spiro[4.5]dec-1-yl]-essigsäureethylester,
[75] 3-(2-Methylsulfanyl-ethyl)-1-naphthalin-2-ylmethyl-2-oxo-1,4,8-triaza-spiro[4.5]decan-8-sulfonsäure dimethylamid,
[76] 1-(3-Cyano-benzyl)-3-isopropyl-2-oxo-1,4,8-triaza-spiro[4.5]decan-8-sulfonsäure dimethylamid,
[77] 3-{3-Isopropyl-2-oxo-8-[2-(2,2,2-trifluor-acetyl)-1,2,3,4-tetrahydro-isoquinolin-7-sulfonyl]-1,4,8-triaza-spiro[4.5]dec-1-ylmethyl}-benzonitril,
[78] 1-Allyl-3-(2-methylsulfanyl-ethyl)-8-[2-(2,2,2-trifluor-acetyl)-1,2,3,4-tetrahydro-isoquinolin-7-sulfonyl]-1,4,8-triaza-spiro[4.5]decan-2-on,
[79] 1-Allyl-8-(4-methoxy-2,3,6-trimethyl-benzolsulfonyl)-3-(2-methylsulfanyl-ethyl)-1,4,8-triaza-spiro[4.5]decan-2-on,
[80] 2-(3-Benzyl-1-ethoxycarbonylmethyl-2-oxo-1,4,8-triaza-spiro[4.5]decan-8-sulfonyl)-benzoesäuremethylester,
[81] 3-Benzyl-1-(2-fluvr-benzyl)-8-(2-trifluormethyl-benzolsulfonyl)-1,4,8-triaza-spiro[4.5]decan-2-on,
[82] 8-(2-Chlor-benzolsulfonyl)-3-(2-methylsulfanyl-ethyl)-1-(2-nitro-benzyl)-1,4 ,8-triaza-spiro[4.5]decan-2-on,
[83] 1-Allyl-3-(2-methylsulfanyl-ethyl)-8-(toluol-3-sulfonyl)-1,4,8-triaza-spiro[4.5]decan-2-on,
[84] [8-(2-Chlor-benzolsulfonyl)-3-(2-methylsulfanyl-ethyl)-2-oxo-1,4,8-triaza-spiro[4.5]dec-1-yl]-essigsäureethylester,
[85] 3-Benzyl-1-(2-fluor-benzyl)-8-(toluol-4-sulfonyl)-1,4,8 triaza-spiro[4.5]decan-2-on,
[86] 4-[8-(2,5-Dichlor-thiophen-3-sulfonyl)-2-oxo-1,4,8-triaza-spiro[4.5]dec-1-ylmethyl]-benzoesäuremethylester,
[87] 3-[8-(4-Fluor-benzolsulfonyl)-3-isopropyl-2-oxo-1,4,8-triaza-spiro[4.5]dec-1-ylmethyl]-benzonitril,
[88] 8-(2,5-Dimethoxy-benzotsulfonyl)-3-(2-methylsulfanyl-ethyl)-1-(2-nitro-benzyl)-1,4,8-triaza-spiro[4.5]decan-2-on,
[89] 8-(3-Chlor-4-fluor-benzolsulfonyl)-1-(2-fluor-benzyl)-1,4,8-triaza-spiro[4.5]decan-2-on,
[90] 1-(2-Fluor-benzyl)-8-(toluol-3-sulfonyl)-1,4,8-triaza-spiro[4.5]decan-2-on,
[91] 1-(2,6-Dichlor-benzyl)-8-(4,5-dichlor-thiophen-2-sulfonyl)-3-isobutyl-1,4,8-triaza-spiro[4.5]decan-2-on,
[92] 8-(5-Chlor-thiophen-2-sulfonyl)-1-(2-fluor-benzyl)-1,4,8-triaza-spiro[4.5]decan-2-on,
[93] 3-Benzyl-1-(2-fluor-benzyl)-8-(4-propyl-benzolsulfonyl)-1,4,8-triaza-spiro[4.5]decan-2-on,
[94] 3-[8-(Butan-1-sulfonyl)-3-isopropyl-2-oxo-1,4,8-triaza-spiro[4.5]dec-1-ylmethyl]-benzonitril,
[95] 3-[8-(2-Methansulfonyl-benzolsulfonyl)-2-oxo-1,4,8-triaza-spiro[4.5]dec-1-ylmethyl]-benzonitril,
[96] 8-(2-Fluor-benzolsulfonyl)-1-(2-fluor-benzyl)-1,4,8-triaza-spiro[4.5]decan-2-on,
[97] 2-[1-(3-Cyano-benzyl)-3-isopropyl-2-oxo-1,4,8-triaza-spiro[4.5]decan-8-sulfonylj-benzoesäuremethylester,
[98] 3-Benzyl-1-(2-fluor-benzyl)-8-(5-fluor-2-methyl-benzolsulfonyl)-1,4,8-triaza-spiro[4.5]decan-2-on,
[99] 1-Allyl-8-(4-methoxy-benzolsulfonyl)-3-(2-methylsulfanyl-ethyl)-1,4,8-triaza-spiro[4.5]decan-2-on,
[100] 1-(3,5-Dimethyl-benzyl)-3-isobutyl-8-(thiophen-2-sulfonyl)-1,4,8-triaza-spiro[4.5]decan-2-on,
[101] 3-(2-Methylsulfanyl-ethyl)-8-(4-nitro-benzolsulfonyl)-1-(2-nitro-benzyl)-1,4,8-triaza-spiro[4.5]decan-2-on,
[102] 2-isobutyl-3-oxo-1,4,8-triaza-spiro[4.5]decan-8-thiocarbonsäure-o-tolylamid,
[103]2-Benzyl-3-oxo-1,4,8-triaza-spiro[4.5]decan-8-thiocarbonsäure isopropylamid,
[104] 1-Butyl-3-isopropyl-2-oxo-1,4,8-triaza-spiro[4.5]decan-8-thiocarbonsäure (tetrahydro-furan-2-ylmethyl)-amid,
[105] 3-{[1-Methyl-3-(2-methylsulfanyl-ethyl)-2-oxo-1,4,8-triaza-spiro[4.5]decan-8-carbothioyl]-amino}-buttersäureethylester,
[106] 1-Allyl-3-isopropyl-2-oxo-1,4,8-triaza-spiro[4.5]decan-8-thiocarbonsäure isopropylamid,
[107] 1-Butyl-3-isopropyl-2-oxo-1,4,8-triaza-spiro[4.5]decan-8-thiocarbonsäure isopropylamid,
[108] 3-Benzyl-1-(2-fluor-benzyl)-2-oxo-1,4,8-triaza-spiro[4.5]decan-8-thiocarbonsäure (2-methoxy-benzol)-amid,
[109] [8-Isopropylthiocarbamoyl-3-(2-methylsulfanyl-ethyl)-2-oxo-1,4,8-triazaspiro[4.5]dec-1-yl]-essigsäureethylester,
[110] 1-Methyl-3-(2-methylsulfanyl-ethyl)-2-oxo-1,4,8-triaza-spiro[4.5]decan-8-thiocarbonsäure m-tolylamid,
[111] 1-Methyl-3-(2-methylsulfanyl-ethyl)-2-oxo-1,4,8-triaza-spiro[4.5]decan-8-thiocarbonsäure 4-methoxy-benzylamid,
[112]2-Isobutyl-3-oxo-1,4,8-triaza-spiro[4.5]decan-8-thiocarbonsäure (4-fluorbenzol)-amid,
[113] [8-Ethoxycarbonylaminocarbothioyl-3-(2-methylsulfanyl-ethyl)-2-oxo-1,4,8-triaza-spiro[4.5]dec-1-yl]-essigsäureethylester
[114] 3-{[1-Ethoxycarbonylmethyl-3-(2-methylsutfanyl-ethyl)-2-oxo-1,4,8-triaza-spiro[4.5]decan-8-carbothioyl]-amino}-buttersäureethylester,
[115] 1-Allyl-3-isopropyl-2-oxo-1,4,8-triaza-spiro[4.5]decan-8-thiocarbonsäure (2-methoxy-benzol)-amid,
[116] 1-Butyl-3-isopropyl-2-oxo-1,4,8-triaza-spiro[4.5]decan-8-thiocarbonsäure benzolamid,
[117] 1-Butyl-3-isopropyl-2-oxo-1,4,8-triaza-spiro[4.5]decan-8-thiocarbonsäure (2,5-dichlor-benzol)-amid,
[118] 3-Benzyl-1-methyl-2-oxo-1,4,8-triaza-spiro[4.5]decan-8-thiocarbonsäure (2,5-difluor-benzol)-amid,
[119] 1-Methyl-3-(2-methylsulfanyl-ethyl)-2-oxo-1,4,8-triaza-spiro[4.5]decan-8-thiocarbonsäure (2-brom-benzol)-amid,
[120] 1-Butyl-3-(2-methylsulfanyl-ethyl)-2-oxo-1,4,8-triaza-spiro[4.5]decan-8-thiocarbonsäure (2,5-dichlor-benzol)-amid, '
[121] 3-(2-Methylsulfanyl-ethyl)-1-naphthalin-2-ylmethyl-2-oxo-1,4,8-triaza-spiro[4.5]decan-8-thiocarbonsäure (2-methoxy-ethyl)-amid,
[122] 1-Butyl-3-isopropyl-2-oxo-1,4,8-triaza-spiro[4.5]decan-8-thiocarbonsäure (2-fluor-benzol)-amid,
[123] 3-isobutyl-2-oxo-1-prop-2-ynyl-1,4,8-triaza-spiro[4.5]decan-8-thiocarbonsäure allylamid,
[124] 1-Butyl-3-isopropyl-2-oxo-1,4,8-triaza-spiro[4.5]decan-8-thiocarbonsäure m-tolylamid,
[125] 1-Allyl-3-isopropyl-2-oxo-1,4,8-triaza-spiro[4.5]decan-8-thiocarbonsäure (4-fluor-benzol)-amid,
[126] 4-(8-Allylthiocarbamoyl-3-isopropyl-2-oxo-1,4,8-triaza-spiro[4.5]dec-1-ylmethyl)-benzoesäuremethylester,
[127] 1-Butyl-3-(2-methylsulfanyl-ethyl)-2-oxo-1,4,8-triaza-spiro[4.5]decan-8-thiocarbonsäure (2-methoxy-benzol)-amid,
[128] 1-Allyl-3-isopropyl-2-oxo-1,4,8-triaza-spiro[4.5]decan-8-thiocarbonsäure (2,5-difluor-benzol)-amid,
[129]1-(2-Cyano-benzyl)-3-(2-methylsulfanyl-ethyl)-2-oxo-1,4,8-triaza-spiro[4.5]decan-8-thiocarbonsäure allylamid,
[130] 1-(3-Cyano-benzyl)-3-(2-methylsulfanyl-ethyl)-2-oxo-1,4,8-triaza-spiro[4.5]decan-8-thiocarbonsäure allylamid,
[131] 3-[(3-Benzyl-1-methyl-2-oxo-1,4,8-triaza-spiro[4.5]decan-8-carbothioyl)-amino]-buttersäureethylester,
[132] 1-Butyl-3-(2-methylsulfanyl-ethyl)-2-oxo-1,4,8-triaza-spiro[4.5]decan-8-thiocarbonsäure cyclohexylmethyl-amid,
[133] 1-Methyl-3-(2-methylsulfanyl-ethyl)-2-oxo-1,4,8-triaza-spiro[4.5]decan-8-thiocarbonsäure (4-chlor-benzol)-amid,
[134] 1-Methyl-3-(2-methylsulfanyl-ethyl)-2-oxo-1,4,8-triaza-spiro[4.5]decan-8-thiocarbonsäure 4-fluor-benzylamid,
[135] 4-[8-(2,6-Dichlor-benzolthiocarbamoyl)-3-isopropyl-2-oxo-1,4,8-triaza-spiro[4.5]dec-1-ylmethyl-benzoesäuremethylester,
[136] 1-Benzyl-3-isopropyl-2-oxo-1,4,8-triaza-spiro[4.5]decan-8-thiocarbonsäure (2,5-dichlor-benzol)-amid,
[137] 4-{3-isopropyl-2-oxo-8-[(tetrahydro-furan-2-ylmethyl)-thiocarbamoyl]-1,4,8-triaza-spiro[4.5]dec-1-ylmethyl}-benzoesäuremethylester,
[138] 1-Methyl-3-(2-methylsulfanyl-ethyl)-2-oxo-1,4,8-triaza-spiro[4.5]decan-8-thiocarbonsäure (2,5-difluor-benzol)-amid,
[139] 1-Benzyl-3-(2-methylsulfanyl-ethyl)-2-oxo-1,4,8-triaza-spiro[4.5]decan-8-thiocarbonsäure (4-isopropyl-benzol)-amid,
[140] 1-Benzyl-3-(2-methylsulfanyl-ethyl)-2-oxo-1,4,8-triaza-spiro[4.5]decan-8-thiocarbonsäure (2,5-difluor-benzol)-amid,
[141] 1-(2-Cyano-benzyl)-3-isobutyl-2-oxo-1,4,8-triaza-spiro[4.5]decan-8-thiocarbonsäure (3-acetyl-benzol)-amid,
[142] 1-Butyl-3-isopropyl-2-oxo-1,4,8-triaza-spiro[4.5]decan-8-thiocarbonsäure (3-chlor-benzolramid,
[143] 2-[(1-Butyl-3-isopropyl-2-oxo-1,4,8-triaza-spiro[4.5]decan-8-carbothioyl)-amino]-benzoesäuremethylester,
[144] (3-Benzyl-8-ethoxycarbonylaminocarbothioyl-2-oxo-1,4,8-triaza-spiro[4.5]dec-1-yl)-essigsäureethylester,
[145] 2-[(3-Benzyl-1-ethoxycarbonylmethyl-2-oxo-1,4,8-triaza-spiro[4.5]decan-8-carbothioyl)-amino]-benzoesäuremethylester,
[146] [1-(2-Cyano-benzyl)-3-(2-methylsulfanyl-ethyl)-2-oxo-1,4,8-triaza-spiro[4.5]decan-8-carbothioyl]-carbamidsäureethylester,
[147] 1-(2-Cyano-benzyl)-3-(2-methylsulfanyl-ethyl)-2-oxo-1,4,8-triaza-spiro[4.5]decan-8-thiocarbonsäure (2-chlor-5-trifluormethyl-benzol)-amid,
[148] 3-(2-Methylsulfanyl-ethyl)-1-naphthalin-2-ylmethyl-2-oxo-1,4,8-triaza-spiro[4.5]decan-8-thiocarbonsäure (1-benzol-ethyl)-amid,
[149] 1-Allyl-3-isopropyl-2-oxo-1,4,8-triaza-spiro[4.5]decan-8-thiocarbonsäure pentafluorbenzol-amid,
[150] 1-Butyl-3-isopropyl-2-oxo-1,4,8-triaza-spiro[4.5]decan-8-thiocarbonsäure 4-chlor-benzylamid,
[151] 3-(2-Methylsulfanyl-ethyl)-naphthalin-2-ylmethyl-2-oxo-1,4,8-triaza-spiro[4.5]decan-8-thiocarbonsäure o-tolylamid,
[152] 1-Butyl-3-isopropyl-2-oxo-1,4,8-triaza-spiro[4.5]decan-8-thiocarbonsäure pentafluorbenzol-amid,
[153] 2-{[1-(2-Cyano-benzyl)-3-(2-methylsulfanyl-ethyl)-2-oxo-1,4,8-triaza-spiro[4.5]decan-8-carbothioyl]-amino}-benzoesäuremethylester,
[154] 1-Benzyl-3-isopropyl-2-oxo-1,4,8-triaza-spiro[4.5]decan-8-thiocarbonsäure pentafluorbenzol-amid,
[155] 1-Benzyl-3-isopropyl-2-oxo-1,4,8-triaza-spiro[4.5]decan-8-thiocarbonsäure (2-methoxy-benzol)-amid,
[156] 1-Benzyl-3-isopropyl-2-oxo-1,4,8-triaza-spiro[4.5]decan-8-thiocarbonsäure benzolamid,
[157] 1-Benzyl-3-isopropyl-2-oxo-1,4,8-triaza-spiro[4.5]decan-8-thiocarbonsäure (2,5-difluor-benzol)-amid,
[158] 1-Butyl-3-(2-methylsulfanyl-ethyl)-2-oxo-1,4,8-triaza-spiro[4.5]decan-8-thiocarbonsäure (4-ethoxy-benzol)-amid,
[159] [3-Benzyl-8-cyclohexylmethyl-thiocarbamoyl)-2-oxo-1,4,8-triaza-spiro[4.5]dec-1-yl]-essigsäureethylester,
[160] 1-Butyl-3-(2-methylsulfanyl-ethyl)-2-oxo-1,4,8-triaza-spiro[4.5]decan-8-thiocarbonsäure benzylamid,
[161] 1-(2-Cyano-benzyl)-3-(2-methylsulfanyl-ethyl)-2-oxo-1,4,8-triaza-spiro[4.5]decan-8-thiocarbonsäure m-tolylamid,
[162] 1-Allyl-3-isopropyl-2-oxo-1,4,8-triaza-spiro[4.5]decan-8-thiocarbonsäure 4-fluor benzylamid,
[163] 3-Isobutyl-2-oxo-1-prop-2-ynyl-1,4,8-triaza-spiro[4.5]decan-8-thiocarbonsäure (3-acetyl-benzol)amid,
[164] 4-(3-Isopropyl-2-oxo-8-pentafluorbenzolthiocarbamoyl-1,4,8-triaza-spiro[4.5]dec-1-ylmethyl)-benzoesäuremethylester,
[165] 3-(2-Methylsulfanyl-ethyl)-1-naphthalin-2-ylmethyl-2-oxo-1,4,8-triaza-spiro[4.5]decan-8-thiocarbonsäure (3-acetyl-benzol)-amid,
[166] 3-Benzyl-1-(2-fluor-benzyl)-2-oxo-1,4,8-triaza-spiro[4.5]decan-8-thiocarbonsäure m-tolylamid,
[167] 4-(8-Ethoxycarbonylaminocarbothioyl-3-isopropyl-2-oxo-1,4,8-triaza-spiro[4.5]dec-1-ylmethyl)-benzoesäuremethylester,
[168] 1-(4-Fluor-benzyl)-3-(2-methylsulfanyl-ethyl)-2-oxo-1,4,8-triaza-spiro[4.5]decan-8-thiocarbonsäure (tetrahydro-furan-2-ylmethyl)-amid,
[169] 1-(2-Cyano-benzyl)-3-isobutyl-2-oxo-1,4,8-triaza-spiro[4.5]decan-8-thiocarbonsäure (3,5-bis-trifluormethyl-benzol)-amid,
[170] 1-(2-Cyano-benzyl)-3-isobutyl-2-oxo-1,4,8-triaza-spiro[4.5]decan-8-thiocarbonsäure benzylamid,
[171] 3-Benzyl-1-(2-fluor-benzyl)-2-oxo-1,4,8-triaza-spiro[4.5]decan-8-thiocarbonsäure cyclohexylmethyl-amid,
[172] 1-(3-Cyano-benzyl)-3-(2-methylsulfanyl-ethyl)-2-oxo-1,4,8-triaza-spiro[4.5]decan-8-thiocarbonsäure (4-chlor-benzol)-amid,
[173] 1-Butyl-3-(2-methylsulfanyl-ethyl)-2-oxo-1,4,8-triaza-spiro[4.5]decan-8-thiocarbonsäure pentafluorbenzol-amid,
[174] [3-Benzyl-1-(2-fluor-benzyl)-2-oxo-1,4,8-triaza-spiro[4.5]decan-8-carbothioyl]-carbamidsäureethylester,
[175] 3-Benzyl-1-methyl-2-oxo-1,4,8-triaza-spiro[4.5]decan-8-thiocarbonsäure 4-chlor-benzylamid,
[176]1-(4-Fluor-benzyl)-3-(2-methylsulfanyl-ethyl)-2-oxo-1,4,8-triaza-spiro[4.5]decan-8-thiocarbonsäure isopropylamid,
[177] 1-Benzyl-3-isopropyl-2-oxo-1,4,8-triaza-spiro[4.5]decan-8-thiocarbonsäure (3,5-dichlor-benzol)-amid,
[178] [1-(3-Cyano-benzyl)-3-(2-methylsulfanyl-ethyl)-2-oxo-1,4,8-triazaspiro[4.5]decan-8-carbothioyl]-carbamidsäureethylester,
[179] 1-Benzyl-3-isopropyl-2-oxo-1,4,8-triaza-spiro[4.5]decan-8-thiocarbonsäure 4-fluor-benzylamid,
[180] 1-Allyl-3-isopropyl-2-oxo-1,4,8-triaza-spiro[4.5]decan-8-thiocarbonsäure (3,5-dichlor-benzol)-amid,
[181] 1-(3-Cyano-benzyl)-3-(2-methylsulfanyl-ethyl)-2-oxo-1,4,8-triaza-spiro[4.5]decan-8-thiocarbonsäure (1-benzol-ethyl)-amid,
[182] 1-(4-Fluor-benzyl)-3-(2-methylsulfanyl-ethyl)-2-oxo-1,4,8-triaza-spiro[4.5]decan-8-thiocarbonsäure 4-fluor-benzylamid,
[183] 1-(2-Cyano-benzyl)-3-(2-methylsulfanyl-ethyl)-2-oxo-1,4,8-triaza-spiro[4.5]decan-8-thiocarbonsäure (2-methoxy-benzol)-amid,
[184] 1-(3-Cyano-benzyl)-3-(2-methylsulfanyl-ethyl)-2-oxo-1,4,8-triaza-spiro[4.5]decan-8-thiocarbonsäure (2-methoxy-benzol)-amid,
[185]1-(3-Cyano-benzyl)-3-(2-methylsulfanyl-ethyl)-2-oxo-1,4,8-triaza-spiro[4.5]decan-8-thiocarbonsäure 4-chlor-benzylamid,
[186]1-(4-Fluor-benzyl)-3-(2-methylsulfanyl-ethyl)-2-oxo-1,4,8-triaza-spiro[4.5]decan-8-thiocarbonsäure o-tolylamid,
[187] 3-Benzyl-1-(2-fluor-benzyl)-2-oxo-1,4,8-triaza-spiro[4.5]decan-8-thiocarbonsäure (2,6-dichlor-benzol)-amid,
[188] 3-Benzyl-1-(2-fluor benzyl)-2-oxo-1,4,8-triaza-spiro[4.5]decan-8-thiocarbonsäure (2-chlor-5-trifluormethyl-benzol)-amid,
[189] 1-Butyl-3-(2-methylsulfanyl-ethyl)-2-oxo-1,4,8-triaza-spiro[4.5]decan-8-thiocarbonsäure (4-fluor-benzol)-amid,
[190] 3-Isobutyl-2-oxo-1-prop-2-ynyl-1,4,8-triaza-spiro[4.5]decan-8-thiocarbonsäure (2,6-dichlor-benzol)-amid,
[191] 4-[8-(4-Chlor-benzylthiocarbamoyl)-3-isopropyl-2-oxo-1,4,8-triaza-spiro[4.5]dec-1-ylmethyl]-benzoesäuremethylester,
[192] 1-(4-Fluor-benzyl)-3-(2-methylsulfanyl-ethyl)-2-oxo-1,4,8-triaza-spiro[4.5]decan-8-thiocarbonsäure (3-cyano-benzol)-amid,
[193] 1-(2-Cyano-benzyl)-3-(2-methylsulfanyl-ethyl)-2-oxo-1,4,8-triaza-spiro[4.5]decan-8-thiocarbonsäure 4-chlor-benzylamid,
[194] 1-Methyl-3-(2-methylsulfanyl-ethyl)-2-oxo-1,4,8-triaza-spiro[4.5]decan-8-thiocarbonsäure (3-trifluormethyl-benzol)-amid,
[195] 3-Isobutyl-2-oxo-1-prop-2-ynyl-1,4,8 triaza-spiro[4.5]decan-8-thiocarbonsäure (3,5-dichlor-benzol)-amid,
[196] 1-(4-Fluor-benzyl)-3-(2-methylsulfanyl-ethyl)-2-oxo-1,4,8-triaza-spiro[4.5]decan-8-thiocarbonsäure (1-benzol-ethyl)-amid,
[197] 3-Benzyl-1-(2-fluor-benzyl)-2-oxo-1,4,8-triaza-spiro[4.5]decan-8-thiocarbonsäure 4-chlor-benzylamid,
[198] 2-Isopropyl-3-oxo-1,4,8-triaza-spifo[4.5]decan-8-thiocarbonsäure (4-fluorbenzol)-amid,
[199] 1-Benzyl-3-isopropyl-2-oxo-1,4,8-triaza-spiro[4.5]decan-8-thiocarbonsäure benzylamid,
[200] 1-(4-Fluor-benzyl)-3-(2-methylsulfanyl-ethyl)-2-oxo-1,4,8-triaza-spiro[4.5]decan-8-thiocarbonsäure (4-ethoxy-benzol)-amid,
[201] 3-Benzyl-1-(2-fluor-benzyl)-2-oxo-1,4,8-triaza-spiro[4.5]decan-8-thiocarbonsäure (3-acetyl-benzol)-amid,
[202] 3-Benzyl-1-methyl-2-oxo-1,4,8-triaza-spiro[4.5]decan-8-carbonsäure cyclohexylamid,
[203] [3-(2-Methylsulfanyl-ethyl)-2-oxo-8-benzolcarbamoyl-1,4,8-triaza-spiro[4.5]dec-1-yl]-essigsäureethylester,
[204] 1-Allyl-3-isopropyl-2-oxo-1,4,8-triaza-spiro[4.5]decan-8-carbonsäure (2,5-dimethoxy-benzol)-amid,
[205] 1-Methyl-3-(2-methylsulfanyl-ethyl)-2-oxo-1,4,8-triaza-spiro[4.5]decan-8-carbonsäure (3-fluor-benzol)-amid,
[206] 4-(8-Cyclohexylcarbamoyl-3-isopropyl-2-oxo-1,4,8-triaza-spiro[4.5]dec-1-ylmethyl)-benzoesäuremethylester,
[207] 1-Butyl-3-(2-methylsulfanyl-ethyl)-2-oxo-1,4,8-triaza-spiro[4.5]decan-8-carbonsäure benzolamid,
[208] 1-Benzyl-3-(2-methylsulfanyl-ethyl)-2-oxo-1,4,8-triaza-spiro[4.5]decan-8-carbonsäure (3-cyano-benzol)-amid,
[209]1-Allyl-3-isopropyl-2-oxo-1,4,8-triaza-spiro[4.5]decan-8-carbonsäure (2,5-difluor-benzol)-amid,
[210] 1-Butyl-3-(2-methylsulfanyl-ethyl)-2-oxo-1,4,8-triaza-spiro[4.5]decan-8-carbonsäure (3,4,5-trimethoxy-benzol)-amid,
[211] 1-Benzyl-3-(2-methylsulfanyl-ethyl)-2-oxo-1,4,8-triaza-spiro[4.5]decan-8-carbonsäure (4-chlor-3-trifluormethyl-benzol)-amid,
[212] 1-Methyl-3-(2-methylsulfanyl-ethyl)-2-oxo-1,4,8-triaza-spiro[4.5]decan-8-carbonsäure (5-chlor-2-methoxy-benzol)-amid,
[213] 1-Benzyl-3-(2-methylsulfanyl-ethyl)-2-oxo-1,4,8-triaza-spiro[4.5]decan-8-carbonsäure (4-ethoxy-benzol)-amid,
[214] 1-Benzyl-3-(2-methylsulfanyl-ethyl)-2-oxo-1,4,8-triaza-spiro[4.5]decan-8-carbonsäure (3-acetyl-benzol)-amid,
[215] 3-[(1-Butyl-3-isopropyl-2-oxo-1,4,8-triaza-spiro[4.5]decan-8-carbonyl)-amino]-benzoesäureethylester,
[216] 1-Butyl-3-isopropyl-2-oxo-1,4,8-triaza-spiro[4.5]decan-8-carbonsäure (2,5-dimethoxy-benzol)-amid,
[217] 1-(2-Cyano-benzyl)-3-isobutyl-2-oxo-1,4,8-triaza-spiro[4.5]decan-8-carbonsäure 3,4-dichlor-benzylamid,
[218] 1-(2-Cyano-benzyl)-3-isobutyl-2-oxo-1,4,8-triaza-spiro[4.5]decan-8-carbonsäure (2,5-difluor-benzol)-amid,
[219] 1-Butyl-3-(2-methylsulfanyl-ethyl)-2-oxo-1,4,8-triaza-spiro[4.5]decan-8-carbonsäure 3,4-dichlor-benzylamid,
[220] 1-Allyl-3-isopropyl-2-oxo-1,4,8-triaza-spiro[4.5]decan-8-carbonsäure (4-trifluormethoxy-benzol)-amid,
[221] 1-Butyl-3-(2-methylsulfanyl-ethyl)-2-oxo-1,4,8-triaza-spiro[4.5]decan-8-carbonsäure (4-ethoxy-benzol)-amid,
[222] 1-Methyl-3-(2-methylsulfanyl-ethyl)-2-oxo-1,4,8-triaza-spiro[4.5]decan-8-carbonsäure (4-trifluormethoxy benzol)-amid,
[223] 4-[3-Isopropyl-8-(3-methoxy-benzolcarbamoyl)-2-oxo-1,4,8-triaza-spiro[4.5]dec-1-ylmethyl]-benzoesäuremethylester,
[224] 1-(2-Cyano-benzyl)-3-(2-methylsulfanyl-ethyl)-2-oxo-1,4,8-triaza-spiro[4.5]decan-8-carbonsäure benzolamid,
[225] [3-Benzyl-8-(3,4-dichlor-benzylcarbamoyl)-2-oxo-1,4,8-triaza-spiro[4.5]dec-1-yl]-essigsäureethylester,
[226] 1-(2-Cyano-benzyl)-3-(2-methylsulfanyl-ethyl)-2-oxo-1,4,8-triaza-spiro[4.5]decan-8-carbonsäure (3-fluor-benzol)-amid,
[227] 1-Butyl-3-(2-methylsulfanyl-ethyl)-2-oxo-1,4,8-triaza-spiro[4.5]decan-8-carbonsäure (4-chlor-3-trifluormethyl-benzol)-amid,
[228] 1-(4-Fluor-benzyl)-3-(2-methylsulfanyl-ethyl)-2-oxo-1,4,8-triaza-spiro[4.5]decan-8-carbonsäure 3,4-dichlor-benzylamid,
[229] 1-(2-Cyano-benzyl)-3-(2-methylsulfanyl-ethyl)-2-oxo-1,4,8-triaza-spiro[4.5]decan-8-carbonsäure 3,4-dichlor-benzylamid,
[230] 3-Benzyl-1-(2-fluor-benzyl)-2-oxo-1,4,8-triaza-spiro[4.5]decan-8-carbonsäure (2,5-difluor-benzol)-amid,
[231] 3-(2-Methylsulfanyl-ethyl)-1-naphthalin-2-ylmethyl-2-oxo-1,4,8-triaza-spiro[4.5]decan-8-carbonsäure (4-ethoxy-benzol)-amid,
[232] 4-[8-(3,4-Dichlor benzylcarbamoyl)-3-isopropyl-2-oxo-1,4,8-triaza-spiro[4.5]dec-1-ylmethyl]-benzoesäuremethylester,
[233] 3-Benzyl-1-(2-fluor-benzyl)-2-oxo-1,4,8-triaza-spiro[4.5]decan-8-carbonsäure 3,4-dichlor-benzylamid,
[234] 1-(2-Cyano-benzyl)-3-(2-methylsulfanyl-ethyl)-2-oxo-1,4,8-triaza-spiro[4.5]decan-8-carbonsäure (3-acetyl-benzol)-amid,
[235] 4-{[3-Benzyl-1-(2-fluor-benzyl)-2-oxo-1,4,8-triaza-spiro[4.5]decan-8-carbonyl]-amino}-benzoesäureethylester,
[236] 1-(3-Cyano-benzyl)-3-(2-methylsulfanyl-ethyl)-2-oxo-1,4,8-triaza-spiro[4.5]decan-8-carbonsäure (3-methoxy-benzol)-amid,
[237] 1-(4-Fluor-benzyl)-3-(2-methylsulfanyl-ethyl)-2-oxo-1,4,8-triaza-spiro[4.5]decan-8-carbonsäure (4-trifluormethoxy-benzol)-amid,
[238] 3-Benzyl-1-(2-fluor-benzyl)-2-oxo-1,4,8-triaza-spiro[4.5]decan-8-carbonsäure cyclohexylamid,
[239] 1-(4-Fluor-benzyl)-3-(2-methylsulfanyl-ethyl)-2-oxo-1,4,8-triaza-spiro[4.5]decan-8-carbonsäure phenethyl-amid,
[240] 3-(2-Methylsulfanyl-ethyl)-1-naphthalin-2-ylmethyl-2-oxo-1,4,8-triaza-spiro[4.5]decan-8-carbonsäure (3-fluor-benzol)-amid,
[241] 4-[8-(4-Chlor-3-trifluormethyl-benzolcarbamoyl)-3-isopropyl-2-oxo-1,4,8-triaza-spiro[4.5]dec-1-ylmethyl]-benzoesäuremethylester,
[242] 1-(3-Cyano-benzyl)-3-(2-methylsulfanyl-ethyl)-2-oxo-1,4,8-triaza-spiro[4.5]decan-8-carbonsäure (2,5-dimethoxy-benzol)-amid,
[243] 1-(4-Fluor-benzyl)-3-(2-methylsulfanyl-ethyl)-2-oxo-1,4,8-triaza-spiro[4.5]decan-8-carbonsäure (4-ethoxy-benzol)-amid,
[244] 1-(4-Fluor-benzyl)-3-(2-methylsulfanyl-ethyl)-2-oxo-1,4,8-triaza-spiro[4.5]decan-8-carbonsäure (3-cyano-benzol)-amid,
[245] [3-Benzyl-8-(3-fluor-benzolcarbamoyl)-2-oxo-1,4,8-triaza-spiro[4.5]dec-1-yl]-essigsäureethylester,
[246] 1,3-Dibenzyl-8-methansulfonyl-1,4,8-triaza-spiro[4.5]decan-2-on,
[247] 8-Benzolsulfonyl-1,3-dibenzyl-1,4,8-triaza-spiro[4.5]decan-2-on,
[248] 1,3-Dibenzyl-8-(4-chlor-benzolsulfonyl)-1,4,8-triaza-spiro[4.5]decan-2-on,
[249] 1,3-Dibenzyl-8-(4-methoxy-benzolsulfonyl)-1,4,8-triaza-spiro[4.5]decan-2-on,
[250] 1,3-Dibenzyl-2-oxo-1,4,8-triaza-spiro[4.5]decan-8-carbonsäurephenylamid,
[251] 1,3-Dibenzyl-2-oxo-1,4,8-triaza-spiro[4.5]decan-8-carbonsäure-(4-methoxyphenyl)-amid,
[252] 1,3-Dibenzyl-2-oxo-1,4,8-triaza-spiro[4.5]decan-8-thiocarbonsäurephenylamid,
[253] 1,3-Dibenzyl-2-oxo-1,4,8-triaza-spiro[4.5]decan-8-thiocarbonsäure-(2,4-difluorphenyl)-amid,

### Pharmakologische Daten:

Die Affinität der erfindungsgemäßen substituierten 1,4,8-Triazaspiro[4.5]decan-2-on-Verbindungen der allgemeinen Formel I zur Batrachotoxin-(BTX)-Bindungsstelle bzw. zum Cannabinoid-Rezeptor CB2, sowie die Hemmung der Noradrenalin- bzw. 5-HT-Wiederaufnahme, wurde wie vorstehend beschrieben bestimmt.

Die erfindungsgemäßen substituierten 1,4,8-Triazaspiro[4.5]decan-2-on-Verbindungen der vorstehend angegebenen allgemeinen Formel I zeigen eine gute bis sehr gute Inhibierung der Noradrenalin-Wiederaufnahme sowie eine gute bis sehr gute Inhibierung der 5-Hydroxy-Tryptophan-Wiederaufnahme.

Darüber hinaus zeigen diese erfindungsgemäßen Verbindungen auch ausgezeichnete Affinitäten für die Batrachotoxin-(BTX)-Bindungsstette des Natriumkanals.

Ebenfalls weisen die erfindungsgemäßen substituierten 1,4,8-Triazaspiro[4.5]decan-2-on-Verbindungen auch eine Hohe Affinität zum Cannabinoidrezeptor CB2 (CB2-Rezeptor) auf.

In den nachfolgenden Tabellen I, II und III sind die jeweiligen pharmakologischen Daten für die beispielgemäßen substituierten 1,4,8-Triazaspiro[4.5]decan-2-on-Verbindungen wiedergegeben.

**Tabelle I:**

| **Beispiel** | BTX Inhibierung | Uptake_5-HT, Ratte, 1µM,% Hemmung | Uptake (NA), Ratte, 10 µM, % Hemmung |
|---|---|---|---|
| **1** | | 55 | |
| **4** | 53 | | 34 |
| **5** | 59 | 30 | |
| **6** | 56 | 57 | |
| **7** | | 54 | 32 |
| **8** | | 55 | |
| **9** | | | 40 |
| **10** | | 35 | 39 |
| **11** | | 52 | 37 |
| **12** | | 58 | |
| **13** | | 56 | |
| **14** | 68 | 61 | |
| **15** | | 44 | |
| **16** | 50 | 82 | |
| **17** | 41 | | |
| **18** | 32 | 68 | |
| **19** | 30 | | |
| **20** | 54 | 81 | |
| **21** | 54 | | |
| **22** | 38 | 66 | |
| **23** | 40 | 69 | |
| **24** | 67 | 66 | |
| **25** | 57 | 58 | |
| **26** | 38 | 68 | |
| **27** | 59 | | |
| **28** | 41 | 75 | |
| **29** | 47 | 75 | |
| **30** | | 53 | |
| **31** | 82 | 67 | |
| **32** | | 64 | |
| **33** | 54 | 67 | |
| **34** | | | |
| **35** | 54 | 79 | |
| **36** | 49 | 65 | |
| **37** | 61 | 73 | |
| **38** | | 40 | |
| **39** | 39 | 38 | 41 |
| **40** | | 57 | |
| **41** | | 56 | |
| **42** | 56 | | |
| **43** | 51 | 71 | |
| **44** | 42 | 42 | |
| **45** | 37 | 74 | |
| **46** | 30 | 90 | 33 |
| **47** | 31 | 71 | 47 |
| **48** | 30 | 85 | |
| **50** | 32 | 79 | 38 |
| **51** | 31 | 62 | |
| **52** | | 53 | |
| **53** | 31 | 45 | 30 |
| **54** | | 30. | 46 |
| **55** | | 57 | |
| **56** | 32 | 60 | |
| **57** | 34 | 32 | |
| **58** | | 56 | |
| **59** | 50 | 50 | |
| **60** | 59 | 65 | |
| **61** | 50 | 61 | |
| **62** | | 70 | |
| **63** | 36 | 59 | |
| **64** | 42 | | |
| **65** | 51 | 71 | 33 |
| **66** | 39 | 64 | |
| **67** | 40 | 64 | |
| **68** | 47 | 69 | 31 |
| **69** | 73 | 62 | |
| **70** | 60 | | |
| **71** | 85 | 76 | |
| **72** | 75 | 75 | |
| **73** | 52 | 80 | |
| **74** | 30 | 74 | |
| **75** | 62 | 74 | |
| **76** | | | |
| **77** | 36 | 32 | |
| **78** | 32 | | |
| **79** | 67 | 37 | |
| **80** | 33 | 84 | |
| **81** | 87 | 81 | |
| **82** | 71 | 49 | |
| **83** | | | |
| **84** | | | |
| **86** | | | |
| **91** | | | |
| **92** | | | |
| **93** | | | |
| **100** | | | |
| **101** | | | |

**Tabelle II:**

| **Beispiel** | BTX-Inhibierung | Uptake_5-HT, Ratte, 1µM, % Hemmung | Uptake (NA), Ratte, 10 µM, % Hemmung |
|---|---|---|---|
| **102** | | - | |
| **103** | | 41 | |
| **107** | | 28 | |
| **108** | 86 | 86 | 31 |
| **111** | 51 | | - |
| **112** | 30 | 31 | |
| **116** | 30 | 40 | |
| **117** | 44 | 33 | |
| **118** | 42 | | |
| **120** | 49 | 58 | |
| **121** | 47 | 39 | |
| **122** | 35 | 32 | |
| **123** | | | |
| **124** | 41 | 47 | |
| **125** | | 34 | |
| **126** | 37 | | |
| **127** | 44 | | |
| **128** | 33 | 57 | |
| **129** | 36 | 41 | |
| **131** | 42 | | 42 |
| **132** | 71 | 30 | |
| **133** | 44 | | |
| **134** | 50 | | |
| **135** | 38 | | |
| **136** | 66 | 44 | |
| **137** | 37 | | 30 |
| **138** | 34 | | |
| **139** | 97 | 63 | |
| **140** | 53 | 59 | |
| **141** | 50 | | |
| **142** | 63 | | |
| **143** | 51 | 32 | |
| **144** | | 47 | |
| **145** | 69 | 67 | |
| **146** | 30 | 30 | |
| **147** | 81 | 33 | |
| **148** | 88 | 49 | |
| **149** | | 55 | |
| **150** | 73 | 51 | 30 |
| **151** | 84 | 64 | 42 |
| **152** | 39 | | 37 |
| **153** | 53 | | 30 |
| **154** | 61 | 34 | 43 |
| **155** | 32 | | 52 |
| **156** | | | 33 |
| **157** | 39 | | 38 |
| **158** | 35 | 33 | 41 |
| **159** | 84 | 33 | 36 |
| **160** | 63 | | 42 |
| **161** | 53 | | 33 |
| **162** | | | 26 |
| **163** | | 38 | 34 |
| **164** | 42 | | 58 |
| **165** | 64 | 42 | 48 |
| **166** | 85 | 64 | 39 |
| **167** | | | 42 |
| **168** | 38 | | 35 |
| **169** | 91 | | 44 |
| **170** | 53 | | 46 |
| **171** | 86 | | 34 |
| **172** | 59 | | 47 |
| **173** | 40 | | 45 |
| **174** | 38 | 31 | 39 |
| **175** | 63 | | 32 |
| **176** | | 32 | |
| **177** | 46 | 36 | |
| **178** | | | |
| **179** | 58 | 47 | |
| **180** | 54 | | |
| **181** | 57 | | 40 |
| **182** | 69 | 30 | |
| **183** | 37 | | 29 |
| **184** | 36 | | 32 |
| **185** | 72 | | |
| **186** | 52 | 55 | |
| **187** | 86 | 54 | 39 |
| **188** | 77 | 59 | |
| **189** | 41 | 66 | 30 |
| **190** | 53 | | |
| **191** | 74 | | |
| **192** | 35 | 38 | |
| **193** | 72 | | 32 |
| **194** | 36 | | |
| **195** | 77 | | 30 |
| **196** | 70 | | |
| **197** | 77 | | |
| **198** | | | |
| **199** | 44 | | |
| **200** | 30 | 30 | |
| **201** | 63 | 54 | |
| **202** | 56 | 34 | |
| **203** | 32 | | 40 |
| **204** | | | |
| **205** | | | |
| **206** | | | 39 |
| **207** | 35 | 39 | |
| **208** | 41 | 45 | 49 |
| **209** | 31 | | - |
| **210** | 44 | 32 | 36 |
| **211** | 85 | 45 | 40 |
| **212** | | | |
| **213** | 41 | 39 | 37 |
| **214** | 40 | 51 | 36 |
| **215** | 51 | 29 | 33 |
| **216** | | 46 | 30 |
| **217** | 78 | | 39 |
| **218** | 40 | | |
| **219** | 70 | 37 | 42 |
| **220** | 44 | 39 | 39 |
| **221** | 44 | 47 | |
| **222** | 48 | | 30 |
| **223** | | | 31 |
| **224** | 30 | 37 | 38 |
| **225** | 77 | 43 | 33 |
| **226** | | 55 | |
| **227** | 85 | 58 | |
| **228** | 87 | | |
| **229** | 69 | | 40 |
| **230** | 78 | 63 | |
| **231** | 79 | 59 | |
| **232** | 55 | | |
| **233** | 85 | 44 | |
| **234** | 31 | 45 | |
| **235** | 77 | 65 | |
| **236** | 42 | 32 | 34 |
| **2377** | 87 | 39 | |
| **238** | 73 | 43 | |
| **239** | 69 | 36 | |
| **240** | 78 | 63 | |
| **241** | 78 | 31 | |
| **242** | 46 | 30 | |
| **243** | 48 | 40 | |
| **244** | 47 | 52 | 32 |
| **245** | 31 | 51 | |

**Tabelle III:**

| **Beispiel** | **Inhibierung CB2** 10µM, % Hemmung | **Kᵢ** |
|---|---|---|
| 8 | 41 | |
| 16 | 58 | |
| 20 | 66 | |
| 24 | 56 | |
| 28 | 40 | |
| 31 | 57 | |
| 35 | 61 | |
| 36 | 40 | |
| 42 | 48 | |
| 43 | 62 | |
| 50 | 52 | |
| 54 | 56 | |
| 73 | 46 | |
| 82 | 56 | |
| 107 | 56 | |
| 147 | 48 | |
| 171 | 41 | |
| 179 | 43 | |
| 182 | 64 | |
| 187 | 84 | |
| 188 | 49 | |
| 231 | 49 | |
| 246 | 50 | |
| 247 | 72 | 4,0 |
| 248 | 87 | 1,2 |
| 249 | 80 | 3,5 |

## Patentansprüche

1. Substituierte 1,4,8-Triazaspiro[4.5]decan-2-on-Verbindungen der allgemeinen Formel I, 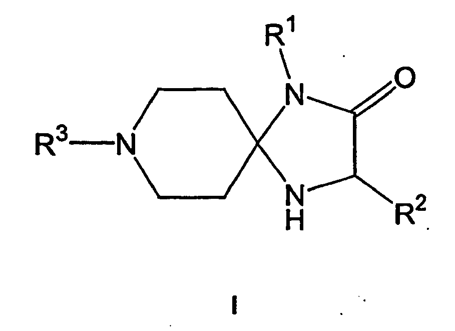 worin
R¹ für einen Wasserstoff-Rest, für einen linearen oder verzweigten, unsubstituierten oder wenigstens einfach substituierten, ggf. wenigstens ein Heteroatom als Kettenglied aufweisenden Alkyl-Rest, für einen linearen oder verzweigten, unsubstituierten oder wenigstens einfach substituierten, ggf. wenigstens ein Heteroatom als Kettenglied aufweisenden Alkenyl-Rest, für einen linearen oder verzweigten, unsubstituierten oder wenigstens einfach substituierten, ggf. wenigstens ein Heteroatom als Kettenglied aufweisenden Alkinyl-Rest, für einen unsubstituierten oder wenigstens einfach substituierten Aryl- oder Heteroaryl-Rest, der über eine lineare oder verzweigte, ggf. wenigstens ein Heteroatom als Kettenglied aufweisende Alkylen-Gruppe gebunden ist, oder für einen über eine lineare oder verzweigte Alkylen-Gruppe gebundenen -C(=O)-OR⁷-Rest steht,
R² für einen Wasserstoff-Rest, für einen linearen oder verzweigten, unsubstituierten oder wenigstens einfach substituierten, ggf. wenigstens ein Heteroatom als Kettenglied aufweisenden Alkyl-Rest, für einen linearen oder verzweigten, unsubstituierten oder wenigstens einfach substituierten, ggf. wenigstens ein Heteroatom als Kettenglied aufweisenden Alkenyl-Rest, für einen linearen oder verzweigten, unsubstituierten oder wenigstens einfach substituierten, ggf. wenigstens ein Heteroatom als Kettenglied aufweisenden Alkinyl-Rest, oder für einen unsubstituierten oder wenigstens einfach substituierten Aryl- oder Heteroaryl-Rest steht, der über eine lineare oder verzweigte, ggf. wenigstens ein Heteroatom als Kettenglied aufweisende Alkylen-Gruppe gebunden sein kann,
R³ für eine -S(=O)₂R⁴-Gruppe, für eine -C(=S)-NH-R⁵-Gruppe oder für eine -C(=O)-NH-R⁶-Gruppe steht,
R⁴ für einen -NR¹⁰R¹¹-Rest, für einen linearen oder verzweigten, unsubstituierten oder wenigstens einfach substituierten, ggf. wenigstens ein Heteroatom als Kettenglied aufweisenden Alkyl-Rest, für einen linearen oder verzweigten, unsubstituierten oder wenigstens einfach substituierten, ggf. wenigstens ein Heteroatom als Kettenglied aufweisenden Alkenyl-Rest, für einen linearen oder verzweigten, unsubstituierten oder wenigstens einfach substituierten, ggf. wenigstens ein Heteroatom als Kettenglied aufweisenden Alkinyl-Rest, für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-Gruppe gebunden und/oder mit einem unsubstituierten oder wenigstens einfach substituierten monozyklischen Ringsystem kondensiert sein kann, oder für einen unsubstituierten oder wenigstens einfach substituierten, ggf. wenigstens ein Heteroatom als Ringglied aufweisenden, cycloaliphatischen Rest steht, der über eine lineare oder verzweigte, unsubstituierte oder wenigstens einfach substituierte, ggf. wenigstens ein Heteroatom als Kettenglied aufweisende Alkylen-Gruppe gebunden und/oder mit einer linearen oder verzweigten, unsubstituierten oder wenigstens einfach substituierten Alkylen-Gruppe überbrückt sein kann,
R⁵ für einen linearen oder verzweigten, unsubstituierten oder wenigstens einfach substituierten, ggf. wenigstens ein Heteroatom als Kettenglied aufweisenden Alkyl-Rest, für einen linearen oder verzweigten, unsubstituierten oder wenigstens einfach substituierten, ggf. wenigstens ein Heteroatom als Kettenglied aufweisenden Alkenyl-Rest, für einen linearen oder verzweigten, unsubstituierten oder wenigstens einfach substituierten, ggf. wenigstens ein Heteroatom als Kettenglied aufweisenden Alkinyl-Rest, für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-Gruppe gebunden sein kann, für einen unsubstituierten oder wenigstens einfach substituierten, ggf. wenigstens ein Heteroatom als Ringglied aufweisenden, cycloaliphatischen Rest steht, der über eine lineare oder verzweigte, unsubstituierte oder wenigstens einfach substituierte, ggf. wenigstens ein Heteroatom als Kettenglied aufweisende Alkylen-Gruppe gebunden sein kann, für einen -C(=O)-OR⁸-Rest oder für einen über eine lineare oder verzweigte Alkylen-Gruppe gebundenen -C(=O)-OR⁹-Rest steht,
R⁶ für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-Gruppe gebunden sein kann, oder für einen unsubstituierten oder wenigstens einfach substituierten, ggf. wenigstens ein Heteroatom als Ringglied aufweisenden, cycloaliphatischen Rest steht, der über eine lineare oder verzweigte, unsubstituierte oder wenigstens einfach substituierte, ggf. wenigstens ein Heteroatom als Kettenglied aufweisende Alkylen-Gruppe gebunden sein kann,
R⁷, R⁸, R⁹, R¹⁰, R¹¹, unabhängig voneinander, jeweils für einen linearen oder verzweigten Alkyl-Rest, einen linearen oder verzweigten Alkenyl-Rest oder einen linearen oder verzweigten Alkinyl-Rest stehen,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

2. Verbindungen gemäß Anspruch 1, **dadurch gekennzeichnet, daß** R¹ für einen Wasserstoff-Rest, für einen linearen oder verzweigten, unsubstituierten oder wenigstens einfach substituierten, ggf. wenigstens ein Heteroatom als Kettenglied aufweisenden C₁₋₁₀-Alkyl-Rest, für einen linearen oder verzweigten, unsubstituierten oder wenigstens einfach substituierten, ggf. wenigstens ein Heteroatom als Kettenglied aufweisenden C₂₋₁₀-Alkenyl-Rest, für einen linearen oder verzweigten, unsubstituierten oder wenigstens einfach substituierten, ggf. wenigstens ein Heteroatom als Kettenglied aufweisenden C₂₋₁₀-Alkinyl-Rest, für einen unsubstituierten oder wenigstens einfach substituierten, 5- bis 14-gliedrigen Aryl- oder Heteroaryl-Rest, der über eine lineare oder verzweigte, ggf. wenigstens ein Heteroatom als Kettenglied aufweisende C₁₋₅-Alkylen-Gruppe gebunden ist, oder für einen über eine lineare oder verzweigte C₁₋₅-Alkylen-Gruppe gebundenen -C(=O)-OR⁷-Rest steht,
vorzugsweise für einen Wasserstoff-Rest, für einen linearen oder verzweigten, unsubstituierten C₁₋₅-Alkyl-Rest, für einen linearen oder verzweigten, unsubstituierten C₂₋₅-Alkenyl-Rest, für einen linearen oder verzweigten, unsubstituierten C₂₋₅-Alkinyl-Rest, für einen unsubstituierten oder wenigstens einfach substituierten Phenyl- oder Naphthyl-Rest, der über eine lineare oder verzweigte, ggf. wenigstens ein Sauerstoffatom als Kettenglied aufweisende C₁-₅-Alkylen-Gruppe gebunden ist, oder für einen über eine lineare oder verzweigte C₁₋₃-Alkylen-Gruppe gebundenen -C(=O)-OR⁷-Rest steht,
besonders bevorzugt für einen Wasserstoff-Rest, für einen linearen oder verzweigten, unsubstituierten C₁₋₄-Alkyl-Rest, für einen linearen oder verzweigten, unsubstituierten C₂₋₃-Alkenyl-Rest, für einen linearen oder verzweigten, unsubstituierten C₂₋₃-Alkinyl-Rest, für einen Phenyl- oder Naphthyl-Rest, der über eine -(CH₂)-, -(CH₂)₂, -(CH₂)₃- oder -(CH₂)₂-O-Brücke gebunden und ggf. einfach oder mehrfach, gleich oder verschieden, mit einem Substituenten ausgewählt aus der Gruppe bestehend aus F, Cl, Br, CN, NO₂, C₁₋₅-Alkyl, C₁₋₅-Alkoxy, -C(=O)-C₁₋₅-Alkyl, -C(=O)-O-C₁₋₅-Alkyl, -S(=O)₂-C₁₋₆ alkyl, -C(=O)- C₁₋₅-Perfluoroalkyl, -CF₃, CHF₂ und CH₂F substituiert ist, oder für einen über eine -(CH₂)-Gruppe gebundenen -C(=O)-OR⁷-Rest steht.

3. Verbindungen gemäß Anspruch 1 oder 2, **dadurch gekennzeichnet, daß** der Rest R² für einen Wasserstoff-Rest, für einen linearen oder verzweigten, unsubstituierten oder wenigstens einfach substituierten, ggf. wenigstens ein Heteroatom als Kettenglied aufweisenden C₁₋₁₀-Alkyl-Rest, für einen linearen oder verzweigten, unsubstituierten oder wenigstens einfach substituierten, ggf. wenigstens ein Heteroatom als Kettenglied aufweisenden C₂₋₁₀-Alkenyl-Rest, für einen linearen oder verzweigten, unsubstituierten oder wenigstens einfach substituierten, ggf. wenigstens ein Heteroatom als Kettenglied aufweisenden C₂₋₁₀-Alkinyl-Rest, oder für einen unsubstituierten oder wenigstens einfach substituierten, 5- bis 14-gliedrigen Aryl- oder Heteroaryl-Rest steht, der über eine lineare oder verzweigte, ggf. wenigstens ein Heteroatom als Kettenglied aufweisende C₁₋₅-Alkylen-Gruppe gebunden sein kann,
vorzugsweise für einen Wasserstoff-Rest, für einen linearen oder verzweigten, unsubstituierten ggf. wenigstens ein Sauerstoffatom und/oder wenigstens ein Schwefelatom als Kettenglied aufweisenden C₁₋₅-Alkyl-Rest oder für einen Phenyl- oder Naphthyl-Rest- steht, der einfach oder mehrfach, gleich oder verschieden, mit einem Substituenten ausgewählt aus der Gruppe bestehend aus F, Cl, Br, CN, NO₂, C₁₋₅-Alkyl, C₁₋₅-Alkoxy, -C(=O)-C₁₋₅-Alkyl, -C(=O)-O-C₁₋₅-Alkyl, -S(=O)₂-C₁₋₆-alkyl. -C(=O)- C₁₋₅-Perfluoroalkyl, -CF₃, CHF₂. und CH₂F substituiert und/oder über eine -(CH₂)-, -(CH₂)₂-, -(CH₂)₃- oder -(CH₂)₂-O-Brücke gebunden sein kann,
besonders bevorzugt für einen Wasserstoff-Rest, für einen linearen oder verzweigten, unsubstituierten, ggf. wenigstens ein Schwefelatom als Kettenglied aufweisenden C₁₋₅-Alkyl-Rest oder für einen Phenyl-Rest steht, wobei der Phenyl-Rest einfach oder mehrfach, gleich oder verschieden, mit einem Substituenten ausgewählt aus der Gruppe bestehend aus F, Cl, Br, CN, NO₂, C₁₋₅-Alkyl, C₁₋₅-Alkoxy, -C(=O)-C₁₋₅-Alkyl, -C(=O)-O-C₁₋₅-Alkyl, -S(=O)₂-C₁-₆-alkyl, -C(=O)- C₁₋₅-Perfiuoroalkyl, -CF₃, CHF₂ und CH₂F substituiert und/oder über eine -(CH₂)-Brücke gebunden sein kann.

4. Verbindungen gemäß einem oder mehreren der Ansprüche 1-3, **dadurch gekennzeichnet, daß** R⁴ für einen -NR¹⁰R¹¹-Rest, für einen linearen oder verzweigten, unsubstituierten oder wenigstens einfach substituierten, ggf. wenigstens ein Heteroatom als Kettenglied aufweisenden C₁₋₁₀-Alkyl-Rest, für einen linearen oder verzweigten, unsubstituierten oder wenigstens einfach substituierten, ggf. wenigstens ein Heteroatom als Kettenglied aufweisenden C₂₋₁₀-Alkenyl-Rest, einen linearen oder verzweigten, unsubstituierten oder wenigstens einfach substituierten, ggf. wenigstens ein Heteroatom als Kettenglied aufweisenden C₂₋₁₀-Alkinyl-Rest, für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte, ggf. wenigstens ein Heteroatom als Kettenglied aufweisende C₁₋₅-Alkylen-Gruppe gebunden und/oder mit einem 5- oder 6-gliedrigen monozyklischen Ringsystem kondensiert sein kann, oder für einen unsubstituierten oder wenigstens einfach substituierten, ggf. wenigstens ein Heteroatom als Ringglied aufweisenden, C₃₋₈-cycloaliphatischen Rest steht, der über eine lineare oder verzweigte, unsubstituierte oder wenigstens einfach substituierte, ggf. wenigstens ein Heteroatom als Kettenglied aufweisende C₁-₅-Alkylen-Gruppe gebunden und/oder mit einer linearen oder verzweigten, unsubstituierten oder wenigstens einfach substituierten C₁₋₅-Alkylen-Gruppe überbrückt sein kann,
vorzugsweise für einen -NR¹⁰R¹¹-Rest, für einen linearen oder verzweigten, unsubstituierten, ggf. wenigstens ein Sauerstoffatom und/oder wenigstens ein Schwefelatom als Kettenglied aufweisenden C₁₋₄-Alkyl-Rest, für einen Furyl-(Furanyl-)-Rest, Thienyl-(Thiophenyl-)-Rest, Phenyl-Rest, Naphthyl-Rest oder 1,2,3,4-Tetrahydro-isochinolin-Rest steht, wobei der zyklische Rest jeweils einfach oder mehrfach, gleich oder verschieden, mit einem Substituenten ausgewählt aus der Gruppe bestehend aus F, Cl, Br, CN, NO₂, C₁-₅-Alkyl, C₁-₅-Alkoxy, -C(=O)-C₁-₅-Alkyl, -C(=O)-O-C₁-₅-Alkyl, -S(=O)₂-C₁-₆-alkyl, -C(=O)- C₁-₅. Perfluoroalkyl, -CF₃, CHF₂ und CH₂F substituiert und/oder über eine -(CH₂)-, -(CH₂)₂- oder -(CH₂)₃-Brücke gebunden sein kann, oder für einen unsubstituierten oder wenigstens einfach mit einer oxo-Gruppe substituierten, ggf. wenigstens ein Heteroatom als Ringglied aufweisenden, C₅- oder C₆-cycloaliphatischen Rest steht, der über eine -(CH₂)-, -(CH₂)₂- oder -(CH₂)₃-Brücke gebunden und/oder einer-(C(CH₃)₂)-Gruppe überbrückt sein kann,
besonders bevorzugt für einen -N(CH₃)₂-Rest, für einen linearen oder verzweigten, unsubstituierten C₁-₄-Alkyl-Rest, für einen Thienyl-(Thiophenyl-)-, Phenyl-, oder 1,2,3,4-Tetrahydro-isochinolin-Rest steht, wobei der zyklische Rest jeweils einfach oder mehrfach, gleich oder verschieden, mit einem Substituenten ausgewählt aus der Gruppe bestehend aus F, Cl, Br, CN, NO₂, C₁-₅-Alkyl, C₁₋₅-Alkoxy, -C(=O)-C₁-₅-Alkyl, -C(=O)-O-C₁-₅-Alkyl, -S(=O)₂-C₁₋₆ alkyl, -C(=O)- C₁-₅-Perfluoroalkyl, -CF₃, CHF₂ und CH₂F substituiert und/oder über eine -(CH₂)-Brücke gebunden sein kann, oder für einen unsubstituierten oder wenigstens einfach mit einer oxo-Gruppe substituierten C₅- oder C₆-cycloaliphatischen Rest steht, der über eine -(CH₂)-Brücke gebunden und/oder mit einer -(C(CH₃)₂)-Gruppe überbrückt sein kann.

5. Verbindungen gemäß einem oder mehreren der Ansprüche 1 bis 4, **dadurch gekennzeichnet, daß** der Rest R⁵ für einen linearen oder verzweigten, unsubstituierten oder wenigstens einfach substituierten, ggf. wenigstens ein Heteroatom als Kettenglied aufweisenden C₁-₁₀-Alkyl-Rest, für einen linearen oder verzweigten, unsubstituierten oder wenigstens einfach substituierten, ggf. wenigstens ein Heteroatom als Kettenglied aufweisenden C₂-₁₀-Alkenyl-Rest, für einen linearen oder verzweigten, unsubstituierten oder wenigstens einfach substituierten, ggf. wenigstens ein Heteroatom als Kettenglied aufweisenden C₂-₁₀-Alkinyl-Rest, für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte, ggf. wenigstens ein Heteroatom als Kettenglied aufweisende C₁-₅-Alkylen-Gruppe gebunden sein kann, für einen unsubstituierten oder wenigstens einfach substituierten, ggf. wenigstens ein Heteroatom als Ringglied aufweisenden, C₃-₈-cycloaliphatischen Rest steht, der über eine lineare oder verzweigte, unsubstituierte oder wenigstens einfach substituierte, ggf. wenigstens ein Heteroatom als Kettenglied aufweisende C₁-₅-Alkylen-Gruppe sein kann, für einen -C(=O)-OR⁸-Rest oder für einen über eine lineare oder verzweigte C₁₋₅-Alkylen-Gruppe gebundenen -C(=O)-OR⁹-Rest steht, vorzugsweise für einen linearen oder verzweigten, ggf. wenigstens ein Sauerstoffatom und/oder wenigstens ein Schwefelatom als Kettenglied aufweisenden C₁₋₅-Alkyl-Rest, für einen linearen oder verzweigten, ggf. wenigstens ein Sauerstoffatom und/oder wenigstens ein Schwefelatom als Kettenglied aufweisenden C₂-₅-Alkenyl-Rest, für einen linearen oder verzweigten, ggf. wenigstens ein Sauerstoffatom und/oder wenigstens ein Schwefelatom als Kettenglied aufweisenden C₂-₅-Alkinyl-Rest, für einen Furyl-(Furanyl-)-Rest, Thienyl-(Thiophenyl-)-Rest, Phenyl- oder Naphthyl-Rest steht, wobei der zyklische Rest jeweils einfach oder mehrfach, gleich oder verschieden, mit einem Substituenten ausgewählt aus der Gruppe bestehend aus F, Cl, Br, CN, NO₂, C₁₋₅-Alkyl, C₁₋₅-Alkoxy, -C(=O)-C₁₋₅-Alkyl, -C(=O)-O-C₁₋₅-Alkyl, -S(=O)₂-C₁-₆-alkyl, -C(=O)- C₁-₅-Perfluoroalkyl, -CF₃, CHF₂ und CH₂F substituiert und/oder über eine -(CH₂)-, -(CH(CH₃))-, -(CH₂)₂- oder -(CH₂)₃-Brücke gebunden sein kann, für einen unsubstituierten, ggf. wenigstens ein Sauerstoffatom als Ringglied aufweisenden, C₄-, C₅- oder C₆-cycloaliphatischen Rest steht, der über eine -(CH₂)-, -(CH(CH₃))-, -(CH₂)₂- oder -(CH₂)₃-Brücke gebunden sein kann, für einen -C(=O)-O-R⁸-Rest oder für einen über eine -(CH₂)-, -(CH(CH₃))-, -(CH₂)₂- oder -(CH₂)₃-Brücke gebundenen -C(=O)-OR⁹-Rest steht,
besonders bevorzugt für einen linearen oder verzweigten, unsubstituierten, ggf. wenigstens ein Sauerstoffatom als Kettenglied aufweisenden C₁₋₃-Alkyl-Rest, für einen linearen oder verzweigten, unsubstituierten C₂₋₃-Alkenyl-Rest, für einen linearen oder verzweigten, unsubstituierten C₂₋₃-Alkinyl-Rest, für einen Phenyl-Rest steht, wobei der Phenyl-Rest einfach oder mehrfach, gleich oder verschieden, mit einem Substituenten ausgewählt aus der Gruppe bestehend aus F, Cl, Br, CN, NO₂, C₁₋₅-Alkyl, C₁₋₅-Alkoxy, -C(=O)-C₁₋₅-Alkyl, -C(=O)-O-C₁₋₅-Alkyl, -S(=O)₂-C₁₋₆-alkyl, -C(=O)- C₁₋₅-Perfluoroalkyl, -CF₃, CHF₂ und CH₂F substituiert und/oder über eine -(CH₂)-, -(CH(CH₃))-, -(CH₂)₂- oder -(CH₂)₃-Brücke gebunden sein kann, für einen unsubstituierten, ggf. wenigstens ein Sauerstoffatom als Ringglied aufweisenden, C₅- oder C₆-cycloaliphatischen Rest steht, der über eine -(CH₂)-, -(CH(CH₃))-, -(CH₂)₂- oder -(CH₂)₃-Brücke gebunden sein kann, für einen C(=O)-O-C₂H₅-Rest oder für einen über eine -(CH₂)-, -(CH(CH₃))-, -(CH₂)₂- oder -(CH₂)₃-Brücke gebundenen -C(=O)-O-R⁹-Rest steht.

6. Verbindungen gemäß einem oder mehreren der Ansprüche 1-5, **dadurch gekennzeichnet, daß** der Rest R⁶ für einen unsubstituierten oder wenigstens einfach substituierten, 5- bis 14-gliedrigen Aryl- oder Heteroaryl-Rest, der über eine lineare oder verzweigte, unsubstituierte oder wenigstens einfach substituierte, ggf. wenigstens ein Heteroatom als Kettenglied aufweisende C₁₋₅-Alkylen-Gruppe gebunden sein kann, oder für einen unsubstituierten oder wenigstens einfach substituierten, ggf. wenigstens ein Heteroatom als Ringglied aufweisenden, C₃₋₈-cycloaliphatischen Rest steht, der über eine lineare oder verzweigte, unsubstituierte oder wenigstens einfach substituierte, ggf. wenigstens ein Heteroatom als Kettenglied aufweisende C₁₋₅-Alkylen-Gruppe sein kann,
vorzugsweise für einen Phenyl-Rest steht, wobei der Phenyl-Rest einfach oder mehrfach, gleich oder verschieden, mit einem Substituenten ausgewählt aus der Gruppe bestehend aus F, Cl, Br, CN, NO₂, C₁₋₅-Alkyl, C₁₋₅-Alkoxy, -C(=O)-C₁₋₅-Alkyl, -C(=O)-O-C₁₋₅-Alkyl, -S(=O)₂-C₁₋₆-alkyl, -C(=O)- C₁₋₅-Perfluoroalkyl, -CF₃, CHF₂ und CH₂F substituiert und/oder über eine -(CH₂)-, -(CH(CH₃))-, (-CH₂)₂- oder -(CH₂)₃-Brücke gebunden sein kann, oder für einen unsubstituierten C₅- oder C₆-cycloaliphatischen Rest steht, der über eine -(CH₂)-, -(CH(CH₃))-, -(CH₂)₂- oder-(CH₂)₃-Brücke gebunden sein kann,
besonders bevorzugt für einen Phenyl-Rest steht, wobei der Phenyl-Rest einfach oder mehrfach, gleich oder verschieden, mit einem Substituenten ausgewählt aus der Gruppe bestehend aus F, Cl, Br, CN, NO₂, C₁₋₅-Alkyl, C₁₋₅-Alkoxy, -C(=O)-C₁₋₅-Alkyl, -C(=O)-O-C₁₋₅-Alkyl, -S(=O)₂-C₁₋₆alkyl, -C(=O)- C₁₋₅-Perfluoroalkyl, -CF₃, CHF₂ und CH₂F substituiert und/oder über eine -(CH₂)- oder (CH₂)₂-Brücke gebunden sein kann, oder für einen Cyclohexyl-Rest steht, der über eine -(CH₂)- oder -(CH₂)₂-Brücke gebunden sein kann.

7. Verbindungen gemäß einem oder mehreren der Ansprüche 1-6, **dadurch gekennzeichnet, daß** die Reste R⁷, R⁸, R⁹, R¹⁰, R¹¹, jeweils unabhängig voneinander, für einen linearen oder verzweigten C₁₋₅-Alkyl-Rest, einen linearen oder verzweigten C₂₋₅-Alkenyl-Rest oder einen linearen oder verzweigten C₂₋₅-Alkinyl-Rest, vorzugsweise für einen linearen oder ggf. verzweigten Methyl-, Ethyl-, n-Propyl oder iso-Propyl-Rest stehen.

8. Verbindungen gemäß einem oder mehreren der Ansprüche 1-7, **dadurch gekennzeichnet, daß**
R¹ für einen Wasserstoff-Rest, für einen Alkyl-Rest ausgewählt aus der Gruppe bestehend aus Methyl, Ethyl, n-Propyl, iso-Propyl, n-Butyl, Isobutyl, sec-Butyl, und tert-Butyl, für einen Alkenyl-Rest ausgewählt aus der Gruppe bestehend aus Vinyl und Allyl, für einen Propinyl-Rest, für einen Phenyl- 1-Naphthyl oder 2-Naphthyl-Rest, wobei der zyklische Rest über eine -(CH₂)-, -(CH₂)₂, -(CH₂)₂- oder -(CH₂)₂-O-Brücke gebunden und ggf. einfach oder mehrfach, gleich oder verschieden, mit einem Substituenten ausgewählt aus der Gruppe bestehend aus F, Cl, Br, CN, NO₂, C₁₋₅-Alkyl, C₁₋₅-Alkoxy, -C(=O)-C₁₋₅-Alkyl, -C(=O)-O-C₁₋₅-Alkyl, -S(=O)₂-C₁₋₆-alkyl, -C(=O)- C₁₋₅-Perfluoroalkyl, -CF₃, CHF₂ und CH₂F substituiert ist, oder für einen über eine -(CH₂)-Gruppe gebundenen -C(=O)-OR⁷ -Rest steht,
R² für einen Wasserstoff-Rest, für einen linearen oder verzweigten, unsubstituierten, ggf. wenigstens ein Schwefelatom als Kettenglied aufweisenden Alkyl-Rest ausgewählt aus der Gruppe bestehend aus Methyl, Ethyl, n-Propyl, iso-Propyl, n-Butyl, Isobutyl, sec-Butyl, tert-Butyl und 2-Methylsulfanylethyl oder für einen Phenyl- oder Benzyl-Rest steht, der jeweils unsubstituiert oder einfach oder mehrfach, gleich oder verschieden, mit einem Substituenten ausgewählt aus der Gruppe bestehend aus F, Cl, Br, CN, NO₂, C₁₋₅-Alkyl, C₁₋₅-Alkoxy, -C(=O)-C₁₋₅-Alkyl, -C(=O)-O-C₁₋₅-Alkyl, -S(=O)₂-C₁₋₆-alkyl, -C(=O)- C₁₋₅-Perfluoroalkyl, -CF₃, CHF₂ und CH₂F substituiert sein kann,
R³ für eine -S(=O)₂-R⁴-Gruppe, für eine -C(=S)-NH-R⁵-Gruppe oder für eine -C(=O)-NH-R⁶-Gruppe steht,
R⁴ für einen -N(CH₃)₂-Rest, für einen Alkyl-Rest ausgewählt aus der Gruppe bestehend aus Methyl, Ethyl, n-Propyl, iso-Propyl, n-Butyl, Isobutyl, sec-Butyl, tert-Butyl, für einen Thienyl-(Thiophenyl-)-, Phenyl-, oder 1,2,3,4-Tetrahydro-isochinolin-Rest steht, wobei der zyklische Rest jeweils unsubstituiert oder einfach oder mehrfach, gleich oder verschieden, mit einem Substituenten ausgewählt aus der Gruppe bestehend aus F, Cl, Br, CN, NO₂, C₁₋₅-Alkyl, C₁₋₅-Alkoxy, -C(=o)-C₁₋₅-Alkyl, -C(=O)O-C₁₋₅-Alkyl, -S(=O)₂-C₁₋₆-alkyl, -C(=O)-C₁₋₅-Perfluoroalkyl, -CF₃, CHF₂ und CH₂F substituiert und/oder über eine -(CH₂)-Brücke gebunden sein kann, oder für einen 7,7-Dimethyl-2-oxo-bicyclo-[2.2.1]-hept-1-yl-methyl-Rest steht,
R⁵ für einen Alkyl-Rest ausgewählt aus der Gruppe bestehend aus Methyl, Ethyl, n-Propyl, iso-Propyl, n-Butyl, Isobutyl, sec-Butyl, tert-Butyl und 2-Methoxyethyl, für einen Alkenyl-Rest ausgewählt aus der Gruppe bestehend aus Vinyl und Allyl, für einen Propinyl-Rest, für einen Phenyl-Rest steht, wobei der Phenyl-Rest einfach oder mehrfach, gleich oder verschieden, mit einem Substituenten ausgewählt aus der Gruppe bestehend aus F, Cl, Br, CN, NO₂, C₁₋₅-Alkyl, C₁₋₅-Alkoxy, -C(=O)-C₁₋₅-Alkyl, -C(=O)-O-C₁₋₅-Alkyl, -S(=O)₂-C₁₋₆-alkyl, -C(=O)- C₁₋₅-Peffluoroalkyl, -CF₃, CHF₂ und CH₂F substituiert und/oder über eine -(CH₂)-, -(CH(CH₃))-, -(CH₂)₂- oder -(CH₂)₃-Brücke gebunden sein kann, für einen Cyclohexyl- oder Tetrahydrofuryl-Rest, der über eine -(CH₂)-Brücke gebunden sein kann, für einen C(=O)-O-C₂H₅-Rest oder für einen über eine -(CH₂)-, -(CH(CH₃))-, -(CH₂)₂- oder -(CH₂)₃-Brücke gebundenen -C(=O)-O-C₂H₅-Rest steht,
R⁶ für einen Phenyl-Rest, der unsubstituiert oder einfach oder mehrfach, gleich oder verschieden, mit einem Substituenten ausgewählt aus der Gruppe bestehend aus F, Cl, Br, CN, NO₂, C₁₋₅-Alkyl, C₁₋₅-Alkoxy, -C(=O)-C₁₋₅-Alkyl, -C(=O)-O-C₁₋₅-Alkyl, -S(=O)₂-C₁₋₆-alkyl, -C(=O)- C₁₋₅-Perfluoroalkyl, -CF₃, CHF₂ und CH₂F substituiert und/oder über eine -(CH₂)- oder (CH₂)₂-Brücke gebunden sein kann, oder für einen Cyclohexyl-Rest steht, der über eine -(CH₂)- oder -(CH₂)₂-Brücke gebunden sein kann,
R⁷, R⁸, R⁹, R¹⁰, R¹¹, unabhängig voneinander, jeweils für einen linearen oder verzweigten C₁₋₅-Alkyl-Rest, einen linearen oder ggf. verzweigten C₂₋₅-Alkenyl-Rest oder einen linearen oder verzweigten C₂₋₅-Alkinyl-Rest stehen, vorzugsweise für einen linearen oder verzweigten Methyl,-, Ethyl-, n-Propyl oder iso-Propyl-Rest stehen.
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

9. Verbindungen gemäß einem oder mehreren der Ansprüche 1 bis 8, **dadurch gekennzeichnet, dass**
R¹ für einen Wasserstoff-Rest steht;
für einen jeweils linearen oder verzweigten, ggf. substituierten C₁₋₁₀ Alkyl-Rest, C₂₋₁₀ Alkenyl-Rest oder C₂₋₁₀ Alkinyl-Rest steht;
für einen ggf. substituierten 5- bis 14-gliedrigen Aryl- oder Heteroaryl-Rest steht;
für-(CH₂)ₐₐ-C(=O)-O-R⁷ mit aa = 1, 2, 3, 4 oder 5 steht;
oder für -(CH₂)-Uₐ-(CH₂)_{b}-V_{c}-(CH₂)_{d}-R¹² mit a = 0 oder 1, b = 0 oder 1, c = 0 oder 1 und d = 0 oder 1 steht, worin U und V unabhängig voneinander jeweils für O, S, NH, N(CH₃) oder N(C₂H₅) stehen;
R² für einen Wasserstoff-Rest steht;
für einen jeweils linearen oder verzweigten, ggf. substituierten C₁₋₁₀ Alkyl-Rest, C₂₋₁₀ Alkenyl-Rest oder C₂₋₁₀ Alkinyl-Rest steht;
für einen ggf. substituierten 5- bis 14-gliedrigen Aryl- oder Heteroaryl-Rest steht;
oder für -(CH₂)-Wₑ-(CH₂)_{f}-X_{g}-(CH₂)ₕ-R¹³ mit e = 0 oder 1, f = 0 oder 1, g = 0 oder 1 und h = 0 oder 1 steht, worin W und X unabhängig voneinander jeweils für O, S, NH, N(CH₃) oder N(C₂H₅) stehen;
R³ für eine -S(=O)₂-R⁴-Gruppe, für eine -C(=S)-NH-R⁵-Gruppe oder für eine -C(=O)-NH-R⁶-Gruppe steht,
R⁴ für einen Rest -NR¹⁰R¹¹ steht;
für einen jeweils linearen oder verzweigten, ggf. substituierten C₁₋₁₀ Alkyl-Rest, C₂₋₁₀ Alkenyl-Rest oder C₂₋₁₀ Alkinyl-Rest steht;
für einen ungesättigten oder gesättigten, ggf. substituierten 3-, 4-, 5-, 6-, 7-, 8- oder 9-gliedrigen cycloaliphatischen Rest steht, der mit einer oder zwei linearen oder verzweigten, ggf. substituierten C₁₋₅-Alkylen-Gruppe überbrückt sein kann,
für einen ggf. substituierten 5- bis 14-gliedrigen Aryl- oder Heteroaryl-Rest steht, der mit einem gesättigten oder ungesättigten, ggf. substituierten monozyklischen Ringsystem kondensiert sein kann;
oder für -(CH₂)-Yₖ-(CH₂)ₘ-Zₙ-(CH₂)ₚ-R¹⁴ mit k = 0 oder 1, m = 0 oder 1, n = 0 oder 1 und p = 0 oder 1 steht, worin Y und Z unabhängig voneinander jeweils für O, S, NH, N(CH₃) oder N(C₂H₅) stehen;
R⁵ für einen jeweils linearen oder verzweigten, ggf. substituierten C₁₋₁₀ Alkyl-Rest, C₂₋₁₀ Alkenyl-Rest oder C₂₋₁₀ Alkinyl-Rest steht;
für einen ungesättigten oder gesättigten, ggf. substituierten 3-,4-, 5-. 6-, 7-, 8- oder 9-gliedrigen cycloaliphatischen Rest steht;
für einen ggf. substituierten 5- bis 14-gliedrigen Aryl- oder Heteroaryl-Rest steht;
für -(CHR¹⁵)-A_{q}-(CH₂)ᵣ-Bₛ-(CH₂)ₜ-R¹⁶ mit q = 0 oder 1, r = 0 oder 1, s = 0 oder 1 und t = 0 oder 1 steht, worin A und B unabhängig voneinander jeweils für O, S, NH, N(CH₃) oder N(C₂H₅) stehen;
für einen -C(=O)-OR⁸-Rest steht;
oder für einen -(CHR¹⁷)-(CH₂)ᵥ-C(=O)-OR⁹-Rest steht, worin v für 0,1, 2 oder 3 steht;
R⁶ für einen ungesättigten oder gesättigten, ggf. substituierten 3-, 4-, 5-, 6-, 7-, 8- oder 9-gliedrigen cycloaliphatischen Rest steht;
für einen ggf. substituierten 5- bis 14-gliedrigen Aryl- oder Heteroaryl-Rest steht;
oder für -(CH₂)-D_{w}-(CH₂)ₓ-E_{y}-(CH₂)_{z}-R¹⁸ mit w = 0 oder 1, x = 0 oder 1, y = 0 oder 1 und z = 0 oder 1 steht, worin D und E unabhängig voneinander jeweils für O, S, NH, N(CH₃) oder N(C₂H₅) stehen;
R⁷ R⁸, R⁹, R¹⁰ und R¹¹ unabhängig voneinander jeweils
für einen jeweils linearen oder verzweigten, ggf. substituierten C₁₋₁₀ Alkyl-Rest, C₂₋₁₀ Alkenyl-Rest oder C₂₋₁₀ Alkinyl-Rest stehen;
R¹² und R¹³ unabhängig voneinander jeweils
für einen jeweils linearen oder verzweigten, ggf. substituierten C₁₋₁₀Alkyl-Rest, C₂₋₁₀ Alkenyl-Rest oder C₂₋₁₀ Alkinyl-Rest stehen;
oder für einen ggf. substituierten 5- bis 14-gliedrigen Aryl- oder Heteroaryl-Rest stehen;
R¹⁴ für einen jeweils linearen oder verzweigten, ggf. substituierten C₁₋₁₀ Alkyl-Rest, C₂₋₁₀ Alkenyl-Rest oder C₂₋₁₀ Alkinyl-Rest steht;
für einen ungesättigten oder gesättigten, ggf. substituierten 3-, 4-, 5-, 6-, 7-, 8- oder 9-gliedrigen cycloaliphatischen Rest steht, der mit einer oder zwei linearen oder verzweigten, ggf. substituierten C₁₋₅-Alkylen-Gruppe überbrückt sein kann,
oder für einen ggf. substituierten 5- bis 14-gliedrigen Aryl- oder Heteroaryl-Rest steht, der mit einem gesättigten oder ungesättigten, ggf. substituierten monozyklischen Ringsystem kondensiert sein kann;
R¹⁵ für einen Wasserstoff-Rest steht
für einen jeweils linearen oder verzweigten, ggf. substituierten C₁₋₁₀ Alkyl-Rest, C₂₋₁₀ Alkenyl-Rest oder C₂₋₁₀ Alkinyl-Rest steht;
R¹⁶ für einen jeweils linearen oder verzweigten, ggf. substituierten C₁₋₁₀Alkyl-Rest, C₂₋₁₀ Alkenyl-Rest oder C₂₋₁₀ Alkinyl-Rest steht;
für einen ungesättigten oder gesättigten, ggf. substituierten 3-, 4-, 5-, 6-, 7-, 8- oder 9-gliedrigen cycloaliphatischen Rest steht;
oder für einen ggf. substituierten 5- bis 14-gliedrigen Aryl- oder Heteroaryl-Rest steht;
R¹⁷ für einen jeweils linearen oder verzweigten, ggf. substituierten C₁₋₁₀ Alkyl-Rest, C₂₋₁₀ Alkenyl-Rest oder C₂₋₁₀ Alkinyl-Rest steht;
und
R¹⁸ für einen ungesättigten oder gesättigten, ggf. substituierten 3-, 4-, 5-, 6-, 7-, 8- oder 9-gliedrigen cycloaliphatischen Rest steht,
oder für einen ggf. substituierten 5- bis 14-gliedrigen Aryl- oder Heteroaryl-Rest steht;
wobei
die vorstehend genannten C₁₋₁₀ Alkyl-Reste unsubstituiert oder jeweils mit 1, 2, 3, 4 oder 5 Substituenten unabhängig voneinander ausgewählt aus der Gruppe bestehend aus F, Cl, Br, I, -CN, -NO₂, -OH, -SH, -NH₂ und -N(C₁₋₅-Alkyl)(C₁₋₅-Alkyl) substituiert sein können;
die vorstehend genannten C₂₋₁₀ Alkenyl-Reste unsubstituiert oder jeweils mit 1, 2, 3, 4 oder 5 Substituenten unabhängig voneinander ausgewählt aus der Gruppe bestehend aus F, Cl, Br, I, -CN, -NO₂, -OH, -SH, -NH₂ und -N(C₁₋₅-Alkyl)(C₁₋₅-Alkyl) substituiert sein können;
die vorstehend genannten C₂₋₁₀ Alkinyl-Reste unsubstituiert oder jeweils mit 1, 2, 3, 4 oder 5 Substituenten unabhängig voneinander ausgewählt aus der Gruppe bestehend aus F, Cl, Br, I, -CN, -NO₂, -OH, -SH, -NH₂ und -N(C₁₋₅-Alkyl)(C₁₋₅-Alkyl) substituiert sein können;
die vorstehend genannten C₁₋₅-Alkylen-Gruppen unsubstituiert oder jeweils mit 1, 2, 3, 4 oder 5 Substituenten unabhängig voneinander ausgewählt aus der Gruppe bestehend aus F, Cl, Br, -OH, -SH, -NH₂, -CN, NO₂ und Phenyl substituiert sein können,
die vorstehend genannten cycloaliphatischen Reste unsubstituiert oder jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-H, -C(=O)-C₁₋₅-Alkyl, -C(=O)-C₁₋₅-Perflouralkyl, -C(=O)-OH, -C(=O)-O-C₁₋₅-Alkyl, -(CH₂)-C(=O)-OH, -(CH₂)-C(=O)-O-C₁₋₅-Alkyl, -NH-C₁₋₅-Alkyl, -N(C₁₋₅-Alkyl)₂, -(CH₂)-Benzo[b]furanyl, -O-Phenyl, -O-Benzyl, Phenyl, Benzyl, Naphthyl und -(CH₂)-Naphthyl substituiert sein können, wobei jeweils der zyklische Teil der Reste -O-Phenyl, -O-Benzyl, Phenyl, -(CH₂)-Benzo[b]furanyl, Benzyl, Naphthyl und -(CH₂)-Naphthyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann,
und die vorstehend genannten cycloaliphatischen Reste jeweils 1, 2, 3, 4 oder 5 Heteroatom(e) unabhängig voneinander ausgewählt aus der Gruppe bestehend aus Sauerstoff, Stickstoff und Schwefel aufweisen können; der Ring der vorstehend genannten monozyklischen Ringsysteme unsubstituiert oder mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -O-C₂₋₅-Alkenyl, -NH₂, -NO₂, -O-CF₃, -O-CHF₂, -O-CH₂F, -S-CF₃, -S-CHF₂, -S-CH₂F, -SH, -S-C₁₋₅-Alkyl, -C₁₋₅-Alkyl, -C(=O)-OH, -C(=OFO-C₁₋₅-Alkyl, -O-C(=O)-C₁₋₅-Alkyl, -O-C(=O)-Phenyl, -(CH₂)-O-C(=O)-C₁₋₅-Alkyl, -(CH₂)-O-C(=O)-Phenyl, -NH-C₁₋₅-Alkyl, -N(C₁₋₅-Alkyl)₂, -NH-C(=O)-O-C₁₋₅-Alkyl, -NH-C(=O)-C₁₋₅-Alkyl, -C(=O)-H, -C(=O)-C₁₋₅-Alkyl, -C(=O)-C₁₋₅-Perflouralkyl, -C(=O)-NH₂, -C(=O)-NH-C₁₋₅-Alkyl, C(=O)-N-(C₁₋₅-Alkyl)₂, -S(=O)₂-NH₂, -S(=O)₂-NH-C₁₋₅-Alkyl, -S(=O)₂-NH-Phenyl, -S(=O)₂-C₁₋₅-Alkyl, -(CH₂)-Benzo[b]furanyl, -O-Phenyl, -S-Phenyl, -S-Benzyl, -O-Benzyl, Phenyl und Benzyl substituiert sein kann, wobei jeweils der zyklische Teil der Reste -O-C(=O)-Phenyl, -(CH₂)-O-C(=O)-Phenyl, -S(=O)₂-NH-Phenyl, -O-Phenyl, -S-Phenyl, -S-Benzyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann,
und der Ring der vorstehend genannten monozyklischen Ringsysteme jeweils 5-, 6- oder 7-gliedrig ist und jeweils 1, 2, 3, 4 oder 5 Heteroatom(e) als Ringglied(er) aufweisen kann, die unabhängig voneinander aus der Gruppe bestehend aus Sauerstoff, Stickstoff und Schwefel ausgewählt sind;
und die vorstehend genannten Aryl- oder Heteroaryl-Reste unsubstituiert oder jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -O-C₂₋₅-Alkenyl, -NH₂, -NO₂, -O-CF₃, -O-CHF₂, -O-CH₂F, -S-CF₃, -S-CHF₂, -S-CH₂F, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl, -O-C(=O)-Phenyl, -(CH₂)-O-C(=O)-C₁₋₅-Alkyl, -(CH₂)-O-C(=O)-Phenyl, -NH-C₁₋₅-Alkyl, -N(C₁₋₅-Alkyl)₂, -NH-C(=O)-O-C₁₋₅-Alkyl, -NH-C(=O)-C₁₋₅-Alkyl, -C(=O)-H, -C(=O)-C₁₋₅-Alkyl, -C(=O)-C₁₋₅-Perflouralkyl, -C(=O)-NH₂, -C(=O)-NH-C₁₋₅-Alkyl, C(=O)-N-(C₁₋₅-Alkyl)₂, -S(=O)₂-NH₂, -S(=O)₂-NH-C₁₋₅-Alkyl, -S(=O)₂-NH-Phenyl, -S(=O)₂-C₁₋₅-Alkyl, -(CH₂)-Benzo[b]furanyl, -O-Phenyl, -S-Phenyl, -S-Benzyl, -O-Benzyl, Phenyl und Benzyl substituiert sein können, wobei jeweils der zyklische Teil der Reste -O-C(=O)-Phenyl, -(CH₂)-O-C(=O)-Phenyl, -S(=O)₂-NH-Phenyl, -O-Phenyl, -S-Phenyl, -S-Benzyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann,
und die vorstehend genannten Heteroaryl-Reste jeweils 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

10. Verbindungen gemäß Anspruch 9, **dadurch gekennzeichnet, dass**
R¹ für einen Wasserstoff-Rest steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, -(CH₂)-(C(CH₃)₃), n-Hexyl, 3-Hexyl, -(CH₂)-(CH₂)-(C(CH₃)₃), n-Heptyl, 3-Heptyl, 4-Heptyl, n-Octyl, -(CH₂)-(CH)(C₂H₅)-(CH₂)-(CH₂)-(CH₂)-(CH₃), Vinyl, 1-Propenyl, 2-Propenyl, 1-Butenyl, 2-Butenyl, 3-Butenyl, 1-Propinyl, 2-Propinyl, 1-Butinyl, 2-Butinyl und 3-Butinyl steht, wobei der Rest jeweils mit 1, 2, 3, 4 oder 5 Substituenten unabhängig voneinander ausgewählt aus der Gruppe bestehend aus F, Cl, Br, I, -CN, -NO₂, -OH, -SH, -NH₂, -N(CH₃)₂, -N(C₂H₅)₂ und -N(CH₃)(C₂H₅) substituiert sein kann;
für einen Rest ausgewählt aus der Gruppe bestehend aus Phenyl, Naphthyl, Thiophenyl, Furanyl, Pyrrolyl, Pyrazolyl, Pyrazinyl, Triazolyl, Pyridinyl, Imidazolyl, Indolyl, Isoindolyl, Benzo[b]furanyl, Benzo[b]thiophenyl, Thiazolyl, Thiadiazolyl, Oxazolyl, Isoxazolyl, Pyridazinyl, Pyrazinyl, Pyrimidinyl, Indazolyl, Chinazolinyl, Chinolinyl, Isochinolinyl, Phenazinyl, Phenothiazinyl, Oxadiazolyl, Benzo[1,2,5]oxadiazolyl und Chromanyl steht, wobei der Rest jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, -S-CH(CH₃)₂, -S-C(CH₃)₃, -S-CH₂-CH₂-CH₂-CH₃, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-Phenyl, -(CH₂)-O-C(=O)-CH₃, -(CH₂)-O-C(=O)-C₂H₅, -(CH₂)-O-C(=O)-Phenyl, -N(CH₃)₂, -N(C₂H₅)₂, -N(H)(CH₃), -N(H)(C₂H₅), -NH-C(=O)-O-CH₃, -NH-C(=O)-O-C₂H₅, -NH-C(=O)-CH₃, -NH-C(=OFC₂H₅, -C(=O)-H, -C(=O)-CH₃, -C(=O)-C₂H₅, -C(=O)-CF₃, -C(=O)-NH₂, -C(=O)-NH-CH₃, -C(=O)-NH₋C₂H₅, -S(=O)₂-NH₂, -S(=O)₂-NH-CH₃, -S(=O)₂-NH-C₂H₅, -S(=O)₂-NH-Phenyl, -S(=O)₂-CH₃, -S(=O)₂-C₂H₅, -(CH₂)-Benzo[b]furanyl, -O-Phenyl, -S-Phenyl, -S-Benzyl, -O-Benzyl, Phenyl und Benzyl substituiert sein kann, wobei jeweils der zyklische Teil der Reste -O-C(=O)-Phenyl, -(CH₂)-O-C(=O)-Phenyl, -S(=O)₂-NH-Phenyl, -O-Phenyl, -S-Phenyl, -S-Benzyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-CH₃, (O-C₂H₅, -O-CH(CH₃)₂, -O-C(CH₃)₃, Methyl, Ethyl, n-Propyl, Isopropyl, n-Butyl, sec-Butyl, Isobutyl, tert-Butyl, -O-CF₃, -S-CF₃, Phenyl und -O-Benzyl substituiert sein kann,
für -(CH₂)ₐₐ-C(=O)-O-R⁷ mit aa = 1, 2, 3, 4 oder 5 steht;
oder für -(CH²)-R¹², -(CH₂)-(CH₂)-R¹², -(CH₂)-O-R¹², -(CH₂)-S-R¹² (CH₂)-N(CH₃)-R¹², -(CH₂)-(CH₂)-(CH₂)-R¹², -(CH₂)-(CH₂)-O-R¹². -(CH₂)-(CH₂)-S-R¹², -(CH₂)-(CH₂)-NH-R¹², -(CH₂)-(CH₂)-N(CH₃)-R¹², -(CH₂)-(CH₂)-O-(CH₂)-R¹², -(CH₂)-(CH₂)-S-(CH₂)-R¹² oder -(CH₂)-(CH₂)-N(CH₃)-R¹² steht;
R² für einen Wasserstoff-Rest steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, -(CH₂)-(C(CH₃)₃), n-Hexyl, 3-Hexyl, -(CH₂)-(CH₂)-(C(CH₃)₃), n-Heptyl, 3-Heptyl, 4-Heptyl, n-Octyl, -(CH₂)-(CH)(C₂H₅)-(CH₂)-(CH₂)-(CH₂)-(CH₃), Vinyl, 1-Propenyl, 2-Propenyl, 1-Butenyl, 2-Butenyl, 3-Butenyl, 1-Propinyl, 2-Propinyl, 1-Butinyl, 2-Butinyl und 3-Butinyl steht, wobei der Rest jeweils mit 1, 2, 3, 4 oder 5 Substituenten unabhängig voneinander ausgewählt aus der Gruppe bestehend aus F, Cl, Br, I, -CN, -NO₂, -OH, -SH, -NH₂, -N(CH₃)₂, -N(C₂H₅)₂ und -N(CH₃)(C₂H₅) substituiert sein kann;
für einen Rest ausgewählt aus der Gruppe bestehend aus Phenyl, Naphthyl, Thiophenyl, Furanyl, Pyrrolyl, Pyrazolyl, Pyrazinyl, Triazolyl, Pyridinyl, Imidazolyl, Indolyl, Isoindolyl, Benzo[b]furanyl, Benzo[b]thiophenyl, Thiazolyl, Thiadiazolyl, Oxazolyl, Isoxazolyl, Pyridazinyl, Pyrazinyl, Pyrimidinyl, Indazolyl, Chinazolinyl, Chinolinyl, Isochinolinyl, Phenazinyl, Phenothiazinyl, Oxadiazolyl, Benzo[1,2,5]oxadiazolyl und Chromanyl steht, wobei der Rest jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, .-S-CH₂F, -SH, -S-CH₃, -S-C₂H₅, -S-CH(CH₃)₂, -S-C(CH₃)₃, -S-CH₂-CH₂-CH₂-CH₃, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-Phenyl, -(CH₂)-O-C(=O)-CH₃, -(CH₂)-O-C(=O)-C₂H₅, -(CH₂)-O-C(=O)-Phenyl, -N(CH₃)₂, -N(C₂H₅)₂, -N(H)(CH₃), -N(H)(C₂H₅), -NH-C(=O)-O-CH₃, -NH-C(=O)-O-C₂H₅, -NH-C(=O)-CH₃, -NH-C(=O)-C₂H₅, -C(=O)-H, -C(=O)-CH₃, -C(=O)-C₂H₅, -C(=O)-CF₃, -C(=O)-NH₂, -C(=O)-NH-CH₃, -C(=O)-NH-C₂H₅, -S(=O)₂-NH₂, -S(=O)₂-NH-CH₃, -S(=O)₂-NH-C₂H₅, -S(=O)₂-NH-Phenyl, -S(=O)₂-CH₃, -S(=O)₂-C₂H₅, -(CH₂)-Benzo[b]furanyl, -O-Phenyl, -S-Phenyl, -S-Benzyl, -O-Benzyl, Phenyl und Benzyl substituiert sein kann, wobei jeweils der zyklische Teil der Reste -O-C(=O)-Phenyl, -(CH₂)-O-C(=O)-Phenyl, -S(=O)₂-NH-Phenyl, -O-Phenyl, -S-Phenyl, -S-Benzyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-CH₃, -O-C₂H₅, -O-CH(CH₃)₂, -O-C(CH₃)₃, Methyl, Ethyl, n-Propyl, Isopropyl, n-Butyl, sec-Butyl, Isobutyl, tert-Butyl, -O-CF₃, -S-CF₃, Phenyl und -O-Benzyl substituiert sein kann,
oder für -(CH₂)-R¹³, -(CH₂)-(CH₂)-R¹³ -(CH₂)-O-R¹³, -(CH₂)-S-R¹³, -(CH₂)-N(CH₃)-R¹³, (CH₂)-(CH₂)-(CH₂) R¹³, -(CH₂)-(CH₂)-O-R¹³, -(CH₂)-(CH₂)-S-R¹³. -(CH₂)-(CH₂)-NH-R¹³, -(CH₂)-(CH₂)-N(CH₃)-R¹³, -(CH₂)-(CH₂)-O-(CH₂)-R¹³. -(CH₂)-(CH₂)-S-(CH₂)-R¹³ oder -(CH₂)-(CH₂)-N(CH₃)-R¹³ steht;
R³ für eine -S(=O)₂-R⁴-Gruppe, für eine -C(=S)-NH-R⁵-Gruppe oder für eine -C(=O)-NH-R⁶-Gruppe steht,
R⁴ für einen Rest -NR¹⁰R¹¹ steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, -(CH₂)-(C(CH₃)₃), n-Hexyl, 3-Hexyl, -(CH₂)-(CH₂)-(C(CH₃)₃), n-Heptyl, 3-Heptyl, 4-Heptyl, n-Octyl, -(CH₂)-(CH)(C₂H₅)-(CH₂)-(CH₂₎-CH₂)-(CH₃), Vinyl, 1-Propenyl, 2-Propenyl, 1-Butenyl, 2-Butenyl, 3-Butenyl, 1-Propinyl, 2-Propinyl, 1-Butinyl, 2-Butinyl und 3-Butinyl steht, wobei der Rest jeweils mit 1, 2, 3, 4 oder 5 Substituenten unabhängig voneinander ausgewählt aus der Gruppe bestehend aus F, Cl, Br, I, -CN, -NO₂, -OH, -SH, -NH₂, -N(CH₃)₂, -N(C₂H₅)₂ und -N(CH₃)(C₂H₅) substituiert sein kann;
für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Adamantyl und 7,7-Dimethyl-bicyclo[2.2.1]heptyl steht, wobei der (hetero)cycloaliphatische Rest jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -O-C(CH₃)₃, -NH₂, -NO₂, -O-CF₃, -S-CF₃, -SH, S-CH₃, -S-C₂H₅, -S-C(CH₃)₃, Methyl, Ethyl, n-Propyl, Isopropyl, n-Butyl, sec-Butyl, Isobutyl, tert-Butyl, -C(=O)-H, -C(=O)-CH₃, -C(=O)-C₂H₅, -C(=O)-OH, -C(=O)-O-CH₃, -C(=O)-O-C₂H₅, -C(=O)-O-C(CH₃)₃, -(CH₂)-C(=O)-OH, -(CH₂)-C(=O)-O-CH₃, -(CH₂)-C(=O)-O-C₂H₅, -N(CH₃)₂, -N(C₂H₅)₂, -N(H)(CH₃), -N(H)(C₂H₅), -(CH₂)-Benzo[b]furanyl, -O-Phenyl, -O-Benzyl, Phenyl, Benzyl, Naphthyl und -(CH₂)-Naphthyl substituiert sein kann, wobei jeweils der zyklische Teil der Reste -O-Phenyl, -O-Benzyl, Phenyl, -(CH₂)-Benzo[b]furanyl, Benzyl, Naphthyl und -(CH₂)-Naphthyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-CH₃, -O-C₂H₅, Methyl, Ethyl, n-Propyl, Isopropyl, n-Butyl, sec-Butyl, Isobutyl, tert-Butyl, -O-CF₃, -S-CF₃, Phenyl und -O-Benzyl substituiert sein kann,
für einen Rest ausgewählt aus der Gruppe bestehend aus Phenyl, Naphthyl, Thiophenyl, Furanyl, Pyrrolyl, Pyrazolyl, Pyrazinyl, Triazolyl, Pyridinyl, Imidazolyl, Indolyl, Isoindolyl, Benzo[b]furanyl, Benzo[b]thiophenyl, Thiazolyl, Thiadiazolyl, Oxazolyl, Isoxazolyl, Pyridazinyl, Pyrazinyl, Pyrimidinyl, Indazolyl, Chinazolinyl, Chinolinyl, Isochinolinyl, Phenazinyl, Phenothiazinyl, Oxadiazolyl, Benzo[1,2,5]oxadiazolyl, Chromanyl und (1,2,3,4)-Tetrahydrochinolinyl steht, wobei der Rest jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, -S-CH(CH₃)₂, -S-C(CH₃)₃, -S-CH₂-CH₂-CH₂-CH₃, Methyl, Ethyl, n-Propyl, Isopropyl, n-Butyl, sec-Butyl, Isobutyl, tert-Butyl, (1,1)-Dimethylpropyl, -C(=O)-OH, -C(=O)-O-CH₃, -C(=O)-O-C₂H₅, -C(=O)-O-C(CH₃)₃, **-**O-C(=O)-CH₃, -O-C(=O)-C₂H₅, -O-C(=O)-Phenyl, -(CH₂)-O-C(=O)-CH₃, -(CH₂)-O-C(=O)-C₂H₅, -(CH₂)-O-C(=O)-Phenyl, -N(CH₃)₂, -N(C₂H₅)₂, -N(H)(CH₃), -N(H)(C₂H₅), -NH-C(=O)-O-CH₃, -NH-C(=O)-O-C₂H₅, -NH-C(=O)-CH₃, -NH-C(=O)-C₂H₅, -C(=O)-H, -C(=O)-CH₃, -C(=O)-C₂H₅, -C(=O)-CF₃, -C(=O)-NH₂, -C(=O)-NH-CH₃, -C(=O)-NH-C₂H₅, -S(=O)₂-NH₂, -S(=O)₂-NH-CH₃, -S(=O)₂-NH-C₂H₅, -S(=O)₂-NH-Phenyl, -S(=O₂-CH₃, -S(=O)₂-C₂H₅, -(CH₂)-Benzo[b]furanyl, -O-Phenyl, -S-Pheny), -S-Benzyl, -O-Benzyl, Phenyl und Benzyl substituiert sein kann, wobei jeweils der zyklische Teil der Reste -O-C(=O)-Phenyl, -(CH₂)-O-C(=O)-Phenyl, -S(=O)₂-NH-Phenyl, -O-Phenyl, -S-Phenyl, -S-Benzyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-CH₃, -O-C₂H₅, -O-CH(CH₃)₂, -O-C(CH₃)₃, Methyl, Ethyl, n-Propyl, Isopropyl, n-Butyl, sec-Butyl, Isobutyl, tert-Butyl, -O-CF₃, -S-CF₃, Phenyl und -O-Benzyl substituiert sein kann,
oder für -(CH₂)-R¹⁴, -(CH₂)-(CH₂)-R¹⁴, -(CH₂)-O-R¹⁴, -(CH₂)-S-R¹⁴, -(CH₂)-N(CH₃)-R¹⁴, -(CH₂)-(CH₂)-(CH2)-R¹⁴, -(CH₂)-(CH₂)-O-R¹⁴, -(CH₂)-(CH₂)-S-R¹⁴, -(CH₂)-(CH₂)-NH-R¹⁴, -(CH₂)-(CH₂)-N(CH₃)-R¹⁴. -(CH₂)-(CH₂)-O-(CH₂)-R¹⁴, -(CH₂)-(CH₂)-S-(CH₂)-R¹⁴ oder -(CH₂)-(CH₂)-N(CH₃)-R¹⁴ steht;
R⁵ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, -(CH₂)-(C(CH₃)₃), n-Hexyl, 3-Hexyl, -(CH₂)-(CH₂)-(C(CH₃)₃), n-Heptyl, 3-Heptyl, 4-Heptyl, n-Octyl, -(CH₂)-(CH)(C₂H₅)-(CH₂)-(CH₂)-(CH₂)-(CH₃), Vinyl, 1-Propenyl, 2-Propenyl, 1-Butenyl, 2-Butenyl, 3-Butenyl, 1-Propinyl, 2-Propinyl, 1-Butinyl, 2-Butinyl und 3-Butinyl steht, wobei der Rest jeweils mit 1, 2, 3, 4 oder 5 Substituenten unabhängig voneinander ausgewählt aus der Gruppe bestehend aus F, Cl, Br, I, -CN, -NO₂, -OH, -SH, -NH₂, -N(CH₃)₂, -N(C₂H₅)₂ und -N(CH₃)(C₂H₅) substituiert sein kann;
für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht, wobei der (hetero)cycloaliphatische Rest jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -O-C(CH₃)₃, -NH₂, -NO₂, -O-CF₃, -S-CF₃, -SH, S-CH₃, -S-C₂H₅, -S-C(CH₃)₃, Methyl, Ethyl, n-Propyl, Isopropyl, n-Butyl, sec-Butyl, Isobutyl, tert-Butyl, -C(=O)-H, -C(=O)-CH₃, -C(=O)-C₂H₅, -C(=O)-OH, -C(=O)-O-CH₃, -C(=O)-O-C₂H₅, -C(=O)-O-C(CH₃)₃, -(CH₂)-C(=O)-OH, -(CH₂)-C(=O)-O-CH₃, -(CH₂)-C(=O)-O-C₂H₅, -N(CH₃)₂, -N(C₂H₅)₂, -N(H)(CH₃), -N(H)(C₂H₅), -(CH₂)-Benzo[b]furanyl, -O-Phenyl, -O-Benzyl, Phenyl, Benzyl, Naphthyl und -(CH₂)-Naphthyl substituiert sein kann, wobei jeweils der zyklische Teil der Reste -O-Phenyl, -O-Benzyl, Phenyl, -(CH₂)-Benzo[b]furanyl, Benzyl, Naphthyl und -(CH₂)-Naphthyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-CH₃, -O-C₂H₅, Methyl, Ethyl, n-Propyl, Isopropyl, n-Butyl, sec-Butyl, Isobutyl, tert-Butyl, -O-CF₃, -S-CF₃, Phenyl und -O-Benzyl substituiert sein kann,
für einen Rest ausgewählt aus der Gruppe bestehend aus Phenyl, Naphthyl, Thiophenyl, Furanyl, Pyrrolyl, Pyrazolyl, Pyrazinyl, Triazolyl, Pyridinyl, Imidazolyl, Indolyl, Isoindolyl, Benzo[b]furanyl, Benzo[b]thiophenyl, Thiazolyl, Thiadiazolyl, Oxazolyl, Isoxazolyl, Pyridazinyl, Pyrazinyl, Pyrimidinyl, Indazolyl, Chinazolinyl, Chinolinyl, Isochinolinyl, Phenazinyl, Phenothiazinyl, Oxadiazolyl, Benzo[1,2,5]oxadiazolyl und Chromanyl steht, wobei der Rest jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, -S-CH(CH₃)₂, -S-C(CH₃)₃, -S-CH₂-CH₂-CH₂-CH₃, Methyl, Ethyl, n-Propyl, Isopropyl, n-Butyl, sec-Butyl, Isobutyl, tert-Butyl, (1,1)-Dimethylpropyl, -C(=O)-OH, -C(=O)-O-CH₃, -C(=O)-O-C₂H₅, -C(=O)-O-C(CH₃)₃, -O-C(=O)CH₃, -O-C(=O)-C₂H₅, -O-C(=O)-Phenyl, -(CH₂)-O-C(=O)-CH₃, -(CH₂)-O-C(=O)-C₂H₅, -(CH₂)-O-C(=O)-Phenyl, -N(CH₃)₂, -N(C₂H₅)₂, -N(H)(CH₃), -N(H)(C₂H₅), -NH-C(=O)-O-CH₃, -NH-C(=O)-O-C₂H₅, -NH-C(=O)-CH₃, -NH-C(=O)-C₂H₅, -C(=O)-H, -C(=O)-CH₃, -C(=O)-C₂H₅, -C(=O)-CF₃, -C(=O)-NH₂, -C(=O)-NH-CH₃, -C(=O)-NH-C₂H₅, -S(=O)₂-NH₂, -S(=O)₂-NH-CH₃, -S(=O)₂-NH-C₂H₅, -S(=O)₂-NH-Phenyl, -S(=O)₂-CH₃, -S(=O)₂-C₂H₅, -(CH₂)-Benzo[b]furanyl, -O-Phenyl, -S-Phenyl, -S-Benzyl, -O-Benzyl, Phenyl und Benzyl substituiert sein kann, wobei jeweils der zyklische Teil der Reste -O-C(=O)-Phenyl, -(CH₂)-O-C(=O)-Phenyl, -S(=O)₂-NH-Phenyl, -O-Phenyl, -S-Phenyl, -S-Benzyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-CH₃, -O-C₂H₅, -O-CH(CH₃)₂, -O-C(CH₃)₃, Methyl, Ethyl, n-Propyl; lsopropyl, n-Butyl, sec-Butyl, Isobutyl, tert-Butyl, -O-CF₃, -S-CF₃, Phenyl und -O-Benzyl substituiert sein kann,
für -(CHR¹⁵)-R¹⁶, -(CHR¹⁵)-(CH₂)-R¹⁶, -(CHR¹⁵)-O-R¹⁶, -(CHR¹⁵)-S-R¹⁶, -(CHR¹⁵)-N(CH₃)-R¹⁶, -(CHR¹⁵-(CH₂)-(CH₂)-R¹⁶ -(CHR¹⁵)-(CH₂)-O-R¹⁶, -(CHR¹⁵)-(CH₂)-S-R¹⁶,-(CHR¹⁵)-(CH₂)-NH-R¹⁶, -(CHR¹⁵)-(CH₂)-N(CH₃)-R¹⁶, -(CHR¹⁵-(CH₂)-O-(CH₂)-R¹⁶,-(CHR¹⁵)-(CH₂)-S-(CH₂)-R¹⁶ oder -(CHR¹⁵)-(CH₂)-N(CH₃)-R¹⁶ steht;
für einen -C(=O)-OR⁸-Rest steht;
oder für einen -(CHR¹⁷)-C(=O)-OR⁹-Rest oder für einen -(CHR¹⁶)-(CH₂)-C(=O)-OR⁹-Rest steht;
R⁶ für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht, wobei der (hetero)cycloaliphatische Rest jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -O-C(CH₃)₃, -NH₂, -NO₂, -O-CF₃, -S-CF₃, -SH, S-CH₃, -S-C₂H₅, -S-C(CH₃)₃, Methyl, Ethyl, n-Propyl, Isopropyl, n-Butyl, sec-Butyl, Isobutyl, tert-Butyl, -C(=O)-H, -C(=O)-CH₃, -C(=O)-C₂H₅, -C(=O)-OH, -C(=O)-O-CH₃, -C(=O)-O-C₂H₅, -C(=OFO-C(CH₃)₃, -(CH₂)-C(=O)-OH, -(CH₂)-C(=O)-O-CH₃, -(CH₂)-C(=O)-O-C₂H₅, -N(CH₃)₂, -N(C₂H₅)₂, -N(H)(CH₃), -N(HHC₂H₅), -(CH₂)-Benzo[b]furanyl, -O-Phenyl, -O-Benzyl, Phenyl, Benzyl, Naphthyl und -(CH₂)-Naphthyl substituiert sein kann, wobei jeweils der zyklische Teil der Reste -O-Phenyl, -O-Benzyl, Phenyl, -(CH₂)-Benzo[b]furanyl, Benzyl, Naphthyl und --(CH₂)-Naphthyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-CH₃, -O-C₂H₅, Methyl, Ethyl, n-Propyl, Isopropyl, n-Butyl, sec-Butyl, Isobutyl, tert-Butyl, -O-CF₃, -S-CF₃, Phenyl und -O-Benzyl substituiert sein kann,
für einen Rest ausgewählt aus der Gruppe bestehend aus Phenyl, Naphthyl, Thiophenyl, Furanyl, Pyrrolyl, Pyrazolyl, Pyrazinyl, Triazolyl, Pyridinyl, Imidazolyl, Indolyl, Isoindolyl, Benzo[b]furanyl, Benzo[b]thiophenyl, Thiazolyl, Thiadiazolyl, Oxazolyl, Isoxazolyl, Pyridazinyl, Pyrazinyl, Pyrimidinyl, Indazolyl, Chinazolinyl, Chinolinyl, Isochinolinyl, Phenazinyl, Phenothiazinyl, Oxadiazolyl, Benzo[1,2,5]oxadiazolyl und Chromanyl steht, wobei der Rest jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, -S-CH(CH₃)₂, -S-C(CH₃)₃, -S-CH₂-CH₂-CH₂-CH₃, Methyl, Ethyl, n-Propyl, Isopropyl, n-Butyl, sec-Butyl, Isobutyl, tert-Butyl, (1,1)-Dimethylpropyl, -C(=O)-OH, -C(=O)-O-CH₃, -C(=O)-O-C₂H₅, -C(=OFO-O(CH₃)₃, -O-C(=O)-CH₃, -O-C(=O)-C₂H₅, -O-C(=O)-Phenyl, -(CH₂)-O-C(=O)-CH₃, -(CH₂)-O-C(=O)-C₂H₅, -(CH₂)-O-C(=O)-Phenyl, -N(CH₃)₂, -N(C₂H₅)₂, -N(H)(CH₃), -N(H)(C₂H₅), NH-C(=O)-O-CH₃, -NH-C(=O)-O-C₂H₅, -NH-C(=O)-CH₃, -NH-C(=O)-C₂H₅, -C(=O)-H, -C(=O)-CH₃, -C(=O)-C₂H₅, -C(=O)-CF₃, -C(=O)-NH₂, -C(=O)-NH-CH₃, -C(=O)-NH-C₂H₅, -S(=O)₂-NH₂, -S(=O)₂-NH-CH₃, -S(=O)₂-NH-C₂H₅, -S(=O)₂-NH-Phenyl, -S(=O)₂-CH₃, -S(=O)₂-C₂H₅, -(CH₂)-Benzo[b]furanyl, -O-Phenyl, -S-Phenyl, -S-Benzyl, -O-Benzyl, Phenyl und Benzyl substituiert sein kann, wobei jeweils der zyklische Teil der Reste -O-C(=O)-Phenyl, -(CH₂)-O-C(=O)-Phenyl, -S(=O)₂-NH-Phenyl, -O-Phenyl, -S-Phenyl, -S-Benzyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-CH₃, -O-C₂H₅, -O-CH(CH₃)₂, -O-C(CH₃)₃, Methyl, Ethyl, n-Propyl, Isopropyl, n-Butyl, sec-Butyl, Isobutyl, tert-Butyl, -O-CF₃, -S-CF₃, Phenyl und -O-Benzyl substituiert sein kann,
oder für -(CH₂)-R¹⁸, -(CH₂)-(CH₂)-R¹⁸, -(CH₂)-O-R¹⁸, -(CH₂)-S-R¹⁸, -(CH₂)-N(CH₃)-R¹⁸, -(CH₂)-(CH₂)-(CH₂₎₋R¹⁸, -(CH₂)-(CH₂-O-R¹⁸, -(CH₂)-(CH₂)-S-R¹⁸, -(CH₂)-(CH₂)-NH-R¹⁸, -(CH₂)-(CH₂)-N(CH₃)-R¹⁸, -(CH₂)-(CH₂)-O-(CH₂)-R¹⁸, -(CH₂)-(CH₂)-SCH₂)-R¹⁸ oder -(CH₂)-(CH₂)-N(CH₃)-R¹⁸ steht;
R⁷ R⁸, R⁹, R¹⁰ und R¹¹ unabhängig voneinander jeweils
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, -(CH₂)-(C(CH₃)₃), n-Hexyl, 3-Hexyl, -(CH₂)-(CH₂)-(C(CH₃)₃), n-Heptyl, 3-Heptyl, 4-Heptyl, n-Octyl, -(CH₂)-(CH)(C₂H₅)-(CH₂)-(CH₂)-(CH₂)-(CH₃), Vinyl, 1-Propenyl, 2-Propenyl, 1-Butenyl, 2-Butenyl, 3-Butenyl, 1-Propinyl, 2-Propinyl, 1-Butinyl, 2-Butinyl und 3-Butinyl stehen, wobei der Rest jeweils mit 1, 2, 3, 4 oder 5 Substituenten unabhängig voneinander ausgewählt aus der Gruppe bestehend aus F, Cl, Br, I, -CN, -NO₂, -OH, -SH, -NH₂, -N(CH₃)₂, -N(C₂H₅)₂ und -N(CH₃)(C₂H₅) substituiert sein kann;
R¹² und R¹³ unabhängig voneinander jeweils
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, -(CH₂)-(C(CH₃)₃), n-Hexyl, 3-Hexyl, -(CH₂)-(CH₂)-(C(CH₃)₃), n-Heptyl, 3-Heptyl, 4-Heptyl, n-Octyl, -(CH₂)-(CH)(C₂H₅)-(CH₂)-(CH₂)-(CH₂)-(CH₃), Vinyl, 1-Propenyl, 2-Propenyl, 1-Butenyl, 2-Butenyl, 3-Butenyl, 1-Propinyl, 2-Propinyl, 1-Butinyl, 2-Butinyl und 3-Butinyl stehen, wobei der Rest jeweils mit 1, 2, 3, 4 oder 5 Substituenten unabhängig voneinander ausgewählt aus der Gruppe bestehend aus F, Cl, Br, I, -CN, **-**NO₂**,** -OH, -SH, -NH₂, -N(CH₃)₂, -N(C₂H₅)₂ und -N(CH₃)(C₂H₅) substituiert sein kann;
oder für einen Rest ausgewählt aus der Gruppe bestehend aus Phenyl, Naphthyl, Thiophenyl, Furanyl, Pyrrolyl, Pyrazolyl, Pyrazinyl, Triazolyl, Pyridinyl, Imidazolyl, Indolyl, Isoindolyl, Benzo[b]furanyl, Benzo[b]thiophenyl, Thiazolyl, Thiadiazolyl, Oxazolyl, Isoxazolyl, Pyridazinyl, Pyrazinyl, Pyrimidinyl, Indazolyl, Chinazolinyl, Chinolinyl, Isochinolinyl, Phenazinyl, Phenothiazinyl, Oxadiazolyl, Benzo[1,2,5]oxadiazolyl und Chromanyl stehen, wobei der Rest jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, -S-CH(CH₃)₂, -S-C(CH₃)₃, -S-CH₂-CH₂-CH₂-CH₃, Methyl, Ethyl, n-Propyl, Isopropyl, n-Butyl, sec-Butyl, Isobutyl, tert-Butyl, (1,1)-Dimethylpropyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-Phenyl, -(CH₂)-O-C(=O)-CH₃, -(CH₂)-O-C(=O)-C₂H₅, -(CH₂)-O-C(=O)-Phenyl, -N(CH₃)₂, -N(C₂H₅)₂, -N(H)(CH₃), -N(H)(C₂H₅), -NH-C(=O)-O-CH₃, -NH-C(=O)-O-C₂H₅, -NH-C(=O)-CH₃, -NH-C(=O)-C₂H₅, -C(=O)-H, -C(=O)-CH₃, -C(=O)-C₂H₅, -C(=O)-CF₃, -C(=O)-NH₂, -C(=O)-NH-CH₃, -C(=O)-NH-C₂H₅, -S(=O)₂-NH₂, -S(=O)₂-NH-CH₃, -S(=O)₂-NH-C₂H₅, -S(=O)₂-NH-Phenyl, -S(=O)₂-CH₃, -S(=O)₂-C₂H₅, (CH₂)-Benzo[b]furanyl, -O-Phenyl, -S-Phenyl, -S-Benzyl, -O-Benzyl, Phenyl und Benzyl substituiert sein kann, wobei jeweils der zyklische Teil der Reste -O-C(=O)-Phenyl, -(CH₂)-O-C(=O)-Phenyl, -S(=O)₂-NH-Phenyl, -O-Phenyl, -S-Phenyl, -S-Benzyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-CH₃, -O-C₂H₅, -O-CH(CH₃)₂, -O-C(CH₃)₃, Methyl, Ethyl, n-Propyl, Isopropyl, n-Butyl, sec-Butyl, Isobutyl, tert-Butyl, -O-CF₃, -S-CF₃, Phenyl und -O-Benzyl substituiert sein kann,
R¹⁴ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, -(CH₂)-(C(CH₃)₃), n-Hexyl, 3-Hexyl, -(CH₂)-(CH₂)-(C(CH₃)₃), n-Heptyl, 3-Heptyl, 4-Heptyl, n-Octyl, -(CH₂)-(CH)(C₂H₅)-(CH₂)-(CH₂₎-(CH₂₎-(CH₃), Vinyl, 1-Propenyl, 2-Propenyl, 1-Butenyl, 2-Butenyl, 3-Butenyl, 1-Propinyl, 2-Propinyl, 1-Butinyl, 2-Butinyl und 3-Butinyl steht, wobei der Rest jeweils mit 1, 2, 3, 4 oder 5 Substituenten unabhängig voneinander ausgewählt aus der Gruppe bestehend aus F, Cl, Br, I, -CN, -NO₂, -OH, -SH, -NH₂, -N(CH₃)₂, -N(C₂H₅)₂ und -N(CH₃)(C₂H₅) substituiert sein kann;
für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Adamantyl und 7,7-Dimethyl-bicyclo[2.2.1]heptyl steht, wobei der (hetero)cycloaliphatische Rest jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, OH, -O-CH₃, -O-C₂H₅, -O-C(CH₃)₃, -NH₂, -NO₂, -O-CF₃, -S-CF₃, -SH, S-CH₃, -S-C₂H₅, -S-C(CH₃)₃, Methyl, Ethyl, n-Propyl, Isopropyl, n-Butyl, sec-Butyl, Isobutyl, tert-Butyl, -C(=O)-H, -C(=O)-CH₃, -C(=O)-C₂H₅, -C(=O)-OH, -C(=O)-O-CH₃, -C(=O)-O-C₂H₅, -C(=OFO-C(CH₃)₃, -(CH₂)-C(=O)-OH, -(CH₂)-C(=OFO-CH₃, -(CH₂)-C(=O)-O-C₂H₅, -N(CH₃)₂, -N(C₂H₅)₂, -N(H)(CH₃), -N(H)(C₂H₅), -(CH₂)-Benzo[b]furanyl, -O-Phenyl, -O-Benzyl, Phenyl, Benzyl, Naphthyl und -(CH₂)-Naphthyl substituiert sein kann, wobei jeweils der zyklische Teil der Reste -O-Phenyl, -O-Benzyl, Phenyl, -(CH₂)-Benzo[b]furanyl, Benzyl, Naphthyl und -(CH₂)-Naphthyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-CH₃, -O-C₂H₅, Methyl, Ethyl, n-Propyl, Isopropyl, n-Butyl, sec-Butyl, Isobutyl, tert-Butyl, -O-CF₃, -S-CF₃, Phenyl und -O-Benzyl substituiert sein kann,
oder für einen Rest ausgewählt aus der Gruppe bestehend aus Phenyl, Naphthyl, Thiophenyl, Furanyl, Pyrrolyl, Pyrazolyl, Pyrazinyl, Triazolyl, Pyridinyl, Imidazolyl, Indolyl, Isoindolyl, Benzo[b]furanyl, Benzo[b]thiophenyl, Thiazolyl, Thiadiazolyl, Oxazolyl, Isoxazolyl, Pyridazinyl, Pyrazinyl, Pyrimidinyl, Indazolyl, Chinazolinyl, Chinolinyl, Isochinolinyl, Phenazinyl, Phenothiazinyl, Oxadiazolyl, Benzo[1,2,5]oxadiazolyl, Chromanyl und (1,2,3,4)-Tetrahydrochinolinyl steht, wobei der Rest jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, -S-CH(CH₃)₂, -S-C(CH₃)₃, -S-CH₂-CH₂-CH₂-CH₃, Methyl, Ethyl, n-Propyl, Isopropyl, n-Butyl, sec-Butyl, Isobutyl, tert-Butyl, (1,1)-Dimethylpropyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-Phenyl, -(CH₂)-O-C(=O)-CH₃, -(CH₂)-O-C(=O)-C₂H₅, -(CH₂)-O-C(=O)-Phenyl, -N(CH₃)₂, -N(C₂H₅)₂, -N(H)(CH₃), -N(H)(C₂H₅), -NH-C(=O)-O-CH₃, -NH-C(=O)-O-C₂H₅, -NH-C(=O)-CH₃, -NH-C(=O)-C₂H₅, -C(=O)-H, -C(=O)-CH₃, -C(=O)-C₂H₅, -C(=O)-CF₃, -C(=O)-NH₂, -C(=O)-NH-CH₃, -C(=O)-NH-C₂H₅, -S(=O)₂-NH₂, -S(=O)₂-NH-CH₃, -S(=O)₂-NH-C₂H₅, -S(=O)₂-NH-Phenyl, -S(=O)₂-CH₃, -S(=O)₂-C₂H₅, -(CH₂)-Benzo[b]furanyl, -O-Phenyl, -S-Phenyl, -S-Benzyl, -O-Benzyl, Phenyl und Benzyl substituiert sein kann, wobei jeweils der zyklische Teil der Reste -O-C(=O)-Phenyl, -(CH₂)-O-C(=O)-Phenyl, -S(=O)₂-NH-Phenyl, -O-Phenyl, -S-Phenyl, -S-Benzyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-CH₃, -O-C₂H₅, -O-CH(CH₃)₂, -O-C(CH₃)₃, Methyl, Ethyl, n-Propyl, Isopropyl, n-Butyl, sec-Butyl, Isobutyl, tert-Butyl, -O-CF₃, -S-CF₃, Phenyl und -O-Benzyl substituiert sein kann,
R¹⁵ für einen Wasserstoff-Rest
oder für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, -(CH₂)-(C(CH₃)₃), n-Hexyl, 3-Hexyl, -(CH₂)-(CH₂)-(C(CH₃)₃), n-Heptyl, 3-Heptyl, 4-Heptyl, n-Octyl, -(CH₂)-(CH)(C₂H₅)-(CH₂)-(CH₂)-(CH₂)-(CH₃), Vinyl, 1-Propenyl, 2-Propenyl, 1-Butenyl, 2-Butenyl, 3-Butenyl, 1-Propinyl, 2-Propinyl, 1-Butinyl, 2-Butinyl und 3-Butinyl steht, wobei der Rest jeweils mit 1, 2, 3, 4 oder 5 Substituenten unabhängig voneinander ausgewählt aus der Gruppe bestehend aus F, Cl, Br, I, -CN, -NO₂, -OH, -SH, -NH₂, -N(CH₃)₂, -N(C₂H₅)₂ und -N(CH₃)(C₂H₅) substituiert sein kann;
R¹⁶ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, -(CH₂)-(C(CH₃)₃), n-Hexyl, 3-Hexyl, -(CH₂)-(CH₂)-(C(CH₃)₃), n-Heptyl, 3-Heptyl, 4-Heptyl, n-Octyl, -(CH₂)-(CH)(C₂H₅)-(CH₂)-(CH₂)-(CH₂)-(CH₃), Vinyl, 1-Propenyl, 2-Propenyl, 1-Butenyl, 2-Butenyl, 3-Butenyl, 1-Propinyl, 2-Propinyl, 1-Butinyl, 2-Butinyl und 3-Butinyl steht, wobei der Rest jeweils mit 1, 2, 3, 4 oder 5 Substituenten unabhängig voneinander ausgewählt aus der Gruppe bestehend aus F, Cl, Br, I, -CN, -NO₂, -OH, -SH, -NH₂, -N(CH₃)₂, -N(C₂H₅)₂ und -N(CH₃)(C₂H₅) substituiert sein kann;
für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht, wobei der (hetero)cycloaliphatische Rest jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -O-C(CH₃)₃, -NH₂, -NO₂, -O-CF₃, -S-CF₃, -SH, S-CH₃, -S-C₂H₅, -S-C(CH₃)₃, Methyl, Ethyl, n-Propyl, Isopropyl, n-Butyl, sec-Butyl, Isobutyl, tert-Butyl, -C(=O)-H, -C(=O)-CH₃, -C(=O)-C₂H₅, -C(=O)-OH, -C(=O)-O-CH₃, -C(=O)-O-C₂H₅, -C(=OFO-C(CH₃)₃, -(CH₂)-C(=O)-OH, -(CH₂)-C(=O)-O-CH₃, -(CH₂)-C(=O)-O-C₂H₅, -N(CH₃)₂, -N(C₂H₅)₂, -N(H)(CH₃), -N(H)(C₂H₅), -(CH₂)-Benzo[b]furanyl, -O-Phenyl, -O-Benzyl, Phenyl, Benzyl, Naphthyl und -(CH₂)-Naphthyl substituiert sein kann, wobei jeweils der zyklische Teil der Reste -O-Phenyl, -O-Benzyl, Phenyl, -(CH₂)-Benzo[b]furanyl, Benzyl, Naphthyl und -(CH₂)-Naphthyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-CH₃, -O-C₂H₅, Methyl, Ethyl, n-Propyl, Isopropyl, n-Butyl, sec-Butyl, Isobutyl, tert-Butyl, -O-CF₃, -S-CF₃, Phenyl und -O-Benzyl substituiert sein kann,
oder für einen Rest ausgewählt aus der Gruppe bestehend aus Phenyl, Naphthyl, Thiophenyl, Furanyl, Pyrrolyl, Pyrazolyl, Pyrazinyl, Triazolyl, Pyridinyl, Imidazolyl, Indolyl, Isoindolyl, Benzo[b]furanyl, Benzo[b]thiophenyl, Thiazolyl, Thiadiazolyl, Oxazolyl, Isoxazolyl, Pyridazinyl, Pyrazinyl, Pyrimidinyl, Indazolyl, Chinazolinyl, Chinolinyl, Isochinolinyl, Phenazinyl, Phenothiazinyl, Oxadiazolyl, Benzo[1,2,5]oxadiazolyl, Chromanyl und (1,2,3,4)-Tetrahydrochinolinyl steht, wobei der Rest jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, -S-CH(CH₃)₂, -S-C(CH₃)₃, -S-CH₂-CH₂-CH₂-CH₃, Methyl, Ethyl, n-Propyl, Isopropyl, n-Butyl, sec-Butyl, Isobutyl, tert-Butyl, (1,1)-Dimethylpropyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-Phenyl, -(CH₂)-O-C(=O)-CH₃, -(CH₂)-O-C(=O)-C₂H₅, -(CH₂)-O-C(=O)-Phenyl, -N(CH₃)₂, -N(C₂H₅)₂, -N(H)(CH₃), -N(H)(C₂H₅), -NH-C(=O)-O-CH₃, -NH-C(=O)-O-C₂H₅, -NH-C(=O)-CH₃, -NH-C(=O)-C₂H₅, -C(=O)-H, -C(=O)-CH₃, -C(=O)-C₂H₅, -C(=O)-CF₃, -C(=O)-NH₂, -C(=O)-NH-CH₃, -C(=O)-NH-C₂H₅, -S(=O)₂-NH₂, -S(=O)₂-NH-CH₃, -S(=O)₂-NH-C₂H₅, -S(=O)₂-NH-Phenyl, -S(=O)₂-CH₃, -S(=O)₂-C₂H₅, -(CH₂)-Benzo[b]furanyl, -O-Phenyl, -S-Phenyl, -S-Benzyl, -O-Benzyl, Phenyl und Benzyl substituiert sein kann, wobei jeweils der zyklische Teil der Reste -O-C(=O)-Phenyl, -(CH₂)-O-C(=O)-Phenyl, -S(=O)₂-NH-Phenyl, -O-Phenyl, -S-Phenyl, -S-Benzyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-CH₃, -O-C₂H₅, -O-CH(CH₃)₂, -O-C(CH₃)₃, Methyl, Ethyl, n-Propyl, Isopropyl, n-Butyl, sec-Butyl, Isobutyl, tert-Butyl, -O-CF₃, -S-CF₃, Phenyl und -O-Benzyl substituiert sein kann,
R¹⁷ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, -(CH₂)-(C(CH₃)₃), n-Hexyl, 3-Hexyl, -(CH₂)-(CH₂)-(C(CH₃)₃), n-Heptyl, 3-Heptyl, 4-Heptyl, n-Octyl, -(CH₂)-(CH)(C₂H₅)-(CH₂)-(CH₂)-(CH₂)-(CH₃), Vinyl, 1-Propenyl, 2-Propenyl, 1-Butenyl, 2-Butenyl, 3-Butenyl, 1-Propinyl, 2-Propinyl, 1-Butinyl, 2-Butinyl und 3-Butinyl steht, wobei der Rest jeweils mit 1, 2, 3, 4 oder 5 Substituenten unabhängig voneinander ausgewählt aus der Gruppe bestehend aus F, Cl, Br, I, -CN, -NO₂, -OH, -SH, -NH₂, -N(CH₃)₂, -N(C₂H₅)₂ und -N(CH₃)(C₂H₅) substituiert sein kann;
und
R¹⁸ für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht, wobei der (hetero)cycloaliphatische Rest jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -O-C(CH₃)₃, -NH₂, -NO₂, -O-CF₃, -S-CF₃, -SH, S-CH₃, -S-C₂H₅, -S-C(CH₃)₃, Methyl, Ethyl, n-Propyl, Isopropyl, n-Butyl, sec-Butyl, Isobutyl, tert-Butyl, -C(=O)-H, -C(=O)-CH₃, -C(=O)-C₂H₅, -C(=O)-OH, -C(=O)-O-CH₃, -C(=O)-O-C₂H₅, -C(=O)-O-C(CH₃)₃, -(CH₂)-C(=O)-OH, -(CH₂)-C(=O)-O-CH₃, -(CH₂)-C(=O)-O-C₂H₅, -N(CH₃)₂, -N(C₂H₅)₂, -N(H)(CH₃), -N(H)(C₂H₅), -(CH₂)-Benzo[b]furanyl, -O-Phenyl, -O-Benzyl, Phenyl, Benzyl, Naphthyl und -(CH₂)-Naphthyl substituiert sein kann, wobei jeweils der zyklische Teil der Reste -O-Phenyl, -O-Benzyl, Phenyl, -(CH₂)-Benzo[b]furanyl, Benzyl, Naphthyl und -(CH₂)-Naphthyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-CH₃, -O-C₂H₅, Methyl, Ethyl, n-Propyl, Isopropyl, n-Butyl, sec-Butyl, Isobutyl, tert-Butyl, -O-CF₃, -S-CF₃, Phenyl und -O-Benzyl substituiert sein kann,
oder für einen Rest ausgewählt aus der Gruppe bestehend aus Phenyl, Naphthyl, Thiophenyl, Furanyl, Pyrrolyl, Pyrazolyl, Pyrazinyl, Triazolyl, Pyridinyl, Imidazolyl, Indolyl, Isoindolyl, Benzo[b]furanyl, Benzo[b]thiophenyl, Thiazolyl, Thiadiazolyl, Oxazolyl, Isoxazolyl, Pyridazinyl, Pyrazinyl, Pyrimidinyl, Indazolyl, Chinazolinyl, Chinolinyl, Isochinolinyl, Phenazinyl, Phenothiazinyl, Oxadiazolyl, Benzo[1,2,5]oxadiazolyl, Chromanyl und (1,2,3,4)-Tetrahydrochinolinyl steht, wobei der Rest jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-C(CH₃)₃, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, -S-CH(CH₃)₂, -S-C(CH₃)₃, -S-CH₂-CH₂-CH₂-CH₃, Methyl, Ethyl, n-Propyl, Isopropyl, n-Butyl, sec-Butyl, Isobutyl, tert-Butyl, (1,1)-Dimethylpropyl, -C(=O)-OH, -C(=O)-O-CH₃, -C(=OFO-C₂H₅, -C(=O)-O-C(CH₃)₃, -O-C(=O)-CH₃, -O-C(=O)-C₂H₅, -O-C(=O)-Phenyl, -(CH₂)-O-C(=O)-CH₃, -(CH₂)-O-C(=O)-C₂H₅, -(CH₂)-O-C(=O)-Phenyl, -N(CH₃)₂, -N(C₂H₅)₂, -N(H)(CH₃), -N(H)(C₂H₅), -NH-C(=O)-O-CH₃, -NH-C(=OFO-C₂H₅, -NH-C(=O)-CH₃, -NH-C(=O)-C₂H₅, -C(=O)-H, -C(=O)-CH₃, -C(=O)-C₂H₅, -C(=O)-CF₃, -C(=O)-NH₂, -C(=O)-NH-CH₃, -C(=O)-NH-C₂H₅, -S(=O)₂-NH₂, -S(=O)₂-NH-CH₃, -S(=O)₂-NH-C₂H₅, -S(=O)₂-NH-Phenyl, -S(=O)₂-CH₃, -S(=O)₂-C₂H₅, -(CH₂)-Benzo[b]furanyl, -O-Phenyl, -S-Phenyl, -S-Benzyl, -O-Benzyl, Phenyl und Benzyl substituiert sein kann, wobei jeweils der zyklische Teil der Reste -O-C(=O)-Phenyl, -(CH₂)-O-C(=O)-Phenyl, -S(=O)₂-NH-Phenyl, -O-Phenyl, -S-Phenyl, -S-Benzyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-CH₃, -O-C₂H₅, -O-CH(CH₃)₂, -O-C(CH₃)₃, Methyl, Ethyl, n-Propyl, Isopropyl, n-Butyl, sec-Butyl, Isobutyl, tert-Butyl, -O-CF₃, -S-CF₃, Phenyl und -O-Benzyl substituiert sein kann.

11. Verbindungen gemäß Anspruch 9 oder 10, **dadurch gekennzeichnet, dass**
R¹ für einen Wasserstoff-Rest steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 1-Propenyl, 2-Propenyl, 1-Propinyl und 2-Propinyl steht;
für -(CH₂)-C(=O)-O-R⁷;
oder für -(CH₂)-R¹², -(CH₂)-(CH₂)-R¹², -(CH₂)-O-R¹² , -(CH₂)-S-R¹² (CH₂)-N(CH₃)-R¹², -(CH₂)-(CH₂)-(CH₂)-R¹² oder -(CH₂)-(CH₂)-O-R¹² steht;
R² für einen Wasserstoff-Rest steht;
für einen Rest ausgewählt aus der Gruppe bestehend aus Methyl, Ethyl, n-Propyl, Isopropyl, n-Butyl, sec-Butyl, Isobutyl, tert-Butyl und n-Pentyl steht;
oder für -(CH)-R¹³, -(CH₂)-(CH₂)-R¹³, -(CH₂)-O-R¹³, -(CH₂)-S-R¹³, -(CH₂)-N(CH₃)-R¹³, -(CH₂)-(CH₂)-S-R¹³ oder-(CH₂)-(CH₂)-O-R¹³steht;
R³ für eine -S(=O)₂-R⁴-Gruppe, für eine -C(=S)-NH-R⁵-Gruppe oder für eine -C(=O)-NH-R⁶-Gruppe steht,
R⁴ für einen Rest -NR¹⁰R¹¹ steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, -(CH₂)-(C(CH₃)₃) und n-Hexyl steht;
für einen Rest ausgewählt aus der Gruppe bestehend aus Phenyl, Naphthyl, Thiophenyl, Furanyl und (1,2,3,4)- Tetrahydrochinolinyl steht, wobei der Rest jeweils mit 1, 2, 3, 4 oder 5 Substituenten unabhängig voneinander ausgewählt aus der Gruppe bestehend aus F, Cl, Br, -CF₃, -O-CH₃, -O-C₂H₅, -O-CH(CH₃)₂, -O-C(CH₃)₃, -O-CH₂-CH₂-CH₂-CH₃, -NO₂, -O-CF₃, -O-CHF₂, -O-CH₂F, -S-CF₃, -S-CHF₂, -S-CH₂F, Methyl, Ethyl, n-Propyl, Isopropyl, n-Butyl, sec-Butyl, Isobutyl, tert-Butyl, (1,1)-Dimethyl-propyl, -C(=O)-O-CH₃, -C(=O)-O-C₂H₅, -C(=O)-CH₃, -C(=O)-C₂H₅, -C(=O)-CF₃, -S(=O)₂-CH₃ und -S(=O)₂-C₂H₅, substituiert sein kann,
oder für -(CH₂)-R¹⁴, -(CH₂)-(CH₂)-R¹⁴ und -(CH₂)-(CH₂)-(CH₂)-R¹⁴ steht;
R⁵ für einen Rest ausgewählt aus der Gruppe bestehend aus Methyl, Ethyl, n-Propyl, Isopropyl, 1-Propenyl und 2-Propenyl steht;
für einen Phenyl-Rest steht, wobei der Phenyl-Rest jeweils mit 1, 2, 3, 4 oder 5 Substituenten unabhängig voneinander ausgewählt aus der Gruppe bestehend aus F, Cl, Br, -CN, -CF₃, -O-CH₃, -O-C₂H₅, -O-CH(CH₃)₂, -O-C(CH₃)₃, -O-CH₂-CH₂-CH₂-CH₃, -O-CF₃, -O-CHF₂, -O-CH₂F, -S-CF₃, -S-CHF₂, -S-CH₂F, -SH, Methyl, Ethyl, n-Propyl, Isopropyl, n-Butyl, sec-Butyl, Isobutyl, tert-Butyl, (1,1)-Dimethylpropyl, -C(=O)-O-CH₃, -C(=O)-O-C₂H₅ und C(=O)-CH₃, -C(=O)-C₂H₅ substituiert sein kann,
für-(CHR¹⁵)-R¹⁶,-(CHR¹⁵)-(CH₂₋R¹⁶, -(CHR¹⁵)-(CH₂)-(CH₂)-R¹⁶, -(CHR¹⁵)-(CH₂)-O-R¹⁶ oder -(CHR¹⁵)-(CH₂)S-R¹⁶ steht;
für einen -C(=O)-OR⁸-Rest steht
oder für einen -(CHR¹⁷)-C(=O)-OR⁹-Rest oder für einen -(CHR¹⁷)-(CH₂-C(=O)-OR⁹-Rest steht;
R⁶ für einen cycloaliphatischen Rest ausgewählt aus der Gruppe bestehend aus Cyclopentyl, Cyclohexyl und Cycloheptyl steht;
für einen Phenyl-Rest steht, der mit 1, 2, 3, 4 oder 5 Substituenten unabhängig voneinander ausgewählt aus der Gruppe bestehend aus F, CF, Br, I, -CN, -CF₃, -O-CH₃, -O-C₂H₅, -O-CH(CH₃)₂, -O-C(CH₃)₃, -O-CF₃, -O-CHF₂, -O-CH₂F, -C(=O)-O-CH₃, -C(=O)-O-C₂H₅, -C(=O)-CH₃ und -C(=O)-C₂H₅ substituiert sein kann,
oder für-(CH₂)-R¹⁸, -(CH₂)-(CH₂)-R¹⁸ oder-(CH₂)-(CH₂)-(CH₂)-R¹⁸ steht;
R⁷, R⁸, R⁹, R¹⁰ und R¹¹ unabhängig voneinander jeweils
für einen Methyl- oder Ethyl-Rest stehen;
R¹² für einen Phenyl- oder Naphthyl-Rest steht, wobei der Rest jeweils mit 1, 2, 3, 4 oder 5 Substituenten unabhängig voneinander, ausgewählt aus der Gruppe bestehend aus F, Cl, Br, -CN, -CF₃, -NO₂, Methyl, Ethyl, n-Propyl, Isopropyl, n-Butyl, sec-Butyl, Isobutyl, tert-Butyl, -C(=O)-O-CH₃, -C(=O)-O-C₂H₅, -C(=O)-CH₃ und -C(=O)-C₂H₅ substituiert sein kann,
R¹³ für einen Methyl- oder Ethyl-Rest steht;
oder für einen Phenyl-Rest steht, der Phenyl-Rest jeweils mit 1, 2, 3, 4 oder 5 Substituenten unabhängig voneinander ausgewählt aus der Gruppe bestehend aus F, Cl, Br und -CN substituiert sein kann,
R¹⁴ für einen 7,7-Dimethyl-2-oxo-bicyclo[2.2.1]heptyl- oder Phenyl-Rest steht;
R¹⁵ für einen Wasserstoff-Rest oder für einen Methyl- oder Ethyl-Rest steht;
R¹⁶ für einen Methyl- oder Ethyl-Rest steht;
für einen (hetero)cycloaliphatischen Rest ausgewählt aus der Gruppe bestehend aus Cyclopentyl, Cyclohexyl, Cyloheptyl und Tetrahydrofuranyt steht;
oder für einen Phenyl-Rest steht, der mit 1, 2, 3, 4 oder 5 Substituenten unabhängig voneinander ausgewählt aus der Gruppe bestehend aus F, Cl, Br, -CN, -CF₃, -O-CH₃, -O-C₂H₅ und -O-CH(CH₃)₂ substituiert sein kann,
R¹⁷ für einen Methyl- oder Ethyl-Rest steht;
und
R¹⁸ für einen (hetero)cycloaliphatischen Rest ausgewählt aus der Gruppe bestehend aus Cyclopentyl, Cyclohexyl und Cyloheptyl steht;
oder für einen Phenyl-Rest steht, der mit 1, 2, 3, 4 oder 5 Substituenten unabhängig voneinander ausgewählt aus der Gruppe bestehend aus F, Cl und Br substituiert sein kann.

12. Verbindungen gemäß einem oder mehreren der Ansprüche 1 bis 11 ausgewählt aus der Gruppe bestehend aus
[1] 3-Isopropyl-8-(4-nitro-benzolsulfonyl)-1,4,8-triaza-spiro[4.5]decan-2-on,
[2] 8-(2-Chlor-benzolsulfonyl)-3-isobutyl-1,4,8-triaza-spiro[4.5]decan-2-on,
[3] 3-Benzyl-8-[2-(2,2,2-trifluor-acetyl)-1,2,3,4-tetrahydro-isoquinolin-7-sulfonyl]-1,4,8-triaza-spiro[4.5]decan-2-on,
[4] 3-Benzyl-8-(4-methoxy-2,3,6-trimethyl-benzolsulfonyl)-1,4,8-triazaspiro[4.5]decan-2-on,
[5] 2-[8-(4-Butoxy-benzolsulfonyl)-2-oxo-1,4,8-triaza-spiro[4.5]dec-1-ylmethyl]-benzonitril,
[6] 2-{8-[4-(1,1-Dimethyl-propyl)-benzolsulfonyl]-2-oxo-1,4,8-triaza-spiro[4.5]dec-1-ylmethyl}-benzonitril,
[7] 3-Benzyl-8-(2-fluor-benzolsulfonyl)-1,4,8-triaza-spiro[4.5]decan-2-on,
[8] 8-(2-Chlor-benzolsuffonyl)-3-isopropyl-1,4,8-triaza-spiro[4.5]decan-2-on,
[9] 1-Benzyl-2-oxo-1,4,8-triaza-spiro[4.5]decan-8-sulfonsäuredimethylamid,
[10] 3-[8-(4-Acetyl-benzolsulfonyl)-3-(2-methylsulfanyl-ethyl)-2-oxo-1,4,8-triaza-spiro[4.5]dec-1-ylmethyl]-benzonitril,
[11] 1-(2-Fluor-benzyl)-3-isopropyl-8-(4-methoxy-2,3,6-trimethyl-benzolsulfonyl)-1,4,8-triaza-spiro[4.5]decan-2-on,
[12] 1-Butyl-8-(5-chlor-thiophen-2-sulfonyl)-3-(2-methylsulfanyl-ethyl)-1,4,8-triaza-spiro[4.5]decan-2-on,
[13] 8-(4-Acetyl-benzolsulfonyl)-1-butyl-3-(2-methylsulfanyl-ethyl)-1,4,8-triaza-spiro[4.5]decan-2-on,
[14] 1-Benzyl-3-isopmpyl-8-(2,4,6-trimethyl-benzolsulfonyl),4,8-triaza-spiro[4.5]decan-2-on,
[15] 2-(1-Butyl-3-isopropyl-2-oxo-1,4,8-triaza-spiro[4.5]decan-8-sulfonyl)-benzoesäuremethylester,
[16] 1,3-Dibenzyl-8-(thiophen-2-sulfonyl)-1,4,8-triaza-spiro[4.5]decan-2-on,
[17] 8-(3-Chlor-4-fluor-benzolsulfonyl-3-isopropyl-1-(2-phenoxy-ethyl)-1,4,8-triaza-spiro[4.5]decan-2-on,
[18] 1-Butyl-3-(2-methylsulfanyl-ethyl)-8-(toluol-3-sulfonyl)-1,4,8-triaza-spiro[4.5]decan-2-on,
[19] 1-Butyl-8-(2,4-difluor-benzolsulfonyl)-3-isopropyl-1,4,8-triaza-spiro[4.5]decan-2-on,
[20] 1,3-Dibenzyl-8-(2,5-dichlor-thlophen-3-sulfonyl)-1,4,8-triaza-spiro[4.5]decan-2-on,
[21] 3-Isopropyl-1-(2-phenoxy-ethyl)-8-(toluol-4-sulfonyl)-1,4,8-triaza-spiro[4.5]decan-2-on,
[22] 3-Benzyl-1-butyl-8-(2-trifluormethyl-benzolsulfonyl)-1,4,8-triaza-spiro[4.5]decan-2-on,
[23] 2-[1-(4-Fluor-benzyl)-3-(2-methylsulfanyl-ethyl)-2-oxo-1,4,8-triaza-spiro[4.5]decan-8-sulfonyl]-benzoesäuremethylester,
[24] 1,3-Dibenzyl-8-(2,3,5,6-tetramethyl-benzolsulfonyl)-1,4,8-triaza-spiro[4.5]decan-2-on,
[25] 1-(4-Fluor benzyl)-3-(2-methylsulfanyl-ethyl)-8-(2-trifluormethyl-benzolsulfonyl)-1,4,8-triaza-spiro[4.5]decan-2-on,
[26] 8-(4-Chlor-2,5-dimethyl-benzolsulfonyl)-1-(4-fluor-benzyl)-3-(2-methylsulfanyl-ethyl)-1,4,8-triaza-spiro[4.5]decan-2-on,
[27] 1-Benzyl-8-(4-chlor 2,5-dimethyl-benzolsulfonyl)-3-isobutyl-1,4,8-triaza-spiro[4.5]decan-2-on,
[28] 3-Benzyl-1-butyl-8-[4-(1,1-dimethyl-propyl)-benzolsulfonyl]-1,4,8-triaza-spiro[4.5]decan-2-on,
[29] 1,3-Dibenzyl-8-(3,4-dimethoxy-benzolsulfonyl)-1,4,8-triaza-spiro[4.5]decan-2-on,
[30] 1-Butyl-8-(3,4-dimethoxy-benzolsulfonylf-3-(2-methylsulfanyl-ethyl)-1,4,8-triaza-spiro[4.5]decan-2-on,
[31] 1,3-Dibenzyl-8-(4-methoxy-2,3,6-trimethyl-benzolsulfonyl)-1,4,8-triaza-spiro[4.5]decan-2-on,
[32] 1-Benzyl-8-ethanesuffonyl-3-(2-methylsulfanyl-ethyl)-1,4,8-triaza-spiro[4.5]decan-2-on,
[33] 3-Benzyl-1-butyl-8-(4-ethyl-benzolsulfonyl)-1,4,8-triaza-spiro[4.5]decan-2-on,
[34] 3-(2-Methylsuffanyl-ethyl)-1-prop-2-ynyl-8-(3-trifluomnethyl-benzolsulfonyl)-1,4,8-triaza-spiro[4.5]decan-2-on,
[35] 1,3-Dibenzyl-8-(4-ethyl-benzolsulfonyl-1,4,8-triaza-spiro[4.5]decan-2-on,
[36] 3-Benzyl-1-butyl-8-(3-chlor-benzolsulfonyl)-1,4,8-triaza-spiro[4.5]decan-2-on,
[37] 1,3-Dibenzyl-8-(2-fluor-benzolsulfonyl)-1,4,8-triaza-spiro[4.5]decan-2-on,
[38] 2-[1-(2-Cyano-benzyl)-3-(2-methylsulfanyl-ethyl)-2-oxo-1,4,8-triaza-spiro[4.5]decan-8-sulfonyl]-benzoesäuremethylester,
[39] 3-Benzyl-1-prop-2-ynyl-8-(2-trifluormethyl-benzolsulfonyl)-1,4,8-triaza-spiro[4.5]decan-2-on,
[40] 3-Benzyl-8-(3-chlor-benzolsuifonyl)-1-methyl-1,4,8-triaza-spiro[4.5]decan-2-on,
[41] 1-Benzyl-8-[4-(1,1-dimethyl-propyl)-benzolsulfonyl]-3-(2-methylsulfanyl-ethyl)-1,4,8-triaza-spiro[4.5]decan-2-on,
[42] 8-(2,4-Difluor-benzolsulfonyl)-1-(3,5-dimethyl-benzyl)-3-(2-methylsulfanyl-ethyl)-1,4,8-triaza-spiro[4.5]decan-2-on,
[43] 1,3-Dibenzyl-8-(4-nitm-benzolsulfonyl)-1,4,8-triaza-spiro[4.5]decan-2-on,
[44] 1-(4-Fluor-benzyl)-3-(2-methylsulfanyl-ethyl)-8-(toluol-4-sulfonyl)-1,4,8-triaza-spiro[4.5]decan-2-on,
[45] 1,3-Dibenzyl-8-(toluol-4-sulfonyl)-1,4,8-triaza-spiro[4.5]decan-2-on,
[46] [3-Benzyl-8-(2-methyl-5-nitro-benzolsulfonyl)-2-oxo-1,4,8-triaza-spiro[4.5]dec-1-yl]-essigsäureethylester,
[47] [3-Benzyl-8-(2-methansulfonyl-benzolsulfonyl)-2-oxo-1,4,8-triaza-spiro[4.5]dec-1-yl]-essigsäureethylester,
[48] [3-Benzyl-2-oxo-8-(thiophen-2-sulfonyl)-4,8-triaza-spiro[4.5]dec-1-yl]-essigsäureethylester,
[49] 4-(8-Ethanesulfonyl-2-oxo-1,4,8-triaza-spiro[4.5]dec-1-ylmethyl)-benzoesäuremethylester,
[50] [3-Benzyl-8-(4-methoxy-benzolsuffonyl)-2-oxo-1,4,8-triaza-spiro[4.5]dec-1-yl]-essigsäureethylester,
[51] 4-(2-Oxo-8-benzolmethansulfonyl-1,4,8-triaza-spiro[4.5]dec-1-ylmethyl)-benzoesäuremethylester,
[52] 3-[3-Isopropyl-8-(4-nitro-benzolsuffonyl)-2-oxo-1,4,8-triaza-spiro[4.5]dec-1-ylmethyl]-benzonitril,
[53] 4-[8-(Butan-1-sulfonyl)-2-oxo-1,4,8-triaza-spiro[4.5]dec-1-ylmethyl]-benzoesäuremethylester,
[54] 1-Allyl-8-(2-methansulfonyl-benzolsulfonyl)-3-(2-methylsuffanyl-ethyl)-1,4,8-triaza-spiro[4.5]decan-2-on,
[55] 1-(2-Fluor-benzyl)-2-oxo-1,4,8-triaza-spiro[4.5]decan-8-sulfonsäure dimethylamid,
[56] 8-(7,7-Dimethyl-2-oxo-bicyclo[2.2.1]hept-1-ylmethansulfonyl)-1-(2-fluorbenzyl)-1,4,8-triaza-spiro[4.5]decan-2-on,
[57] [3-(2-Methylsulfanyl-ethyl)-2-oxo-8-benzolmethansulfonyl-1,4,8-triaza-spiro[4.5]dec-1-yl]-essigsäureethylester,
[58] 1-Allyl-3-(2-methylsulfanyl-ethyl)-8-benzolmethansulfonyl-1,4,8-triaza-spiro[4.5]decan-2-on,
[59] 3-[2-Oxo-8-(2,4,6-trimethyl-benzolsulfonyl),4,8-triaza-spiro[4.5]dec-1-ylmethyl]-benzonitril,
[60] 4-[2-Oxo-8-(2,4,6-trimethyl-benzolsulfonyl)-1,4,8-triaza-spiro[4.5]dec-1-ylmethyl]-benzoesäuremethylester,
[61] [3-Benzyl-8-(5-chlor-thiophen-2-sulfonyl)-2-oxo-1,4,8-triaza-spiro[4.5]dec-1-yl]-essigsäureethylester,
[62] [3-Benzyl-8-(butan-1-sulfonyl)-2-oxo-1,4,8-triaza-spiro[4.5]dec-1-yl]-essigsäureethylester,
[63] [3-Benzyl-2-oxo-8-(toluol-3-sulfonyl)-1,4,8-triaza-spiro[4.5]dec-1-yl]-essigsäureethylester,
[64] 1-(2-Fluor-benzyl)-8-(4-methoxy-benzolsulfonyl)-1,4,8-triaza-spiro[4.5]decan-2-on,
[65] [3-Benzyl-2-oxo-8-(2-trifluormethyl-benzolsulfonyl)-1,4,8-triaza-spiro[4.5]dec-1-yl]-essigsäureethylester,
[66] (8-Benzolsulfonyl-3-benzyl-2-oxo-1,4,8-triaza-spiro[4.5]dec-1-yl)-essigsäureethylester,
[67] [3-Benzyl-2-oxo-8-(4-propyl-benzolsulfonyl)-1,4,8-triaza-spiro[4.5]dec-1-yl]-essigsäureethylester,
[68] (3-Benzyl-2-oxo-8-benzolmethansulfonyl-1,4,8-triaza-spiro[4.5]dec-1-yl)-essigsäureethylester,
[69] [3-Benzyl-8-(3-chlor-4-fluor benzotsulfonyl)-2-oxo-1,4,8-triaza-spiro[4.5]dec-1-yl]-essigsäureethylester,
[70] 1-Allyl-3-isopropyl-8-(2,4,6-trimethyl-benzolsulfonyl)-1,4,8-triaza-spiro[4.5]decan-2-on,
[71] 8-Benzolsulfonyl-3-benzyl-1-(2-fluor-benzyl)-1,4,8-triaza-spiro[4.5]decan-2-on,
[72] [3-Benzyl-2-oxo-8-(2,4,6-trimethyl-benzolsulfonyl)-1,4,8-triaza-spiro[4.5]dec-1-yl]-essigsäureethylester,
[73] [3-Benzyl-8-(4-butoxy-benzolsulfonyl)-2-oxo-1,4,8-triaza-spiro[4.5]dec-1-yl]-essigsäureethylester,
[74] [3-Benzyl-8-(2,5-dimethoxy-benzolsulfonyl)-2-oxo-1,4,8-triaza-spiro[4.5]dec-1-yl]-essigsäureethylester,
[75] 3-(2-Methylsulfanyl-ethyl)-1-naphthalin-2-ylmethyl-2-oxo-1,4,8-triaza-spiro[4.5]decan-8-suffonsäure dimethylamid,
[76] 1-(3-Cyano-benzyl)-3-isopropyl-2-oxo-1,4,8-triaza-spiro[4.5]decan-8-sulfonsäure dimethylamid,
[77] 3-{3-Isopropyl-2-oxo-8-[2-(2,2,2-trifluor-acetyl)-1,2,3,4-tetrahydro-isoquinolin-7-sulfonyl]-1,4,8-triaza-spiro[4.5]dec-1-ylmethyl}-benzonitril,
[78] 1-Allyl-3-(2-methylsulfanyl-ethyl)-8-[2-(2,2 ,2-trifluor-acetyl)-1,2,3,4-tetrahydro-isoquinolin-7-sulfonyl]-1,4,8-triaza-spiro[4.5]decan-2-on,
[79] 1-Allyl-8-(4-methoxy-2,3,6-trimethyl-benzolsulfonyl)-3-(2-methylsulfanylethyl)-1,4,8-triaza-spiro[4.5]decan-2-on,
[80] 2-(3-Benzyl-1-ethoxycarbonylmethyl-2-oxo-1,4,8-triaza-spiro[4.5]decan-8-sulfonylrbenzoesäuremethylester,
[81] 3-Benzyl-1-(2-fluor-benzyl)-8-(2-trifluormethyl-benzolsulfonyl)-1,4,8-triaza-spiro[4.5]decan-2-on,
[82] 8-(2-Chlor-benzolsufonyl)-3-(2-methylsufanyl-ethyl)-1-(2-nitro-benzyl)-1,4,8-triaza-spiro[4.5]decan-2-on,
[83] 1-Allyl-3-(2-methylsulfanyl-ethyl)-8-(toluol-3-sulfonyl)-1,4,8-triaza-spiro[4.5]decan-2-on,
[84] [8-(2-Chtor-benzolsulfonyl)-3-(2-methylsutfanyl-ethyl)-2-oxo-1,4,8-triaza-spiro[4.5]dec-1-A-essigsäureethylester,
[85] 3-Benzyl-1-(2-fluor benzyl)-8-(toluol-4-sulfonyl)-1,4,8-triaza-spiro[4.5]decan-2-on,
[86] 4-[8-(2,5-Dichlor-thiophen-3-sulfonyl)-2-oxo-1,4,8-triaza-spiro[4.5]dec-1-ylmethyl]-benzoesäuremethylester,
[87] 3-[8-(4-Fluor-benzolsuffonyl)-3-isopropyl-2-oxo-1,4,8-triaza-spiro[4.5]dec-1-ylmethyl]-benzonitril,
[88] 8-(2,5-Dimethoxy-benzolsulfonyl)-3-(2-methylsulfanyl-ethyl)-1-(2-nitrobenzyl)-1,4,8-triaza-spiro[4.5]decan-2-on,
[89] 8-(3-Chlor-4-fluor-benzolsulfonyl)-1-(2-fluor-benzyl)-1,4,8-triaza-spiro[4.5]decan-2-on,
[90] 1-(2-Fluor-benzyl)-8-(toluol-3-sulfonyl)-1,4,8-triaza-spiro[4.5]decan-2-on,
[91] 1-(2,6-Dichlor benzyl)-8-(4,5-dichlor thiophen-2-sulfonyl)-3-isobutyl-1,4,8-triaza-spiro[4.5]decan-2-on,
[92] 8-(5-Chlor-thiophen-2-sulfonyl-(2-fluor-benzyl)-1,4,8-triaza-spiro[4.5]decan-2-on,
[93] 3-Benzyl-1-(2-fluor-benzyl)-8-(4-propyl-benzolsulfonyl-1,4,8-triaza-spiro[4.5]decan-2-on,
[94] 3-[8-(Butan-1-sulfonyl)-3-isopropyl-2-oxo-1,4,8-triaza-spiro[4.5]dec-1-ylmethyl]-benzonitril,
[95] 3-[8-(2-Methansulfonyl-benzolsulfonyl)-2-oxo-1,4,8-triaza-spiro[4.5]dec-1-ylmethyl]-benzonitril,
[96] 8-(2-Ftuor-benzo)sulfonyl)-1-(2-fluor-benzyl)-1,4,8-triaza-spiro[4.5]decan-2-on,
[97] 2-[1-(3-Cyano-benzyl)-3-isopropyl-2-oxo-1,4,8-triaza-spiro[4.5]decan-8-sulfonyl]-benzoesäuremethylester,
[98] 3-Benzyl-1-(2-fluor-benzyl)-8-(5-fluor-2-methyl-benzolsulfony)-1,4,8-triaza-spiro[4.5]decan-2-on,
[99] 1-Allyl-8-(4-methoxy-benzolsulfonyl)-3-(2-methylsulfanyl-ethyl)-1,4,8-triaza-spiro[4.5]decan-2-on,
[100] 1-(3,5-Dimethyl-benzyl)-3-isobutyl-8-(thiophen-2-suffonyl)-1,4,8-triazaspiro[4.5]decan-2-on,
[101] 3-(2-Methylsulfanyl-ethyl)-8-(4-nitro-benzolsufonyl)-1-(2-nitro-benzyl)-1,4,8-triaza-spiro[4.5]decan-2-on,
[102] 2-Isobutyl-3-oxo-1,4,8-triaza-spiro[4.5]decan-8-thiocarbonsäure-o-tolylamid,
[103] 2-Benzyl-3-oxo-1,4,8-triaza-spiro[4.5]decan-8-thiocarbonsäure isopropylamid,
[104] 1-Butyl-3-isopropyl-2-oxo-1,4,8-triaza-spiro[4.5]decan-8-thiocarbonsäure (tetrahydro-furan-2-ylmethyl)-amid,
[105] 3-{[1-Methyl]-3-(2-methylsulfanyl-ethyl)-2-oxo-1,4,8-triaza-spiro[4.5]decan-8-carbothioyl]-amino}-buttersäureethylester,
[106] 1-Allyl-3-isopropyl-2-oxo-1,4,8-triaza-spiro[4.8]decan-8-thiocarbonsäure isopropylamid,
[107] 1-Butyl-3-isopropyl-2-oxo-1,4,8-triaza-spiro[4.5]decan-8-thiocarbonsäure isopropylamid,
[108] 3-Benzyl-1-(2-fluor-benzyl)-2-oxo-1,4,8-triaza-spiro[4.5]decan-8-thiocarbonsäure (2-methoxy-benzol)-amid,
[109] [8-Isopropylthiocarbamoyl-3-(2-methylsulfanyl-ethyl-2-oxo-1,4,8-triaza-spiro[4.5]dec-1-yl]-essigsäureethylester,
[110] 1-Methyl-3-(2-methylsulfanyl-ethyl)-2-oxo-1,4,8-triaza-spiro[4.5]decan-8-thiocarbonsäure m-tolylamid,
[111] 1-Methyl-3-(2-methylsulfanyl-ethyl)-2-oxo-1,4,8-triaza-spiro[4.5]decan-8-thiocarbonsäure 4-methoxy-benzylamid,
[112] 2-Isobutyl-3-oxo-1,4,8-triaza-spiro[4.5]decan-8-thiocarbonsäure (4-fluorbenzol)-amid,
[113] [8-Ethoxycarbonylaminocarbothioyl-3-(2-methylsulfanyl-ethyl)-2-oxo-1,4,8-triaza-spiro[4.5]dec-1-yl]-essigsäureethylester
[114] 3-{[1-Ethoxycarbonylmethyl-3-(2-methylsuffanyl-ethyl)-2-oxo-1,4,8-triaza-spiro[4.5]decan-8-carbothioyl]-amino}-buttersäureethylester,
[115] 1-Allyl-3-isopropyl-2-oxo-1,4,8-triaza-spiro[4.5]decan-8-thiocarbonsäure (2-methoxy-benzol)-amid,
[116] 1-Butyl-3-isopropyl-2-oxo-1,4,8-triaza-spiro[4.5]decan-8-thiocarbonsäure benzolamid,
[117] 1-Butyl-3-isopropyl-2-oxo-1,4,8-triaza-spiro[4.5]decan-8-thiocarbonsäure (2,5-dichlor-benzol)-amid,
[118] 3-Benzyl-1-methyl-2-oxo-1,4,8-triaza-spiro[4.5]decan-8-thiocarbonsäure (2.5-difluor-benzol)-amid,
[119] 1-Methyl-3-(2-methylsulfanyl-ethyl)-2-oxo-1,4,8-triaza-spiro[4.5]decan-8-thiocarbonsäure (2-brom-benzol)-amid,
[120] 1-Butyl-3-(2-methylsulfanyl-ethyl)-2-oxo-1,4,8-triaza-spiro[4.5]decan-8-thiocarbonsäure (2,5-dichlor benzol)-amid,
[121] 3-(2-Methylsulfanyl-ethyl)-1-naphthalin-2-ylmethyl-2-oxo-1,4,8-triaza-spiro[4.5]decan-8-thiocarbonsäure (2-methoxy-ethyl)-amid,
[122] 1-Butyl-3-isopropyl-2-oxo-1,4,8-triaza-spiro[4.5]decan-8-thiocarbonsäure (2-fluor-benzol)-amid,
[123] 3-Isobutyl-2-oxo-1-prop-2-ynyl-1,4,8-triaza-spiro[4.5]decan-8-thiocarbonsäure allylamid,
[124] 1-Butyl-3-Isopropyl-2-oxo-1,4,8-triaza-spiro[4.5]decan-8-thiocarbonsäure m-tolylamid,
[125] 1-Allyl-3-isopropyl-2-oxo-1,4,8-triaza-spiro[4.5]decan-8-thiocarbonsäure (4-fluor benzol)-amid,
[126] 4-(8-Allylthiocarbamoyl-3-isopropyl-2-oxo-1,4,8-triaza-spiro[4.5]dec-1-ylmethyl)-benzoesäuremethylester,
[127] 1-Butyl-3-(2-methylsulfanyl-ethyl)-2-oxo-1,4,8-triaza-spiro[4.5]decan-8-thiocarbonsäure (2-methoxy-benzol)-amid,
[128] 1-Allyl-3-isopropyl-2-oxo-1,4,8-triaza-spiro[4.5]decan-8-thiocarbonsäure (2,5-difluor-benzol)-amid,
[129] 1-(2-Cyano-benzyl)-3-(2-methylsulfanyl-ethyl)-2-oxo-1,4,8-triaza-spiro[4.5]decan-8-thiocarbonsäure allylamid,
[130] 1-(3-Cyano-benzyl)-3-(2-methylsulfanyl-ethyl)-2-oxo-1,4,8-triaza-spiro[4.5]decan-8-thiocarbonsäure allylamid,
[131] 3-[(3-Benzyl-1-methyl-2-oxo-1,4,8-triaza-spiro[4.5]decan-8-carbothioyl)-amino]-buttersäureethylester,
[132] 1-Butyl-3-(2-methylsulfanyl-ethyl)-2-oxo-1,4,8-triaza-spiro[4.5]decan-8-thiocarbonsäure cyclohexylmethyl-amid,
[133] 1-Methyl-3-(2-methylsulfanyl-ethyl)-2-oxo-1,4,8-triaza-spiro[4.5]decan-8-thiocarbonsäure (4-chlor-benzol)-amid,
[134] 1-Methyl-3-(2-methylsulfanyl-ethyl)-2-oxo-1,4,8-triaza-spiro[4.5]decan-8-thiocarbonsäure 4-fluor-benzylamid,
[135] 4-[8-(2,6-Dichlor-benzolthlocarbamoyl)-3-isopropyl-2-oxo-1,4,8-triaza-spiro[4.5]dec-1-ylmethy]-benzoesäuremethylester,
[136] 1-Benzyl-3-isopropyl-2-oxo-1,4,8-triaza-spiro[4.5]decan-8-thiocarbonsäure (2,5-dichlor-benzol)-amid,
[137] 4-{3-Isopropyl-2-oxo-8-[(tetrahydro-furan-2-ylmethyl)-thiocarbamoyl]-1,4,8-triaza-spiro[4.5]dec-1-ylmethyl}-benzoesäuremethylester,
[138] 1-Methyl-3-(2-methylsulfanyl-ethyl)-2-oxo-1,4,8-triaza-spiro[4.5]decan-8-thiocarbonsäure (2,5-difluor-benzol)-amid,
[139] 1-Benzyl-3-(2-methylsulfanyl-ethyl)-2-oxo-1,4,8-triaza-spiro[4.5]decan-8-thiocarbonsäure (4-isopropyl-benzol)-amid,
[140] 1-Benzyl-3-(2-methylsulfanyl-ethyl)-2-oxo-1,4,8-triaza-spiro[4.5]decan-8-thiocarbonsäure (2,5-difluor-benzol)-amid,
[141] 1-(2-Cyano-benzyl)-3-isobutyl-2-oxo-1,4,8-triaza-spiro[4.5]decan-8-thiocarbonsäure (3-acetyl-benzol)-amid,
[142] 1-Butyl-3-Isopropyl-2-oxo-1,4,8-triaza-spiro[4.5]decan-8-thiocarbonsäure (3-chlor-benzol)-amid,
[143] 2-[(1-Butyl-3-isopropyl-2-oxo-1,4,8-triaza-spiro[4.5]decan-8-carbothioyl)-amino]-benzoesäuremethylester,
[144] (3-Benzyl-8-ethoxycarbonylaminocarbothioyl-2-oxo-1,4,8-triaza-spiro[4.5]dec-1-yl)-essigsäureethylester,
[145] 2-[(3-Benzyl-1-ethoxycarbonylmethyl-2-oxo-1,4,8-triaza-spiro[4.5]decan-8-carbothioyl)-amino]-benzoesäuremethylester,
[146] [1-(2-Cyano-benzyl)-3-(2-methylsulfanyl-ethyl)-2-oxo-1,4,8-triaza-spiro[4.5]decan-8-carbothioyl]-carbamidsäureethylester,
[147] 1-(2-Cyano-benzyl)-3-(2-methylsulfanyl-ethyl)-2-oxo-1,4,8-triaza-spiro[4.5]decan-8-thiocarbonsäure (2-chlor-5-trifluormethyl-benzol)-amid,
[148] 3-(2-Methylsulfanyl-ethyl)-1-naphthalin-2-ylmethyl-2-oxo-1,4,8-triazaspiro[4.5]decan-8-thiocarbonsäure (1-benzol-ethyl)-amid,
[149] 1-Allyl-3-isopropyl-2-oxo-1,4,8-triaza-spiro[4.5]decan-8-thiocarbonsäure pentafluorbenzol-amid,
[150] 1-Butyl-3-isopropyl-2-oxo-1,4,8-triaza-spiro[4.5]decan-8-thiocarbonsäure 4-chlor-benzylamid,
[151] 3-(2-Methylsulfanyl-ethyl)-1-naphthalin-2-ylmethyl-2-oxo-1,4,8-triaza-spiro[4.5]decan-8-thiocarbonsäure o-tolylamid,
[152] 1-Butyl-3-isopropyl-2-oxo-1,4,8-triaza-spiro[4.5]decan-8-thiocarbonsäure pentafluorbenzol-amid,
[153] 2-{[1-(2-Cyano-benzyl)-3-(2-methytsulfanyl-ethyl)-2-oxo-1,4,8-triaza-spiro[4.5]decan-8-carbothioyl]-amino}-benzoesäuremethylester,
[154] 1-Benzyl-3-isopropyl-2-oxo-1,4,8-triaza-spiro[4.5]decan-8-thiocarbonsäure pentafluorbenzol-amid,
[155] 1-Benzyl-3-isopropyl-2-oxo-1,4,8-triaza-spiro[4.5]decan-8-thiocarbonsäure (2-methoxy-benzol)-amid,
[156] 1-Benzyl-3-Isopropyl-2-oxo-1,4,8-triaza-spiro[4.5]decan-8-thiocarbonsäure benzolamid,
[157] 1-Benzyl-3-isopropyl-2-oxo-1,4,8-triaza-spiro[4.5]decan-8-thiocarbonsäure (2,5-difluor-benzol)-amid,
[158] 1-Butyl-3-(2-methylsulfanyl-ethyl)-2-oxo-1,4,8-triaza-spiro[4.5]decan-8-thiocarbonsäure (4-ethoxy-benzol)-amid,
[159] [3-Benzyl-8-(cyclohexylmethyl-thiocarbamoyl)-2-oxo-1,4,8-triaza-spiro[4.5]dec-1-yl]-essigsäureethylester,
[160] 1-Butyl-3-(2-methylsulfanyl-ethyl)-2-oxo-1,4,8-triaza-spiro[4.5]decan-8-thiocarbonsäure benzylamid,
[161] 1-(2-Cyano-benzyl)-3-(2-methylsulfanyl-ethyl)-2-oxo-1,4,8-triaza-spiro[4.5]decan-8-thiocarbonsäure m-tolylamid,
[162] 1-Allyl-3-isopropyl-2-oxo-1,4,8-triaza-spiro[4.5]decan-8-thiocarbonsäure 4-fluor-benzylamid,
[163] 3-Isobutyl-2-oxo-1-prop-2-ynyl-1,4,8-triaza-spiro[4.5]decan-8-thiocarbonsäure (3-acetyl-benzol)-amid,
[164] 4-(3-Isopropyl-2-oxo-8-pentafluorbenzolthiocarbamoyl-1,4,8-triaza-spiro[4.5]dec-1-ylmethyl)-benzoesäuremethylester,
[165] 3-(2-Methylsulfanyl-ethyl)-1-naphthalin-2-ylmethyl-2-oxo-1,4,8-triaza-spiro[4.5]decan-8-thiocarbonsäure (3-acetyl-benzol)-amid,
[166] 3-Benzyl-1-(2-triaza-benzyl)-2-oxo-1,4,8-triaza-spiro[4.5]decan-8-thiocarbonsäure m-tolylamid,
[167] 4-(8-Ethoxycarbonylaminocarbothioyl-3-isopropyl-2-oxo-1,4,8-triaza-spiro[4.5]dec-1-ylmethyl)-benzoesäuremethylester,
[168] 1-(4-Fluor-benzyl)-3-(2-methylsulfanyl-ethyl)-2-oxo-1,4,8-triazaspiro[4.5]decan-8-thiocarbonsäure (tetrahydro-furan-2-ylmethyl)-amid,
[169] 1-(2-Cyano-benzyl)-3-isobutyl-2-oxo-1,4,8-triaza-spiro[4.5]decan-8-thiocarbonsäure (3,5-bis-trifluormethyl-benzol)-amid,
[170] 1-(2-Cyano-benzyl)-3-isobutyl-2-oxo-1,4,8-triaza-splro[4.5]decan-8-thiocarbonsäure benzylamid,
[171] 3-Benzyl-1-(2-fluor-benzyl)-2-oxo-1,4,8-triaza-spiro[4.5]decan-8-thiocarbonsäure cyclohexylmethyl-amid,
[172] 1-(3-Cyano-benzyl)-3-(2-methylsulfanyl-ethyl)-2-oxo-1,4,8-triaza-spiro[4.5]decan-8-thiocarbonsäure (4-chlor-benzol)-amid,
[173] 1-Butyl-3-(2-methylsulfanyl-ethyl-2-oxo-1,4,8-triaza-spiro[4.5]decan-8-thiocarbonsäure pentafluorbenzol-amid,
[174] [3-Benzyl-1-(2-fluor-benzyl)-2-oxo-1,4,8-triaza-spiro[4.5]decan-8-carbothioyl]-carbamidsäureethylester,
[175] 3-Benzyl-1-methyl-2-oxo-1,4,8-triaza-spiro[4.5]decan-8-thiocarbonsäure 4-chlor-benzylamid,
[176] 1-(4-Fluor-benzyl)-3-(2-methylsulfanyl-ethyl)-2-oxo-1,4,8-triaza-spiro[4.5]decan-8-thiocarbonsäure isopropylamid,
[177] 1-Benzyl-3-isopropyl-2-oxo-1,4,8-triaza-spiro[4.5]decan-8-thiocarbonsäure (3,5-dichlor-benzol)-amid,
[178] [1-(3-Cyano-benzyl)-3-(2-methylsulfanyl-ethyl)-2-oxo-1,4,8-triaza-spiro[4.5]decan-8-carbothioyl]-carbamidsäureethylester,
[179] 1-Benzyl-3-isopropyl-2-oxo-1,4,8-triaza-spiro[4.5]decan-8-thiocarbonsäure 4-fluor-benzylamid,
[180] 1-Allyl-3-isopropyl-2-oxo-1,4,8-triaza-spiro[4.5]decan-8-thiocarbonsäure (3,5-dichlor-benzol)-amid,
[181] 1-(3-Cyano-benzyl)-3-(2-methylsulfanyl-ethyl)-2-oxo-1,4,8-triaza-spiro[4.5]decan-8-thiocarbonsäure (1-benzol-ethyl)-amid,
[182] 1-(4-Fluor-benzyl)-3-(2-methylsulfanyl-ethyl)-2-oxo-1,4,8-triaza-spiro[4.5]decan-8-thiocarbonsäure 4-fluor-benzylamid,
[183]1-(2-Cyano-benzyl)-3-(2-methylsulfanyl-ethyl)-2-oxo-1,4,8-triaza-spiro[4.5]decan-8-thiocarbonsäure (2-methoxy-benzol)-amid,
[184] 1-(3-Cyano-benzyl)-3-(2-methytsuffanyl-ethyl)-2-oxo-1,4,8-triaza-spiro[4.5]decan-8-thiocarbonsäure (2-methoxy-benzol)-amid,
[185] 1-(3-Cyano-benzyl)-3-(2-methylsulfanyl-ethyl)-2-oxo-1,4,8-triaza-spiro[4.5]decan-8-thiocarbonsäure 4-chlor-benzylamid,
[186] 1-(4-Fluor-benzyl)-3-(2-methylsulfanyl-ethyl)-2-oxo-1,4,8-triaza-spiro[4.5]decan-8-thiocarbonsäure o-tolylamid,
[187]3-Benzyl-1-(2-fluor benzyl)-2-oxo-1,4,8-triaza-spiro[4.5]decan-8-thiocarbonsäure (2,6-dichlor-benzol)-amid,
[188] 3-Benzyl-1-(2-fluor-benzyl)-2-oxo-1,4,8-triaza-spiro[4.5]decan-8-thiocarbonsäure (2-chlor-5-trifluormethyl-benzol)-amid,
[189] 1-Butyl-3-(2-methylsulfanyl-ethyl)-2-oxo-1,4,8-triaza-spiro[4.5]decan-8-thiocarbonsäure (4-fluor-benzol)-amid,
[190] 3-Isobutyl-2-oxo-1-prop-2-ynyl-1,4,8-triaza-spiro[4.5]decan-8-thiocarbonsäure (2,6-dichlor-benzol)-amid,
[191]4-[8-(4-Chlor benzylthiocarbamoyl]-3-isopropyl-2-oxo-1,4,8-triaza-spiro[4.5]dec-1-ylmethyl]-benzoesäuremethylester,
[192] 1-(4-Fluor benzyl)-3-(2-methylsulfanyl-ethyl)-2-oxo-1,4,8-triaza-spiro[4.5]decan-8-thiocarbonsäure (3-cyano-benzol)-amid,
[193]1-(2-Cyano-benzyl)-3-(2-methylsulfanyl-ethyl)-2-oxo-1,4,8-triaza-spiro[4.5]decan-8-thiocarbonsäure 4-chlor-benzylamid,
[194] 1-Methyl-3-(2-methylsulfanyl-ethyl)-2-oxo-1,4,8-triaza-spiro[4.5]decan-8-thiocarbonsäure (3-trifluormethyl-benzol)-amid,
[195] 3-Isobutyl-2-oxo-1-prop-2-ynyl-1,4,8-triaza-spiro[4.5]decan-8-thiocarbonsäure (3,5-dichlor-benzol)-amid,
[196] 1-(4-Fluor-benzyl)-3-(2-methylsulfanyl-ethyl)-2-oxo-1,4,8-triaza-spiro[4.5]decan-8-thiocarbonsäure (1-benzol-ethyl)-amid,
[197] 3-Benzyl-1-(2-fluor-benzyl)-2-oxo-1,4,8-triaza-spiro[4.5]decan-8-thiocarbonsäure 4-chlor-benzylamid,
[198] 2-Isopropyl-3-oxo-1,4,8-triaza-spiro[4.5]decan-8-thiocarbonsäure (4-fluorbenzol)-amid,
[199] 1-Benzyl-3-isopropyl-2-oxo-1,4,8-triaza-spiro[4.5]decan-8-thiocarbonsäure benzylamid,
[200] 1-(4-Fluor-benzyl)-3-(2-methylsulfanyl-ethyl)-2-oxo-1,4,8-triazaspiro[4.5]decan-8-thiocarbonsäure (4-ethoxy-benzol)-amid,
[201] 3-Benzyl-1-(2-fluor-benzyl)-2-oxo-1,4,8-triaza-spiro[4.5]decan-8-thiocarbonsäure (3-acetyl-benzol)-amid,
[202] 3-Benzyl-1-methyl-2-oxo-1,4,8-triaza-spiro[4.5]decan-8-carbonsäure cyclohexylamid,
[203] [3-(2-Methylsulfanyl-ethyl)-2-oxo-8-benzolcarbamoyl-1,4,8-triazaspiro[4.5]dec-1-yl]-essigsäureethylester,
[204] 1-Allyl-3-isopropyl-2-oxo-1,4,8-triaza-spiro[4.5]decan-8-carbonsäure (2,5-dimethoxy-benzol)-amid,
[205] 1-Methyl-3-(2-methylsulfanyl-ethyl)-2-oxo-1,4,8-triaza-spiro[4.5]decan-8-carbonsäure (3-fluor-benzol)-amid,
[206] 4-(8-Cyclohexylcarbamoyl-3-isopropyl-2-oxo-1,4,8-triaza-spiro[4.5]dec-1-ylmethyl)-benzoesäuremethylester,
[207] 1-Butyl-3-(2-methylsulfanyl-ethyl)-2-oxo-1,4,8-triaza-spiro[4.5]decan-8-carbonsäure benzolamid,
[208] 1-Benzyl-3-(2-methylsulfanyl-ethyl)-2-oxo-1,4,8-triaza-spiro[4.5]decan-8-carbonsäure (3-cyano-benzol)-amid,
[209] 1-Allyl-3-isopropyl-2-oxo-1,4,8-triaza-spiro[4.5]decan-8-carbonsäure (2,5-difluor-benzol)-amid,
[210] 1-Butyl-3-(2-methylsulfanyl-ethyl)-2-oxo-1,4,8-triaza-spiro[4.5]decan-8-carbonsäure (3,4,5-trimethoxy-benzol)-amid,
[211] 1-Benzyl-3-(2-methylsulfanyl-ethyl-2-oxo-1,4,8-triaza-spiro[4.5]decan-8-carbonsäure (4-chlor-3-trifluormethyl-benzol)-amid,
[212] 1-Methyl-3-(2-methylsulfanyl-ethyl)-2-oxo-1,4,8-triaza-spiro[4.5]decan-8-carbonsäure (5-chlor-2-methoxy-benzol)-amid,
[213] 1-Benzyl-3-(2-methylsulfanyl-ethyl)-2-oxo-1,4,8-triaza-spiro[4.5]decan-8-carbonsäure (4-ethoxy-benzol)-amid,
[214] 1-Benzyl-3-(2-methylsulfanyl-ethyl)-2-oxo-1,4,8-triaza-spiro[4.5]decan-8-carbonsäure (3-acetyl-benzol)-amid,
[215]3-[(1-Butyl-3-isopropyl-2-oxo-1,4,8-triaza-spiro[4.5]decan-8-carbonyl)-amino]-benzoesäureethylester,
[216] 1-Butyl-3-isopropyl-2-oxo-1,4,8-triaza-spiro[4.5]decan-8-carbonsäure (2,5-dimethoxy-benzol)-amid,
[217] 1-(2-Cyano-benzyl)-3-isobutyl-2-oxo-1,4,8-triaza-spiro[4.5]decan-8-carbonsäure 3,4-dichlor-benzylamid,
[218] 1-(2-Cyano-benzyl)-3-isobutyl-2-oxo-1,4,8-triaza-spiro[4.5]decan-8-carbonsäure (2,5-difluor-benzol)-amid,
[219] 1-Butyl-3-(2-methylsulfanyl-ethyl)-2-oxo-1,4,8-triaza-spiro[4.5]decan-8-carbonsäure 3,4-dichlor-benzylamid,
[220] 1-Allyl-3-isopropyl-2-oxo-1,4,8-triaza-spiro[4.5]decan-8-carbonsäure (4-trifluormethoxy-benzolfamid,
[221] 1-B utyl-3-(2-methylsulfanyl-ethyl)-2-oxo-1,4,8-triaza-spiro[4.5]decan-8-carbonsäure (4-ethoxy-benzol)-amid,
[222] 1-Methyl-3-(2-methylsulfanyl-ethyl)-2-oxo-1,4,8-triaza-spiro[4.5]decan-8-carbonsäure (4-trifluormethoxy-benzolramid,
[223] 4-[3-Isopropyl-8-(3-methoxy-benzolcarbamoyl)-2-oxo-1,4,8-triaza-spiro[4.5]dec-1-ylmethyl]-benzoesäuremethylester,
[224] 1-(2-Cyano-benzy)-3-(2-methylsufanyl)-ethyl)-2-oxo-1,4,8-triaza-spiro[4.5]decan-8-carbonsäure benzolamid,
[225][3-Benzyl-8-(3,4-dichlor benzylcarbamoyl)-2-oxo-1,4,8-triaza-spiro[4.5]dec-1-yl]-essigsäureethylester,
[226] 1-(2-Cyano-benzyl)-3-(2-methylsulfanyl-ethyl)-2-oxo-1,4,8-triaza-spiro[4.5]decan-8-carbonsäure (3-fluor-benzol)-amid,
[227] 1-Butyl-3-(2-methylsulfanyl-ethyl)-2-oxo-1,4,8-triaza-spiro[4.5]decan-8-carbonsäure (4-chlor-3-trifluormethyl-benzol)-amid,
[228] 1-(4-Fluor benzyl)-3-(2-methylsulfanyl-ethyl)-2-oxo-1,4,8-triaza-spiro[4.5]decan-8-carbonsäure 3,4-dichlor-benzylamid,
[229] 1-(2-Cyano-benzyl)-3-(2-methylsulfanyl-ethyl)-2-oxo-1,4,8-triaza-spiro[4.5]decan-8-carbonsäure 3,4-dichlor-benzylamid,
[230] 3-Benzyl-1-(2-fluor-benzyl)-2-oxo-1,4,8-triaza-spiro[4.5]decan-8-carbonsäure (2,5-difluor-benzol)-amid,
[231] 3-(2-Methylsulfanyl-ethyl)-1-naphthalen-2-ylmethyl-2-oxo-1,4,8-triaza-spiro[4.5]decan-8-carbonsäure (4-ethoxy-benzot)-amid,
[232] 4-[8-(3,4-Dichlor-benzylcarbamoyl)-3-isopropyl-2-oxo-1,4,8-triaza-spiro[4.5]dec-1-ylmethyl]-benzoesäuremethylester,
[233] 3-Benzyl-(2-fluor-benzyl)-2-oxo-1,4,8-triaza-spiro[4.5]decan-8-carbonsäure 3,4-dichlor-benzylamid,
[234] 1-(2-Cyano-benzyl)-3-(2-methylsulfanyl-ethyl)-2-oxo-1,4,8-triaza-spiro[4.5]decan-8-carbonsäure (3-acetyl-benzol)-amid,
[235] 4-{[3-Benzyl-1-(2-fluor benzyl)-2-oxo-1,4,8-triaza-spiro[4.5]decan-8-carbonyl]-amino}-benzoesäureethylester,
[236] 1-(3-Cyano-benzyl)-3-(2-methylsulfanyl-ethyl)-2-oxo-1,4,8-triaza-spiro[4.5]decan-8-carbonsäure (3-methoxy-benzol)-amid,
[237] 1-(4-Fluor-benzyl)-3-(2-methylsulfanyl-ethyl)-2-oxo-1,4,8-triaza-spiro[4.5]decan-8-carbonsäure (4-trifluormethoxy-benzol)-amid,
[238] 3-Benzyl-1-(2-fluor-benzyl)-2-oxo-1,4,8-triaza-spiro[4.5]decan-8-carbonsäure cyclohexylamid,
[239] 1-(4-Fluor-benzyl)-3-(2-methysulfanyl-ethyl)-2-oxo-1,4,8-triaza-spiro[4.5]decan-8-carbonsäure phenethyl-amid,
[240] 3-(2-Methylsulfanyl-ethyl)-1-naphthalen-2-ylmethyI-2-oxo-1 ,4.8-triaza-spiro[4.5]decan-8-carbonsäure (3-fluor-benzol)-amid,
[241] 4-[8-(4-Chlor-3-trifluormethyl-benzolcarbamoyl)-3-isopropyl-2-oxo-1,4,8-triaza-spiro[4.5]dec-1-ylmethyl]-benzoesäuremethylester,
[242] 1-(3-Cyano-benzyl)-3-(2-methylsulfanyl-ethyl)-2-oxo-1,4,8-triaza-spiro[4.5]decan-8-carbonsäure (2,5-dimethoxy-benzol)-amid,
[243] 1-(4-Fluor-benzyl)-3-(2-methylsulfanyl-ethyl)2-oxo-1,4,8-triaza-spiro[4.5]decan-8-carbonsäure (4-ethoxy-benzol)-amid,
[244] 1-(4-Fluor-benzyl)-3-(2-methylsulfanyl-ethyl)-2-oxo-1,4,8-triaza-spiro[4.5]decan-8-carbonsäure (3-cyano-benzol)-amid und
[245] [3-Benzyl-8-(3-fluor-benzolcarbamoyl)-2-oxo-1,4,8-triaza-spiro[4.5]dec-1-yl]-essigsäureethylester,
[246] 1,3-Dibenzyl-8-methansulfonyl-1,4,8-triaza-spiro[4.5]decan-2-on,
[247] 8-Benzolsulfonyl-1,3-dibenzyl-1,4,8-triaza-spiro[4.5]decan-2-on,
[248] 1,3-Dibenzyl-8-(4-chlor-benzolsulfonyl)-1,4,8-triaza-spiro[4.5]decan-2-on,
[249] 1,3-Dibenzyl-8-(4-methoxy-benzolsulfonyl)-1,4,8-triaza-spiro[4.5]decan-2-on,
[250] 1,3-Dibenzyl-2-oxo-1,4,8-triaza-spiro[4.5]decan-8-carbonsäurephenylamid,
[251] 1,3-Dibenzyl-2-oxo-1,4,8-triaza-spiro[4.5]decan-8-carbonsäure-(4-methoxy-phenyl)-amid,
[252] 1,3-Dibenzyl-2-oxo-1,4,8-triaza-spiro[4.5]decan-8-thiocarbonsäurephenylamid,
[253] 1,3-Dibenzyl-2-oxo-1,4,8-triaza-spiro[4.5]decan-8-thiocarbonsäure-(2,4-difluor-phenyl)-amid,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

13. Verfahren zur Herstellung substituierter 1,4,8-Triazaspiro[4.5]decan-2-on-Verbindungen gemäß einem oder mehreren der Ansprüche 1 bis 12, **dadurch gekennzeichnet, daß** ein geschütztes Piperidin-4-on der allgemeinen Formel 11, 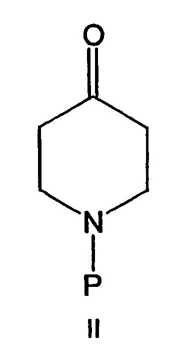 worin P für eine Schutzgruppe steht, durch Umsetzung mit wenigstens einer Verbindung der allgemeinen Formel III, 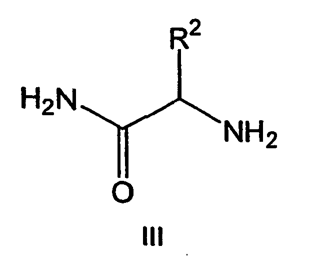 worin R² die Bedeutung gemäß einem oder mehreren der Ansprüche 1 bis 12 hat, in wenigstens eine Verbindung der allgemeinen Formel IV, 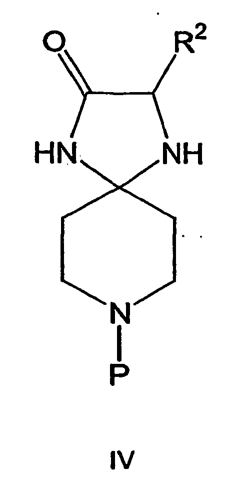 worin P und R² die vorstehend genannte Bedeutung haben, überführt wird, welche ggf. gereinigt und/oder ggf. isoliert wird, und gegebenenfalls durch Umsetzung mit wenigstens einer Verbindung der allgemeinen Formel R¹-X¹, worin R¹ die Bedeutung gemäß einem oder mehrerer der Ansprüche 1 bis 12 hat und X¹ für eine geeignete Austrittsgruppe, vorzugsweise für einen Halogen-Rest, steht, ggf. in Gegenwart wenigstens einer Base, in wenigstens eine Verbindung der allgemeinen Formel V, 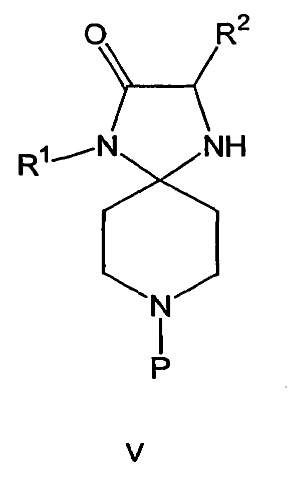 worin R¹, R² und P die vorstehend genannte Bedeutung haben, überführt wird, und diese ggf. gereinigt und/oder ggf. isoliert wird, und ggf. durch Abspaltung der Schutzgruppe P in wenigstens eine Verbindung der allgemeinen Formel VI, 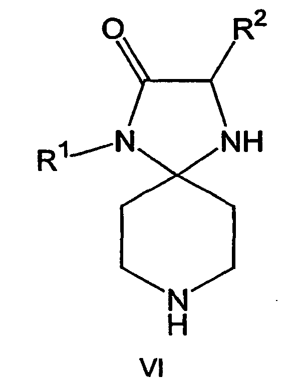 überführt wird, diese ggf. gereinigt und/oder ggf. isoliert wird, und wenigstens eine Verbindung der allgemeinen Formel IV, V oder VI durch Umsetzung mit einer Sulfonylverbindung der allgemeinen Formel R⁴-SO₂-X², einem Isothiocyanat der allgemeinen Formel R⁵-NCS oder einem Isocyanat der allgemeinen Formel R⁶-NCO, worin die Reste R⁴, R⁵ und R⁶ jeweils die Bedeutung gemäß einem oder mehreren der Ansprüche 1 bis 12 haben und und X² für eine geeignete Austrittsgruppe, vorzugsweise für einen Halogen-Rest, in wenigstens eine Verbindung der allgemeinen Formel I gemäß Anspruch 1 überführt wird, worin die Reste R¹-A³ jeweils die Bedeutung gemäß einem oder mehreren der Ansprüche 1 bis 12 haben, und diese Verbindung ggf. gereinigt und/oder ggf. isoliert wird.

14. Arzneimittel enthaltend wenigstens eine substituierte 1,4,8- Triazaspiro[4.5]decan-2-on-Verbindung gemäß einem oder mehreren der Ansprüche 1 bis 12 und ggf. einen oder mehrere pharmazeutisch verträgliche Hilfsstoffe.

15. Arzneimittel gemäß Anspruch 14 zur Regulation, insbesondere zur Hemmung, der Noradrenalin-Wiederaufnahme (Noradrenalin-Uptake), zur Regulation, insbesondere zur Hemmung, der 5-Hydroxy Tryptophan-Wiederaufnahme (5-HT-Uptake) und/oder zur Batrachotoxin-(BTX)-Rezeptor-Regulation und/oder zur CB2-Rezeptor-Regulation.

16. Arzneimittel gemäß Anspruch 14 oder 15 zur Prophylaxe und/oder Behandlung von Depressionen.

17. Arzneimittel gemäß Anspruch 14 oder 15 zur Prophylaxe und/oder Behandlung von Schmerzen, vorzugsweise zur Behandlung und/oder Prophylaxe von akuten Schmerzen, chronischen Schmerzen, neuropathischen Schmerzen und/oder Clusterkopfschmerzen.

18. Arzneimittel gemäß Anspruch 14 oder 15 zur kombinierten Prophylaxe und/oder Behandlung von Depressionen und Schmerzen, vorzugsweise zur kombinierten Behandlung von Depressionen und Schmerzen ausgewählt aus der Gruppe bestehend aus akuten Schmerzen, chronischen Schmerzen, neuropathischen Schmerzen und Clusterkopfschmerzen.

19. Arzneimittel gemäß Anspruch 14 oder 15 zur Prophylaxe und/oder Behandlung von Akohol- und/oder Drogen- und/oder Medikamentenmißbrauch, zur Prophylaxe und/oder Behandlung von Akohol- und/oder Drogen- und/oder Medikamentenabhängigkeit, zur Prophylaxe und/oder Behandlung von Entzündungen, zur Prophylaxe und/oder Behandlung von Antriebslosigkeit, zur Prophylaxe und/oder Behandlung von Störungen der Nahrungsmittelaufnahme, vorzugsweise ausgewählt aus der Gruppe bestehend aus Bulimie, Anorexie, Fettsucht und Kachexie, zur Prophylaxe und/oder Behandlung von Katalepsie, zur Vigilanzsteigerung, zur Libidosteigerung oder zur Anxiolyse, zur Prophylaxe und/oder Behandlung von neurodegenerativen Erkrankungen, vorzugsweise einer oder mehrerer neurodegenerativer Erkrankungen ausgewählt aus der Gruppe bestehend aus Morbus Parkinson, Morbus Huntington, Morbus Alzheimer und Multipler Sklerose, zur Prophylaxe und/oder Behandlung von Ischämie und/oder zur Lokalanästhesie.

20. Arzneimittel gemäß Anspruch 14 oder 15 zur Behandlung und/oder Prophylaxe einer oder mehrerer Erkrankungen ausgewählt aus der Gruppe bestehend aus nicht-akuten allergischen Erkrankungen, vorzugsweise allergischer Dermatitis; Asthma; Rhinitis; Konjunktivitis; Erkrankungen die durch 2-arachidonglycerol und/oder entsprechender Ether wie Hematol vermittelt werden; Septikämie; Krebs, insbesondere Blutkrebs und/oder Himtumore; Kreislaufstörungen; grünem Star, Entzündungen, vorzugsweise immunologisch vermittelten Entzündungserkrankungen, besonders bevorzugt rheumatoide Arthritis, Lupus erythematodes, Psoriasis und Thyroiditis; Diabetes; Blutvergiftung; Epilepsie; Tourettes Syndrom; Osteoporose; Morbus Bechterew; Gicht; gichtartiger Arthritis; Osteoarthritis; Durchblutungsstörungen; Ischämien, vorzugsweise Nierenischämie und renale Ischämie, und/oder zur Regulation des Immunsystems, vorzugsweise zur Unterdrückung des Immunsystems.

21. Verwendung wenigstens einer substituierten 1,4.8-Triazaspiro[4.5]decan-2-on-Verbindung gemäß einem der Ansprüche 1 bis 12 zur Herstellung eines Arzneimittels zur Regulation der Noradrenalin-Wiederaufnahme (Noradrenalin-Uptake), vorzugsweise zur Hemmung der Noradrenalin-Wiederaufnahme (Noradrenalin-Uptake).

22. Verwendung wenigstens einer substituierten 1,4,8-Triazaspiro[4.5]decan-2-on-Verbindung gemäß einem der Ansprüche 1 bis 12 zur Herstellung eines Arzneimittels zur Regulation der 5-Hydroxy-Tryptophan-Wiederaufnahme (5-HT-Uptake), vorzugsweise zur Hemmung der 5-Hydroxy-Tryptophan-Wiederaufnahme (5-HT-Uptake).

23. Verwendung wenigstens einer substituierten 1,4,8-Triazaspiro[4.5]decan-2-on-Verbindung gemäß einem der Ansprüche 1 bis 12 zur Herstellung eines Arzneimittels zur Batrachotoxin-(BTX)-Rezeptor-Regulation.

24. Verwendung wenigstens einer substituierten 1,4,8-Triazaspiro[4.5]decan-2-on-Verbindung gemäß einem der Ansprüche 1 bis 12 zur Herstellung eines Arzneimittels zur CB2-Rezeptor-Regulation.

25. Verwendung wenigstens einer substituierten 1,4,8-Triazaspiro[4.5]decan-2-on-Verbindung gemäß einem der Ansprüche 1 bis 12 zur Herstellung eines Arzneimittels zur Prophylaxe und/oder Behandlung von Depressionen.

26. verwendung wenigstens einer substituierten 1,4,8-Triazaspiro[4.5]decan-2-on-Verbindung gemäß einem der Ansprüche 1 bis 12 zur Herstellung eines Arzneimittels zur Prophylaxe und/oder Behandlung von Schmerzen, vorzugsweise zur Behandlung und/oder Prophylaxe von akuten Schmerzen, chronischen Schmerzen, neuropathischen Schmerzen und/oder Clusterkopfschmerzen.

27. Verwendung wenigstens einer substituierten 1,4,8-Triazaspiro[4.5]decan-2-on-Verbindung gemäß einem der Ansprüche 1 bis 12 zur Herstellung eines Arzneimittels zur kombinierten Prophylaxe und/oder Behandlung von Depressionen und Schmerzen, vorzugsweise zur kombinierten Prophylaxe und/oder Behandlung von Depressionen und Schmerzen ausgewählt aus der Gruppe bestehend aus akuten Schmerzen, chronischen Schmerzen, neuropathischen Schmerzen und Clusterkopfschmerzen.

28. Verwendung wenigstens einer substituierten 1,4,8-Triazaspiro[4.5]decan-2-on-Verbindung gemäß einem der Ansprüche 1 bis 12 zur Herstellung eines Arzneimittels zur Prophylaxe und/oder Behandlung von Akohol- und/oder Drogen- und/oder Medikamentenmißbrauch, zur Prophylaxe und/oder Behandlung von Akohol- und/oder Drogen- und/oder Medikamentenabhängigkeit, zur Prophylaxe und/oder Behandlung von Entzündungen, zur Prophylaxe und/oder Behandlung von Antriebslosigkeit, zur Prophylaxe und/oder Behandlung von Störungen der Nährungsmittelaufnahme, vorzugsweise ausgewählt aus der Gruppe bestehend aus Bulimie, Anorexie, Fettsucht und Kachexie, zur Prophylaxe und/oder Behandlung von Katalepsie, zur Vigilanzsteigerung, zur Libidosteigerung oder zur Anxiolyse, zur Prophylaxe und/oder Behandlung von neurodegenerativen Erkrankungen, vorzugsweise einer oder mehrerer neurodegenerativer Erkrankungen ausgewählt aus der Gruppe bestehend aus Morbus Parkinson, Morbus Huntington, Morbus Alzheimer und Multipler Sklerose, zur Prophylaxe und/oder Behandlung von Ischämie und/oder zur Lokalanästhesie.

29. Verwendung wenigstens einer substituierten 1,4,8-Triazaspiro[4.5]decan-2-on-Verbindung gemäß einem der Ansprüche 1 bis 12 zur Herstellung eines Arzneimittels zur Behandlung und/oder Prophylaxe einer oder mehrerer Erkrankungen ausgewählt aus der Gruppe bestehend aus nicht-akuten allergischen Erkrankungen, vorzugsweise allergischer Dermatitis; Asthma; Rhinitis; Konjunktivitis: Erkrankungen die durch 2-arachidonglycerol und/oder entsprechender Ether wie Hematol vermittelt werden; Septikämie; Krebs, insbesondere Blutkrebs und/oder Himtumore; Kreislaufstörungen; grünem Star: Entzündungen, vorzugsweise immunologisch vermittelten Entzündungserkrankungen, besonders bevorzugt rheumatoide Arthritis, Lupus erythematodes, Psoriasis und Thyroiditis; Diabetes; Blutvergiftung; Epilepsie; Tourettes Syndrom; Osteoporose Morbus Bechterew; Gicht; gichtartiger Arthritis; Osteoarthritis; Durchblutungsstörungen; Ischämien, vorzugsweise Nierenischämle und renale Ischämie, und/oder zur Regulation des Immunsystems, vorzugsweise zur Unterdrückung des Immunsystems.

## Claims

1. Substituted 1,4,8-triazaspiro[4.5]decan-2-one compounds of the general formula 1, 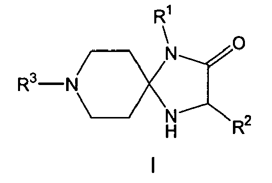 in which
R¹ stands for a hydrogen residue, or for a linear or branched unsubstituted or at least monosubstituted alkyl residue optionally comprising at least one heteroatom as a link, or for a linear or branched unsubstituted or at least monosubstituted alkenyl residue optionally comprising at least one heteroatom as a link, or for a linear or branched unsubstituted or at least monosubstituted alkynyl residue optionally comprising at least one heteroatom as a link, or for an unsubstituted or at least monosubstituted aryl residue or an unsubstituted or at least monosubstituted heteroaryl residue, which aryl or heteroaryl residue can be bonded via a linear or branched alkylene group optionally comprising at least one heteroatom as a link, or for a -C(=O)OR⁷ residue bonded via a linear or branched alkylene group,
R² stands for a hydrogen residue, or for a linear or branched unsubstituted or at least monosubstituted alkyl residue optionally comprising at least one heteroatom as a link, or for a linear or branched unsubstituted or at least monosubstituted alkenyl residue optionally comprising at least one heteroatom as a link, or for a linear or branched unsubstituted or at least monosubstituted alkynyl residue optionally comprising at least one heteroatom as a link, or for an unsubstituted or at least monosubstituted aryl residue or an unsubstituted or at least monosubstituted heteroaryl residue, which aryl or heteroaryl residue can be bonded via a linear or branched alkylene group optionally comprising at least one heteroatom as a link,
R³ stands or for an -S(=O)₂-R⁴ group, or for a -C(=S)NH-R⁵ group or for a -C(=O)NH-R⁶ group,
R⁴ stands for an -NR¹⁰R¹¹ residue, or for a linear or branched unsubstituted or at least monosubstituted alkyl residue optionally comprising at least one heteroatom as a link, or for a linear or branched unsubstituted or at least monosubstituted alkenyl residue optionally comprising at least one heteroatom as a link, or for a linear or branched unsubstituted or at least monosubstituted alkynyl residue optionally comprising at least one heteroatom as a link, or for an unsubstituted or at least monosubstituted aryl residue or an unsubstituted or at least monosubstituted heteroaryl residue, which aryl monosubstituted alkylene group optionally comprising at least one heteroatom as a link and/or condensed with an unsubstituted or at least monosubstituted monocyclic ring system, or for an unsubstituted or at least monosubstituted cycloaliphatic residue, which optionally comprises at least one heteroatom as a ring member and which can be bonded via a linear or branched unsubstituted or at least monosubstituted alkylene group optionally comprising at least one heteroatom as a link and/or bridged by a linear or branched unsubstituted or at least monosubstituted alkylene group,
R⁵ stands for a linear or branched unsubstituted or at least monosubstituted alkyl residue optionally comprising at least one heteroatom as a link, or for a linear or branched unsubstituted or at least monosubstituted alkenyl residue optionally comprising at least one heteroatom as a link, or for a linear or branched unsubstituted or at least monosubstituted alkynyl residue optionally comprising at least one heteroatom as a link, or for an unsubstituted or at least monosubstituted aryl residue or an unsubstituted or at least monosubstituted heteroaryl residue, which residue can be bonded via a linear or branched unsubstituted or at least monosubstituted alkylene group optionally comprising at least one heteroatom as a link, or for an unsubstituted or at least monosubstituted cycloaliphatic residue, which optionally comprises at least one heteroatom as a ring member or which can be bonded via a linear or branched unsubstituted or at least monosubstituted alkylene group optionally comprising at least one heteroatom as a link, or for a -C(=O)OR⁸ residue or for a -C(=O)OR⁹ residue bonded via a linear or branched alkylene group,
R⁶ stands for an unsubstituted or at least monosubstituted aryl residue or an unsubstituted or at least monosubstituted heteroaryl residue, which aryl or heteroaryl residue can be bonded via a linear or branched unsubstituted or at least monosubstituted alkylene group optionally comprising at least one heteroatom as a link, or for an unsubstituted or at least monosubstituted cycloaliphatic residue, which optionally comprises at least one heteroatom as a ring member or which can be bonded via a linear or branched unsubstituted or at least monosubstituted alkylene group optionally comprising at least one heteroatom as a link,
R⁷, R⁸, R⁹, R¹⁰, and R¹¹, in each case independently stand for a linear or branched alkyl residue, a linear or branched alkenyl residue, or a linear or branched alkynyl residue,
in each case optionally in the form of one of the pure stereoisomers thereof, particularly enantiomers or diastereoisomers thereof, the racemates thereof or in the form of a mixture of stereoisomers, particularly the enantiomers and/or diastereoisomers thereof, in any desired mixing ratio, or in each case in the form of appropriate salts, or in each case in the form of appropriate solvates.

2. Compounds as defined in Claim 1, **characterised in that**
R¹ stands for a hydrogen residue, for a linear or branched unsubstituted or at least monosubstituted C₁₋₁₀ alkyl residue optionally comprising at least one heteroatom as a link, for a linear or branched unsubstituted or at least monosubstituted C₂-₁₀ alkenyl residue optionally comprising at least one heteroatom as a link, for a linear or branched unsubstituted or at least monosubstituted C₂-₁₀ alkynyl residue optionally comprising at least one heteroatom as a link, for an unsubstituted or at least monosubstituted five-membered to fourteen-membered aryl residue or heteroaryl residue, which is bonded via a linear or branched C₁₋₅ alkylene group optionally comprising at least one heteroatom as a link, or for a -C(=O)OR⁷ residue bonded via a linear or branched C₁-₅ alkylene group,
preferably for a hydrogen residue, or for a linear or branched unsubstituted C₁₋₅ alkyl residue, or for a linear or branched unsubstituted C₂₋₅ alkenyl residue, or for a linear or branched unsubstituted C₂₋₅ alkynyl residue, or for an unsubstituted or at least monosubstituted phenyl residue or an unsubstituted or at least monosubstituted naphthyl residue, which phenyl or naphthyl residue is bonded via a linear or branched C₁₋₅ alkylene group optionally comprising at least one oxygen atom as a link, or for a -C(=O)OR⁷ residue bonded via a linear or branched C₁₋₃ alkylene group,
more preferably for a hydrogen residue, or for a linear or branched unsubstituted C₁₋₄ alkyl residue, or for a linear or branched unsubstituted C₂₋₃ alkenyl residue, or for a linear or branched unsubstituted C₂₋₃ alkynyl residue, or for a phenyl residue or naphthyl residue, which phenyl or naphthyl residue is bonded via a -(CH₂) bridge, a -(CH₂)₂- bridge, a -(CH₂)₃- bridge or a -(CHF₂)₂-O- bridge and is monosubstituted or polysubstituted by the same or different substituents selected from the group consisting of F, Cl, Br, CN, NO₂, C₁₋₅ alkyl, C₁₋₅ alkoxy, -C(=O)C₁₋₅-alkyl, -C(=O)O-C₁₋₅-alkyl, -S(=O)₂-C₁₋₆-alkyl, -C(=O)-C₁₋₅-perfluoroalkyl, -CF₃, CHF₂, and CH₂F, or stands for a -C(=O)OR⁷ residue bonded via a -(CH₂) group.

3. Compounds as defined in Claim 1 or Claim 2, **characterised in that**
R² stands for a hydrogen residue, or for a linear or branched unsubstituted or at least monosubstituted C₁₋₁₀ alkyl residue optionally comprising at least one heteroatom as a link, or for a linear or branched unsubstituted or at least monosubstituted C₂₋₁₀ alkenyl residue optionally comprising at least one heteroatom as a link, or for a linear or branched unsubstituted or at least monosubstituted C₂₋₁₀ alkynyl residue optionally comprising at least one heteroatom as a link, or for an unsubstituted or at least monosubstituted five-membered to fourteen-membered aryl or heteroaryl residue, which can be bonded via a linear or branched C₁₋₅ alkylene group optionally comprising at least one heteroatom as a link,
preferably for a hydrogen residue, or for a linear or branched unsubstituted C₁₋₅ alkyl residue optionally comprising at least one oxygen atom and/or at least one sulfur atom as a link or for a phenyl or naphthyl residue, which can be monosubstituted or polysubstituted by the same or different substituents selected from the group consisting of F, Cl, Br, CN, NO₂, C₁₋₅ alkyl, C₁₋₅ alkoxy, -C(=O)C₁₋₅-alkyl, -C(=O)O-C₁₋₅-alkyl, -S(=O)₂-C₁₋₆-alkyl, -C(=O)-C₁₋₅-perfluoroalkyl, -CF₃, CHF₂, and CH₂F and/or can be bonded via a -(CH₂) bridge, a -(CH₂)₂- bridge, a -(CH₂)₃- bridge or a -(CH₂)₂-O- bridge,
more preferably for a hydrogen residue, or for a linear or branched unsubstituted C₁₋₅ alkyl residue optionally comprising at least one sulfur atom as a link or for a phenyl residue, wherein the phenyl residue can be monosubstituted or polysubstituted by the same or different substituents selected from the group consisting of F, Cl, Br, CN, NO₂, C₁₋₅ alkyl, C₁₋₅ alkoxy, -C(=O)C₁₋₅-alkyl, -C(=O)O-C₁₋₅-alkyl, -S(=O)₂-C₁₋₆-alkyl, -C(=O)-C₁₋₅-perfluoroalkyl, -CF₃, CHF₂, and CH₂F and/or can be bonded via a -(CH₂) bridge,

4. Compounds as defined in one or more of Claims 1 to 3, **characterised in that**
R⁴ stands for an NR¹⁰R¹¹ residue, or for a linear or branched unsubstituted or at least monosubstituted C₁₋₁₀ alkyl residue optionally comprising at least one heteroatom as a link, or for a linear or branched unsubstituted or at least monosubstituted C₂₋₁₀ alkenyl residue optionally comprising at least one heteroatom as a link, or for a linear or branched unsubstituted or at least monosubstituted C₂₋₁₀ alkynyl residue optionally comprising at least one heteroatom as a link, or for an unsubstituted or at least monosubstituted five-membered to fourteen-membered aryl residue or heteroaryl residue, which can be bonded via a linear or branched unsubstituted or at least monosubstituted C₁₋₅ alkylene group optionally comprising at least one heteroatom as a link and/or condensed with a five-membered or six-membered monocyclic ring system, or for an unsubstituted or at least monosubstituted C₃₋₈-cycloaliphatic residue, which optionally comprises at least one heteroatom as a ring member or which can be bonded via a linear or branched unsubstituted or at least monosubstituted C₁₋₅ alkylene group optionally comprising at least one heteroatom as a link and/or bridged by a linear or branched unsubstituted or at least monosubstituted C₁₋₅ alkylene group,
preferably for an -NR¹⁰R¹¹ residue, or for a linear or branched unsubstituted C₁₋₄ alkyl residue optionally comprising at least one oxygen atom and/or at least one sulfur atom as a link, or for a furyl (furanyl) residue, thienyl (thiophenyl) residue, phenyl residue, naphthyl residue, or 1,2,3,4-tetrahydroisoquinoline residue, while the cyclic residue can in each case be monosubstituted or polysubstituted by the same or different substituents selected from the group consisting of F, Cl, Br, CN, NO₂, C₁₋₅ alkyl, C₁₋₅ alkoxy, -C(=O)C₁₋₅-alkyl, -C(=O)O-C₁₋₅-alkyl, -S(=O)₂-C₁₋₆-alkyl, -C(=O)-C₁₋₅-perfluoroalkyl, -CF₃, CHF₂, and CH₂F and/or be can be bonded via a -(CH₂)₂- or -(CH₂)₃ bridge, or for an unsubstituted C₅ or C₆ cycloaliphatic residue or one which is at least monosubstituted by an oxo group and which optionally comprises at least one heteroatom as a ring member, which can be bonded via a -(CH₂) bridge, a -(CH₂)₂- bridge, or a -(CH₂)₃- bridge and/or can be bridged by a -(C(CH₃)₂) group,
more preferably for an -N(CH₃)₂ residue, or for a linear or branched unsubstituted C₁₋₄ alkyl residue, or for a thienyl (thiophenyl) residue, phenyl residue, or 1,2,3,4-tetrahydroisoquinoline residue, and the cyclic residue can in each case be monosubstituted or polysubstituted by the same or different substituents selected from the group consisting of F, Cl, Br, CN, NO₂, C₁₋₅ alkyl, C₁₋₅ alkoxy, -C(=O)C₁₋₅-alkyl, -C(=O)O-C₁₋₅-alkyl, -S(=O)₂-C₁₋₆-alkyl, -C(=O)-C₁₋₅-perfluoroalkyl, -CF₃, CHF₂, and CH₂F and/or be can be bonded via a -(CH₂) bridge, or for an unsubstituted C₅ or C₁₋₆ cycloaliphatic residue or one which is at least monosubstituted by an oxo group and which can be bonded via a -(CH₂) bridge and/or can be bridged by a -(C(CH₃)₂) group,

5. Compounds as defined in one or more of Claims 1 to 4, **characterised in that** the residue
R⁵ stands for a linear or branched unsubstituted or at least monosubstituted C₁₋₁₀ alkyl residue optionally comprising at least one heteroatom as a link, or for a linear or branched unsubstituted or at least monosubstituted C₂₋₁₀ alkenyl residue optionally comprising at least one heteroatom as a link, or for a linear or branched unsubstituted or at least monosubstituted C₂₋₁₀ alkynyl residue optionally comprising at least one heteroatom as a link, or for an unsubstituted or at least monosubstituted five-membered to fourteen-membered aryl or heteroaryl residue, which residue can be bonded via a linear or branched unsubstituted or at least monosubstituted C₁₋₅ alkylene group optionally comprising at least one heteroatom as a link, for an unsubstituted or at least monosubstituted C₃₋₈ cycloaliphatic residue optionally comprising at least one heteroatom as a ring member and which can be bonded via a linear or branched unsubstituted or at least monosubstituted C₁₋₅ alkylene group optionally comprising at least one heteroatom as a link, or for a -C(=O)OR⁸ residue or for a -C(=O)OR⁹ residue bonded via a linear or branched C₁₋₅ alkylene group,
preferably for a linear or branched C₁₋₅ alkyl residue optionally comprising at least one oxygen atom and/or at least one sulfur atom as a link, or for a linear or branched C₂₋₅ alkenyl residue optionally comprising at least one oxygen atom and/or at least one sulfur atom as a link, or for a linear or branched C₂₋₅ alkynyl residue optionally comprising at least one oxygen atom and/or at least one sulfur atom as a link, or for a furyl (furanyl) residue, thienyl (thiophenyl) residue, phenyl, or naphthyl residue, and the cyclic residue can in each case be monosubstituted or polysubstituted by the same or different substituents selected from the group consisting of F, Cl, Br, CN, NO₂, C₁₋₅ alkyl, C₁₋₅ alkoxy, -C(=O)C₁₋₅-alkyl, -C(=O)O-C₁₋₅-alkyl, -S(=O)₂-C₁₋₆-alkyl, -C(=O)-C₁₋₅-perfluoroalkyl, -CF₃, CHF₂, and CH₂F and/or can be bonded via a -(CH₂) bridge, a -(CH(CH₃)) bridge, a -(CH₂)₂- bridge or a -(CH₂)₃ bridge, or for an unsubstituted C₄, C₅ or C₆ cycloaliphatic residue optionally comprising at least one oxygen atom as a ring member, which can be bonded via a -(CH₂) bridge, a -(CH(CH₃)) bridge, a -(CH₂)₂- bridge or a -(CH₂)₃ bridge, or for a -C(=O)O-R⁸ residue or a -C(=O)OR⁹ residue bonded via a -(CH₂) bridge, a -(CH(CH₃)) bridge, a -(CH₂)₂- bridge or a -(CH₂)₃- bridge,
more preferably for a linear or branched unsubstituted C₁₋₃ alkyl residue optionally comprising at least one oxygen atom as a link, or for a linear or branched unsubstituted C₂₋₃ alkenyl residue, or for a linear or branched unsubstituted C₂₋₃ alkynyl residue, or for a phenyl residue, while the phenyl residue can be monosubstituted or polysubstituted by the same or different substituents selected from the group consisting of F, Cl, Br, CN, NO₂, C₁₋₅ alkyl, C₁₋₅ alkoxy, -C(=O)C₁₋₅-alkyl, -C(=O)O-C₁₋₅-alkyl, -S(=O)₂-C₁₋₆-alkyl, -C(=O)-C₁₋₅-perfluoroalkyl, -CF₃, CHF₂, and CH₂F and/or can be bonded via a (CH₂) bridge, a -(CH(CH₃)) bridge, a -(CH₂)₂- bridge or a -(CH₂)₃ bridge, or for an unsubstituted C₅ or C₆cycloaliphatic residue which optionally comprises at least one oxygen atom as a ring member and which can be bonded via a -(CH₂) bridge, a -(CH(CH₃)) bridge, a -(CH₂)₂- bridge or a -(CH₂)₃ bridge, or for a C(=O)-O-C₂H₅ residue or for a -C(=O)O-R⁹ residue bonded via a -(CH₂) bridge, a -(CH(CH₃)) bridge, a -(CH₂)₂- bridge or a -(CH₂)₃ bridge,

6. Compounds as defined in one or more of Claims 1 to 5, **characterised in that** the residue
R⁶ stands for an unsubstituted or at least monosubstituted five-membered to fourteen-membered aryl or heteroaryl residue, which aryl or heteroaryl residue can be bonded via a linear or branched unsubstituted or at least monosubstituted C₁₋₅ alkylene group optionally comprising at least one heteroatom as a link, or for an unsubstituted or at least monosubstituted C₃₋₈ cycloaliphatic residue which optionally comprises at least one heteroatom as a ring member, or which can be bonded via a linear or branched unsubstituted or at least monosubstituted C₁₋₅ alkylene group optionally comprising at least one heteroatom as a link,
preferably for a phenyl residue, which phenyl residue can be monosubstituted or polysubstituted by the same or different substituents selected from the group consisting of F, Cl, Br,CN, NO₂, C₁₋₅ alkyl, C₁₋₅ alkoxy, -C(=O)C₁₋₅-alkyl, -C(=O)O-C₁₋₅-alkyl, -S(=O)₂-C₁₋₆-alkyl, -C(=O)-C₁₋₅-perfluoroalkyl, -CF₃, CHF₂, and CH₂F and/or can be bonded via a -(CH₂) bridge, a -(CH(CH₃)) bridge, a -(CH₂)₂- bridge, or a -(CH₂)₃- bridge, or for an unsubstituted C₅- or C₆-cycloaliphatic residue, which can be bonded via a (CH₂) bridge, a -(CH(CH₃)) bridge, a -(CH₂)₂- bridge, or a -(CH₂)₃- bridge,
more preferably for a phenyl residue, which phenyl residue can be monosubstituted or polysubstituted by the same or different substituents selected from the group consisting of F, Cl, Br, CN, NO₂, C₁₋₅ alkyl, C₁₋₅ alkoxy, -C(=O)C₁₋₅-alkyl, -C(=O)O-C₁₋₅-alkyl, -S(=O)₂-C₁₋₆-alkyl, -C(=O)-C₁₋₅-perfluoroalkyl, -CF₃, CHF₂, and CH₂F and/or can be bonded via a (CH₂) bridge or a -(CH₂)₂- bridge, or for a cyclohexyl residue, which can be bonded via a (CH₂) bridge or a -(CH₂)₂ bridge,

7. Compounds as defined in one or more of Claims 1 to 6, **characterised in that** the residues
R⁷, R⁸, R⁹, and R¹⁰, R¹¹, independently stand for a linear or branched C₁₋₅ alkyl residue, a linear or branched C₂₋₅ alkenyl residue, or a linear or branched C₂-₅ alkynyl residue, preferably for a linear or optionally branched methyl residue, ethyl residue, n-propyl residue or isopropyl residue,

8. Compounds as defined in one or more of Claims 1 to 7, **characterised in that**
R¹ stands for a hydrogen residue, or for an alkyl residue selected from the group consisting of methyl, ethyl, n-propyl, isopropyl, n-butyl, isobutyl, sec-butyl, and tert-butyl, or for an alkenyl residue selected from the group consisting of vinyl and allyl, or for a propynyl residue, or for a phenyl residue, a 1-naphthyl residue, or a 2-naphthyl residue, while the cyclic residue is can be bonded via a -(CH₂) bridge, a -(CH₂)₂- bridge, a -(CH₂)₂ - bridge, or a -(CH₂)₂-O- bridge and is optionally monosubstituted or polysubstituted by the same or different substituents selected from the group consisting of F, Cl, Br, CN, NO₂, C₁₋₅ alkyl, C₁₋₅ alkoxy, -C(=O)C₁₋₅-alkyl, -C(=O)O-C₁₋₅-alkyl, -S(=O)₂-C₁₋₆-alkyl, -C(=O)-C₁₋₅-perfluoroalkyl, -CF₃, CHF₂, and CH₂F, or for a -C(=O)OR⁷ residue bonded via a (CH₂) group,
R² stands for a hydrogen residue, or for a linear or branched unsubstituted alkyl residue optionally comprising at least one sulfur atom as a link selected from the group consisting of methyl, ethyl, n-propyl, isopropyl, n-butyl, isobutyl, sec-butyl, tert-butyl, and 2-methylsulfanylethyl or for a phenyl residue or benzyl residue, which can in each case be unsubstituted or monosubstituted or polysubstituted by the same or different substituents selected from the group consisting of F, Cl, Br, CN, NO₂, C₁₋₅ alkyl, C₁₋₅ alkoxy, -C(=O)C₁₋₅-alkyl, -C(=O)O-C₁₋₅-alkyl, -S(=O)₂-C₁-₆-alkyl, -C(=O)-C₁₋₅-perfluoroalkyl, -CF₃, CHF₂, and CH₂F,
R³ stands for an S(=O)₂-R⁴ group, or for a -C(=S)NH-R⁵ group or for a -C(=O)NH-R⁶ group,
R⁴ stands for a N-(CH₃)₂ residue, or for an alkyl residue selected from the group consisting of methyl, ethyl, n-propyl, isopropyl, n-butyl, isobutyl, sec-butyl, tert-butyl, or for a thienyl (thiophenyl) residue, phenyl residue, or 1,2,3,4-tetrahydroisoquinoline residue, and each cyclic residue can be unsubstituted or monosubstituted or polysubstituted by the same or different substituents selected from the group consisting of F, Cl, Br, CN, NO₂, C₁₋₅ alkyl, C₁₋₅ alkoxy, -C(=O)C₁₋₅-alkyl, -C(=O)O-C₁₋₅-alkyl, -S(=O)₂-C₁₋₆-alkyl, -C(=O)-C₁₋₅-perfluoroalkyl, -CF₃, CHF₂, and CH₂F and/or can be bonded via a (CH₂) bridge, or for a 7,7-dimethyl-2-oxobicyclo[2.2.1]hept-1-ylmethyl residue,
R⁵ stands for an alkyl residue selected from the group consisting of methyl, ethyl, n-propyl, isopropyl, n-butyl, isobutyl, sec-butyl, tert-butyl, and 2-methoxyethyl, or for an alkenyl residue selected from the group consisting of vinyl and allyl, or for a propynyl residue, or for a phenyl residue, and the phenyl residue can be monosubstituted or polysubstituted by the same or different substituents selected from the group consisting of F, Cl, Br, CN, NO₂, C₁₋₅ alkyl, C₁₋₅ alkoxy, -C(=O)C₁₋₅-alkyl , -C(=O)O-C₁₋₅-alkyl, -S(=O)₂-C₁₋₆-alkyl, -C(=O)-C₁₋₅-perfluoroalkyl, -CF₃, CHF₂, and CH₂F and/or can be bonded via a (CH₂) bridge, a -(CH(CH₃)) bridge, a -(CH₂)₂- bridge, or a -(CH₂)₃ bridge, or for a cyclohexyl or tetrahydrofuryl residue, which can be bonded via a (CH₂) bridge, cyclohexyl or tetrahydrofuryl residue, which can be bonded via a (CH₂) bridge, or for a C(=O)O-C₂H₅ residue or for a -C(=O)O-C₂H₅ residue bonded via a (CH₂) bridge, a -(CH(CH₃)) bridge, a -(CH₂)₂- bridge, or a -(CH₂)₃ bridge,
R⁶ stands for a phenyl residue, which can be unsubstituted or monosubstituted or polysubstituted by the same or different substituents selected from the group consisting of F, Cl, Br, CN, NO₂, C₁₋₅ alkyl, C₁₋₅ alkoxy, -C(=O)C₁₋₅-alkyl, -C(=O)O-C₁₋₅-alkyl, -S(=O)₂-C₁₋₆-alkyl, -C(=O)C₁₋₅-perfluoroalkyl, -CF₃, CHF₂, and CH₂F and/or be can be bonded via a (CH₂) bridge or a -(CH₂)₂- bridge, or for a cyclohexyl residue, which can be bonded via a (CH₂) bridge or a -(CH₂)₂ bridge,
R⁷, R⁸, R⁹, R¹⁰, and R¹¹ in each case independently stand for a linear or branched C₁₋₅ alkyl residue, a linear or optionally branched C₂₋₅ alkenyl residue or a linear or branched C₂₋₅ alkynyl residue, preferably for a linear or branched methyl residue, ethyl residue, n-propyl residue or isopropyl residue.
in each case optionally in the form of one of the pure stereoisomers thereof, particularly enantiomers or diastereoisomers thereof, the racemates thereof or in the form of a mixture of stereoisomers, particularly the enantiomers and/or diastereoisomers thereof, in any desired mixing ratio, or in each case in the form of appropriate salts, or in each case in the form of appropriate solvates.

9. Compounds as defined in any one or more of Claims 1 to 8, **characterised in that**
R¹ stands for a hydrogen residue;
or for a linear or branched, optionally substituted C₁₋₁₀ alkyl residue, linear or branched, optionally substituted C₂₋₁₀ alkenyl residue, or linear or branched, optionally substituted C₂₋₁₀ alkynyl residue;
or for an optionally substituted 5-membered to 14-membered aryl or heteroaryl residue;r
or for -(CH₂)ₐₐ-C(=O)-O-R⁷ in which aa is 1, 2, 3, 4 or 5;
or for -(CH₂)-Uₐ-(CH₂)_{b}-V_{c}-(CH₂)_{d}-R¹² in which a is 0 or 1, b is 0 or 1, c is 0 or 1 and d is 0 or 1, wherein U and V in each case independently stand for O, S, NH, N(CH₃) or N(C₂H₅);
R² stands for a hydrogen residue;
or for a linear or branched, optionally substituted C₁₋₁₀ alkyl residue, a linear or branched, optionally substituted C₂₋₁₀ alkenyl residue or a linear or branched, optionally substituted C₂₋₁₀ alkynyl residue;
or for an optionally substituted 5-membered to 14-membered aryl or heteroaryl residue;
or for -(CH₂)-Wₑ-(CH₂)_{f}-X_{g}-(CH₂)ₕR¹³ in which e is 0 or 1, f is 0 or 1, g is 0 or 1 and h is 0 or 1, wherein W and X in each case independently stand for O, S, NH, N(CH₃) or N(C₂H₅);
R³ stands for an -S(=O)₂-R⁴ group, for a -C(=S)-NH-R⁵ group or for a -C(=O)-NH-R⁶ group,
R⁴ stands for a residue -NR¹⁰R¹¹;
or for a linear or branched, optionally substituted C₁₋₁₀ alkyl residue, a linear or branched, optionally substituted C₂₋₁₀ alkenyl residue, or a linear or branched, optionally substituted C₂₋₁₀ alkynyl residue;
or for an unsaturated or saturated, optionally substituted 3-membered, 4-membered, 5-membered, 6-membered, 7-membered, 8-membered, or 9-membered cycloaliphatic residue, which can be bridged by one or two linear or branched, optionally substituted C₁₋₅ alkylene groups,
or for an optionally substituted 5-membered to 14-membered aryl or heteroaryl residue, which can be condensed with a saturated or unsaturated, optionally substituted monocyclic ring system;
or for -(CH₂)-Yₖ-(CH₂)ₘ-Zₙ-(CH₂)ₚ-R¹⁴ in which k is 0 or 1, m is 0 or 1, n is 0 or 1 and p is 0 or 1, in which Y and Z in each case independently stand for O, S, NH, N(CH₃) or N(C₂H₅);
R⁵ stands for a linear or branched, optionally substituted C₁₋₁₀ alkyl residue, a linear or branched, optionally substituted C₂₋₁₀ alkenyl residue or a linear or branched, optionally substituted C₂₋₁₀ alkynyl residue;
or for an unsaturated or saturated, optionally substituted 3-membered, 4-membered, 5-membered, 6-membered, 7-membered, 8-membered, or 9-membered cycloaliphatic residue;
or for an optionally substituted 5-membered to 14-membered aryl or heteroaryl residue;
or for -(CHR¹⁵)-A_{q}-(CH₂)ᵣ-Bₛ-(CH₂)ₜ-R¹⁶ in which q is 0 or 1, r is 0 or 1, s is 0 or 1 and t is 0 or 1, in which A and B in each case independently stand for O, S, NH, N(CH₃) or N(C₂H₅);
or for a -C(=O)-OR⁸ residue;
or for a -(CHR¹⁷)-(CH₂)ᵥC(=O)-OR⁹ residue, in which v stands for 0, 1, 2, or 3;
R⁶ stands for an unsaturated or saturated, optionally substituted 3-membered, 4-membered, 5-membered, 6-membered, 7-membered, 8-membered, or 9-membered cycloaliphatic residue;
or for an optionally substituted 5-membered to 14-membered aryl or heteroaryl residue;
or for -(CH₂)-D_{w}-(CH₂)ₓ-E_{y}-(CH₂)_{z}-R¹⁸ in which w is 0 or 1, x is 0 or 1, y is 0 or 1 and z is 0 or 1, in which D and E in each case independently stand for O, S, NH, N(CH₃) or N(C₂H₅);
R⁷, R⁸, R⁹, R¹⁰ and R¹¹ in each case independently
stand for a linear or branched, optionally substituted C₁₋₁₀ alkyl residue, a linear or branched, optionally substituted C₂₋₁₀ alkenyl residue, or a linear or branched, optionally substituted C₂₋₁₀ alkynyl residue;
R¹² and R¹³ in each case independently
stand for a linear or branched, optionally substituted C₁₋₁₀ alkyl residue, a linear or branched, optionally substituted C₂₋₁₀ alkenyl residue, or a linear or branched, optionally substituted C₂₋₁₀ alkynyl residue;
or for an optionally substituted 5-membered to 14-membered aryl or heteroaryl residue;
R¹⁴ stands for a linear or branched, optionally substituted C₁₋₁₀ alkyl residue, a linear or branched, optionally substituted C₂₋₁₀ alkenyl residue, or a linear or branched, optionally substituted C₂₋₁₀ alkynyl residue;
or for an unsaturated or saturated, optionally substituted 3-membered, 4-membered, 5-membered, 6-membered, 7-membered, 8-membered, or 9-membered cycloaliphatic residue, which can be bridged by one or two linear or branched, optionally substituted C₁₋₅ alkylene groups,
or for an optionally substituted 5-membered to 14-membered aryl or heteroaryl residue, which can be condensed with a saturated or unsaturated, optionally substituted monocyclic ring system;
R¹⁵ stands for a hydrogen residue
or for a linear or branched, optionally substituted C₁₋₁₀ alkyl residue, a linear or branched, optionally substituted C₂₋₁₀ alkenyl residue, or a linear or branched, optionally substituted C₂₋₁₀ alkynyl residue;
R¹⁶ stands for a linear or branched, optionally substituted C₁₋₁₀ alkyl residue, a linear or branched, optionally substituted C₂₋₁₀ alkenyl residue, or a linear or branched, optionally substituted C₂₋₁₀ alkynyl residue;
or for an unsaturated or saturated, optionally substituted 3-membered, 4-membered, 5-membered, 6-membered, 7-membered, 8-membered, or 9-membered cycloaliphatic residue;
or for an optionally substituted 5-membered to 14-membered aryl or heteroaryl residue;
R¹⁷ stands for a linear or branched, optionally substituted C₁₋₁₀ alkyl residue, a linear or branched, optionally substituted C₂₋₁₀ alkenyl residue, or a linear or branched, optionally substituted C₂-₁₀ alkynyl residue;
and
R¹⁸ stands for an unsaturated or saturated, optionally substituted 3-membered, 4-membered, 5-membered, 6-membered, 7-membered, 8-membered, or 9-membered cycloaliphatic residue,
or for an optionally substituted 5-membered to 14-membered aryl or heteroaryl residue;
wherein
the aforementioned C₁₋₁₀ alkyl residues are unsubstituted or can each be substituted by 1, 2, 3, 4 or 5 substituents in each case independently selected from the group consisting of F, Cl, Br, I, -CN, -NO₂, -OH, -SH, -NH₂ and -N(C₁₋₅ alkyl)(C₁₋₅ alkyl);
the aforementioned C₂₋₁₀ alkenyl residues are unsubstituted or can be substituted by 1, 2, 3, 4 or 5 substituents in each case independently selected from the group consisting of F, Cl, Br, I, -CN, -NO₂, -OH, -SH, -NH₂ and -N(C₁₋₅ alkyl)(C₁₋₅ alkyl);
the aforementioned C₂₋₁₀ alkynyl residues are unsubstituted or can be substituted by 1, 2, 3, 4 or 5 substituents in each case independently selected from the group consisting of F, Cl, Br, I, -CN, -NO₂, -OH, -SH, -NH₂ and -N(C₁₋₅ alkyl)(C₁₋₅ alkyl);
the aforementioned C₁₋₅ alkylene groups are unsubstituted or can be substituted by 1, 2, 3, 4 or 5 substituents in each case independently selected from the group consisting of F, Cl, Br, -OH, -SH, -NH₂, -CN, NO₂ and phenyl,
the aforementioned cycloaliphatic residues are unsubstituted or can be substituted by 1, 2, 3, 4 or 5 substituents in each case independently selected from the group consisting of oxo (=O), thioxo (=S), F, Cl, Br, I, -CN, -CF₃, -SF₅, -OH, -O-C₁₋₅-alkyl, -NH₂, -NO₂, -O-CF₃, -S-CF₃, -SH, -S-C₁₋₅-alkyl, -C₁₋₅ alkyl, -C(=O)-H, -C(=O)-C₁₋₅-alkyl, -C(=O)-C₁₋₅-perfluoroalkyl, -C(=O)-OH, -C(=O)-O-C₁₋₅-alkyl, -(CH₂)-C(=O)-OH, -(CH₂)-C(=O)-O-C₁₋₅-alkyl, -NH-C₁₋₅ alkyl, -N(C₁₋₅-alkyl)₂, -(CH₂)-benzo[b]furanyl, -O-phenyl, -O-benzyl, phenyl, benzyl, naphthyl and -(CH₂)-naphthyl, while in each case the cyclic moiety of the residues -O-phenyl, -O-benzyl, phenyl, -(CH₂)-benzo[b]furanyl, benzyl, naphthyl, and -(CH₂)-naphthyl can be substituted by 1, 2, 3, 4 or 5 substituents in each case independently selected from the group consisting of F, Cl, Br, -OH, -CF₃, -SF₅, -CN, -NO₂, -O-C₁₋₅ alkyl, -C₁₋₅ alkyl, -O-CF₃, -S-CF₃, phenyl and -O-benzyl,
and the aforementioned cycloaliphatic residues 1, 2, 3, 4 or 5 can in each case independently comprise heteroatom(s) selected from the group consisting of oxygen, nitrogen, and sulfur;
the ring of the aforementioned monocyclic ring systems are unsubstituted or can be substituted by 1, 2, 3, 4 or 5 substituents in each case independently selected from the group consisting of oxo (=O), thioxo (=S), F, Cl, Br, I, -CN, -CF₃, -SF₅, -OH, -O-C₁₋₅ alkyl, -O-C₂₋₅-alkenyl, -NH₂, -NO₂, -O-CF₃, -O-CHF₂, -O-CH₂F, -S-CF₃, -S-CHF₂, -S-CH₂F, -SH, -S-C₁₋₅-alkyl, -C₁₋₅ alkyl, -C(=O)-OH, -C(=O)-O-C₁₋₅-alkyl, -O-C(=O)-C₁₋₅-alkyl, -O-C(=O)-phenyl, -(CH₂)-O-C(=O)-C₁₋₅-alkyl, -(CH₂)-O-C(=O)-phenyl, -NH-C₁₋₅-alkyl, -N(C₁₋₅-alkyl)₂, -NH-C(=O)-O-C₁₋₅-alkyl, -NH-C(=O)-C₁₋₅-alkyl, -C(=O)-H, -C(=O)-C₁₋₅-alkyl, -C(=O)-C₁₋₅-perfluoroalkyl, -C(=O)-NH₂, -C(=O)-NH-C₁₋₅-alkyl, C(=O)-N-(C₁₋₅-alkyl)₂, -S(=O)₂-NH₂, -S(=O)₂-NH-C₁₋₅-alkyl, -S(=O)₂-NH-phenyl, -S(=O)₂-C₁₋₅-alkyl, -(CH₂)-benzo[b]furanyl, -O-phenyl, -S-phenyl, -S-benzyl, -O-benzyl, phenyl and benzyl, while in each case the cyclic moiety of the residues -O-C(=O)-phenyl, -(CH₂)-O-C(=O)-phenyl, -S(=O)₂-NH-phenyl, -O-phenyl, -S-phenyl, -S-benzyl, -O-benzyl, phenyl, -(CH₂)-benzo[b]furanyl and benzyl can be substituted by 1, 2, 3, 4 or 5 substituents in each case independently selected from the group consisting of F, Cl, Br, -OH, -CF₃, -SF₅, -CN, -NO₂, -O-C₁₋₅-alkyl, -C₁₋₅ alkyl, -O-CF₃, -S-CF₃, phenyl and -O-benzyl,
and the ring of the aforementioned monocyclic ring systems is 5-membered, 6-membered, or 7-membered and can comprise 1, 2, 3, 4 or 5 heteroatom(s) as ring member(s), which are independently selected from the group consisting of oxygen, nitrogen, and sulfur;
and the aforementioned aryl or heteroaryl residues are unsubstituted or can be substituted by 1, 2, 3, 4 or 5 substituents in each case independently selected from the group consisting of F, Cl, Br, I, -CN, -CF₃, -SF₅, -OH, -O-C₁₋₅-alkyl, -O-C₂₋₅-alkenyl, -NH₂, -NO₂, -O-CF₃, -O-CHF₂, -O-CH₂F, -S-CF₃, -S-CHF₂, -S-CH₂F, -SH, -S-C₁₋₅-alkyl, -C₁₋₅alkyl, -C(=O)-OH, -C(=O)-O-C₁₋₅-alkyl, -O-C(=O)-C₁₋₅-alkyl, -O-C(=O)-phenyl, -(CH₂)-O-C(=O)-C₁₋₅-alkyl, -(CH₂)-O-C(=O)-phenyl, -NH-C₁₋₅-alkyl, -N(C₁₋₅-alkyl)₂, -NH-C(=O)-O-C₁₋₅-alkyl, -NH-C(=O)-C₁₋₅-alkyl, -C(=O)-H, -C(=O)-C₁₋₅-alkyl, -C(=O)-C₁₋₅-perfluoroalkyl, -C(=O)-NH₂, -C(=O)-NH-C₁₋₅-alkyl, C(=O)-N-(C₁₋₅-alkyl)₂, -S(=O)₂-NH₂, -S(=O)₂-NH-C₁-₅-alkyl, -S(=O)₂-NH-phenyl, -S(=O)₂-C₁₋₅-alkyl, -(CH₂)-benzo[b]furanyl, -O-phenyl, -S-phenyl, -S-benzyl, -0-benzyl, phenyl and benzyl, while in each case the cyclic moiety of the residues -O-C(=O)-phenyl, -(CH₂)-O-C(=O)-phenyl, -S(=O)₂-NH-phenyl, -O-phenyl, -S-phenyl, -S-benzyl, -O-benzyl, phenyl, -(CH₂)-benzo[b]furanyl, and benzyl can be substituted by 1, 2, 3, 4 or 5 substituents in each case independently selected from the group consisting of F, Cl, Br, -OH, -CF₃, -SF₅, -CN, -NO₂, -O-C₁₋₅-alkyl, -C₁₋₅ alkyl, -O-CF₃, -S-CF₃, phenyl and -O-benzyl,
and the aforementioned heteroaryl residues can comprise 1, 2, 3, 4 or 5 heteroatom(s) as ring member(s), which are independently selected from the group consisting of oxygen, nitrogen, and sulfur;
in each case optionally in the form of one of the pure stereoisomers thereof, particularly enantiomers or diastereoisomers thereof, the racemates thereof, or in the form of a mixture of stereoisomers, particularly enantiomers and/or diastereoisomers thereof, in any desired mixing ratio, or in each case in the form of appropriate salts or in each case in the form of appropriate solvates.

10. Compounds as defined in Claim 9, **characterised in that**
R¹ stands for a hydrogen residue;
or for a residue selected from the group consisting of methyl, ethyl, n-propyl, isopropyl, n-butyl, sec-butyl, isobutyl, tert-butyl, n-pentyl, 2-pentyl, 3-pentyl, -(CH₂)-(C(CH₃)₃), n-hexyl, 3-hexyl, -(CH₂)-(CH₂)-(C(CH₃)₃), n-heptyl, 3-heptyl, 4-heptyl, n-octyl, -(CH₂)-(CH)(C₂H₅)-(CH₂)-(CH₂)-(CH₂)-(CH₃), vinyl, 1-propenyl, 2-propenyl, 1-butenyl, 2-butenyl, 3-butenyl, 1-propynyl, 2-propynyl, 1-butynyl, 2-butynyl and 3-butynyl, while each of these residues can be substituted by 1, 2, 3, 4 or 5 substituents in each case independently selected from the group consisting of F, Cl, Br, I, -CN, -NO₂, -OH, -SH, -NH₂, -N(CH₃)₂, -N(C₂H₅)₂ and -N(CH₃)(C₂H₅);
or for a residue selected from the group consisting of phenyl, naphthyl, thiophenyl, furanyl, pyrrolyl, pyrazolyl, pyrazinyl, triazolyl, pyridinyl, imidazolyl, indolyl, isoindolyl, benzo[b]furanyl, benzo[b]thiophenyl, thiazolyl, thiadiazolyl, oxazolyl, isoxazolyl, pyridazinyl, pyrazinyl, pyrimidinyl, indazolyl, quinazolinyl, quinolinyl, isoquinolinyl, phenazinyl, phenothiazinyl, oxadiazolyl, benzo[1,2,5]oxadiazolyl and chromanyl, while each of these residues can be substituted by 1, 2, 3, 4 or 5 substituents in each case independently selected from the group consisting of F, Cl, Br, I, -CN, -CF₃, -SF₅, -OH, -O-CH₃, -O-C₂H₅, -O-CH(CH₃)₂, -O-C(CH₃)₃, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, -S-CH(CH₃)₂, -S-C(CH₃)₃, -S-CH₂-CH₂-CH₂-CH₃, methyl, ethyl, n-propyl, isopropyl, n-butyl, sec-butyl, isobutyl, tert-butyl, -C(=O)-OH, -C(=O)-O-CH₃, -C(=O)-O-C₂H₅; -C(=O)-O-C(CH₃)₃, -O-C(=O)-CH₃, -O-C(=O)-C₂H₅, -O-C(=O)-phenyl, -(CH₂)-O-C(=O)-CH₃, -(CH₂)-O-C(=O)-C₂H₅, -(CH₂)-O-C(=O)-phenyl, -N(CH₃)₂, -N(C₂H₅)₂, -N(H)(CH₃), -N(H)(C₂H₅), -NH-C(=O)-O-CH₃, -NH-C(=O)-O-C₂H₅, -NH-C(=O)-CH₃, -NH-C(=O)-C₂H₅, -C(=O)-H, -C(=O)-CH₃, -C(=O)-C₂H₅, -C(=O)-CF₃, -C(=O)-NH₂, -C(=O)-NH-CH₃, -C(=O)-NH-C₂H₅, -S(=O)₂-NH₂, -S(=O)₂-NH-CH₃, -S(=O)₂-NH-C₂H₅, -S(=O)₂-NH-phenyl, -S(=O)₂-CH₃, -S(=O)₂-C₂H₅, -(CH₂)-benzo[b]furanyl, -O-phenyl, -S-phenyl, -S-benzyl, -O-benzyl, phenyl and benzyl, while in each case the cyclic moiety of the residues -O-C(=O)-phenyl, -(CH₂)-O-C(=O)-phenyl, -S(=O)₂-NH-phenyl, -O-phenyl, -S-phenyl, -S-benzyl, -O-benzyl, phenyl, -(CH₂)-benzo[b]furanyl and benzyl can be substituted by 1, 2, 3, 4 or 5 substituents in each case independently selected from the group consisting of F, Cl, Br, -OH, -CF₃, -SF₅, -CN, -NO₂, -O-CH₃, -O-C₂H₅, -O-CH(CH₃)₂, -O-C(CH₃)₃, methyl, ethyl, n-propyl, isopropyl, n-butyl, sec-butyl, isobutyl, tert-butyl, -O-CF₃, -S-CF₃, phenyl and -O-benzyl,
or for -(CH₂)ₐₐ-C(=O)-O-R⁷ in which aa is 1, 2, 3, 4 or 5;
or for -(CH₂)-R¹² -(CH₂)-(CH₂)-R¹², -(CH₂)-O-R¹², -(CH₂)-S-R¹², -(CH₂)-N(CH₃)-R¹² -(CH₂)-(CH₂)-(CH₂)-R¹² -(CH₂)-(CH₂)-O-R¹², -(CH₂)-(CH₂)-S-R¹². -(CH₂)-(CH₂)-NH-R¹², -(CH₂)-(CH₂)-N(CH₃)-R¹², -(CH₂)-(CH₂)-O-(CH₂)-R¹², -(CH₂)-(CH₂)-S-(CH₂)-R¹² or -(CH₂)-(CH₂)-N(CH₃)-R¹²;
R² stands for a hydrogen residue;
or for a residue selected from the group consisting of methyl, ethyl, n-propyl, isopropyl, n-butyl, sec-butyl, isobutyl, tert-butyl, n-pentyl, 2-pentyl, 3-pentyl, -(CH₂)-(C(CH₃)₃), n-hexyl, 3-hexyl, -(CH₂)-(CH₂)-(C(CH₃)₃), n-heptyl, 3-heptyl, 4-heptyl, n-octyl, -(CH₂)-(CH)(C₂H₅)-(CH₂)-(CH₂)-(CH₂)-(CH₃), vinyl, 1-propenyl, 2-propenyl, 1-butenyl, 2-butenyl, 3-butenyl, 1-propynyl, 2-propynyl, 1-butynyl, 2-butynyl and 3-butynyl, while the residue can in each case be substituted by 1, 2, 3, 4 or 5 substituents in each case independently selected from the group consisting of F, Cl, Br, I, -CN, -NO₂, -OH, -SH, -NH₂, -N(CH₃)₂, -N(C₂H₅)₂, and -N(CH₃)(C₂H₅);
or for a residue selected from the group consisting of phenyl, naphthyl, thiophenyl, furanyl, pyrrolyl, pyrazolyl, pyrazinyl, triazolyl, pyridinyl, imidazolyl, indolyl, isoindolyl, benzo[b]furanyl, benzo[b]thiophenyl, thiazolyl, thiadiazolyl, oxazolyl, isoxazolyl, pyridazinyl, pyrazinyl, pyrimidinyl, indazolyl, quinazolinyl, quinolinyl, isoquinolinyl, phenazinyl, phenothiazinyl, oxadiazolyl, benzo[1,2,5]oxadiazolyl and chromanyl, while the residue can in each case be substituted by 1, 2, 3, 4 or 5 substituents in each case independently selected from the group consisting of F, Cl, Br, I, -CN, -CF₃, -SF₅, -OH, -O-CH₃, -O-C₂H₅, -O-CH(CH₃)₂, -O-C(CH₃)₃, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, -S-CH(CH₃)₂, -S-C(CH₃)₃, -S-CH₂-CH₂-CH₂-CH₃, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-phenyl, -(CH₂)-O-C(=O)-CH₃, -(CH₂)-O-C(=O)-C₂H₅, -(CH₂)-O-C(=O)-phenyl, -N(CH₃)₂, -N(C₂H₅)₂, -N(H)(CH₃), -N(H)(C₂H₅), -NH-C(=O)-O-CH₃, -NH-C(=O)-O-C₂H₅, -NH-C(=O)-CH₃, -NH-C(=O)-C₂H₅, -C(=O)-H, -C(=O)-CH₃, -C(=O)-C₂H₅, -C(=O)-CF₃, -C(=O)-NH₂, -C(=O)-NH-CH₃, -C(=O)-NH-C₂H₅, -S(=O)₂-NH₂, -S(=O)₂-NH-CH₃, -S(=O)₂-NH-C₂H₅, -S(=O)₂-NH-phenyl, -S(=O)₂-CH₃, -S(=O)₂-C₂H₅, -(CH₂)-benzo[b]furanyl, -O-phenyl, -S-phenyl, -S-benzyl, -O-benzyl, phenyl and benzyl, while in each case the cyclic moiety of the residues -O-C(=O)-phenyl, -(CH₂)-O-C(=O)-phenyl, -S(=O)₂-NH-phenyl, -O-phenyl, -S-phenyl, -S-benzyl, -O-benzyl, phenyl, -(CH₂)-benzo[b]furanyl and benzyl can be substituted by 1, 2, 3, 4 or 5 substituents in each case independently selected from the group consisting of F, Cl, Br, -OH, -CF₃, -SF₅, -CN, -NO₂, -O-CH₃, -O-C₂H₅, -O-CH(CH₃)₂, -O-C(CH₃)₃, methyl, ethyl, n-propyl, isopropyl, n-butyl, sec-butyl, isobutyl, tert-butyl, -O-CF₃, -S-CF₃, phenyl and -O-benzyl,
or for -(CH₂)-R¹³. -(CH₂)-(CH₂)-R¹³, -(CH₂)-O-R¹³, -(CH₂)-S-R¹³, -(CH₂)-N(CH₃)-R¹³, -(CH₂)-(CH₂)-(CH₂)-R¹³, -(CH₂)-(CH₂)-O-R¹³, -(CH₂)-(CH₂)-S-R¹³, -(CH₂)-( CH₂)-NH-R¹³, -(CH₂)-(CH₂)-N(CH₃)-R¹³, -(CH₂)-(CH₂)-O-(CH₂)-R¹³, -(CH₂)-(CH₂)-S-(CH₂)-R¹³ or -(CH₂)-(CH₂)-N(CH₃)-R¹³;
R³ stands for an -S(=O)₂-R⁴ group, for a -C(=S)-NH-R⁵ group or for a -C(=O)-NH-R⁶ group,
R⁴ stands for a residue -NR¹⁰R¹¹;
or for a residue selected from the group consisting of methyl, ethyl, n-propyl, isopropyl, n-butyl, sec-butyl, isobutyl, tert-butyl, n-pentyl, 2-pentyl, 3-pentyl, -(CH₂)-(C(CH₃)₃), n-hexyl, 3-hexyl, -(CH₂)-(CH₂)-(C(CH₃)₃), n-heptyl, 3-heptyl, 4-heptyl, n-octyl, -(CH₂)-(CH)(C₂H₅)-(CH₂)-(CH₂)-(CH₂)-(CH₃), vinyl, 1-propenyl, 2-propenyl, 1-butenyl, 2-butenyl, 3-butenyl, 1-propynyl, 2-propynyl, 1-butynyl, 2-butynyl and 3-butynyl, while the residue can in each case be substituted by 1, 2, 3, 4 or 5 substituents in each case independently selected from the group consisting of F, Cl, Br, I, -CN, -NO₂, -OH, -SH, -NH₂, -N(CH₃)₂, -N(C₂H₅)₂ and -N(CH₃)(C₂H₅);
or for a (hetero)cycloaliphatic residue selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, adamantyl and 7,7-dimethyl-bicycto[2.2.1]hepty!, while the (hetero)cycloaliphatic residue can in each case be substituted by 1, 2, 3, 4 or 5 substituents in each case independently selected from the group consisting of oxo (=O), thioxo (=S), F, Cl, Br, I, -CN, -CF₃, -SF₅, -OH, -O-CH₃, -O-C₂H₅, -O-C(CH₃)₃, -NH₂, -NO₂, -O-CF₃, -S-CF₃, -SH, S-CH₃, -S-C₂H₅, -S-C(CH₃)₃, methyl, ethyl, n-propyl, isopropyl, n-butyl, sec-butyl, isobutyl, tert-butyl, -C(=O)-H, -C(=O)-CH₃, -C(=O)-C₂H₅, -C(=O)-OH, -C(=O)-O-CH₃, -C(=O)-O-C₂H₅, -C(=O)-O-C(CH₃)₃, -(CH₂)-C(=O)-OH, -(CH₂)-C(=O)-O-CH₃, -₍CH₂₎-C(=O)-O-C₂H₅, -N(CH₃)₂, -N(C₂H₅)₂, -N(H)(CH₃), -N(H)(C₂H₅), -(CH₂)-benzo[b]furanyl, -O-phenyl, -O-benzyl, phenyl, benzyl, naphthyl and -(CH₂)-naphthyl, while in each case the cyclic moiety of the residues -O-phenyl, -O-benzyl, phenyl, -(CH₂)-benzo[b]furanyl, benzyl, naphthyl and -(CH₂)-naphthyl can be substituted by 1, 2, 3, 4 or 5 substituents in each case independently selected from the group consisting of F, Cl, Br, -OH, -CF₃, -SF₅, -CN, -NO₂, -O-CH₃, -O-C₂H₅, methyl, ethyl, n-propyl, isopropyl, n-butyl, sec-butyl, isobutyl, tert-butyl, -O-CF₃, -S-CF₃, phenyl and -O-benzyl,
or for a residue selected from the group consisting of phenyl, naphthyl, thiophenyl, furanyl, pyrrolyl, pyrazolyl, pyrazinyl, triazolyl, pyridinyl, imidazolyl, indolyl, isoindolyl, benzo[b]furanyl, benzo[b]thiophenyl, thiazolyl, thiadiazolyl, oxazolyl, isoxazolyl, pyridazinyl, pyrazinyl, pyrimidinyl, indazolyl, quinazolinyl, quinolinyl, isoquinolinyl, phenazinyl, phenothiazinyl, oxadiazolyl, benzo[1,2,5]oxadiazolyl, chromanyl and (1,2,3,4)-tetrahydroquinolinyl, while the residue can in each case be substituted by 1, 2, 3, 4 or 5 substituents in each case independently selected from the group consisting of F, Cl, Br, I, -CN, -CF₃, -SF₅, -OH, -O-CH₃, -O-C₂H₅, -O-CH(CH₃)₂, -O-C(CH₃)₃, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, -S-CH(CH₃)₂, -S-C(CH₃)₃, -S-CH₂-CH₂-CH₂-CH₃, methyl, ethyl, n-propyl, isopropyl, n-butyl, sec-butyl, isobutyl, tert-butyl, (1,1)-dimethylpropyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-phenyl, -(CH₂)-O-C(=O)-CH₃, -(CH₂)-O-C(=O)-C₂H₅, -(CH₂)-O-C(=O)-phenyl, -N(CH₃)₂, -N(C₂H₅)₂, -N(H)(CH₃), -N(H)(C₂H₅), -NH-C(=O)-O-CH₃, -NH-C(=O)-O-C₂H₅, -NH-C(=O)-CH₃, -NH-C(=O)-C₂H₅, -NH-C(=O)-CH₃, -NH-C(=O)-C₂H₅, -C(=O)-H, -C(=O)-CH3, -C(=O)-C₂H₅, -C(=O)-CF₃, -C(=O)-NH₂, -C(=O)-NH-CH₃, -C(=O)-NH-C₂H₅, -S(=O)₂-NH₂, -S(=O)₂-NH-CH₃, -S(=O)₂-NH-C₂H₅, -S(=O)₂-NH-phenyl, -S(=O)₂-CH₃, -S(=O)₂-C₂H₅, -(CH₂)-benzo[b]furanyl, -O-phenyl, -S-phenyl, -S-benzyl, -O-benzyl, phenyl and benzyl, while in each case the cyclic moiety of the residues -O-C(=O)-phenyl, -(CH₂)-O-C(=O)-phenyl, -S(=O)₂-NH-phenyl, -O-phenyl, -S-phenyl, -S-benzyl, -O-benzyl, phenyl, -(CH₂)-benzo[b]furanyl and benzyl can be substituted by 1, 2, 3, 4 or 5 substituents in each case independently selected from the group consisting of F, Cl, Br, -OH, -CF₃, -SF₅, -CN, -NO₂, -O-CH₃, -O-C₂H₅, O-CH(CH₃)₂, -O-C(CH₃)₃, methyl, ethyl, n-propyl, isopropyl, n-butyl, sec-butyl, isobutyl, tert-butyl, -O-CF₃, -S-CF₃, phenyl and -O-benzyl,
or for -(CH₂)-R¹⁴, -(CH₂)-(CH₂)-R¹⁴, -(CH₂)-O-R¹⁴, -(CH₂)-S-R¹⁴, -(CH₂)-N(CH₃)-R¹⁴, -(CH₂)-(CH₂)-(CH₂)-R¹⁴, -(CH₂)-(CH₂)-O-R¹⁴, -(CH₂)-(CH₂)-S-R¹⁴, -(CH₂)-(CH₂)-NH-R¹⁴, -(CH₂)-(CH₂)-N(CH₃)-R¹⁴, -(CH₂)-(CH₂)-O-(CH₂)-R¹⁴, -(CH₂)-(CH₂)-S-(CH₂)-R¹⁴ or -(CH₂)-(CH₂)-N(CH₃)-R¹⁴;
R⁵ stands for a residue selected from the group consisting of methyl, ethyl, n-propyl, isopropyl, n-butyl, sec-butyl, isobutyl, tert-butyl, n-pentyl, 2-pentyl, 3-pentyl, -(CH₂)-(C(CH₃)₃), n-hexyl, 3-hexyl, -(CH₂)-(CH₂)-(C(CH₃)₃), n-heptyl, 3-heptyl, 4-heptyl, n-octyl, -(CH₂)-(CH)(C₂H₅)-(CH₂)-(CH₂)-(CH₂)-(CH₃), vinyl, 1-propenyl, 2-propenyl, 1-butenyl, 2-butenyl, 3-butenyl, 1-propynyl, 2-propynyl, 1-butynyl, 2-butynyl and 3-butynyl, while the residue can in each case be substituted by 1, 2, 3, 4 or 5 substituents in each case independently selected from the group consisting of F, Cl, Br, I, -CN, -NO₂, -OH, -SH, -NH₂, -N(CH₃)₂, -N(C₂H₅)₂ and -N(CH₃)(C₂H₅);
or for a (hetero)cycloaliphatic residue selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl and dithiolanyl, while the (hetero)cycloaliphatic residue can in each case be substituted by 1, 2, 3, 4 or 5 substituents in each case independently selected from the group consisting of oxo (=O), thioxo (=S), F, Cl, Br, I, -CN, -CF₃, -SF₅, -OH, -O-CH₃, -O-C₂H₅, -O-C(CH₃)₃, -NH₂, -NO₂, -O-CF₃, -S-CF₃, -SH, S-CH₃, -S-C₂H₅, -S-C(CH₃)₃, methyl, ethyl, n-propyl, isopropyl, n-butyl, sec-butyl, isobutyl, tert-butyl, -C(=O)-H, -C(=O)-CH₃, -C(=O)-C₂H₅, -C(=O)-OH, -C(=O)-O-CH₃, -C(=O)-O-C₂H₅, -C(=O)-O-C(CH₃)₃, -(CH₂)-C(=O)-OH, -(CH₂)-C(=O)-O-CH₃, -(CH₂)-C(=O)-O-C₂H₅, -N(CH₃)₂, -N(C₂H₅)₂, -N(H)(CH₃), -N(H)(C₂H₅), -(CH₂)-benzo[b]furanyl, -O-phenyl, -O-benzyl, phenyl, benzyl, naphthyl and -(CH₂)-naphthyl, while in each case the cyclic moiety of the residues -O-phenyl, -O-benzyl, phenyl, -(CH₂)-benzo[b]furanyl, benzyl, naphthyl and -(CH₂)-naphthyl can be substituted by 1, 2, 3, 4 or 5 substituents in each case independently selected from the group consisting of F, Cl, Br, -OH, -CF₃, -SF₅, -CN, -NO₂, -O-CH₃, -O-C₂H₅, methyl, ethyl, n-propyl, isopropyl, n-butyl, sec-butyl, isobutyl, tert-butyl, -O-CF₃, -S-CF₃, phenyl and -O-benzyl,
or for a residue selected from the group consisting of phenyl, naphthyl, thiophenyl, furanyl, pyrrolyl, pyrazolyl, pyrazinyl, triazolyl, pyridinyl, imidazolyl, indolyl, isoindolyl, benzo[b]furanyl, benzo[b]thiophenyl, thiazolyl, thiadiazolyl, oxazolyl, isoxazolyl, pyridazinyl, pyrazinyl, pyrimidinyl, indazolyl, quinazolinyl, quinolinyl, isoquinolinyl, phenazinyl, phenothiazinyl, oxadiazolyl, benzo[1,2,5]oxadiazolyl and chromanyl, while the residue can in each case be substituted by 1, 2, 3, 4 or 5 substituents in each case independently selected from the group consisting of F, Cl, Br, I, -CN, -CF₃, -SF₅, -OH, -O-CH₃, -O-C₂H₅, -O-CH(CH₃)₂, -O-C(CH₃)₃, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, -S-CH(CH₃)₂, -S-C(CH₃)₃, -S-CH₂-CH₂-CH₂-CH₃, methyl, ethyl, n-propyl, isopropyl, n-butyl, sec-butyl, isobutyl, tert-butyl, (1,1)-dimethylpropyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-phenyl, -(CH₂)-O-C(=O)-CH₃, -(CH₂)-O-C(=O)-C₂H₅, -(CH₂)-O-C(=O)-phenyl, -N(CH₃)₂, -N(C₂H₅)₂, -N(H)(CH₃), -N(H)(C₂H₅), -NH-C(=O)-O-CH₃, -NH-C(=O)-O-C₂H₅, -NH-C(=O)-CH₃, -NH-C(=O)-C₂H₅, -C(=O)-H, -C(=O)-CH₃, -C(=O)-C₂H₅, -C(=O)-CF₃, -C(=O)-NH₂, -C(=O)-NH-CH₃, -C(=O)-NH-C₂H₅, -S(=O)₂-NH₂, -S(=O)₂-NH-CH₃, -S(=O)₂-NH-C₂H₅, -S(=O)₂-NH-phenyl, -S(=O)₂-CH₃, -S(=O)₂-C₂H₅, -(CH₂)-benzo[b]furanyl, -O-phenyl, -S-phenyl, -S-benzyl, -O-benzyl, phenyl and benzyl, while in each case the cyclic moiety of the residues -O-C(=O)-phenyl, -(CH₂)-O-C(=O)-phenyl, -S(=O)₂-NH-phenyl, -O-phenyl, -S-phenyl, -S-benzyl, -O-benzyl, phenyl, -(CH₂)-benzo[b]furanyl, and benzyl can be substituted by 1, 2, 3, 4 or 5 substituents in each case independently selected from the group consisting of F, Cl, Br, -OH, -CF₃, -SF₅, -CN, -NO₂, -O-CH₃, -O-C₂H₅, -O-CH(CH₃)₂, -O-C(CH₃)₃, methyl, ethyl, n-propyl, isopropyl, n-butyl, sec-butyl, isobutyl, tert-butyl, -O-CF₃, -S-CF₃, phenyl and -O-benzyl,
or for -(CHR¹⁵)-R¹⁶, -(CHR¹⁵)-(CH₂)-R¹⁶, -(CHR¹⁵)-O-R¹⁶, -(CHR¹⁵)-S-R¹⁶, -(CHR¹⁵)-N(CH₃)-R¹⁶, -(CHR¹⁵)-(CH₂)-(CH₂)-R¹⁶, -(CHR¹⁵)-(CH₂)-O-R¹⁶, -(CHR¹⁵)-(CH₂)-S-R¹⁶, -(CHR¹⁵)-(CH₂)-NH-R¹⁶, -(CHR¹⁵)-(CH₂)-N(CH₃)-R¹⁶, -(CHR¹⁵)-(CH₂)-O-(CH₂)-R¹⁶, -(CHR¹⁵)-(CH₂)-S-(CH₂)-R¹⁶ or -(CHR¹⁵)-(CH₂)-N(CH₃)-R¹⁶;
or for a -C(=O)-OR⁸ residue;
or for a -(CHR¹⁷)-C(=O)-OR⁹ residue or for a -(CHR¹⁶)-(CH₂)-C(=O)-OR⁹residue;
R⁶ stands for a (hetero)cycloaliphatic residue selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl and dithiolanyl, while the (hetero)cycloaliphatic residue can in each case be substituted by 1, 2, 3, 4 or 5 substituents in each case independently selected from the group consisting of oxo (=O), thioxo (=S), F, Cl, Br, I, -CN, -CF₃, -SF₅, -OH, -O-CH₃, -O-C₂H₅, -O-C(CH₃)₃, -NH₂, -NO₂, -O-CF₃, -S-CF₃, -SH, S-CH₃, -S-C₂H₅, -S-C(CH₃)₃, methyl, ethyl, n-propyl, isopropyl, n-butyl, sec-butyl, isobutyl, tert-butyl, -C(=O)-H, -C(=O)-CH₃, -C(=O)-C₂H₅, -C(=O)-OH, -C(=O)-O-CH₃, -C(=O)-O-C₂H₅, -C(=O)-O-C(CH₃)₃, -(CH₂)-C(=O)-OH, -(CH₂)-C(=O)-O-CH₃, -(CH₂)-C(=O)-O-C₂H₅, -N(CH₃)₂, -N(C₂H₅)₂, -N(H)(CH₃), -N(H)(C₂H₅), -(CH₂)-benzo[b]furanyl, -O-phenyl, -O-benzyl, phenyl, benzyl, naphthyl and -(CH₂)-naphthyl can be substituted, while in each case the cyclic moiety of the residues -O-phenyl, -O-benzyl, phenyl, -(CH₂)-benzo[b]furanyl, benzyl, naphthyl and -(CH₂)-naphthyl can be substituted by 1, 2, 3, 4 or 5 substituents in each case independently selected from the group consisting of F, Cl, Br, -OH, -CF₃, -SF₅, -CN, -NO_{2,} -O-CH₃, -O-C₂H₅, methyl, ethyl, n-propyl, isopropyl, n-butyl, sec-butyl, isobutyl, tert-butyl, -O-CF₃, -S-CF₃, phenyl and -O-benzyl,
or for a residue selected from the group consisting of phenyl, naphthyl, thiophenyl, furanyl, pyrrolyl, pyrazolyl, pyrazinyl, triazolyl, pyridinyl, imidazolyl, indolyl, isoindolyl, benzo[b]furanyl, benzo[b]thiophenyl, thiazolyl, thiadiazolyl, oxazolyl, isoxazolyl, pyridazinyl, pyrazinyl, pyrimidinyl, indazolyl, quinazolinyl, quinolinyl, isoquinolinyl, phenazinyl, phenothiazinyl, oxadiazolyl, benzo[1,2,5]oxadiazolyl and chromanyl, while the residue can in each case be substituted by 1, 2, 3, 4 or 5 substituents in each case independently selected from the group consisting of F, Cl, Br, I, -CN, -CF₃, -SF₅, -OH, -O-CH₃, -O-C₂H₅, -O-CH(CH₃)₂, -O-C(CH₃)₃, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, -S-CH(CH₃)₂, -S-C(CH₃)₃, -S-CH₂-CH₂-CH₂-CH₃, methyl, ethyl, n-propyl, isopropyl, n-butyl, sec-butyl, isobutyl, tert-butyl, (1,1)-dimethylpropyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-phenyl, -(CH₂)-O-C(=O)-CH₃, -(CH₂)-O-C(=O)-C₂H₅, -(CH₂)-O-C(=O)-phenyl, -N(CH₃)₂, -N(C₂H₅)₂, -N(H)(CH₃), -N(H)(C₂H₅), -NH-C(=O)-O-CH₃, -NH-C(=O)-O-C₂H₅, -NH-C(=O)-CH₃, -NH-C(=O)-C₂H₅, -C(=O)-H, -C(=O)-CH₃, -C(₌O)-C₂H₅, -C(=O)-CF₃, -C(=O)-NH₂, -C(=O)-NH-CH₃, -C(=O)-NH-C₂H₅, -S(=O)₂-NH₂, -S(=O)₂-NH-CH₃, -S(=O)2-NH-C₂H₅, -S(=O)₂-NH-phenyl, -S(=O)₂-CH₃, -S(=O)-C₂H₅, -(CH₂)-benzo[b]furanyl, -O-phenyl, -S-phenyl, -S-benzyl, -O-benzyl, phenyl and benzyl, while in each case the cyclic moiety of the residues -O-C(=O)-phenyl, -(CH₂)-O-C(=O)-phenyl, -S(=O)₂-NH-phenyl, -O-phenyl, -S-phenyl, -S-benzyl, -O-benzyl, phenyl, -(CH₂)-benzo[b]furanyl and benzyl can be substituted by 1, 2, 3, 4 or 5 substituents in each case independently selected from the group consisting of F, Cl, Br, -OH, -CF₃, -SF₅, -CN, -NO₂, -O-CH₃, -O-C₂H₅, -O-CH(CH₃)₂, -O-C(CH₃)₃, methyl, ethyl, n-propyl, isopropyl, n-butyl, sec-butyl, isobutyl, tert-butyl, -O-CF₃, -S-CF₃, phenyl and -O-benzyl,
or for -(CH₂)-R¹⁸, -(CH₂)-(CH₂)-R¹⁸, -(CH₂)-O-R2¹⁸, -(CH₂)-S-R¹⁸, -(CH₂)-N(CH₃)-R¹⁸, -(CH₂)-(CH₂)-(CH₂)-R¹⁸, -(CH₂)-(CH₂)-O-R¹⁸, -(CH₂)-(CH2)-S-R¹⁸, -(CH₂)-(CH₂)-NH-R¹⁸, -(CH₂)-(CH₂)-N(CH₃)-R¹⁸, -(CH₂)-(CH₂)-O-(CH₂)-R¹⁸, -(CH₂)-(CH₂)-S-(CH₂)-R¹⁸ or -(CH₂)-(CH₂)-N(CH₃)-R¹⁸;
R⁷, R⁸, R⁹, R¹⁰ and R¹¹ in each case independently
stand for a residue selected from the group consisting of methyl, ethyl, n-propyl, isopropyl, n-butyl, sec-butyl, isobutyl, tert-butyl, n-pentyl, 2-pentyl, 3-pentyl, -(CH₂)-(C(CH₃)₃), n-hexyl, 3-hexyl, -(CH₂)-(CH₂)-(C(CH₃)₃), n-heptyl, 3-heptyl, 4-heptyl, n-octyl, -(CH₂)-(CH)(C₂H₅)-(CH₂)-(CH₂)-(CH₂)-(CH₃), vinyl, 1-propenyl, 2-propenyl, 1-butenyl, 2-butenyl, 3-butenyl, 1-propynyl, 2-propynyl, 1-butynyl, 2-butynyl and 3-butynyl, while the residue can in each
case be substituted by 1, 2, 3, 4 or 5 substituents in each case independently selected from the group consisting of F, Cl, Br, I, -CN, -NO₂, -OH, -SH, -NH₂, -N(CH₃)₂, -N(C₂H₅)₂ and -N(CH₃)(C₂H₅);
R¹² and R¹³ in each case independently
stand for a residue selected from the group consisting of methyl, ethyl, n-propyl, isopropyl, n-butyl, sec-butyl, isobutyl, tert-butyl, n-pentyl, 2-pentyl, 3-pentyl, -(CH₂)-(C(CH₃)₃), n-hexyl, 3-hexyl, -(CH₂)-(CH₂)-(C(CH₃)₃), n-heptyl, 3-heptyl, 4-heptyl, n-octyl, -(CH₂)-(CH)(C₂H₅)-(CH₂)-(CH₂)-(CH₂)-(CH₃), vinyl, 1-propenyl, 2-propenyl, 1-butenyl, 2-butenyl, 3-butenyl, 1-propynyl, 2-propynyl, 1-butynyl, 2-butynyl and 3-butynyl, while the residue can in each case be substituted by 1, 2, 3, 4 or 5 substituents in each case independently selected from the group consisting of F, Cl, Br, I, -CN, -NO₂, -OH, -SH, -NH₂, -N(CH₃)₂, -N(C₂H₅)₂ and -N(CH₃)(C₂H₅);
or for a residue selected from the group consisting of phenyl, naphthyl, thiophenyl, furanyl, pyrrolyl, pyrazolyl, pyrazinyl, triazolyl, pyridinyl, imidazolyl, indolyl, isoindolyl, benzo[b]furanyl, benzo[b]thiophenyl, thiazolyl, thiadiazolyl, oxazolyl, isoxazolyl, pyridazinyl, pyrazinyl, pyrimidinyl, indazolyl, quinazolinyl, quinolinyl, isoquinolinyl, phenazinyl, phenothiazinyl, oxadiazolyl, benzo[1,2,5]oxadiazolyl and chromanyl, while the residue can in each case be substituted by 1, 2, 3, 4 or 5 substituents in each case independently selected from the group consisting of F, Cl, Br, I, -CN, -CF₃, -SF₅, -OH, -O-CH₃, -O-C₂H₅, -O-CH(CH₃)₂, -O-C(CH₃)₃, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, -S-CH(CH₃)₂, -S-C(CH₃)₃, -S-CH₂-CH₂CH₂-CH₃, methyl, ethyl, n-propyl, isopropyl, n-butyl, sec-butyl, isobutyl, tert-butyl, (1,1)-dimethylpropyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-phenyl, -(CH₂)-O-C(=O)-CH₃, -(CH₂)-O-C(=O)-C₂H₅, -(CH₂)-O-C(=O)-phenyl, -N(CH₃)₂, -N(C₂H₅)₂, -N(H)(CH₃), -N(H)(C₂H₅), -NH-C(=O)-O-CH₃, -NH-C(=O)-O-C₂H₅, -NH-C(=O)-CH₃, -NH-C(=O)-C₂H₅, -C(=O)-H, -C(=O)-CH₃, -C(=O)-C₂H₅, -C(=O)-CF₃, -C(=O)-NH₂, -C(=O)-NH-CH₃, -C(=O)-NH-C₂H₅, -S(=O)₂-NH₂, -S(=O)₂-NH-CH₃, -S(=O)₂-NH-C₂H₅, -S(=O)₂-NH-phenyl, -S(=O)₂-CH₃, -S(=O)₂-C₂H₅, -(CH₂)-benzo[b]furanyl, -O-phenyl, -S-phenyl, -S-benzyl, -O-benzyl, phenyl and benzyl, while in each case the cyclic moiety of the residues -O-C(=O)-phenyl, -(CH₂)-O-C(=O)-phenyl, -S(=O)₂-NH-phenyl, -O-phenyl, -S-phenyl, -S-benzyl, -O-benzyl, phenyl, -(CH₂)-benzo[b]furanyl and benzyl can be substituted by 1, 2, 3, 4 or 5 substituents in each case independently selected from the group consisting of F, Cl, Br, -OH, -CF₃, -SF₅, -CN, -NO₂, -O-CH₃, -O-C₂H₅, -O-CH(CH₃)₂, -O-C(CH₃)₃, methyl, ethyl, n-propyl, isopropyl, n-butyl, sec-butyl, isobutyl, tert-butyl, -O-CF₃, -S-CF₃, phenyl and -O-benzyl,
R¹⁴ stands for a residue selected from the group consisting of methyl, ethyl, n-propyl, isopropyl, n-butyl, sec-butyl, isobutyl, tert-butyl, n-pentyl, 2-pentyl, 3-pentyl, -(CH₂)-(C(CH₃)₃), n-hexyl, 3-hexyl, -(CH₂)-(CH₂)-(C(CH₃)₃), n-heptyl, 3-heptyl, 4-heptyl, n-octyl, -(CH₂)-(CH)(C₂H₅)-(CH₂)-(CH₂)-(CH₂)-(CH₃), vinyl, 1-propenyl, 2-propenyl, 1-butenyl, 2-butenyl, 3-butenyl, 1-propynyl, 2-propynyl, 1-butynyl, 2-butynyl and 3-butynyl, while the residue can in each case be substituted by 1, 2, 3, 4 or 5 substituents in each case independently selected from the group consisting of F, Cl, Br, I, -CN, -NO₂, -OH, -SH, -NH₂, -N(CH₃)₂, -N(C₂H₅)₂ and -N(CH₃)(C₂H₅);
or for a (hetero)cycloaliphatic residue selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, adamantyl and 7,7-dimethyl-bicyclo[2.2.1]heptyl, while each of the (hetero)cycloaliphatic residues can be substituted by 1, 2, 3, 4 or 5 substituents in each case independently selected from the group consisting of oxo (=O), thioxo (=S), F, Cl, Br, I, -CN, -CF₃, -SF₅, -OH, -O-CH₃, -O-C₂H₅, -O-C(CH₃)₃, -NH₂, -NO₂, -O-CF₃, -S-CF₃, -SH, S-CH₃, -S-C₂H₅, -S-C(CH₃)₃, methyl, ethyl, n-propyl, isopropyl, n-butyl, sec-butyl, isobutyl, tert-butyl, -C(=O)-H, -C(=O)-CH₃, -C(=O)-C₂H₅, -C(=O)-OH, -C(=O)-O-CH₃, -C(₌O)-O-C₂H₅, -C(₌O)-O-C(CH₃)₃, -(CH₂)-C(=O)-OH, -(CH₂)-C(=O)-O-CH₃, -(CH₂)-C(=O)-O-C₂H₅, -N(CH₃)₂, -N(C₂H₅)₂, -N(H)(CH₃), -N(H)(C₂H₅), -(CH₂)-benzo[b]furanyl, -O-phenyl, -O-benzyl, phenyl, benzyl, naphthyl and -(CH₂)-naphthyl, while in each case the cyclic moiety of the residues -O-phenyl, -O-benzyl, phenyl, -(CH₂)-benzo[b]furanyl, benzyl, naphthyl and -(CH₂)-naphthyl can be substituted by 1, 2, 3, 4 or 5 substituents in each case independently selected from the group consisting of F, Cl, Br, -OH, -CF₃, -SF₅, -CN, -NO₂, -O-CH₃, -O-C₂H₅, methyl, ethyl, n-propyl, isopropyl, n-butyl, sec-butyl, isobutyl, tert-butyl, -O-CF₃, -S-CF₃, phenyl and -O-benzyl,
or for a residue selected from the group consisting of phenyl, naphthyl, thiophenyl, furanyl, pyrrolyl, pyrazolyl, pyrazinyl, triazolyl, pyridinyl, imidazolyl, indolyl, isoindolyl, benzo[b]furanyl, benzo[b]thiophenyl, thiazolyl, thiadiazolyl, oxazolyl, isoxazolyl, pyridazinyl, pyrazinyl, pyrimidinyl, indazolyl, quinazolinyl, quinolinyl, isoquinolinyl, phenazinyl, phenothiazinyl, oxadiazolyl, benzo[1,2,5]oxadiazolyl, chromanyl and (1,2,3,4)-tetrahydroquinolinyl, while the residue can in each case be substituted by 1, 2, 3, 4 or 5 substituents in each case independently selected from the group consisting of F, Cl, Br, I, -CN, -CF₃, -SF₅, -OH, -O-CH₃, -O-C₂H₅, -O-CH(CH₃)₂, -O-C(CH₃)₃, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, -S-CH(CH₃)₂, -S-C(CH₃)₃, -S-CH₂-CH₂-CH₂-CH₃, methyl, ethyl, n-propyl, isopropyl, n-butyl, sec-butyl, isobutyl, tert-butyl, (1,1)-dimethytpropyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-phenyl, -(CH₂)-O-C(=O)-CH₃, -(CH₂)-O-C(=O)-C₂H₅, -(CH₂)-O-C(=O)-phenyl, -N(CH₃)₂, -N(C₂H₅)₂, -N(H)(CH₃), -N(H)(C₂H₅), -NH-C(=O)-O-CH₃, -NH-C(=O)-O-C₂H₅, -NH-C(=O)-CH₃, -NH-C(=O)-C₂H₅, -C(=O)-H, -C(=O)-CH₃, -C(=O)-C₂H₅, -C(=O)-CF₃, -C(=O)-NH₂, -C(=O)-NH-CH₃, -C(=O)-NH-C₂H₅, -S(=O)₂-NH₂, -S(=O)₂-NH-CH₃, -S(=O)₂-NH-C₂H₅, -S(=O)₂-NH-phenyl, -S(=O)₂-CH₃, -S(=O)₂-C₂H₅, -(CH₂)-benzo[b]furanyl, -O-phenyl, -S-phenyl, -S-benzyl, -O-benzyl, phenyl and benzyl, while in each case the cyclic moiety of the residues -O-C(=O)-phenyl, -(CH₂)-O-C(=O)-phenyl, -S(=O)₂-NH-phenyl, -O-phenyl, -S-phenyl, -S-benzyl, -O-benzyl, phenyl, -(CH₂)-benzo[b]furanyl and benzyl can be substituted by 1, 2, 3, 4 or 5 substituents in each case independently selected from the group consisting of F, Cl, Br, -OH, -CF₃, -SF₅, -CN, -NO₂, -O-CH₃, -O-C₂H₅, -O-CH(CH₃)₂, -O-C(CH₃)₃, methyl, ethyl, n-propyl, isopropyl, n-butyl, sec-butyl, isobutyl, tert-butyl, -O-CF₃, -S-CF₃, phenyl and -O-benzyl,
R¹⁵ stands for a hydrogen residue
or for a residue selected from the group consisting of methyl, ethyl, n-propyl, isopropyl, n-butyl, sec-butyl, isobutyl, tert-butyl, n-pentyl, 2-pentyl, 3-pentyl, -(CH₂)-(C(CH₃)₃), n-hexyl, 3-hexyl, -(CH₂)-(CH₂)-(C(Ch₃)₃), n-heptyl, 3-heptyl, 4-heptyl, n-octyl, -(CH₂)-(CH)(C₂H₅)-(CH₂)-(Ch₂)-(CH₂)-(CH₃), vinyl, 1-propenyl, 2-propenyl, 1-butenyl, 2-butenyl, 3-butenyl, 1-propynyl, 2-propynyl, 1-butynyl, 2-butynyl and 3-butynyl, while the residue can in each case be substituted by 1, 2, 3, 4 or 5 substituents in each case independently selected from the group consisting of F, Cl, Br, I, -CN, -NO₂, -OH, -SH, -NH₂, -N(CH₃)₂, -N(C₂H₅)₂ and -N(CH₃)(C₂H₅);
R¹⁶ stands for a residue selected from the group consisting of methyl, ethyl, n-propyl, isopropyl, n-butyl, sec-butyl, isobutyl, tert-butyl, n-pentyl, 2-pentyl, 3-pentyl, -(CH₂)-(C(CH₃)₃), n-hexyl, 3-hexyl, -(CH₂)-(CH₂)-(C(CH₃)₃), n-heptyl, 3-heptyl, 4-heptyl, n-octyl, -(CH₂)-(CH)(C₂H₅)-(CH₂)-(CH₂)-(CH₂)-(CH₃), vinyl, 1-propenyl, 2-propenyl, 1-butenyl, 2-butenyl, 3-butenyl, 1-propynyl, 2-propynyl, 1-butynyl, 2-butynyl and 3-butynyl, while the residue can in each case be substituted by1, 2, 3, 4 or 5 substituents in each case independently selected from the group consisting of F, Cl, Br, I, -CN, -NO₂, -OH, -SH, -NH₂, -N(CH₃)₂, -N(C₂H₅)₂ and -N(CH₃)(C₂H₅);
or for a (hetero)cycloaliphatic residue selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl and dithiolanyl, while each of the (hetero)cycloaliphatic residues can be substituted by 1, 2, 3, 4 or 5 substituents in each case independently selected from the group consisting of oxo (=O), thioxo (=S), F, Cl, Br, I, -CN, -CF₃, -SF₅, -OH, -O-CH₃, -O-C₂H₅, -O-C(CH₃)₃, -NH₂, -NO₂, -O-CF₃, -S-CF₃, -SH, S-CH₃, -S-C₂H₅, -S-C(CH₃)₃, methyl, ethyl, n-propyl, isopropyl, n-butyl, sec-butyl, isobutyl, tert-butyl, -C(=O)-H, -C(=O)-CH₃, -C(=O)-C₂H₅, -C(=O)-OH, -C(=O)-O-CH₃, -C(=O)-O-C₂H₅, -C(=O)-O-C(CH₃)₃, -(CH₂)-C(=O)-OH, -(CH₂)-C(=O)-O-CH₃, -(CH₂)-C(=O)-O-C₂H₅, -N(CH₃)₂, -N(C₂H₅)₂, -N(H)(CH₃), -N(H)(C₂H₅), -(CH₂)-benzo[b]furanyl, -O-phenyl, -O-benzyl, phenyl, benzyl, naphthyl and -(CH₂)-naphthyl, while in each case the cyclic moiety of the residues -O-phenyl, -O-benzyl, phenyl, -(CH₂)-benzo[b]furanyl, benzyl, naphthyl and -(CH₂)-naphthyl can be substituted by 1, 2, 3, 4 or 5 substituents in each case independently selected from the group consisting of F, Cl, Br, -OH, -CF₃, -SF₅, -CN, -NO₂, -O-CH₃, -O-C₂H₅, methyl, ethyl, n-propyl, isopropyl, n-butyl, sec-butyl, isobutyl, tert-butyl, -O-CF₃, -S-CF₃, phenyl and -O-benzyl,
or for a residue selected from the group consisting of phenyl, naphthyl, thiophenyl, furanyl, pyrrolyl, pyrazolyl, pyrazinyl, triazolyl, pyridinyl, imidazolyl, indolyl, isoindolyl, benzo[b]furanyl, benzo[b]thiophenyl, thiazolyl, thiadiazolyl, oxazolyl, isoxazolyl, pyridazinyl, pyrazinyl, pyrimidinyl, indazolyl, quinazolinyl, quinolinyl, isoquinolinyl, phenazinyl, phenothiazinyl, oxadiazolyl, benzo[1,2,5]oxadiazolyl, chromanyl and (1,2,3,4)-tetrahydroquinolinyl, while the residue can in each case be substituted by 1, 2, 3, 4 or 5 substituents in each case independently selected from the group consisting of F, Cl, Br, I, -CN, -CF₃, -SF₅, -OH, -O-CH₃, -O-C₂H₅, -O-CH(CH₃)₂, -O-C(CH₃)₃, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, -S-CH(CH₃)₂, -S-C(CH₃)₃, -S-CH₂-CH₂-CH₂-CH₃, methyl, ethyl, n-propyl, isopropyl, n-butyl, sec-butyl, isobutyl, tert-butyl, (1,1)-dimethylpropyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-phenyl, -(CH₂)-O-C(=O)-CH₃, -(CH₂)-O-C(=O)-C₂H₅, -(CH₂)-O-C(=O)-phenyl, -N(CH₃₎₂, -N(C₂H₅)₂, -N(H)(CH₃), -N(H)(C₂H₅), -NH-C(=O)-O-CH₃, -NH-C(=O)-O-C₂H₅, -NH-C(=O)-CH₃, -NH-C(=O)-C₂H₅, -C(=O)-H, -C(=O)-CH₃, -C(=O)-C₂H₅, -C(=O)-CF₃, -C(=O)-NH₂, -C(=O)-NH-CH₃, -C(=O)-NH-C₂H₅, -S(=O)₂-NH₂, -S(=O)₂-NH-CH₃, -S(=O)₂-NH-C₂H₅, -S(=O)₂-NH-phenyl, -S(=O)₂-CH₃, -S(=O)₂-C₂H₅, -(CH₂)-benzo[b]furanyl, -O-phenyl, -S-phenyl, -S-benzyl, -O-benzyl, phenyl and benzyl, while in each case the cyclic moiety of the residues -O-C(=O)-phenyl, -(CH₂)-O-C(=O)-phenyl, -S(=O)₂-NH-phenyl, -O-phenyl, -S-phenyl, -S-benzyl, -O-benzyl, phenyl, -(CH₂)-benzo[b]furanyl, and benzyl can be substituted by 1, 2, 3, 4 or 5 substituents in each case independently selected from the group consisting of F, Cl, Br, -OH, -CF₃, -SF₅, -CN, -NO₂, -O-CH₃, -O-C₂H₅, -O-CH(CH₃)₂, -O-C(CH₃)₃, methyl, ethyl, n-propyl, isopropyl, n-butyl, sec-butyl, isobutyl, tert-butyl, -O-CF₃, -S-CF₃, phenyl and -O-benzyl,
R¹⁷ stands for a residue selected from the group consisting of methyl, ethyl, n-propyl, isopropyl, n-butyl, sec-butyl, isobutyl, tert-butyl, n-pentyl, 2-pentyl, 3-pentyl, -(CH₂)-(C(CH₃)₃), n-hexyl, 3-hexyl, -(CH₂)-(CH₂)-(C(CH₃)₃), n-heptyl, 3-heptyl, 4-heptyl, n-octyl, -(CH₂)-(CH)(C₂H₅)-(CH₂)-(CH₅)-(CH₂)-(CH₃), vinyl, 1-propenyl, 2-propenyl, 1-butenyl, 2-butenyl, 3-butenyl, 1-propynyl, 2-propynyl, 1-butynyl, 2-butynyl and 3-butynyl, while the residue can in each case be substituted by 1, 2, 3, 4 or 5 substituents in each case independently selected from the group consisting of F, Cl, Br, I, -CN, -NO₂, -OH, -SH, -NH₂, -N(CH₃)₂, -N(C₂H₅)₂ and -N(CH₃)(C₂H₅);
and
R¹⁸ stands for a (hetero)cycloaliphatic residue selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl and dithiolanyl, while the (hetero)cycloaliphatic residue can in each case be substituted by 1, 2, 3, 4 or 5 substituents in each case independently selected from the group consisting of oxo (=O), thioxo (=S), F, Cl, Br, I, -CN, -CF₃, -SF₅, -OH, -O-CH₃, -O-C₂H₅, -O-C(CH₃)₃, -NH₂, -NO₂, -O-CF₃, -S-CF₃, -SH, S-CH₃, -S-C₂H₅, -S-C(CH₃)₃, methyl, ethyl, n-propyl, isopropyl, n-butyl, sec-butyl, isobutyl, tert-butyl, -C(=O)-H, -C(=O)-CH₃, -C(=O)-C₂H₅, -C(=O)-OH, -C(=O)-O-CH₃, -C(=O)-O-C₂H₅, -C(=O)-O-C(CH₃)₃, -(CH₂)-C(=O)-OH, -(CH₂)-C(=O)-O-CH₃, -(CH₂)-C(=O)-O-C₂H₅, -N(CH₃)₂, -N(C₂H₅)₂, -N(H)(CH₃), -N(H)(C₂H₅), -(CH₂)-benzo[b]furanyl, -O-phenyl, -O-benzyl, phenyl, benzyl, naphthyl and -(CH₂)-naphthyl, while in each case the cyclic moiety of the residues -O-phenyl, -O-benzyl, phenyl, -(CH₂)-benzo[b]furanyl, benzyl, naphthyl and -(CH₂)-naphthyl can be substituted by 1, 2, 3, 4 or 5 substituents in each case independently selected from the group consisting of F, Cl, Br, -OH, -CF₃, -SF₅, -CN, -NO₂, -O-CH₃, -O-C₂H₅, methyl, ethyl, n-propyl, isopropyl, n-butyl, sec-butyl, isobutyl, tert-butyl, -O-CF₃, -S-CF₃, phenyl and -O-benzyl,
or for a residue selected from the group consisting of phenyl, naphthyl, thiophenyl, furanyl, pyrrolyl, pyrazolyl, pyrazinyl, triazolyl, pyridinyl, imidazolyl, indolyl, isoindolyl, benzo[b]furanyl, benzo[b]thiophenyl, thiazolyl, thiadiazolyl, oxazolyl, isoxazolyl, pyridazinyl, pyrazinyl, pyrimidinyl, indazolyl, quinazolinyl, quinolinyl, isoquinolinyl, phenazinyl, phenothiazinyl, oxadiazolyl, benzo[1,2,5]oxadiazolyl, chromanyl and (1,2,3,4)-tetrahydroquinolinyl, while the residue can in each case be substituted by 1, 2, 3, 4 or 5 substituents in each case independently selected from the group consisting of F, Cl, Br, I, -CN, -CF₃, -SF₅, -OH, -O-CH₃, -O-C₂H₅, -O-CH(CH₃)₂, -O-C(CH₃)₃, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, -S-CH(CH₃)₂, -S-C(CH₃)₃, -S-CH₂-CH₂-CH₂-CH₃, methyl, ethyl, n-propyl, isopropyl, n-butyl, sec-butyl, isobutyl, tert-butyl, (1,1)-dimethylpropyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-phenyl, -(CH₂)-O-C(=O)-CH₃, -(CH₂)-O-C(=O)-C₂H₅, -(CH₂)-O-C(=O)-phenyl, -N(CH₃)₂, -N(C₂H₅)₂, -N(H)(CH₃), -N(H)(C₂H₅), -NH-C(=O)-O-CH₃, -NH-C(=O)-O-C₂H₅, -NH-C(=O)-CH₃, -NH-C(=O)-C₂H₅, -C(=O)-H, -C(=O)-CH₃, -C(=O)-C₂H₅, -C(=O)-CF₃, -C(=O)-NH₂, -C(=O)-NH-CH₃, -C(=O)-NH-C₂H₅, -S(=O)₂-NH₂, -S(=O)₂-NH-CH₃, -S(=O)₂-NH-C₂H₅, -S(=O)₂-NH-phenyl, -S(=O)₂-CH₃, -S(=O)₂-C₂H₅, -(CH₂)-benzo[b]furanyl, -O-phenyl, -S-phenyl, -S-benzyl, -O-benzyl, phenyl and benzyl, while in each case the cyclic moiety of the residues -O-C(=O)-phenyl, -(CH₂)-O-C(=O)-phenyl, -S(=O)₂-NH-phenyl, -O-phenyl, -S-phenyl, -S-benzyl, -O-benzyl, phenyl, -(CH₂)-benzo[b]furanyl and benzyl can be substituted by 1, 2, 3, 4 or 5 substituents in each case independently selected from the group consisting of F, Cl, Br, -OH, -CF₃, -SF₅, -CN, -NO₂, -O-CH₃, -O-C₂H₅, -O-CH(CH₃)₂, -O-C(CH₃)₃, methyl, ethyl, n-propyl, isopropyl, n-butyl, sec-butyl, isobutyl, tert-butyl, -O-CF₃, -S-CF₃, phenyl and -O-benzyl.

11. Compounds as defined in Claim 9 or Claim 10, **characterised in that**
R¹ stands for a hydrogen residue;
or for a residue selected from the group consisting of methyl, ethyl, n-propyl, isopropyl, n-butyl, sec-butyl, isobutyl, tert-butyl, n-pentyl, 1-propenyl, 2-propenyl, 1-propynyl und 2-propynyl;
or for -(CH₂)-C(=O)-O-R⁷;
or for -(CH₂)-R¹², -(CH₂)-(CH₂)-R¹², -(CH₂)-O-R¹², -(CH₂)-S-R¹², -(CH₂)-N(CH₃)-R¹², -(CH₂)-(CH₂)-(CH₂)-R¹² or -(CH₂)-(CH₂)-O-R¹²;
R² stands for a hydrogen residue;
or for a residue selected from the group consisting of methyl, ethyl, n-propyl, isopropyl, n-butyl, sec-butyl, isobutyl, tert-butyl und n-pentyl;
or for -(CH₂)-R¹³, -(CH₂)-(CH₂)-R¹³, -(CH₂)-O-R¹³, -(CH₂)-S-R¹³, -(CH₂)-N(CH₃)-R¹³, -(CH₂)-(CH₂)-S-R¹³ or -(CH₂)-(CH₂)-O-R¹³;
R³ stands for an -S(=O)₂-R⁴ group, for a -C(=S)-NH-R⁵ group or for a -C(=O)-NH-R⁶ group,
R⁴ stands for a residue -NR¹⁰R¹¹;
or for a residue selected from the group consisting of methyl, ethyl, n-propyl, isopropyl, n-butyl, sec-butyl, isobutyl, tert-butyl, n-pentyl, 2-pentyl, 3-pentyl, -(CH₂)-(C(CH₃)₃) und n-hexyl;
or for a residue selected from the group consisting of phenyl, naphthyl, thiophenyl, furanyl und (1,2,3,4)-tetrahydroquinolinyl, while the residue can in each case be substituted by 1, 2, 3, 4 or 5 substituents independently selected from the group consisting of F, Cl, Br, -CF₃, -O-CH₃, -O-C₂H₅, -O-CH(CH₃)₂, -O-C(CH₃)₃, -O-CH₂-CH₂-CH₂-CH₃, -NO₂, -O-CF₃, -O-CHF₂, -O-CH₂F, -S-CF₃, -S-CHF₂, -S-CH₂F, methyl, ethyl, n-propyl, isopropyl, n-butyl, sec-butyl, isobutyl, tert-butyl, (1,1)-dimethyl-propyl, -C(=O)-O-CH₃, -C(=O)-O-C₂H₅, -C(=O)-CH₃, -C(=O)-C₂H₅, -C(=O)-CF₃, -S(=O)₂-CH₃, and -S(=O)₂-C₂H₅,
or for -(CH₂)-R¹⁴, -(CH₂)-(CH₂)-R¹⁴ und -(CH₂)-(CH₂)-(CH₂)-R¹⁴;
R⁵ stands for a residue selected from the group consisting of methyl, ethyl, n-propyl, isopropyl, 1-propenyl und 2-propenyl;
or for a phenyl residue, while each of the phenyl residues can be substituted by 1, 2, 3, 4 or 5 substituents independently selected from the group consisting of F, Cl, Br, -CN, -CF₃, -O-CH₃, -O-C₂H₅, -O-CH(CH₃)₂, -O-C(CH₃)₃, -O-CH₂-CH₂-CH₂-CH₃, -O-CF₃, -O-CHF₂, -O-CH₂F, -S-CF₃, -S-CHF₂, -S-CH₂F, -SH, methyl, ethyl, n-propyl, isopropyl, n-butyl, sec-butyl, isobutyl, tert-butyl, (1,1)-dimethylpropyl, -C(=O)-O-CH₃, -C(=O)-O-C₂H₅ und -C(=O)-CH₃, -C(=O)-C₂H₅,
or for -(CHR¹⁵)-R¹⁶, -(CHR¹⁵)-(CH2)-R¹⁶, -(CHR¹⁵)-(CH₂)-(CH₂)-R¹⁶, -(CHR¹⁵)-(CH₂)-O-R¹⁶ or -(CHR¹⁵)-(CH₂)-S-R¹⁶;
or for a -C(=O)-OR⁸ residue
or for a -(CHR¹⁷)-C(=O)-OR⁹ residue or for a -(CHR¹⁷)-(CH₂)-C(=O)-OR⁹ residue;
R⁶ stands for a cycloaliphatic residue selected from the group consisting of cyclopentyl, cyclohexyl und cycloheptyl;
or for a phenyl residue, which can be substituted by 1, 2, 3, 4 or 5 substituents independently selected from the group consisting of F, Cl, Br, I, -CN, -CF₃, -O-CH₃, -O-C₂H₅, -O-CH(CH₃)₂, -O-C(CH₃)₃, -O-CF₃, -O-CHF₂, -O-CH₂F, -C(=O)-O-CH₃, -C(=O)-O-C₂H₅, -C(=O)-CH₃ und -C(=O)-C₂H₅,
or for -(CH₂)-R¹⁸, -(CH₂)-(CH₂)-R¹⁸ or-(CH₂)-(PH₂)-(CH₂)-R¹⁸;
R⁷ R⁸, R⁹, R¹⁰ und R¹¹ in each case independently
stand for for a methyl- or ethyl residue:
R¹² stands for a phenyl or naphthyl residue, while the residue can in each case be substituted by 1, 2, 3, 4 or 5 substituents independently selected from the group consisting of F, Cl, Br, -CN, -CF₃, -NO₂, methyl, ethyl, n-propyl, isopropyl, n-butyl, sec-butyl, isobutyl, tert-butyl, -C(=O)-O-CH₃, -C(=O)-O-C₂H₅, -C(=O)-CH₃ und -C(=O)-C₂H₅,
R¹³ stands for a methyl residue or an ethyl residue;
or for a phenyl residue, which phenyl residue can in each case be substituted by 1, 2, 3, 4 or 5 substituents independently selected from the group consisting of F, Cl, Br und -CN,
R¹⁴ stands for a 7,7-dimethyl-2-oxo-bicyclo[2.2.1]heptyl or phenyl residue;
R¹⁵ stands for a hydrogen residue
or for a methyl residue or ethyl residue;
R¹⁶ stands for a methyl residue or ethyl residue;
or for a (hetero)cycloaliphatic residue selected from the group consisting of cyclopentyl, cyclohexyl, cycloheptyl, and tetrahydrofuranyl;
or for a phenyl residue, which can be substituted by 1, 2, 3, 4 or 5 substituents independently selected from the group consisting of F, Cl, Br, -CN, -CF₃, -O-CH₃, -O-C₂H₅ und -O-CH(CH₃)₂,
R¹⁷ stands for a methyl residue or ethyl residue;
and
R¹⁸ stands for a (hetero)cycloaliphatic residue selected from the group consisting of cyclopentyl, cyclohexyl und cycloheptyl;
or for a phenyl residue, which can be substituted by 1, 2, 3, 4 or 5 substituents independently selected from the group consisting of F, Cl und Br.

12. Compounds as defined in any one of Claims 1 to 11 selected from the group consisting of
[1] 3-Isopropyl-8-(4-nitrobenzenesulfonyl)-1,4,8-triazaspiro[4.5]decan-2-one,
[2] 8-(2-Chlorobenzenesulfonyl)-3-isobutyl-1,4,8-triazaspiro[4.5]decan-2-one,
[3] 3-Benzyl-8-[2-(2,2,2-trifluoroacetyl)-1,2,3,4-tetrahydroisoquinolin-7-sulfonyl]-1,4,8-triazaspiro[4.5]decan-2-one,
[4] 3-Benzyl-8-(4-methoxy-2,3,6-trimethylbenzenesulfonyl)-1,4,8-triazaspiro[4.5]decan-2-one,
[5] 2-[8-(4-Butoxybenzenesulfonyl)-2-oxo-1,4,8-triazaspiro[4.5]dec-1-ylmethyl]-benzonitrile,
[6] 2-{8-[4-(1,1-Dimethylpropyl)benzenesulfonyl]-2-oxo-1,4,8-triazaspiro[4.5]dec-1-ylmethyl}-benzonitrile,
[7] 3-Benzyl-8-(2-fluorobenzenesulfonyl)-1,4,8-triazaspiro[4.5]decan-2-one,
[8] 8-(2-Chlorobenzenesulfonyl)-3-isopropyl-1,4,8-triazaspiro[4.5]decan-2-one,
[9] 1-Benzyl-2-oxo-1,4,8-triazaspiro[4.5]decane-8-sulfonic acid dimethylamide,
[10] 3-[8-(4-Acetylbenzenesulfonyl)-3-(2-methylsulfanylethyl)-2-oxo-1,4,8-triazaspiro[4.5]dec-1-ylmethyl]-benzonitrile,
[11] 1-(2-Fluorobenzyl)-3-isopropyl-8-(4-methoxy-2,3,6-trimethylbenzenesulfonyl)-1,4,8-triazaspiro[4.5]decan-2-one,
[12] 1-Butyl-8-(5-chlorothiophen-2-sulfonyl)-3-(2-methylsulfanylethyl)-1,4,8-triazaspiro[4.5]decan-2-one,
[13] 8-(4-Acetylbenzenesulfonyl)-1-butyl-3-(2-methylsulfanylethyl)-1,4,8-triazaspiro[4.5]decan-2-one,
[14] 1-Benzyl-3-isopropyl-8-(2,4,6-trimethylbenzenesulfonyl)-1,4,8-triazaspiro[4.5]decan-2-one,
[15] Methyl 2-(1-butyl-3-isopropyl-2-oxo-1,4,8-triazaspiro[4.5]decane-8-sulfonyl)benzoate,
[16] 1,3-Dibenzyl-8-(thiophen-2-sulfonyl)-1,4,8-triazaspiro[4.5]decan-2-one,
[17] 8-(3-Chloro-4-fluorobenzenesulfonyl)-3-isopropyl-1-(2-phenoxyethyl)-1,4,8-triazaspiro[4.5]decan-2-one,
[18] 1-Butyl-3-(2-methylsulfanylethyl)-8-(toluene-3-sulfonyl)-1,4,8-triazaspiro[4.5]decan-2-one,
[19] 1-Butyl-8-(2,4-difluorobenzenesulfonyl)-3-isopropyl-1,4,8-triazaspiro[4.5]decan-2-one,
[20] 1,3-Dibenzyl-8-(2,5-dichlorothiophen-3-sulfonyl)-1,4,8-triazaspiro[4.5]decan-2-one,
[21] 3-Isopropyl-1-(2-phenoxyethyl)-8-(toluene-4-sulfonyl)-1,4,8-triazaspiro[4.5]decan-2-one,
[22] 3-Benzyl-1-butyl-8-(2-trifluoromethylbenzenesulfonyl)-1,4,8-triazaspiro[4.5]decan-2-one,
[23] Methyl 2-[1-(4-fluorobenzyl)-3-(2-methylsulfanylethyl)-2-oxo-1,4,8-triazaspiro[4.5]decane-8-sulfonyl]-benzoate,
[24] 1,3-Dibenzyl-8-(2,3,5,6-tetramethylbenzenesulfonyl)-1,4,8-triazaspiro[4.5]decan-2-one,
[25] 1-(4-Fluorobenzyl)-3-(2-methylsulfanylethyl)-8-(2-trifluoromethylbenzenesulfonyl)-1,4,8-triazaspiro[4.5]decan-2-one,
[26] 8-(4-Chloro-2,5-dimethylbenzenesulfonyl)-1-(4-fluorobenzyl)-3-(2-methylsulfanylethyl)-1,4,8-triazaspiro[4.5]decan-2-one,
[27] 1-Benzyl-8-(4-chloro-2,5-dimethylbenzenesulfonyl)-3-isobutyl-1,4,8-triazaspiro[4.5]decan-2-one,
[28] 3-Benzyl-1-butyl-8-[4-(1,1-dimethylpropyl)benzenesulfonyl]-1,4,8-triazaspiro[4.5]decan-2-one,
[29] 1,3-Dibenzyl-8-(3,4-dimethoxybenzenesulfonyl)-1,4,8-triazaspiro[4.5]decan-2-one,
[30] 1-Butyl-8-(3,4-dimethoxybenzenesulfonyl)-3-(2-methylsulfanylethyl)-1,4,8-triazaspiro[4.5]decan-2-one,
[31] 1,3-Dibenzyl-8-(4-methoxy-2,3,6-trimethylbenzenesulfonyl)-1,4,8-triazaspiro[4.5]decan-2-one,
[32] 1-Benzyl-8-ethanesulfonyl-3-(2-methylsulfanylethyl)-1,4,8-triazaspiro[4.5]decan-2-one,
[33] 3-Benzyl-1-butyl-8-(4-ethylbenzenesulfonyl)-1,4,8-triazaspiro[4.5]decan-2-one,
[34] 3-(2-Methylsulfanylethyl)-1-prop-2-ynyl-8-(3-trifluoromethylbenzenesulfonyl)-1,4,8-triazaspiro[4.5]decan-2-one,
[35] 1,3-Dibenzyl-8-(4-ethylbenzenesulfonyl)-1,4,8-triazaspiro[4.5]decan-2-one,
[36] 3-Benzyl-1-butyl-8-(3-chlorobenzenesulfonyl)-1,4,8-triazaspiro[4.5]decan-2-one,
[37] 1,3-Dibenzyl-8-(2-fluorobenzenesulfonyl)-1,4,8-triazaspiro[4.5]decan-2-one,
[38] Methyl 2-[1-(2-cyanobenzyl)-3-(2-methylsulfanylethyl)-2-oxo-1,4,8-triazaspiro[4.5]decane-8-sulfonyl]-benzoate,
[39] 3-Benzyl-1-prop-2-ynyl-8-(2-trifluoromethylbenzenesulfonyl)-1,4,8-triazaspiro[4.5]decan-2-one,
[40] 3-Benzyl-8-(3-chlorobenzenesulfonyl)-1-methyl-1,4,8-triazaspiro[4.5]decan-2-one,
[41] 1-Benzyl-8-[4-(1,1-dimethylpropyl)benzenesulfonyl]-3-(2-methylsulfanylethyl)-1,4,8-triazaspiro[4.5]decan-2-one,
[42] 8-(2,4-Difluorobenzenesulfonyl)-1-(3,5-dimethylbenzyl)-3-(2-methylsulfanylethyl)-1,4,8-triazaspiro[4.5]decan-2-one,
[43] 1,3-Dibenzyl-8-(4-nitrobenzenesulfonyl)-1,4,8-triazaspiro[4.5]decan-2-one,
[44] 1-(4-Fluorobenzyl)-3-(2-methylsulfanylethyl)-8-(toluene-4-sulfonyl)-1,4,8-triazaspiro[4.5]decan-2-one,
[45] 1,3-Dibenzyl-8-(toluene-4-sulfonyl)-1,4,8-triazaspiro[4.5]decan-2-one,
[46] Ethyl [3-benzyl-8-(2-methyl-5-nitrobenzenesulfonyl)-2-oxo-1,4,8-triazaspiro[4.5]dec-1-yl]-acetate,
[47] Ethyl [3-benzyl-8-(2-methanesulfonyl-benzenesulfonyl)-2-oxo-1,4,8-triazaspiro[4.5]dec-1-yl]-acetate,
[48] Ethyl [3-benzyl-2-oxo-8-(thiophen-2-sulfonyl)-1,4,8-triazaspiro[4.5]dec-1-yl]-acetate,
[49] Methyl 4-(8-ethanesulfonyl-2-oxo-1,4,8-triazaspiro[4.5]dec-1-ylmethyl)benzoate,
[50] Ethyl [3-benzyl-8-(4-methoxybenzenesulfonyl)-2-oxo-1,4,8-triazaspiro[4.5]dec-1-yl]-acetate,
[51] Methyl 4-(2-Oxo-8-benzenemethanesulfonyl-1,4,8-triazaspiro[4.5]dec-1-ylmethyl)benzoate,
[52] 3-[3-Isopropyl-8-(4-nitrobenzenesulfonyl)-2-oxo-1,4,8-triazaspiro[4,5]dec-1-ylmethyl]-benzonitrile,
[53] Methyl 4-[8-(butan-1-sulfonyl)-2-oxo-1,4,8-triazaspiro[4.5]dec-1-ylmethyl]-benzoate,
[54] 1-Allyl-8-(2-methanesulfonyl-benzenesulfonyl)-3-(2-methylsulfanylethyl)-1,4,8-triazaspiro[4.5]decan-2-one,
[55] 1-(2-Fluorobenzyl)-2-oxo-1,4,8-triazaspiro[4.5]decane-8-sulfonic acid dimethylamide,
[56] 8-(7,7-Dimethyl-2-oxobicyclo[2.2.1]hept-1-ylmethanesulfonyl)-1-(2-fluorobenzyl)-1,4,8-triazaspiro[4.5]decan-2-one,
[57] Ethyl [3-(2-methylsulfanylethyl)-2-oxo-8-benzenemethanesulfonyl-1,4,8-triazaspiro[4.5]dec-1-yl]-acetate,
[58] 1-Allyl-3-(2-methylsulfanylethyl)-8-benzenemethanesulfonyl-1,4,8-triazaspiro[4.5]decan-2-one,
[59] 3-[2-Oxo-8-(2,4,6-trimethylbenzenesulfonyl)-1,4,8-triazaspiro[4.5]dec-1-ylmethyl]-benzonitrile,
[60] Methyl 4-[2-oxo-8-(2,4,6-trimethylbenzenesulfonyl)-1,4,8-triazaspiro[4.5]dec-1-ylmethyl]-benzoate,
[61] Ethyl [3-benzyl-8-(5-chlorothiophen-2-sulfonyl)-2-oxo-1,4,8-triazaspiro[4.5]dec-1-yl]-acetate,
[62] Ethyl [3-benzyl-8-(butan-1-sulfonyl)-2-oxo-1,4,8-triazaspiro[4.5]dec-1-yl]-acetate,
[63] Ethyl [3-benzyl-2-oxo-8-(toluene-3-sulfonyl)-1,4,8-triazaspiro[4.5]dec-1-yl]-acetate,
[64] 1-(2-Fluorobenzyl)-8-(4-methoxybenzenesulfonyl)-1,4,8-triazaspiro[4.5]decan-2-one,
[65] Ethyl [3-benzyl-2-oxo-8-(2-trifluoromethylbenzenesulfonyl)-1,4,8-triazaspiro[4.5]dec-1-yl]-acetate,
[66] Ethyl (8-benzenesulfonyl-3-benzyl-2-oxo-1,4,8-triazaspiro[4.5]dec-1-yl)acetate,
[67] Ethyl [3-benzyl-2-oxo-8-(4-propylbenzenesulfonyl)-1,4,8-triazaspiro[4.5]dec-1-yl]-acetate,
[68] Ethyl (3-benzyl-2-oxo-8-benzenemethanesulfonyl-1,4,8-triazaspiro[4.5]dec-1-yl)acetate,
[69] Ethyl [3-benzyl-8-(3-chloro-4-fluorobenzenesulfonyl)-2-oxo-1,4,8-triazaspiro[4.5]dec-1-yl]-acetate,
[70] 1-Allyl-3-isopropyl-8-(2,4,6-trimethylbenzenesulfonyl)-1,4,8-triazaspiro[4.5]decan-2-one,
[71] 8-Benzenesulfonyl-3-benzyl-1-(2-fluorobenzyl)-1,4,8-triazaspiro[4.5]decan-2-one,
[72] Ethyl [3-benzyl-2-oxo-8-(2,4,6-trimethylbenzenesulfonyl)-1,4,8-triazaspiro[4,5]dec-1-yl]-acetate,
[73] Ethyl [3-benzyl-8-(4-butoxybenzenesulfonyl)-2-oxo-1,4,8-triazaspiro[4.5]dec-1-yl]-acetate,
[74] Ethyl [3-benzyl-8-(2,5-dimethoxybenzenesulfonyl)-2-oxo-1,4,8-triazaspiro[4.5]dec-1-yl]-acetate,
[75] 3-(2-Methylsulfanylethyl)-1-naphthalen-2-ylmethyl-2-oxo-1,4,8-triazaspiro[4.5]decane-8-sulfonic acid dimethylamide,
[76] 1-(3-Cyanobenzyl)-3-isopropyl-2-oxo-1,4,8-triazaspiro[4.5]decane-8-sulfonic acid dimethylamide,
[77] 3-{3-Isopropyl-2-oxo-8-[2-(2,2,2-trifluoroacetyl)-1,2,3,4-tetrahydroisoquinolin-7-sulfonyl]-1,4,8-triazaspiro[4.5]dec-1-ylmethyl}-benzonitrile,
[78] 1-Allyl-3-(2-methylsulfanylethyl)-8-[2-(2,2,2-trifluoroacetyl)-1,2,3,4-tetrahydroisoquinolin-7-sulfonyl]-1,4,8-triazaspiro[4.5]decan-2-one,
[79] 1-Allyl-8-(4-methoxy-2,3,6-trimethylbenzenesulfonyl)-3-(2-methylsulfanylethyl)-1,4,8-triazaspiro[4.5]decan-2-one,
[80] Methyl 2-(3-benzyl-1-ethoxycarbonylmethyl-2-oxo-1,4,8-triazaspiro[4.5]decane-8-sulfonyl)benzoate,
[81] 3-Benzyl-1-(2-fluorobenzyl)-8-(2-trifluoromethylbenzenesulfonyl)-1,4,8-triazaspiro[4.5]decan-2-one,
[82] 8-(2-Chlorobenzenesulfonyl)-3-(2-methylsulfanylethyl)-1-(2-nitrobenzyl)-1,4,8-triazaspiro[4.5]decan-2-one,
[83] 1-Allyl-3-(2-methylsulfanylethyl)-8-(toluene-3-sulfonyl)-1,4,8-triazaspiro[4.5]decan-2-one,
[84] Ethyl [8-(2-chlorobenzenesulfonyl)-3-(2-methylsulfanylethyl)-2-oxo-1,4,8-triazaspiro[4.5]dec-1-yl]-acetate,
[85] 3-benzyl-1-(2-fluorobenzyl)-8-(toluene-4-sulfonyl)-1,4,8-triazaspiro[4.5]decan-2-one,
[86] Methyl 4-[8-(2,5-dichlorothiophen-3-sulfonyl)-2-oxo-1,4,8-triazaspiro[4.5]dec-1-ylmethyl]-benzoate,
[87] 3-[8-(4-Fluorobenzenesulfonyl)-3-isopropyl-2-oxo-1,4,8-triazaspiro[4.5]dec-1-ylmethyl]-benzonitrile,
[88] 8-(2,5-Dimethoxybenzenesulfonyl)-3-(2-methylsulfanylethyl)-1-(2-nitrobenzyl)-1,4,8-triazaspiro[4.5]decan-2-one,
[89] 8-(3-Chloro-4-fluorobenzenesulfonyl)-1-(2-fluorobenzyl)-1,4,8-triazaspiro[4.5]decan-2-one,
[90] 1-(2-Fluorobenzyl)-8-(toluene-3-sulfonyl)-1,4,8-triazaspiro[4,5]decan-2-one,
[91] 1-(2,6-Dichlorobenzyl)-8-(4,5-dichlorothiophen-2-sulfonyl)-3-isobutyl-1,4,8-triazaspiro[4.5]decan-2-one,
[92] 8-(5-Chlorothiophen-2-sulfonyl)-1-(2-fluorobenzyl)-1,4,8-triazaspiro[4,5]decan-2-one,
[93] 3-Benzyl-1-(2-fluorobenzyl)-8-(4-propylbenzenesulfonyl)-1,4,8-triazaspiro[4.5]decan-2-one,
[94] 3-[8-(Butan-1-sulfonyl)-3-isopropyl-2-oxo-1,4,8-triazaspiro[4.5]dec-1-ylmethyl]-benzonitrile,
[95] 3-[8-(2-Methanesulfonyl-benzenesulfonyl)-2-oxo-1,4,8-triazaspiro[4.5]dec-1-ylmethyl]-benzonitrile,
[96] 8-(2-Fluorobenzenesulfonyl)-1-(2-fluorobenzyl)-1,4,8-triazaspiro[4.5]decan-2-one,
[97] Methyl 2-[1-(3-cyanobenzyl)-3-isopropyl-2-oxo-1,4,8-triazaspiro[4.5]decane-8-sulfonyl]-benzoate,
[98] 3-Benzyl-1-(2-fluorobenzyl)-8-(5-fluoro-2-methylbenzenesulfonyl)-1,4,8-triazaspiro[4.5]decan-2-one,
[99] 1-Allyl-8-(4-methoxybenzenesulfonyl)-3-(2-methylsulfanylethyl)-1,4,8-triazaspiro[4.5]decan-2-one,
[100] 1-(3,5-Dimethylbenzyl)-3-isobutyl-8-(thiophen-2-sulfonyl)-1,4,8-triazaspiro[4.5]decan-2-one,
[101] 3-(2-Methylsulfanylethyl)-8-(4-nitrobenzenesulfonyl)-1-(2-nitrobenzyl)-1,4,8-triazaspiro[4.5]decan-2-one,
[102] 2-Isobutyl-3-oxo-1,4,8-triazaspiro[4.5]decane-8-thiocarboxylic acid o-tolylamide,
[103] 2-Benzyl-3-oxo-1,4,8-triazaspiro[4.5]decane-8-thiocarboxylic acid isopropylamide,
[104] 1-Butyl-3-isopropyl-2-oxo-1,4,8-triazaspiro[4.5]decane-8-thiocarboxylic acid (tetrahydrofuran-2-ylmethyl)amide,
[105] Ethyl 3-{[1-methyl-3-(2-methylsulfanylethyl)-2-oxo-1,4,8-triazaspiro[4.5]decane-8-carbothioyl]-amino}-butyrate,
[106] 1-Allyl-3-isopropyl-2-oxo-1,4,8-triazaspiro[4.5]decane-8-thiocarboxylic acid isopropylamide,
[107] 1-Butyl-3-isopropyl-2-oxo-1,4,8-triazaspiro[4.5]decane-8-thiocarboxylic acid isopropylamide,
[108] 3-Benzyl-1-(2-fluorobenzyl)-2-oxo-1,4,8-triazaspiro[4.5]decane-8-thiocarboxylic acid (2-methoxybenzene)amide,
[109] Ethyl [8-isopropylthiocarbamoyl-3-(2-methylsulfanylethyl)-2-oxo-1,4,8-triazaspiro[4.5]dec-1-yl]-acetate,
[110] 1-Methyl-3-(2-methylsulfanylethyl)-2-oxo-1,4,8-triazaspiro[4.5]decane-8-thiocarboxylic acid m-tolylamide,
[111] 1-Methyl-3-(2-methylsulfanylethyl)-2-oxo-1,4,8-triazaspiro[4,5]decane-8-thiocarboxylic acid 4-methoxybenzylam ide,
[112] 2-Isobutyl-3-oxo-1,4,8-triazaspiro[4.5]decane-8-thiocarboxylic acid (4-fluorobenzene)amide,
[113] Ethyl [8-ethoxycarbonylaminocarbothioyl-3-(2-methylsulfanylethyl)-2-oxo-1,4,8-triazaspiro[4.5]dec-1-yl]-acetate
[114] Ethyl 3-{[1-ethoxycarbonylmethyl-3-(2-methylsulfanylethyl)-2-oxo-1,4,8-triazaspiro[4.5]decane-8-carbothioyl]-amino}-butyrate,
[115] 1-Allyl-3-isopropyl-2-oxo-1,4,8-triazaspiro[4.5]decane-8-thiocarboxylic acid (2-methoxybenzene)amide,
[116] 1-Butyl-3-isopropyl-2-oxo-1,4,8-triazaspiro[4.5]decane-8-thiocarboxylic acid benzeneamide,
[117] 1-Butyl-3-isopropyl-2-oxo-1,4,8-triazaspiro[4.5]decane-8-thiocarboxylic acid (2,5-dichlorobenzene)amide,
[118] 3-Benzyl-1-methyl-2-oxo-1,4,8-triazaspiro[4.5]decane-8-thiocarboxylic acid (2,5-difluorobenzene)amide,
[119] 1-Methyl-3-(2-methylsulfanylethyl)-2-oxo-1,4,8-triazaspiro[4.5]decane-8-thiocarboxylic acid (2-bromobenzene)amide,
[120] 1-Butyl-3-(2-methylsulfanylethyl)-2-oxo-1,4,8-triazaspiro[4.5]decane-8-thiocarboxylic acid (2,5-dichlorobenzene)amide,
[121] 3-(2-Methylsulfanylethyl)-1-naphthalen-2-ylmethyl-2-oxo-1,4,8-triazaspiro[4.5]decane-8-thiocarboxylic acid (2-methoxyethyl)amide,
[122] 1-Butyl-3-isopropyl-2-oxo-1,4,8-triazaspiro[4.5]decane-8-thiocarboxylic acid (2-fluorobenzene)amide,
[123] 3-Isobutyl-2-oxo-1-prop-2-ynyl-1,4,8-triazaspiro[4.5]decane-8-thiocarboxylic acid allylamide,
[124] 1-Butyl-3-isopropyl-2-oxo-1,4,8-triazaspiro[4.5]decane-8-thiocarboxylic acid m-tolylamide,
[125] 1-Allyl-3-isopropyl-2-oxo-1,4,8-triazaspiro[4.5]decane-8-thiocarboxylic acid (4-fluorobenzene)amide,
[126] Methyl 4-(8-allylthiocarbamoyl-3-isopropyl-2-oxo-1,4,8-triazaspiro[4.5]dec-1-ylmethyl)benzoate,
[127] 1-Butyl-3-(2-methylsulfanylethyl)-2-oxo-1,4,8-triazaspiro[4.5]decane-8-thiocarboxylic acid (2-methoxybenzene)amide,
[128] 1-Allyl-3-isopropyl-2-oxo-1,4,8-triazaspiro[4.5]decane-8-thiocarboxylic acid (2,5-difluorobenzene)amide,
[129] 1-(2-Cyanobenzyl)-3-(2-methylsulfanylethyl)-2-oxo-1,4,8-triazaspiro[4.5]decane-8-thiocarboxylic acid allylamide,
[130] 1-(3-Cyanobenzyl)-3-(2-methylsulfanylethyl)-2-oxo-1,4,8-triazaspiro[4.5]decane-8-thiocarboxylic acid allylamide,
[131] Ethyl 3-[(3-benzyl-1-methyl-2-oxo-1,4,8-triazaspiro[4,5]decane-8-carbothioyl)amino]-butyrate,
[132] 1-Butyl-3-(2-methylsulfanylethyl)-2-oxo-1,4,8-triazaspiro[4.5]decane-8-thiocarboxylic acid cyclohexylmethylamide,
[133] 1-Methyl-3-(2-methylsulfanylethyl)-2-oxo-1,4,8-triazaspiro[4.5]decane-8-thiocarboxylic acid (4-chlorobenzene)amide,
[134] 1-Methyl-3-(2-methylsulfanylethyl)-2-oxo-1,4,8-triazaspiro[4.5]decane-8-thiocarboxylic acid 4-fluorobenzylamide,
[135] Methyl 4-[8-(2,6-dichlorobenzenethiocarbamoyl)-3-isopropyl-2-oxo-1,4,8-triazaspiro[4.5]dec-1-ylmethyl]-benzoate,
[136] 1-Benzyl-3-isopropyl-2-oxo-1,4,8-triazaspiro[4.5]decane-8-thiocarboxylic acid (2,5-dichlorobenzene)amide,
[137] Methyl 4-{3-isopropyl-2-oxo-8-[(tetrahydrofuran-2-ylmethyl)thiocarbamoyl]-1,4,8-triazaspiro[4.5]dec-1-ylmethyl}-benzoate,
[138] 1-Methyl-3-(2-methylsulfanylethyl)-2-oxo-1,4,8-triazaspiro[4.5]decane-8-thiocarboxylic acid (2,5-difluorobenzene)amide,
[139] 1-Benzyl-3-(2-methylsulfanylethyl)-2-oxo-1,4,8-triazaspiro[4.5]decane-8-thiocarboxylic acid (4-isopropylbenzene)amide,
[140] 1-Benzyl-3-(2-methylsulfanylethyl)-2-oxo-1,4,8-triazaspiro[4.5]decane-8-thiocarboxylic acid (2,5-difluorobenzene)amide,
[141] 1-(2-Cyanobenzyl)-3-isobutyl-2-oxo-1,4,8-triazaspiro[4.5]decane-8-thiocarboxylic acid (3-acetylbenzene)amide,
[142] 1-Butyl-3-isopropyl-2-oxo-1,4,8-triazaspiro[4.5]decane-8-thiocarboxylic acid (3-chlorobenzene)amide,
[143] Methyl 2-[(1-butyl-3-isopropyl-2-oxo-1,4,8-triazaspiro[4.5]decane-8-carbothioyl)amino]-benzoate,
[144] Ethyl (3-benzyl-8-ethoxycarbonylaminocarbothioyl-2-oxo-1,4,8-triazaspiro[4.5]dec-1-yl)acetate,
[145] Methyl 2-[(3-benzyl-1-ethoxycarbonylmethyl-2-oxo-1,4,8-triazaspiro[4.5]decane-8-carbothioyl)amino]-benzoate,
[146] Ethyl [1-(2-cyanobenzyl)-3-(2-methylsulfanylethyl)-2-oxo-1,4,8-triazaspiro[4.5]decane-8-carbothioyl]-carbamate,
[147] 1-(2-Cyanobenzyl)-3-(2-methylsulfanylethyl)-2-oxo-1,4,8-triazaspiro[4.5]decane-8-thiocarboxylic acid (2-chloro-5-trifluoromethylbenzene)amide,
[148] 3-(2-Methylsulfanylethyl)-1-naphthalen-2-ylmethyl-2-oxo-1,4,8-triazaspiro[4.5]decane-8-thiocarboxylic acid (1-benzene-ethyl)amide,
[149] 1-Allyl-3-isopropyl-2-oxo-1,4,8-triazaspiro[4.5]decane-8-thiocarboxylic acid pentafluorobenzeneamide,
[150] 1-Butyl-3-isopropyl-2-oxo-1,4,8-triazaspiro[4.5]decane-8-thiocarboxylic acid 4-chlorobenzylamide,
[151] 3-(2-Methylsulfanylethyl)-1-naphthalen-2-ylmethyl-2-oxo-1,4,8-triazaspiro[4.5]decane-8-thiocarboxylic acid o-tolylamide,
[152] 1-Butyl-3-isopropyl-2-oxo-1,4,8-triazaspiro[4.5]decane-8-thiocarboxylic acid pentafluorobenzeneamide,
[153] Methyl 2-{[1-(2-cyanobenzyl)-3-(2-methylsulfanylethyl)-2-oxo-1,4,8-triazaspiro[4.5]decane-8-carbothioyl]-amino}-benzoate,
[154] 1-Benzyl-3-isopropyl-2-oxo-1,4,8-triazaspiro[4.5]decane-8-thiocarboxylic acid pentafluorobenzeneamide;
[155] 1-Benzyl-3-isopropyl-2-oxo-1,4,8-triazaspiro[4.5]decane-8-thiocarboxylic acid (2-methoxybenzene)amide,
[156] 1-Benzyl-3-isopropyl-2-oxo-1,4,8-triazaspiro[4.5]decane-8-thiocarboxylic acid benzeneamide,
[157] 1-Benzyl-3-isopropyl-2-oxo-1,4,8-triazaspiro[4.5]decane-8-thiocarboxylic acid (2,5-difluorobenzene)amide,
[158] 1-Butyl-3-(2-methylsulfanylethyl)-2-oxo-1,4,8-triazaspiro[4.5]decane-8-thiocarboxylic acid (4-ethoxybenzene)amide,
[159] Ethyl [3-benzyl-8-(cyclohexylmethylthiocarbamoyl)-2-oxo-1,4,8-triazaspiro[4.5]dec-1-yl]-acetate,
[160] 1-Butyl-3-(2-methylsulfanylethyl)-2-oxo-1,4,8-triazaspiro[4.5]decane-8-thiocarboxylic acid benzylamide,
[161] 1-(2-Cyanobenzyl)-3-(2-methylsulfanylethyl)-2-oxo-1,4,8-triazaspiro[4.5]decane-8-thiocarboxylic acid m-tolylamide,
[162] 1-Allyl-3-isopropyl-2-oxo-1,4,8-triazaspiro[4.5]decane-8-thiocarboxylic acid 4-fluorobenzylamide,
[163] 3-Isobutyl-2-oxo-1-prop-2-ynyl-1,4,8-triazaspiro[4.5]decane-8-thiocarboxylic acid (3-acetylbenzene)amide,
[164] Methyl 4-(3-isopropyl-2-oxo-8-pentafluorobenzenethiocarbamoyl-1,4,8-triazaspiro[4.5]dec-1-ylmethyl)benzoate,
[165] 3-(2-Methylsulfanylethyl)-1-naphthalen-2-ylmethyl-2-oxo-1,4,8-triazaspiro[4.5]decane-8-thiocarboxylic acid (3-acetylbenzene)amide,
[166] 3-Benzyl-1-(2-fluorobenzyl)-2-oxo-1,4,8-triazaspiro[4.5]decane-8-thiocarboxylic acid m-tolylamide,
[167] Methyl 4-(8-ethoxycarbonylaminocarbothioyl-3-isopropyl-2-oxo-1,4,8-triazaspiro[4.5]dec-1-ylmethyl)benzoate,
[168] 1-(4-Fluorobenzyl)-3-(2-methylsulfanylethyl)-2-oxo-1,4,8-triazaspiro[4.5]decane-8-thiocarboxylic acid (tetrahydrofuran-2-ylmethyl)amide,
[169] 1-(2-Cyanobenzyl)-3-isobutyl-2-oxo-1,4,8-triazaspiro[4.5]decane-8-thiocarboxylic acid (3,5-bis-trifluoromethylbenzene)amide,
[170] 1-(2-Cyanobenzyl)-3-isobutyl-2-oxo-1,4,8-triazaspiro[4.5]decane-8-thiocarboxylic acid benzylamide,
[171] 3-Benzyl-1-(2-fluorobenzyl)-2-oxo-1,4,8-triazaspiro[4.5]decane-8-thiocarboxylic acid cyclohexylmethylamide,
[172] 1-(3-Cyanobenzyl)-3-(2-methylsulfanylethyl)-2-oxo-1,4,8-triazaspiro[4.5]decane-8-thiocarboxylic acid (4-chlorobenzene)amide,
[173] 1-Butyl-3-(2-methylsulfanylethyl)-2-oxo-1,4,8-triazaspiro[4.5]decane-8-thiocarboxylic acid pentafluorobenzeneamide,
[174] Ethyl [3-benzyl-1-(2-fluorobenzyl)-2-oxo-1,4,8-triazaspiro[4.5]decane-8-carbothioyl]-carbamate,
[175] 3-Benzyl-1-methyl-2-oxo-1,4,8-triazaspiro[4.5]decane-8-thiocarboxylic acid 4-chlorobenzylamide,
[176] 1-(4-Fluorobenzyl)-3-(2-methylsulfanylethyl)-2-oxo-1,4,8-triazaspiro[4.5]decane-8-thiocarboxylic acid isopropylamide,
[177] 1-Benzyl-3-isopropyl-2-oxo-1,4,8-triazaspiro[4.5]decane-8-thiocarboxylic acid (3,5-dichlorobenzene)amide,
[178] Ethyl [1-(3-cyanobenzyl)-3-(2-methylsulfanylethyl)-2-oxo-1,4,8-triazaspiro[4.5]decane-8-carbothioyl]-carbamate,
[179] 1-Benzyl-3-isopropyl-2-oxo-1,4,8-triazaspiro[4.5]decane-8-thiocarboxylic acid 4-fluorobenzylamide,
[180] 1-Allyl-3-isopropyl-2-oxo-1,4,8-triazaspiro[4.5]decane-8-thiocarboxylic acid (3,5-dichlorobenzene)amide,
[181] 1-(3-Cyanobenzyl)-3-(2-methylsulfanylethyl)-2-oxo-1,4,8-triazaspiro[4.5]decane-8-thiocarboxylic acid (1-benzene-ethyl)amide,
[182] 1-(4-Fluorobenzyl)-3-(2-methylsulfanylethyl)-2-oxo-1,4,8-triazaspiro[4.5]decane-8-thiocarboxylic acid 4-fluorobenzylamide,
[183] 1-(2-Cyanobenzyl)-3-(2-methylsulfanylethyl)-2-oxo-1,4,8-triazaspiro[4.5]decane-8-thiocarboxylic acid (2-methoxybenzene)amide,
[184] 1-(3-Cyanobenzyl)-3-(2-methylsulfanylethyl)-2-oxo-1,4,8-triazaspiro[4.5]decane-8-thiocarboxylic acid (2-methoxybenzene)amide,
[185] 1-(3-Cyanobenzyl)-3-(2-methylsulfanylethyl)-2-oxo-1,4,8-triazaspiro[4.5]decane-8-thiocarboxylic acid 4-chlorobenzylamide,
[186] 1-(4-Fluorobenzyl)-3-(2-methylsulfanylethyl)-2-oxo-1,4,8-triazaspiro[4.5]decane-8-thiocarboxylic acid o-tolylamide,
[187] 3-Benzyl-1-(2-fluorobenzyl)-2-oxo-1,4,8-triazaspiro[4.5]decane-8-thiocarboxylic acid (2,6-dichlorobenzene)amide,
[188] 3-Benzyl-1-(2-fluorobenzyl)-2-oxo-1,4,8-triazaspiro[4.5]decane-8-thiocarboxylic acid (2-chloro-5-trifluoromethylbenzene)amide,
[189] 1-Butyl-3-(2-methylsulfanylethyl)-2-oxo-1,4,8-triazaspiro[4.5]decane-8-thiocarboxylic acid (4-fluorobenzene)amide,
[190] 3-Isobutyl-2-oxo-1-prop-2-ynyl-1,4,8-triazaspiro[4.5]decane-8-thiocarboxylic acid (2,6-dichlorobenzene)amide,
[191] Methyl 4-[8-(4-chlorobenzylthiocarbamoyl)-3-isopropyl-2-oxo-1,4,8-triazaspiro[4.5]dec-1-ylmethyl]-benzoate,
[192] 1-(4-Fluorobenzyl)-3-(2-methylsulfanylethyl)-2-oxo-1,4,8-triazaspiro[4.5]decane-8-thiocarboxylic acid (3-cyanobenzene)amide,
[193] 1-(2-Cyanobenzyl)-3-(2-methylsulfanylethyl)-2-oxo-1,4,8-triazaspiro[4.5]decane-8-thiocarboxylic acid 4-chlorobenzylamide,
[194] 1-Methyl-3-(2-methylsulfanylethyl)-2-oxo-1,4,8-triazaspiro[4.5]decane-8-thiocarboxylic acid (3-trifluoromethylbenzene)am ide,
[195] 3-Isobutyl-2-oxo-1-prop-2-ynyl-1,4,8-triazaspiro[4.5]decane-8-thiocarboxylic acid (3,5-dichlorobenzene)amide,
[196] 1-(4-Fluorobenzyl)-3-(2-methylsulfanylethyl)-2-oxo-1,4,8-triazaspiro[4.5]decane-8-thiocarboxylic acid (1-benzene-ethyl)amide,
[197] 3-Benzyl-1-(2-fluorobenzyl)-2-oxo-1,4,8-triazaspiro[4.5]decane-8-thiocarboxylic acid 4-chlorobenzylamide,
[198] 2-Isopropyl-3-oxo-1,4,8-triazaspiro[4.5]decane-8-thiocarboxylic acid (4-fluorobenzene)amide,
[199] 1-Benzyl-3-isopropyl-2-oxo-1,4,8-triazaspiro[4.5]decane-8-thiocarboxylic acid benzylamide,
[200] 1-(4-Fluorobenzyl)-3-(2-methylsulfanylethyl)-2-oxo-1,4,8-triazaspiro[4.5]decane-8-thiocarboxylic acid (4-ethoxybenzene)amide,
[201] 3-Benzyl-1-(2-fluorobenzyl)-2-oxo-1,4,8-triazaspiro[4.5]decane-8-thiocarboxylic acid (3-acetylbenzene)amide,
[202] 3-Benzyl-1-methyl-2-oxo-1,4,8-triazaspiro[4.5]decane-8-carboxylic acid cyclohexylamide,
[203] Ethyl [3-(2-methylsulfanylethyl)-2-oxo-8-benzenecarbamoyl-1,4,8-triazaspiro[4.5]dec-1-yl]-acetate,
[204] 1-Allyl-3-isopropyl-2-oxo-1,4,8-triazaspiro[4.5]decane-8-carboxylic acid (2,5-dimethoxybenzene)am ide,
[205] 1-Methyl-3-(2-methylsulfanylethyl)-2-oxo-1,4,8-triazaspiro[4.5]decane-8-carboxylic acid (3-fluorobenzene)amide,
[206] Methyl 4-(8-cyclohexylcarbamoyl-3-isopropyl-2-oxo-1,4,8-triazaspiro[4.5]dec-1-ylmethyl)benzoate,
[207] 1-Butyl-3-(2-methylsulfanylethyl)-2-oxo-1,4,8-triazaspiro[4.5]decane-8-carboxylic acid benzeneamide,
[208] 1-Benzyl-3-(2-methylsulfanylethyl)-2-oxo-1,4,8-triazaspiro[4.5]decane-8-carboxylic acid (3-cyanobenzene)amide,
[209] 1-Allyl-3-isopropyl-2-oxo-1,4,8-triazaspiro[4.5]decane-8-carboxylic acid (2,5-difluorobenzene)amide,
[210] 1-Butyl-3-(2-methylsulfanylethyl)-2-oxo-1,4,8-triazaspiro[4.5]decane-8-carboxylic acid (3,4,5-trimethoxybenzene)amide,
[211] 1-Benzyl-3-(2-methylsulfanylethyl)-2-oxo-1,4,8-triazaspiro[4.5]decane-8-carboxylic acid (4-chloro-3-trifluoromethylbenzene)amide,
[212] 1-Methyl-3-(2-methylsulfanylethyl)-2-oxo-1,4,8-triazaspiro[4.5]decane-8-carboxylic acid (5-chloro-2-methoxybenzene)amide,
[213] 1-Benzyl-3-(2-methylsulfanylethyl)-2-oxo-1,4,8-triazaspiro[4.5]decane-8-carboxylic acid (4-ethoxybenzene)amide,
[214] 1-Benzyl-3-(2-methylsulfanylethyl)-2-oxo-1,4,8-triazaspiro[4.5]decane-8-carboxylic acid (3-acetylbenzene)amide,
[215] Ethyl 3-[(1-butyl-3-isopropyl-2-oxo-1,4,8-triazaspiro[4.5]decane-8-carbonyl)amino]-benzoate,
[216] 1-Butyl-3-isopropyl-2-oxo-1,4,8-triazaspiro[4.5]decane-8-carboxylic acid (2,5-dimethoxybenzene)amide,
[217] 1-(2-Cyanobenzyl)-3-isobutyl-2-oxo-1,4,8-triazaspiro[4.5]decane-8-carboxylic acid 3,4-dichlorobenzylamide,
[218] 1-(2-Cyanobenzyl)-3-isobutyl-2-oxo-1,4,8-triazaspiro[4.5]decane-8-carboxylic acid (2,5-difluorobenzene)amide,
[219] 1-Butyl-3-(2-methylsulfanylethyl)-2-oxo-1,4,8-triazaspiro[4.5]decane-8-carboxylic acid 3,4-dichlorobenzylamide,
[220] 1-Allyl-3-isopropyl-2-oxo-1,4,8-triazaspiro[4.5]decane-8-carboxylic acid (4-trifluoromethoxybenzene)amide,
[221] 1-Butyl-3-(2-methylsulfanylethyl)-2-oxo-1,4,8-triazaspiro[4.5]decane-8-carboxylic acid (4-ethoxybenzene)amide,
[222] 1-Methyl-3-(2-methylsulfanylethyl)-2-oxo-1,4,8-triazaspiro[4.5]decane-8-carboxylic acid (4-trifluoromethoxybenzene)amide,
[223] Methyl 4-[3-isopropyl-8-(3-methoxybenzenecarbamoyl)-2-oxo-1,4,8-triazaspiro[4.5]dec-1-ylmethyl]-benzoate,
[224] 1-(2-Cyanobenzyl)-3-(2-methylsulfanylethyl)-2-oxo-1,4,8-triazaspiro[4.5]decane-8-carboxylic acid benzeneamide,
[225] Ethyl [3-benzyl-8-(3,4-dichlorobenzylcarbamoyl)-2-oxo-1,4,8-triazaspiro[4.5]dec-1-yl]-acetate,
[226] 1-(2-Cyanobenzyl)-3-(2-methylsulfanylethyl)-2-oxo-1,4,8-triazaspiro[4.5]decane-8-carboxylic acid (3-fluorobenzene)amide,
[227] 1-Butyl-3-(2-methylsulfanylethyl)-2-oxo-1,4,8-triazaspiro[4.5]decane-8-carboxylic acid (4-chloro-3-trifluoromethylbenzene)amide,
[228] 1-(4-Fluorobenzyl)-3-(2-methylsulfanylethyl)-2-oxo-1,4,8-triazaspiro[4.5]decane-8-carboxylic acid 3,4-dichlorobenzylamide,
[229] 1-(2-Cyanobenzyl)-3-(2-methylsulfanylethyl)-2-oxo-1,4,8-triazaspiro[4.5]decane-8-carboxylic acid 3,4-dichlorobenzylamide,
[230] 3-Benzyl-1-(2-fluorobenzyl)-2-oxo-1,4,8-triazaspiro[4.5]decane-8-carboxylic acid (2,5-difluorobenzene)amide,
[231] 3-(2-Methylsulfanylethyl)-1-naphthalen-2-ylmethyl-2-oxo-1,4,8-triazaspiro[4.5]decane-8-carboxylic acid (4-ethoxybenzene)amide,
[232] Methyl 4-[8-(3,4-d!chtorobenzylcarbamoyl)-3-isopropyl-2-oxo-1,4,8-triazaspiro[4.5]dec-1-ylmethyl]-benzoate,
[233] 3-Benzyl-1-(2-fluorobenzyl)-2-oxo-1,4,8-triazaspiro[4.5]decane-8-carboxylic acid 3,4-dichlorobenzylamide,
[234] 1-(2-Cyanobenzyl)-3-(2-methylsulfanylethyl)-2-oxo-1,4,8-triazaspiro[4.5]decane-8-carboxylic acid (3-acetylbenzene)amide,
[235] Ethyl 4-{[3-benzyl-1-(2-fluorobenzyl)-2-oxo-1,4,8-triazaspiro[4.5]decane-8-carbonyl]-amino}-benzoate,
[236] 1-(3-Cyanobenzyl)-3-(2-methylsulfanylethyl)-2-oxo-1,4,8-triazaspiro[4.5]decane-8-carboxylic acid (3-methoxybenzene)amide,
[237] 1-(4-Fluorobenzyl)-3-(2-methylsulfanylethyl)-2-oxo-1,4,8-triazaspiro[4.5]decane-8-carboxylic acid (4-trifluoromethoxybenzene)amide,
[238] 3-Benzyl-1-(2-fluorobenzyl)-2-oxo-1,4,8-triazaspiro[4,5]decane-8-carboxylic acid cyclohexylamide,
[239] 1-(4-Fluorobenzyl)-3-(2-methylsulfanylethyl)-2-oxo-1,4,8-triazaspiro[4.5]decane-8-carboxylic acid phenethylamide,
[240] 3-(2-Methylsulfanylethyl)-1-naphthalen-2-ylmethyl-2-oxo-1,4,8-triazaspiro[4.5]decane-8-carboxylic acid (3-fluorobenzene)amide,
[241] Methyl 4-[8-(4-chloro-3-trifluoromethylbenzenecarbamoyl)-3-isopropyl-2-oxo-1,4,8-triazaspiro[4.5]dec-1-ylmethyl]-benzoate,
[242] 1-(3-Cyanobenzyl)-3-(2-methylsulfanylethyl)-2-oxo-1,4,8-triazaspiro[4.5]decane-8-carboxylic acid (2,5-dimethoxybenzene)amide,
[243] 1-(4-Fluorobenzyl)-3-(2-methylsulfanylethyl)-2-oxo-1,4,8-triazaspiro[4.5]decane-8-carboxylic acid (4-ethoxybenzene)amide,
[244] 1-(4-Fluorobenzyl)-3-(2-methylsulfanylethyl)-2-oxo-1,4,8-triazaspiro[4.5]decane-8-carboxylic acid (3-cyanobenzene)amide, and
[245] Ethyl [3-benzyl-8-(3-fluorobenzenecarbamoyl)-2-oxo-1,4,8-triazaspiro[4.5]dec-1-yl]-acetate,
[246] 1,3-Dibenzyl-8-methanesulfonyl-1,4,8-triazaspiro[4.5]decan-2-one,
[247] 8-Benzenesulfonyl-1,3-dibenzyl-1,4,8-triazaspiro[4.5]decan-2-one,
[248] 1,3-Dibenzyl-8-(4-chlorobenzenesulfonyl)-1,4,8-triazaspiro[4.5]decan-2-one,
[249] 1,3-Dibenzyl-8-(4-methoxybenzenesulfonyl)-1,4,8-triazaspiro[4.5]decan-2-one,
[250] 1,3-Dibenzyl-2-oxo-1,4,8-triazaspiro[4.5]decane-8-carboxylic acid phenylamide,
[251] 1,3-Dibenzyl-2-oxo-1,4,8-triazaspiro[4.5]decane-8-carboxylic acid (4-methoxyphenyl)amide,
[252] 1,3-Dibenzyl-2-oxo-1,4,8-triazaspiro[4.5]decane-8-thiocarboxylic acid phenylamide,
[253] 1,3-Dibenzyl-2-oxo-1,4,8-triazaspiro[4.5]decane-8-thiocarboxylic acid (2,4-difluorophenyl)amide,
in each case optionally in the form of one of the pure stereoisomers thereof, particularly enantiomers or diastereoisomers thereof, the racemates thereof or in the form of a mixture of stereoisomers, particularly the enantiomers and/or diastereoisomers thereof, in any desired mixing ratio, or in each case in the form of appropriate salts, or in each case in the form of appropriate solvates.

13. A method for the production of substituted 1,4,8-triazaspiro[4.5]decan-2-one compounds as defined in one or more of Claims 1 to 12, **characterised in that** a protected piperidin-4-one of the general formula II, 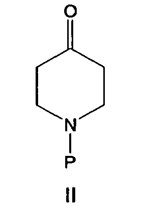 in which P stands for a protective group,
is converted, by reaction with at least one amino acid amide compound of the general formula III, 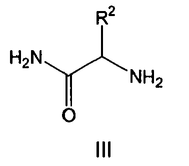 in which R² has the meaning as defined in one or more of Claims 1 to 12,
into at least one compound of the general formula IV, 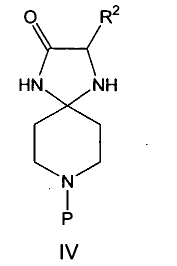 in which P and R² have the aforementioned meanings,
which is optionally purified and/or optionally isolated, and optionally converted,
by reaction with at least one compound of the general formula
R¹-X¹,
in which R¹ has the meaning as defined in one or more of Claims 1 to 12 and X¹ stands for a suitable leaving group, preferably for a halogen residue,
optionally in the presence of at least one base,
into at least one compound of the general formula V, 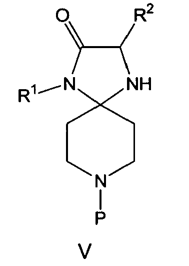 in which R¹, R² and P have the aforementioned meanings,
and this compound is optionally purified and/or optionally isolated, and optionally converted,
by eliminating the protective group P,
into at least one compound of the general formula VI, 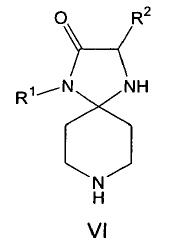 which is optionally purified and/or optionally isolated,
and at least one compound of the general formula IV, V or VI is converted,
by reaction with a sulfonyl compound of the general formula R⁴-SO₂-X², or by reaction with an isothiocyanate compound of the general formula R⁵-NCS or by reaction with an isocyanate compound of the general formula R^{R}-NCO in which the residues R⁴, R⁵ and R⁶ in each case have the meanings as defined in one or more of Claims 1 to 12 and X² stands for a suitable leaving group, preferably for a halogen residue,
into at least one compound of the general formula I as defined in Claim 1, in which the residues R¹ to R³ in each case have meanings as defined in one or more of Claims 1 to 12, and this compound is optionally purified and/or optionally isolated.

14. A medicament containing at least one substituted 1,4,8-triazaspiro[4.5]decan-2-one compound as defined in one or more of Claims 1 to 12 and optionally one or more pharmaceutically acceptable adjuvants.

15. A medicament as defined in Claim 14 for regulation, particularly inhibition, of noradrenalin reuptake (noradrenalin uptake), for regulation, particularly inhibition, of 5-hydroxytryptophan reuptake (5-HT uptake) and/or for batrachotoxin (BTX) receptor regulation and/or for CB2 receptor regulation.

16. A medicament as defined in Claim 14 or Claim 15 for prevention and/or treatment of depression.

17. A medicament as defined in Claim 14 or Claim 15 for prevention and/or treatment of pain, preferably for treatment and/or prevention of acute pain, chronic pain, neuropathic pain, and/or cluster headache.

18. A medicament as defined in Claim 14 or Claim 15 for combined prevention and/or treatment of depression and pain, preferably for combined treatment of depression and pain selected from the group consisting of acute pain, chronic pain, neuropathic pain and cluster headache.

19. A medicament as defined in Claim 14 or Claim 15 for prevention and/or treatment of abuse of alcohol and/or drugs and/or medicaments, for prevention and/or treatment of addiction to alcohol and/or drugs and/or medicines, for prevention and/or treatment of inflammation, for prevention and/or treatment of lethargy, for prevention and/or treatment of disturbances in food intake, preferably selected from the group consisting of bulimia, anorexia, obesity, and cachexia, for prevention and/or treatment of catalepsy, for vigilance enhancement, for libido enhancement or for anxiolysis, for prevention and/or treatment of neurodegenerative disorders, preferably one or more neurodegenerative disorders selected from the group consisting of Parkinson's disease, Huntington's disease, Alzheimer's disease and multiple sclerosis, for prevention and/or treatment of ischaemia and/or for local anaesthesia.

20. A medicament as defined in Claim 14 or Claim 15 for treatment and/or prevention of one or more disorders selected from the group consisting of non-acute allergic disorders, preferably allergic dermatitis; asthma; rhinitis; conjunctivitis; disorders caused by 2-arachidonic glycerol and/or corresponding ethers, such as haematol; sepsis; cancer, particularly leukaemia and/or cerebral tumours; circulatory disorders; green cataract; inflammation, preferably immunologically mediated inflammatory diseases, more preferably rheumatoid arthritis, lupus erythematodes, psoriasis and thyroiditis; diabetes; blood poisoning; epilepsy; Tourette's syndrome; osteoporosis; Bechterew's disease; gout; gouty arthritis; osteoarthritis; disturbances to the blood supply; ischaemia, preferably renal ischaemia, and/or for regulation of the immune system, preferably for suppression of the immune system.

21. Use of at least one substituted 1,4,8-triazaspiro[4.5]decan-2-one compound as defined in any one of Claims 1 to 12 for the production of a medicament for regulation of the noradrenalin reuptake (noradrenalin uptake), preferably for inhibition of the noradrenalin reuptake (noradrenalin uptake).

22. Use of at least one substituted 1,4,8-triazaspiro[4.5]decan-2-one compound as defined in any one of Claims 1 to 12 for the production of a medicament for regulation of 5-hydroxytryptophan reuptake (5-HT uptake), preferably for inhibition of 5-hydroxytryptophan reuptake (5-HT uptake).

23. Use of at least one substituted 1,4,8-triazaspiro[4.5]decan-2-one compound as defined in any one of Claims 1 to 12 for the production of a medicament for batrachotoxin (BTX) receptor regulation.

24. Use of at least one substituted 1,4,8-triazaspiro[4.5]decan-2-one compound as defined in any one of Claims 1 to 12 for the production of a medicament for CB2 receptor regulation.

25. Use of at least one substituted 1,4,8-triazaspiro[4.5]decan-2-one compound as defined in any one of Claims 1 to 12 for the production of a medicament for prevention and/or treatment of depression.

26. Use of at least one substituted 1,4,8-triazaspiro[4.5]decan-2-one compound as defined in any one of Claims 1 to 12 for the production of a medicament for prevention and/or treatment of pain, preferably for treatment and prevention of acute pain, chronic pain, neuropathic pain, and/or cluster headache.

27. Use of at least one substituted 1,4,8-triazaspiro[4.5]decan-2-one compound as defined in any one of Claims 1 to 12 for the production of a medicament for the combined prevention and/or treatment of depression and pain, preferably for the combined prevention and/or treatment of depression and pain selected from the group consisting of acute pain, chronic pain, neuropathic pain, and cluster headache.

28. Use of at least one substituted 1,4,8-triazaspiro[4.5]decan-2-one compound as defined in any one of Claims 1 to 12 for the production of a medicament for prevention and/or treatment of abuse of alcohol and/or drugs and/or medicaments, for prevention and/or treatment of addiction to alcohol and/or drugs and/or medicines, for prevention and/or treatment of inflammation, for prevention and/or treatment of lethargy, for prevention and/or treatment of disturbances in food intake, preferably selected from the group consisting of bulimia, anorexia, obesity, and cachexia, for prevention and/or treatment of catalepsy, for vigilance enhancement, for libido enhancement or for anxiolysis, for prevention and/or treatment of neurodegenerative disorders, preferably one or more neurodegenerative disorders selected from the group consisting of Parkinson's disease, Huntington's disease, Alzheimer's disease and multiple sclerosis, for prevention and/or treatment of ischaemia, and/or for local anaesthesia.

29. Use of at least one substituted 1,4,8-triazaspiro[4.5]decan-2-one compound as defined in any one of Claims 1 to 12 for the preparation of a medicament for treatment and/or prevention one or more disorders selected from the group consisting of non-acute allergic disorders, preferably allergic dermatitis; asthma; rhinitis; conjunctivitis; disorders caused by 2-arachidone glycerol and/or corresponding ethers, such as haematol; sepsis; cancer, particularly leukaemia and/or cerebral tumour; circulatory disorder; green cataract; inflammation, preferably immunologically mediated inflammatory diseases, more preferably rheumatoid arthritis, lupus erythematodes, psoriasis and thyroiditis; diabetes; blood poisoning; epilepsy; Tourette's syndrome; osteoporosis; Bechterew's disease; gout; gouty arthritis; osteoarthritis; disturbances to the blood supply; ischaemia, preferably renal ischaemia, and/or for regulation of the immune system, preferably for suppression of the immune system.

## Revendications

1. Composés substitués de 1,4,8-triazaspiro[4,5]décan-2-one de formule générale I, 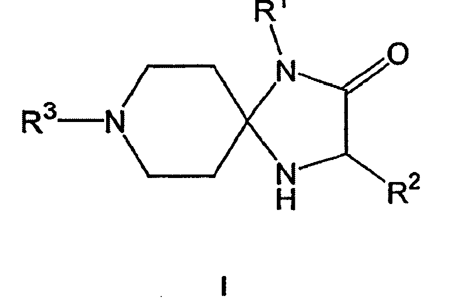 où
R¹ représente un radical hydrogène, un radical alkyle linéaire ou ramifié, non substitué ou au moins monosubstitué, présentant le cas échéant au moins un hétéroatome comme élément de chaîne, un radical alcényle linéaire ou ramifié, non substitué ou au moins monosubstitué, présentant le cas échéant au moins un hétéroatome comme élément de chaîne, un radical alcynyle linéaire ou ramifié, non substitué ou au moins monosubstitué, présentant le cas échéant au moins un hétéroatome comme élément de chaîne, un radical aryle ou hétéroaryle non substitué ou au moins monosubstitué, qui est lié via un groupe alkylène linéaire ou ramifié, présentant le cas échéant au moins un hétéroatome comme élément de chaîne, ou un radical -C(=O)-OR⁷ lié via un groupe alkylène linéaire ou ramifié,
R² représente un radical hydrogène, un radical alkyle linéaire ou ramifié, non substitué ou au moins monosubstitué, présentant le cas échéant au moins un hétéroatome comme élément de chaîne, un radical alcényle linéaire ou ramifié, non substitué ou au moins monosubstitué, présentant le cas échéant au moins un hétéroatome comme élément de chaîne, un radical alcynyle linéaire ou ramifié, non substitué ou au moins monosubstitué, présentant le cas échéant au moins un hétéroatome comme élément de chaîne, un radical aryle ou hétéroaryle non substitué ou au moins monosubstitué, qui peut être lié via un groupe alkylène linéaire ou ramifié, présentant le cas échéant au moins un hétéroatome comme élément de chaîne,
R³ représente un groupe -S(=O)₂-R⁴, un groupe -C(=S)-NH-R⁵ ou un groupe -C(=O)-NH-R⁶,
R⁴ représente un radical -NR¹⁰R¹¹, un radical alkyle linéaire ou ramifié, non substitué ou au moins monosubstitué, présentant le cas échéant au moins un hétéroatome comme élément de chaîne, un radical alcényle linéaire ou ramifié, non substitué ou au moins monosubstitué, présentant le cas échéant au moins un hétéroatome comme élément de chaîne, un radical alcynyle linéaire ou ramifié, non substitué ou au moins monosubstitué, présentant le cas échéant au moins un hétéroatome comme élément de chaîne, un radical aryle ou hétéroaryle non substitué ou au moins monosubstitué, qui peut être lié via un groupe alkylène linéaire ou ramifié, non substitué ou au moins monosubstitué, présentant le cas échéant au moins un hétéroatome comme élément de chaîne et/ou condensé avec un système monocyclique non substitué ou au moins monosubstitué, ou un radical cycloaliphatique non substitué ou au moins monosubstitué, présentant le cas échéant au moins un hétéroatome comme élément de cycle, qui peut être lié via un groupe alkylène linéaire ou ramifié, non substitué ou au moins monosubstitué, présentant le cas échéant au moins un hétéroatome comme élément de chaîne et/ou ponté par un groupe alkylène linéaire ou ramifié, non substitué ou au moins monosubstitué,
R⁵ représente un radical alkyle linéaire ou ramifié, non substitué ou au moins monosubstitué, présentant le cas échéant au moins un hétéroatome comme élément de chaîne, un radical alcényle linéaire ou ramifié, non substitué ou au moins monosubstitué, présentant le cas échéant au moins un hétéroatome comme élément de chaîne, un radical alcynyle linéaire ou ramifié, non substitué ou au moins monosubstitué, présentant le cas échéant au moins un hétéroatome comme élément de chaîne, un radical aryle ou hétéroaryle non substitué ou au moins monosubstitué, qui peut être lié via un groupe alkylène linéaire ou ramifié, non substitué ou au moins monosubstitué, présentant le cas échéant au moins un hétéroatome comme élément de chaîne, un radical cycloaliphatique non substitué ou au moins monosubstitué, présentant le cas échéant au moins un hétéroatome comme élément de cycle, qui peut être lié via un groupe alkylène linéaire ou ramifié, non substitué ou au moins monosubstitué, présentant le cas échéant au moins un hétéroatome comme élément de chaîne, un radical -C(=O)-OR⁸ ou un radical -C(=O)-OR⁹ lié via un groupe alkylène linéaire ou ramifié,
R⁶ représente un radical aryle ou hétéroaryle non substitué ou au moins monosubstitué, qui peut être lié via un groupe alkylène linéaire ou ramifié, non substitué ou au moins monosubstitué, présentant le cas échéant au moins un hétéroatome comme élément de chaîne, ou un radical cycloaliphatique non substitué ou au moins monosubstitué, présentant le cas échéant au moins un hétéroatome comme élément de cycle, qui peut être lié via un groupe alkylène linéaire ou ramifié, non substitué ou au moins monosubstitué, présentant le cas échéant au moins un hétéroatome comme élément de chaîne,
R⁷, R⁸ , R⁹, R¹⁰, R¹¹ représentent indépendamment l'un de l'autre à chaque fois un radical alkyle linéaire ou ramifié, un radical alcényle linéaire
ou ramifié ou un radical alcynyle linéaire ou ramifié,
le cas échéant à chaque fois sous forme d'un de leurs stéréo-isomères purs, en particulier leurs énantiomères ou diastéréo-isomères, leurs racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

2. Composés selon la revendication 1, **caractérisés en ce que** R¹ représente un radical hydrogène, un radical C₁₋₁₀-alkyle linéaire ou ramifié, non substitué ou au moins monosubstitué, présentant le cas échéant au moins un hétéroatome comme élément de chaîne, un radical C₂₋₁₀-alcényle linéaire ou ramifié, non substitué ou au moins monosubstitué, présentant le cas échéant au moins un hétéroatome comme élément de chaîne, un radical C₂₋₁₀-alcynyle linéaire ou ramifié, non substitué ou au moins monosubstitué, présentant le cas échéant au moins un hétéroatome comme élément de chaîne, un radical aryle ou hétéroaryle de 5 à 14 chaînons, non substitué ou au moins monosubstitué, qui est lié via un groupe C₁₋₅-alkylène linéaire ou ramifié, présentant le cas échéant au moins un hétéroatome comme élément de chaîne, ou un radical -C (=O) -OR⁷ lié via un groupe C₁₋₅-alkylène linéaire ou ramifié,
de préférence un radical hydrogène, un radical C₁₋₅-alkyle linéaire ou ramifié, non substitué, un radical C₂₋₅-alcényle linéaire ou ramifié, non substitué, un radical C₂₋₅-alcynyle linéaire ou ramifié, non substitué, un radical phényle ou naphtyle non substitué
ou au moins monosubstitué, qui est lié via un groupe C₁₋₅-alkylène linéaire ou ramifié, présentant le cas échéant au moins un atome d'oxygène comme élément de chaîne, ou un radical -C (=O) -OR⁷ lié via un groupe C₁₋₃-alkylène linéaire ou ramifié,
de manière particulièrement préférée un radical hydrogène, un radical C₁₋₄-alkyle linéaire ou ramifié, non substitué, un radical C₂₋₃-alcényle linéaire ou ramifié, non substitué, un radical C₂₋₃-alcynyle linéaire ou ramifié, non substitué, un radical phényle
ou naphtyle qui est lié via un pont -(CH₂)-, -(CH₂)₂-, -(CH₂)₃- ou -(CH₂)₂-O- et le cas échéant monosubstitué ou polysubstitué, de manière identique ou différente, par un substituant choisi dans le groupe constitué par F, Cl, Br, CN, NO₂, C₁₋₅-alkyle, C₁₋₅-alcoxy, -C(=O)-C₁₋₅-alkyle, -C (=O) -O-C₁₋₅-alkyle, -S (=O) ₂-C₁₋₆-alkyle, -C(=O)-C₁₋₅-perfluoroalkyle, -CF₃, CHF₂ et CH₂F, ou un radical -C(=O)-OR⁷ lié via un groupe -(CH₂)-.

3. Composés selon la revendication 1 ou 2, **caractérisés**
**en ce que** le radical R² représente un radical hydrogène, un radical C₁₋₁₀-alkyle linéaire ou ramifié, non substitué ou au moins monosubstitué, présentant le cas échéant au moins un hétéroatome comme élément de chaîne, un radical C₂₋₁₀-alcényle linéaire ou ramifié, non substitué ou au moins monosubstitué, présentant le cas échéant au moins un hétéroatome comme élément de chaîne, un radical C₂₋₁₀-alcynyle linéaire ou ramifié, non substitué ou au moins monosubstitué, présentant le cas échéant au moins un hétéroatome comme élément de chaîne, un radical aryle ou hétéroaryle de 5 à 14 chaînons, non substitué ou au moins monosubstitué, qui peut être lié via un groupe C₁₋₅-alkylène linéaire ou ramifié, présentant le cas échéant au moins un hétéroatome comme élément de chaîne,
de préférence un radical hydrogène, un radical C₁₋₅-alkyle linéaire ou ramifié, non substitué, présentant le cas échant au moins un atome d'oxygène et/ou au moins un atome de soufre comme élément de chaîne, ou un radical phényle ou naphtyle, qui peut être monosubstitué ou polysubstitué, de manière identique ou différente, par un substituant choisi dans le groupe constitué par F, Cl, Br, CN, NO₂, C₁₋₅-alkyle, C₁₋₅-alcoxy, -C(=O)-C₁₋₅-alkyle, -C(=O)-O-C₁₋₅-alkyle, -S(=O)₂-C₁₋₆-alkyle, -C(=O)-C₁₋₅-perfluoroalkyle, -CF₃, CHF₂ et CH₂F et/ou lié via un pont -(CH₂)-, -(CH₂)₂-, -(CH₂)₃- ou -(CH₂)₂-O-,
de manière particulièrement préférée un radical hydrogène, un radical C₁₋₅-alkyle linéaire ou ramifié, non substitué, présentant le cas échéant au moins un atome de soufre comme élément de chaîne, ou un radical phényle, où le radical phényle peut être monosubstitué ou polysubstitué, de manière identique ou différente, par un substituant choisi dans le groupe constitué par F, Cl, Br, CN, NO₂, C₁₋₅-alkyle, C₁₋₅-alcoxy, -C (=O) -C₁₋₅-alkyle, -C(=O)-O-C₁₋₅-alkyle, -S(=O)₂-C₁₋₆-alkyle, -C(=O)-C₁₋₅-perfluoroalkyle, -CF₃, CHF₂ et CH₂F et/ou lié via un pont -(CH₂)-.

4. Composés selon l'une ou plusieurs des revendications 1 à 3, **caractérisés**
**en ce que** R⁴ représente un radical -NR¹⁰R¹¹, un radical C₁₋₁₀-alkyle linéaire ou ramifié, non substitué ou au moins monosubstitué, présentant le cas échéant au moins un hétéroatome comme élément de chaîne, un radical C₂₋₁₀-alcényle linéaire ou ramifié, non substitué ou au moins monosubstitué, présentant le cas échéant au moins un hétéroatome comme élément de chaîne, un radical C₂₋₁₀-alcynyle linéaire ou ramifié, non substitué ou au moins monosubstitué, présentant le cas échéant au moins un hétéroatome comme élément de chaîne, un radical aryle ou hétéroaryle de 5 à 14 chaînons, non substitué ou au moins monosubstitué, qui peut être lié via un groupe C₁₋₅-alkylène linéaire ou ramifié, non substitué ou au moins monosubstitué, présentant le cas échéant au moins un hétéroatome comme élément de chaîne et/ou condensé avec un système monocyclique de 5 ou 6 chaînons, ou un radical C₃₋₈-cycloaliphatique non substitué ou au moins monosubstitué, présentant le cas échéant au moins un hétéroatome comme élément de cycle, qui peut être lié via un groupe C₁₋₅-alkylène linéaire ou ramifié, non substitué ou au moins monosubstitué, présentant le cas échéant au moins un hétéroatome comme élément de chaîne et/ou ponté par un groupe C₁₋₅-alkylène linéaire ou ramifié, non substitué ou au moins monosubstitué,
de préférence un radical -NR¹⁰R¹¹, un radical C₁₋₄-alkyle linéaire ou ramifié, non substitué, présentant le cas échéant au moins un atome d'oxygène et/ou au moins un atome de soufre comme élément de chaîne, un radical furyle (furannyle), un radical thiényle (thiophényle), un radical phényle, un radical naphtyle ou un radical 1,2,3,4-tétrahydro-isoquinoléine, où le radical cyclique peut être à chaque fois monosubstitué ou polysubstitué, de manière identique ou différente, par un substituant choisi dans le groupe constitué par F, Cl, Br, CN, NO₂, C₁₋₅-alkyle, C₁₋₅-alcoxy, -C(=O)-C₁₋₅-alkyle, -C (=O) -O-C₁₋₅-alkyle, -S(=O)₂-C₁₋₆-alkyle, -C(=O)-C₁₋₅-perfluoroalkyle, -CF₃, CHF₂ et CH₂F et/ou lié via un pont -(CH₂)-, -(CH₂)₂- ou -(CH₂)₃-, ou un radical C₅-cycloaliphatique ou C₆-cycloaliphatique non substitué ou au moins monosubstitué par un groupe oxo, présentant le cas échéant au moins un hétéroatome comme élément de cycle, qui peut être lié via un pont -(CH₂)-, -(CH₂)₂-ou -(CH₂)₃-et/ou ponté par un groupe -(C(CH₃)₂)-,
de manière particulièrement préférée un radical -N(CH₃)₂, un radical C₁₋₄-alkyle linéaire ou ramifié, non substitué, un radical thiényle (thiophényle), phényle ou 1,2,3,4-tétrahydro-isoquinoléine, où le radical cyclique peut à chaque fois être monosubstitué ou polysubstitué, de manière identique ou différente, par un substituant choisi dans le groupe constitué par F, Cl, Br, CN, NO₂, C₁₋₅-alkyle, C₁₋₅-alcoxy, -C(=O)-C₁₋₅-alkyle, -C(=O)-O-C₁₋₅-alkyle, -S(=O)₂-C₁₋₆-alkyle, -C(=O)-C₁₋₅-perfluoroalkyle, -CF₃, CHF₂ et CH₂F et/ou lié via un pont -(CH₂)-, ou un radical C₅-cycloaliphatique ou C₆-cycloaliphatique non substitué ou au moins monosubstitué par un groupe oxo, qui peut être lié via un pont -(CH₂)- et/ou ponté par un groupe -(C(CH₃)₂)-.

5. Composés selon l'une ou plusieurs des revendications 1 à 4, **caractérisés**
**en ce que** le radical R⁵ représente un radical C₁₋₁₀-alkyle linéaire ou ramifié, non substitué ou au moins monosubstitué, présentant le cas échéant au moins un hétéroatome comme élément de chaîne, un radical C₂₋₁₀-alcényle linéaire ou ramifié, non substitué ou au moins monosubstitué, présentant le cas échéant au moins un hétéroatome comme élément de chaîne, un radical C₂₋₁₀-alcynyle linéaire ou ramifié, non substitué ou au moins monosubstitué, présentant le cas échéant au moins un hétéroatome comme élément de chaîne, un radical aryle ou hétéroaryle de 5 à 14 chaînons, non substitué ou au moins monosubstitué, qui peut être lié via un groupe C₁₋₅-alkylène linéaire ou ramifié, non substitué ou au moins monosubstitué, présentant le cas échéant au moins un hétéroatome comme élément de chaîne, un radical C₃₋₈-cycloaliphatique non substitué ou au moins monosubstitué, présentant le cas échéant au moins un hétéroatome comme élément de cycle, qui peut être lié via un groupe C₁₋₅-alkylène linéaire ou ramifié, non substitué ou au moins monosubstitué, présentant le cas échéant au moins un hétéroatome comme élément de chaîne, un radical -C(=O)-OR⁸ ou un radical -C(=O)-OR⁹ lié via un groupe C₁₋₅-alkylène linéaire ou ramifié,
de préférence un radical C₁₋₅-alkyle linéaire ou ramifié, présentant le cas échéant au moins un atome d'oxygène et/ou au moins un atome de soufre comme élément de chaîne, un radical C₂₋₅-alcényle linéaire ou ramifié, présentant le cas échéant au moins un atome d'oxygène et/ou au moins un atome de soufre comme élément de chaîne, un radical C₂₋₅-alcynyle linéaire ou ramifié, présentant le cas échéant au moins un atome d'oxygène et/ou au moins un atome de soufre comme élément de chaîne, un radical furyle (furannyle), thiényle (thiophényle), un radical phényle ou naphtyle, où le radical cyclique peut à chaque fois être monosubstitué ou polysubstitué, de manière identique ou différente, par un substituant choisi dans le groupe constitué par F, Cl, Br, CN, NO₂, C₁₋₅-alkyle, C₁₋₅-alcoxy, -C(=O)-C₁₋₅-alkyle, -C(=O)-O-C₁₋₅-alkyle, -S(=O)₂-C₁₋₆-alkyle, -C(=O)-C₁₋₅-perfluoroalkyle, -CF₃, CHF₂ et CH₂F et/ou lié via un pont -(CH₂)-, -(CH(CH₃))-, -(CH₂)₂-ou -(CH₂)₃-, un radical cycloaliphatique en C₄, C₅ ou C₆, non substitué, présentant le cas échéant au moins un atome d'oxygène comme élément de cycle, qui peut être lié via un pont -(CH₂)-, -(CH(CH₃))-, -(CH₂)₂- ou -(CH₂)₃-, un radical -C(=O)-O-R⁸ ou un radical -C(=O)-OR⁹ lié via un pont -(CH₂)-, -(CH(CH₃))-, -(CH₂)₂- ou -(CH₂)₃-,
de manière particulièrement préférée un radical C₁₋₃-alkyle linéaire ou ramifié, non substitué, présentant le cas échéant au moins un atome d'oxygène comme élément de chaîne, un radical C₂₋₃-alcényle linéaire ou ramifié, non substitué, un radical C₂₋₃-alcynyle linéaire ou ramifié, non substitué, un radical phényle, où le radical phényle peut être monosubstitué ou polysubstitué, de manière identique ou différente, par un substituant choisi dans le groupe constitué par F, Cl, Br, CN, NO₂, C₁₋₅-alkyle, C₁₋₅-alcoxy, -C (=O) -C₁₋₅-alkyle, -C(=O)-O-C₁₋₅-alkyle, -S (=O) ₂-C₁₋₆-alkyle, -C(=O)-C₁₋₅-perfluoroalkyle, -CF₃, CHF₂ et CH₂F et/ou lié via un pont - (CH₂) -, -(CH(CH₃))-, - (CH₂) ₂- ou - (CH₂) ₃-, un radical cycloaliphatique en C₅ ou C₆ non substitué, présentant le cas échéant au moins un atome d'oxygène comme élément de cycle, qui peut être lié via un pont -(CH₂)-, -(CH(CH₃))-, -(CH₂)₂- ou - (CH₂)₃-, un radical C(=O)-O-C₂H₅ ou un radical -C(=O)-O-R⁹ lié via un pont - (CH₂) (CH (CH₃) (CH₂)₂- ou - (CH₂)₃- .

6. Composés selon l'une ou plusieurs des revendications 1 à 5, **caractérisés**
**en ce que** le radical R⁶ représente un radical aryle ou hétéroaryle de 5 à 14 chaînons, non substitué ou au moins monosubstitué, qui peut être lié via un groupe C₁₋₅-alkylène linéaire ou ramifié, non substitué ou au moins monosubstitué, présentant le cas échéant au moins un hétéroatome comme élément de chaîne, ou un radical C₃₋₈-cycloaliphatique non substitué ou au moins monosubstitué, présentant le cas échéant au moins un hétéroatome comme élément de cycle, qui peut être lié via un groupe C₁₋₅-alkylène linéaire ou ramifié, non substitué ou au moins monosubstitué, présentant le cas échéant au moins un hétéroatome comme élément de chaîne,
de préférence un radical phényle, où le radical phényle peut être monosubstitué ou polysubstitué, de manière identique ou différente, par un substituant choisi dans le groupe constitué par F, Cl, Br, CN, NO₂, C₁₋₅-alkyle, C₁₋₅-alcoxy, -C (=O) -C₁₋₅-alkyle, -C (=O) -O-C₁₋₅-alkyle, -S (=O) ₂-C₁₋₆-alkyle, -C(=O)-C₁₋₅-perfluoroalkyle, -CF₃, CHF₂ et CH₂F et/ou lié via un pont -(CH₂)-, -(CH(CH₃))-, - (CH₂) ₂- ou - (CH₂)₃-, ou un radical cycloaliphatique en C₅ ou C₆ non substitué, qui peut être lié via un pont -(CH₂)-, -(CH(CH₃))-, -(CH₂)₂- ou - (CH₂)₃-,
de manière particulièrement préférée un radical phényle, où le radical phényle peut être monosubstitué ou polysubstitué, de manière identique ou différente, par un substituant choisi dans le groupe constitué par F, Cl, Br, CN, NO₂, C₁₋₅-alkyle, C₁₋₅-alcoxy, -C (=O) -C₁₋₅-alkyle, -C(=O)-O-C₁₋₅-alkyle, -S(=O)₂-C₁₋₆-alkyle, -C(=O)-C₁₋₅-perfluoroalkyle, -CF₃, CHF₂ et CH₂F et/ou lié via un pont -(CH₂)-, -(CH(CH₂)₂-, ou un radical cyclohexyle qui peut être lié via un pont -(CH₂)- ou -(CH₂)₂-.

7. Composés selon l'une ou plusieurs des revendications 1 à 6, **caractérisés**
**en ce que** les radicaux R⁷, R⁸, R⁹, R¹⁰, R¹¹, à chaque fois indépendamment l'un de l'autre, représentent un radical C₁₋₅-alkyle linéaire ou ramifié, un radical C₂₋₅-alcényle linéaire ou ramifié ou un radical C₂₋₅-alcynyle linéaire ou ramifié, de préférence un radical méthyle, éthyle, n-propyle ou iso-propyle linéaire ou le cas échéant ramifié.

8. Composés selon l'une ou plusieurs des revendications 1 à 7, **caractérisés en ce que**
R¹ représente un radical hydrogène, un radical alkyle choisi dans le groupe constitué par méthyle, éthyle, n-propyle, iso-propyle, n-butyle, isobutyle, sec-butyle, et tert-butyle, un radical alcényle choisi dans le groupe constitué par vinyle et allyle, un radical propynyle, un radical phényle, 1-naphtyle ou 2-naphtyle, où le radical cyclique est lié via un pont -(CH₂)-, - (CH₂)₂-, - (CH₂)₂- ou - (CH₂)₂-O- et le cas échéant monosubstitué ou polysubstitué, de manière identique ou différente, par un substituant choisi dans le groupe constitué par F, Cl, Br, CN, NO₂, C₁₋₅-alkyle, C₁₋₅-alcoxy, -C (=O) -C₁₋₅-alkyle, -C(=O)-O-C₁₋₅-allkyle, -S(=O)₂-C₁₋₆-alkyle, -C (=O) -C₁₋₅-perfluoroalkyle, -CF₃, CHF₂ et CH₂F, ou un radical -C(=O)-OR⁷ lié via un groupe - (CH₂) -,
R² représente un radical hydrogène, un radical alkyle linéaire ou ramifié, non substitué, présentant le cas échéant au moins un atome de soufre comme élément de chaîne, choisi dans le groupe constitué par méthyle, éthyle, n-propyle, iso-propyle, n-butyle, isobutyle, sec-butyle, tert-butyle et 2-méthylsulfanyléthyle, ou un radical phényle ou benzyle, qui peut être à chaque fois non substitué ou monosubstitué ou polysubstitué, de manière identique ou différente, par un substituant choisi dans le groupe constitué par F, Cl, Br, CN, NO₂, C₁₋₅-alkyle, C₁₋₅-alcoxy, -C(=O)-C₁₋₅-alkyle, -C(=O)-O-C₁₋₅-alkyle, -S (=O) ₂-C₁₋₆-alkyle, -C (=O) -C₁₋₅-perfluoroalkyle, -CF₃, CHF₂ et CH₂F,
R³ représente un groupe -S (=O)₂-R⁴, un groupe -C (=S)-NH-R⁵ ou un groupe -C(=O)-NH-R⁶,
R⁴ représente un radical -N(CH₃)₂, un radical alkyle choisi dans le groupe constitué par méthyle, éthyle, n-propyle, iso-propyle, n-butyle, isobutyle, sec-butyle, tert-butyle, un radical thiényle (thiophényle), phényle, ou 1,2,3,4-tétrahydro-isoquinoléine, où le radical cyclique peut être à chaque fois non substitué ou monosubstitué ou polysubstitué, de manière identique ou différente, par un substituant choisi dans le groupe constitué par F, Cl, Br, CN, NO₂, C₁₋₅-alkyle, C₁₋₅-alcoxy, -C(=O)-C₁₋₅-alkyle, -C(=O)-O-C₁₋₅-alkyle, -S(=O)₂-C₁₋₆-alkyle, -C(=O)-C₁₋₅-perfluoroalkyle, -CF₃, CHF₂ et CH₂F et/ou lié via un pont -(CH₂)-, ou un radical 7,7-diméthyl-2-oxobicyclo-[2,2,1]-hept-1-ylméthyle,
R⁵ représente un radical alkyle choisi dans le groupe constitué par méthyle, éthyle, n-propyle, iso-propyle, n-butyle, isobutyle, sec-butyle, tert-butyle et 2-méthoxyéthyle, un radical alcényle, choisi dans le groupe constitué par vinyle et allyle, un radical propynyle, un radical phényle, où le radical phényle peut être monosubstitué ou polysubstitué, de manière identique ou différente, par un substituant choisi dans le groupe constitué par F, Cl, Br, CN, NO₂, C₁₋₅-alkyle, C₁₋₅-alcoxy, -C (=O) -C₁₋₅-alkyle, -C (=O) -O-C₁₋₅-alkyle, -S (=O)₂-C₁₋₆-alkyle, -C(=O)-C₁₋₅-perfluoroalkyle, -CF₃, CHF₂ et CH₂F et/ou lié via un pont -(CH₂)-, -(CH(CH₃))-, -(CH₂)₂- ou -(CH₂)₃-, un radical cyclohexyle ou tétrahydrofuryle, qui peut être lié via un pont -(CH₂) un radical C(=O)-O-C₂H₅ ou un radical -C(=O)-O-C₂H₅ lié via un pont -(CH₂)-, -(CH(CH₃))-, -(CH₂) ₂- ou -(CH₂)₃-,
R⁶ représente un radical phényle, qui peut être non substitué ou monosubstitué ou polysubstitué, de manière identique ou différente, par un substituant choisi dans le groupe constitué par F, Cl, Br, CN, NO₂, C₁₋₅-alkyle, C₁₋₅-alcoxy, -C(=O)-C₁₋₅-alkyle, -C(=O)-O-C₁₋₅-alkyle, -S (=O) ₂-C₁₋₆-alkyle, -C (=O) -C₁₋₅-perfluoroalkyle, -CF₃, CHF₂ et CH₂F et/ou lié via un pont -(CH₂)- ou -(CH₂)₂-, ou un radical cyclohexyle, qui peut être lié via un pont -(CH₂)- ou -(CH₂)₂-,
R⁷, R⁸, R⁹, R¹⁰, R¹¹, représentent indépendamment l'un de l'autre, à chaque fois un radical C₁₋₅-alkyle linéaire ou ramifié, un radical C₂₋₅-alcényle linéaire ou le cas échéant ramifié ou un radical C₂₋₅-alcynyle linéaire ou ramifié, de préférence un radical méthyle, éthyle, n-propyle ou iso-propyle linéaire ou ramifié.
le cas échéant à chaque fois sous forme d'un de leurs stéréo-isomères purs, en particulier leurs énantiomères ou diastéréo-isomères, leurs racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

9. Composés selon l'une ou plusieurs des revendications 1 à 8, **caractérisés en ce que**
R¹
- représente un radical hydrogène ;
- représente un radical C₁₋₁₀-alkyle, C₂₋₁₀-alcényle ou C₂₋₁₀-alcynyle à chaque fois linéaire ou ramifié, le cas échéant substitué ;
- représente un radical aryle ou hétéroaryle de 5 à 14 chaînons, le cas échéant substitué ;
- représente -(CH₂)ₐₐ-C(=O)-O-R⁷ avec aa = 1, 2, 3, 4 ou 5 ;
- ou représente -(CH₂)-Uₐ-(CH₂)_{b}-V_{c}-(CH₂)_{d}-R¹² avec a = 0 ou 1, b = 0 ou 1, c = 0 ou 1 et d = 0 ou 1, où U et V représentent, indépendamment l'un de l'autre, à chaque fois 0, S, NH, N(CH₃) ou N ( C₂H₅ ) ;
R²
- représente un radical hydrogène ;
- représente un radical C₁₋₁₀-alkyle, C₂₋₁₀-alcényle ou C₂₋₁₀-alcynyle à chaque fois linéaire ou ramifié, le cas échéant substitué ;
- représente un radical aryle ou hétéroaryle de 5 à 14 chaînons, le cas échéant substitué ;
- ou représente -(CH₂)-Wₑ-(CH₂)_{f}-X_{g}-(CH₂)ₕ-R¹³ avec e = 0 ou 1, f = 0 ou 1, g = 0 ou 1 et h = 0 ou 1, où W et X représentent, indépendamment l'un de l'autre, à chaque fois O, S, NH, N(CH₃) ou N(C₂H₅) ;
R³ représente un groupe -S (=O)₂-R⁴, un groupe -C(=S)-NH-R⁵ ou un groupe -C (=O) -NH-R⁶,
R⁴ représente un radical -NR¹⁰R¹¹ ;
- représente un radical C₁₋₁₀-alkyle, C₂₋₁₀-alcényle ou C₂₋₁₀-alcynyle à chaque fois linéaire ou ramifié, le cas échéant substitué ;
- représente un radical cycloaliphatique insaturé ou saturé, le cas échéant substitué, de 3, 4, 5, 6, 7, 8 ou 9 chaînons, qui peut être ponté par un ou deux groupes C₁₋₅-alkylène linéaires ou ramifiés, le cas échéant substitués,
- représente un radical aryle ou hétéroaryle de 5 à 14 chaînons, le cas échéant substitué, qui peut être condensé par un système monocyclique saturé ou insaturé, le cas échéant substitué ;
- ou représente -(CH₂)-Yₖ-(CH₂)ₘ-Zₙ-(CH₂)ₚ-R¹⁴ avec k = 0 ou 1, m = 0 ou 1, n = 0 ou 1 et p = 0 ou 1, où Y et Z représentent, indépendamment l'un de l'autre, à chaque fois O, S, NH, N(CH₃) ou N (C₂H₅) ;
R⁵ représente un radical C₁₋₁₀-alkyle, C₂₋₁₀-alcényle ou C₂₋₁₀-alcynyle à chaque fois linéaire ou ramifié, le cas échéant substitué ;
- représente un radical cycloaliphatique insaturé ou saturé, le cas échéant substitué, de 3, 4, 5, 6, 7, 8 ou 9 chaînons ;
- représente un radical aryle ou hétéroaryle de 5 à 14 chaînons, le cas échéant substitué ;
- représente -(CHR¹⁵)-A_{q}-(CH₂)ᵣ-Bₛ-(CH₂)ₜ-R¹⁶ avec q = 0 ou 1, r = 0 ou 1, s = 0 ou 1 et t = 0 ou 1, où A et B représentent indépendamment l'un de l'autre, à chaque fois O, S, NH, N(CH₃) ou N(C₂H₅) ;
- représente un radical -C(=O)-OR⁸ ;
- ou représente un radical -(CHR¹⁷)-(CH₂)ᵥ-C(=O)-OR⁹, où v vaut 0, 1, 2 ou 3 ;
R⁶ représente un radical cycloaliphatique insaturé ou saturé, le cas échéant substitué, de 3, 4, 5, 6, 7, 8 ou 9 chaînons ;
- représente un radical aryle ou hétéroaryle de 5 à 14 chaînons, le cas échéant substitué ;
- ou représente - (CH₂) -D_{w}- (CH₂)ₓ-E_{y}- (CH₂)_{z}-R¹⁸ avec w = 0 ou 1, x = 0 ou 1, y = 0 ou 1 et z = 0 ou 1, où D et E représentent, indépendamment l'un de l'autre, à chaque fois 0, S, NH, N(CH₃) ou N(C₂H₅)
R⁷, R⁸, R⁹, R¹⁰ et R¹¹ indépendamment l'un de l'autre, à chaque fois
- représentent un radical C₁₋₁₀-alkyle, C₂₋₁₀-alcényle ou C₂₋₁₀-alcynyle à chaque fois linéaire ou ramifié, le cas échéant substitué ;
R¹² et R¹³ indépendamment l'un de l'autre, à chaque fois
- représentent un radical C₁₋₁₀-alkyle, C₂₋₁₀-alcényle ou C₂₋₁₀-alcynyle à chaque fois linéaire ou ramifié, le cas échéant substitué ;
- ou représentent un radical aryle ou hétéroaryle de 5 à 14 chaînons, le cas échéant substitué ;
R¹⁴ représente un radical C₁₋₁₀-alkyle, C₂₋₁₀-alcényle ou C₂₋₁₀-alcynyle à chaque fois linéaire ou ramifié, le cas échéant substitué ;
- représente un radical cycloaliphatique insaturé ou saturé, le cas échéant substitué, de 3, 4, 5, 6, 7, 8 ou 9 chaînons, qui peut être ponté par un ou deux groupes C₁₋₅-alkylène linéaires ou ramifiés, le cas échéant substitués,
- ou représente un radical aryle ou hétéroaryle de 5 à 14 chaînons, le cas échéant substitué, qui peut être condensé par un système monocyclique saturé ou insaturé, le cas échéant substitué ;
R¹⁵ représente un radical hydrogène ;
- représente un radical C₁₋₁₀-alkyle, C₂₋₁₀-alcényle ou C₂₋₁₀-alcynyle à chaque fois linéaire ou ramifié, le cas échéant substitué ;
R¹⁶ représente un radical C₁₋₁₀-alkyle, C₂₋₁₀-alcényle ou C₂₋₁₀-alcynyle à chaque fois linéaire ou ramifié, le cas échéant substitué ;
- représente un radical cycloaliphatique insaturé ou saturé, le cas échéant substitué, de 3, 4, 5, 6, 7, 8 ou 9 chaînons ;
- ou représente un radical aryle ou hétéroaryle de 5 à 14 chaînons, le cas échéant substitué ;
R¹⁷ représente un radical C₁₋₁₀-alkyle, C₂₋₁₀-alcényle ou C₂₋₁₀-alcynyle à chaque fois linéaire ou ramifié, le cas échéant substitué ;
et
R¹⁸ représente un radical cycloaliphatique insaturé ou saturé, le cas échéant substitué, de 3, 4, 5, 6, 7, 8 ou 9 chaînons ;
- ou représente un radical aryle ou hétéroaryle de 5 à 14 chaînons, le cas échéant substitué ;
où
- les radicaux C₁₋₁₀-alkyle susmentionnés peuvent être non substitués ou à chaque fois substitués par 1, 2, 3, 4 ou 5 substituants, choisis indépendamment l'un de l'autre dans le groupe constitué par F, Cl, Br, I, -CN, -NO₂, -OH, -SH, -NH₂ et -N(C₁₋₅-alkyl) (C₁₋₅-alkyle) ;
- les radicaux C₂₋₁₀-alcényle susmentionnées peuvent être non substitués ou à chaque fois substitués par 1, 2, 3, 4 ou 5 substituants, choisis indépendamment l'un de l'autre dans le groupe constitué par F, Cl, Br, I, -CN, -NO₂, -OH, -SH,
- NH₂ et -N(C₁₋₅-alkyl) (C₁₋₅-alkyle) ;
- les radicaux C₂₋₁₀-alcynyle susmentionnés peuvent être non substitués ou à chaque fois substitués par 1, 2, 3, 4 ou 5 substituants, choisis indépendamment l'un de l'autre dans le groupe constitué par F, Cl, Br, I, -CN, -NO₂, -OH, -SH, -NH₂ et -N(C₁₋₅-alkyl) (C₁₋₅-alkyle) ;
- les radicaux C₁₋₅-alkylène susmentionnés peuvent être non substitués ou à chaque fois substitués par 1, 2, 3, 4 ou 5 substituants, choisis indépendamment l'un de l'autre dans le groupe constitué par F, Cl, Br, -OH, -SH, -NH₂, -CN, -NO₂ et phényle ;
- les radicaux cycloaliphatiques susmentionnés peuvent être non substitués ou à chaque fois substitués- par 1, 2, 3, 4 ou 5 substituants choisis indépendamment l'un de l'autre dans le groupe constitué par oxo (=O), thioxo (=S), F, Cl, Br, I, -CN, -CF₃, -SF₅, -OH, -O-C₁₋₅-alkyle, -NH₂, -NO₂, -O-CF₃, -S-CF₃, -SH, -S-C₁₋₅-alkyle, -C₁₋₅-alkyle, -C(=O)-H, -C(=O)-C₁₋₅-alkyle, -C(=O)-C₁₋₅-perfluoroalkyle, -C(=O)-OH, -C(=O)-O-C₁₋₅-alkyle, -(CH₂)-C(=O)-OH, -(CH₂)-C(=O)-O-C₁₋₅-alkyle, -NH-C₁₋₅-alkyle, -N(C₁₋₅-alkyle)₂, -(CH₂)-benzo[b]furannyle, -O-phényle, -O-benzyle, phényle, benzyle, naphtyle et -(CH₂)-naphtyle, où la partie cyclique des radicaux -O-phényle, -O-benzyle, phényle, -(CH₂)-benzo[b]furannyle, benzyle, naphtyle et -(CH₂)-naphtyle peut à chaque fois être substituée par 1, 2, 3, 4 ou 5 substituants choisis indépendamment l'un de l'autre dans le groupe constitué par F, Cl, Br, -OH, -CF₃, -SF₅, -CN, -NO₂, -O-C₁₋₅-alkyle, -C₁₋₅-alkyle, -O-CF₃, -S-CF₃, phényle et -O-benzyle,
- et les radicaux cycloaliphatiques susmentionnés peuvent à chaque fois présenter 1, 2, 3, 4 ou 5 hétéroatome(s) choisi(s) indépendamment l'un de l'autre dans le groupe constitué par oxygène, azote et soufre ;
- le cycle des systèmes monocycliques susmentionnés peut être non substitué ou être substitué par 1, 2, 3, 4 ou 5 substituants choisis indépendamment l'un de l'autre dans le groupe constitué par oxo (=O) , thioxo (=S), F, Cl, Br, I, -CN, -CF₃, -SF₅, -OH, -O-C₁₋₅-alkyle, -O-C₂₋₅-alcényle, -NH₂, -NO₂, -O-CF₃, -O-CHF₂, -O-CH₂F, -S-CF₃, -S-CHF₂, -S-CH₂F, -SH, -S-C₁₋₅-alkyle, -C₁₋₅-alkyle, -C(=O)-OH, -C(=O)-O-C₁₋₅-alkyle, -O-C(=O)-C₁₋₅-alkyle, -OC(=O)-phényle, - (CH₂)-O-C(=O)-C₁₋₅-alkyle, -(CH₂)-OC (=O) -phényle, -NH-C₁₋₅-alkyle, -N (C₁₋₅-alkyle)₂, -NH-C(=O)-O-C₁₋₅-alkyle, -NH-C(=O)-C₁₋₅-alkyle, -C(=O)-H, -C(=O) -C₁₋₅-alkyle, -C (=O) -C₁₋₅-perfluoroalkyle, -C(=O)-NH₂, -C(=O)-NH-C₁₋₅-alkyle, C (=O) -N- (C₁₋₅-alkyle)₂, -S (=O)₂-NH₂, -S (=O)₂-NH-C₁₋₅-alkyle, -S(=O)₂-NH-phényle, -S(=O)₂-C₁₋₅-alkyle, -(CH₂)-benzo[b]furannyle, -O-phényle, -S-phényle, -S-benzyle, -O-benzyle, phényle et benzyle, où la partie cyclique des radicaux -O-C(=O)-phényle, -(CH₂) -O-C (=O) -phényle, -S (=O)₂-NH-phényle, -O-phényle, -S-phényle, -8-benzyle, -O-benzyle, phényle, -(CH₂)-benzo[b]furannyle et benzyle peut à chaque fois être substituée par 1, 2, 3, 4 ou 5 substituants choisis indépendamment l'un de l'autre dans le groupe constitué par F, Cl, Br, -OH, -CF₃, -SF₅, -CN, -NO₂, -O-C₁₋₅-alkyle, -C₁₋₅-alkyle, -O-CF₃, -S-CF₃, phényle et -O-benzyle,
- et le cycle des systèmes monocycliques susmentionnés peut à chaque fois présenter 5, 6 ou 7 chaînons et à chaque fois 1, 2, 3, 4 ou 5 hétéroatome(s) comme élément(s) de cycle, qui sont choisis, indépendamment l'un de l'autre, dans le groupe constitué par oxygène, azote et soufre ;
- et les radicaux aryle ou hétéroaryle susmentionnés peuvent être non substitués ou à chaque fois substitués par 1, 2, 3, 4 ou 5 substituants choisis indépendamment l'un de l'autre dans le groupe constitué par F, Cl, Br, I, -CN, -CF₃, -SF₅, -OH, -O-C₁₋₅-alkyle, -O-C₂₋₅-alcényle, -NH₂, -NO₂, -O-CF₃, -O-CHF₂, -O-CH₂F, -S-CF₃, -S-CHF₂, -S-CH₂F, -SH, -S-C₁₋₅-alkyle, -C₁₋₅-alkyle, -C(=O)-OH, -C (=O) -O-C₁₋₅-alkyle, -O-C(=O)-C₁₋₅-alkyle, -OC(=O)-phényle, -(CH₂)-O-C(=O)-C₁₋₅-alkyle, -(CH₂)-OC(=O)-phényle, -NH-C₁₋₅-alkyle, -N(C₁₋₅-alkyle)₂, -NH-C(=O)-O-C₁₋₅-alkyle, -NH-C(=O)-C₁₋₅-alkyle, -C(=O)-H, -C (=O) -C₁₋₅-alkyle, -C (=O) -C₁₋₅-perfluoroalkyle, -C(=O)-NH₂, -C(=O)-NH-C₁₋₅-alkyle, C (=O) -N- (C₁₋₅-alkyle)₂, -S (=O)₂-NH₂, -S (=O)₂-NH-C₁₋₅-alkyle, -S(=O)₂-NH-phényle, -S(=O)₂-C₁₋₅-alkyle, -(CH₂)-benzo[b]furannyle, -0-phényle, -S-phényle, -S-benzyle, -0-benzyle, phényle et benzyle, où la partie cyclique des radicaux -O-C(=O)-phényle, - (CH₂)-O-C(=O)-phényle, -S(=O)₂-NH-phényle, -O-phényle, -S-phényle, -S-benzyle, -0-benzyle, phényle, -(CH₂)-benzo[b]furannyle et benzyle peut à chaque fois être substituée par 1, 2, 3, 4 ou 5 substituants choisis indépendamment l'un de l'autre dans le groupe constitué par F, Cl, Br, -OH, -CF₃, -SF₅, -CN, -NO₂, -O-C₁₋₅-alkyle, -C₁₋₅-alkyle, -O-CF₃, -S-CF₃, phényle et -O-benzyle,
- et les radicaux hétéroaryle susmentionnés peuvent à chaque fois présenter 1, 2, 3, 4 ou 5 hétéroatome(s) comme élément(s) de cycle, qui est/sont choisi(s) indépendamment l'un de l'autre dans le groupe constitué par oxygène, azote et soufre ;
le cas échéant à chaque fois sous forme d'un de leurs stéréo-isomères purs, en particulier leurs énantiomères ou diastéréo-isomères, leurs racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

10. Composés selon la revendication 9, **caractérisés en ce que**
R¹ représente un radical hydrogène ;
- représente un radical choisi dans le groupe constitué par méthyle, éthyle, n-propyle, isopropyle, n-butyle, sec-butyle, isobutyle, tert-butyle, n-pentyle, 2-pentyle, 3-pentyle, -(CH₂)-(C(CH₃)₃), n-hexyle, 3-hexyle, - (CH₂)-(CH₂)-(C(CH₃)₃), n-heptyle, 3-heptyle, 4-heptyle, n-octyle, -(CH₂)-(CH) (C₂H₅) - (CH₂) - (CH₂) - (CH₂) - (CH₃), vinyle, 1-propényle, 2-propényle, 1-butényle, 2-butényle, 3-butényle, 1-propynyle, 2-propynyle, 1-butynyle, 2-butynyle et 3-butynyle, où le radical peut être substitué à chaque fois par 1, 2, 3, 4 ou 5 substituants choisis indépendamment l'un de l'autre dans le groupe constitué par F, Cl, Br, I, -CN, -NO₂, -OH, -SH, -NH₂, -N(CH₃)₂, -N(C₂H₅)₂ et -N(CH₃) (C₂H₅) ;
- représente un radical choisi dans le groupe constitué par phényle, naphtyle, thiophényle, furannyle, pyrrolyle, pyrazolyle, pyrazinyle, triazolyle, pyridinyle, imidazolyle, indolyle, iso-indolyle, benzo[b]furannyle, benzo[b]thiophényle, thiazolyle, thiadiazolyle, oxazolyle, isoxazolyl, pyridazinyle, pyrazinyle, pyrimidinyle, indazolyle, quinazolinyle, quinoléinyle, isoquinoléinyle, phénazinyle, phénothiazinyle, oxadiazolyle, benzo[1,2,5]oxadiazolyle et chromanyle, où le radical peut être substitué à chaque fois par 1, 2, 3, 4 ou 5 substituants choisis indépendamment l'un de l'autre dans le groupe constitué par F, Cl, Br, I, -CN, -CF₃, -SF₅, -OH, -O-CH₃, -O-C₂H₅, -O-CH(CH₃)₂, -O-C(CH₃)₃, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, -S-CH (CH₃)₂, -S-C(CH₃)₃, -S-CH₂-CH₂-CH₂-CH₃, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C (=O)-phényle, - (CH₂) -O-C (=O)-CH₃, -(CH₂)-O-C(=O)-C₂H₅, -(CH₂)-O-C(=O)-phényle, -N(CH₃)₂, -N(C₂H₅)₂, -N (H) (CH₃), -N (H) (C₂H₅), -NH-C(=O)-O-CH₃, -NH-C (=O) -O-C₂H₅, -NH-C (=O) -CH₃, -NH-C(=O)-C₂H₅, -C(=O)-H, -C(=O)-CH₃, -C(=O)-C₂H₅, -C(=O)-CF₃, -C(=O)-NH₂, -C(=O)-NH-CH₃, -C(=O)-NH-C₂H₅, -S (=O)₂-NH₂, -S(=O)₂-NH-CH₃, -S(=O)₂-NH-C₂H₅, -S (=O)₂-NH-phényle, -S (=O)₂-CH₃, -S (=O)₂-C₂H₅, -(CH₂)-benzo[b]furannyle, -O-phényle, -S-phényle, -S-benzyle, -O-benzyle, phényle et benzyle, où la partie cyclique des radicaux -O-C(=O)-phényle, - (CH₂) -O-C (=O) -phényle, -S (=O)₂-NH-phényle, -O-phényle, -S-phényle, -S-benzyle, -O-benzyle, phényle, -(CH₂) benzo[b] furannyle et benzyle peut être substituée à chaque fois par 1, 2, 3, 4 ou 5 substituants choisis indépendamment l'un de l'autre dans le groupe constitué par F, Cl, Br, -OH, -CF₃, -SF₅, -CN, -NO₂, -O-CH₃, -O-C₂H₅, -0-CH(CH₃)₂, -O-C(CH₃)₃, méthyle, éthyle, n-propyle, isopropyle, n-butyle, sec-butyle, isobutyle, tert-butyle, -O-CF₃, -S-CF₃, phényle et -0-benzyle,
- représente -(CH₂)ₐₐ-C(=O)-O-R⁷ avec aa valant 1, 2, 3, 4 ou 5 ;
- ou représente - (CH₂)-R¹², -(CH₂)-(CH₂)-R¹², -(CH₂)-OR¹², -(CH₂)-S-R¹², (CH₂)-N (CH₃)-R¹², -(CH₂)-(CH₂)-(CH₂)-R¹², -(CH₂)-(CH₂)-O-R¹², -(CH₂)-(CH₂) S-R¹², -(CH₂)-(CH₂)-NH-R¹²_{,} -(CH₂)-(CH₂)-N(CH₃)-R¹²_{,} -(CH₂)-(CH₂)-O-(CH₂)-R¹², -(CH₂)-(CH₂)-S-(CH₂)-R¹² ou -(CH₂)-(CH₂)-N(CH₃)-R¹² ;
R² représente un radical hydrogène ;
- représente un radical choisi dans le groupe constitué par méthyle, éthyle, n-propyle, isopropyle, n-butyle, sec-butyle, isobutyle, tert-butyle, n-pentyle, 2-pentyle, 3-pentyle, -(CH₂)-(C(CH₃)₃), n-hexyle, 3-hexyle, -(CH₂)-(CH₂)-(C(CH₃)₃), n-heptyle, 3-heptyle, 4-heptyle, n-octyle, -(CH₂)-(CH) (C₂H₅) - (CH₂) - (CH₂) - (CH₂) - (CH₃), vinyle, 1-propényle, 2-propényle, 1-butényle, 2-butényle, 3-butényle, 1-propynyle, 2-propynyle, 1-butynyle, 2-butynyle et 3-butynyle, où le radical peut être substitué à chaque fois par 1, 2, 3, 4 ou 5 substituants choisis indépendamment l'un de l'autre dans le groupe constitué par F, Cl, Br, I, -CN, -NO₂, -OH, -SH, -NH₂, -N(CH₃)₂, -N(C₂H₅)₂ et -N (CH₃) (C₂H₅) ;
- représente un radical choisi dans le groupe constitué par phényle, naphtyle, thiophényle, furannyle, pyrrolyle, pyrazolyle, pyrazinyle, triazolyle, pyridinyle, imidazolyle, indolyle, iso-indolyle, benzo[b]furannyle, benzo[b]thiophényle, thiazolyle, thiadiazolyle, oxazolyle, isoxazolyle, pyridazinyle, pyrazinyle, pyrimidinyle, indazolyle, quinazolinyle, quinoléinyle, isoquinoléinyle, phénazinyle, phénothiazinyle, oxadiazolyle, benzo[1,2,5]oxadiazolyle et chromanyle, où le radical peut être substitué à chaque fois par 1, 2, 3, 4 ou 5 substituants choisis indépendamment l'un de l'autre dans le groupe constitué par F, Cl, Br, I, -CN, -CF₃, -SF₅, -OH, -O-CH₃, -O-C₂H₅, -O-CH(CH₃)₂, -O-C(CH₃)₃, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, -S-CH(CH₃)₂, -S-C (CH₃)₃, -S-CH₂-CH₂-CH₂-CH₃, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-phényle, -(CH₂)-O-C(=O)-CH₃, -(CH₂)-O-C(=O)-C₂H₅, -(CH₂)-O-C(=O)-phényle, -N(CH₃)₂, -N(C₂H₅)₂, -N(H) (CH₃), -N (H) (C₂H₅), -NH-C(=O)-O-CH₃, -NH-C(=O)-O-C₂H₅, -NH-C(=O)-CH₃, -NH-C(=O)-C₂H₅, -C(=O)-H, -C(=O)-CH₃, -C(=O)-C₂H₅, -C(=O)-CF₃, -C(=O)-NH₂, -C(=O)-NH-CH₃, -C(=O)-NH-C₂H₅, -S (=O)₂-NH₂, -S (=O)₂-NH-CH₃, -S (=O)₂-NH-C₂H₅, -S(=O)₂-NH-phényle, -S (=O)₂-CH₃, -S(=O)₂-C₂H₅, -(CH₂)-benzo[b]furannyle, -0-phényle, -S-phényle, -S-benzyle, -0-benzyle, phényle et benzyl, où la partie cyclique des radicaux -O-C(=O)-phényle, - (CH₂) -O-C (=O) -phényle, -S (=O)₂-NH-phényle, -O-phényle, -S-phényle, -S-benzyle, -O-benzyle, phényle, -(CH₂)-benzo[b]furannyle et benzyle peut être substituée à chaque fois par 1, 2, 3, 4 ou 5 substituants choisis indépendamment l'un de l'autre dans le groupe constitué par F, Cl, Br, -OH, -CF₃, -SF₅, -CN, -NO₂, -O-CH₃, -O-C₂H₅, -O-CH(CH₃)₂, -O-C(CH₃)₃, méthyle, éthyle, n-propyle, isopropyle, n-butyle, sec-butyle, isobutyle, tert-butyle, -O-CF₃, -S-CF₃, phényle et -O-benzyle,
- ou représente -(CH₂)-R¹³, -(CH₂)-(CH₂)-R¹³, -(CH₂)-OR¹³, -(CH₂)-S-R¹³, - (CH₂) -N (CH₃) -R¹³, -(CH₂) - (CH₂) - (CH₂)-R¹³, -(CH₂)-(CH₂)-O-R¹³, -(CH₂)-(CH₂)-S-R¹³, -(CH₂)-(CH₂)-NH-R¹³, -(CH₂)-(CH₂)-N(CH₃)-R¹³, -(CH₂)-(CH₂) -O-(CH₂) -R¹³, -(CH₂)-(CH₂)-S-(CH₂)-R¹³ ou -(CH₂)-(CH₂)-N(CH₃)-R¹³ _{;}
R³ représente un groupe -S(=O)₂-R⁴, un groupe -C(=S)-NH-R⁵ ou un groupe -C(=O)-NH-R⁶,
R⁴ représente un radical -NR¹⁰R¹¹;
- représente un radical choisi dans le groupe constitué par méthyle, éthyle, n-propyle, isopropyle, n-butyle, sec-butyle, isobutyle, tert-butyle, n-pentyle, 2-pentyle, 3-pentyle, -(CH₂)-(C(CH₃)₃), n-hexyle, 3-hexyle, -(CH₂)-(CH₂)-(C(CH₃)₃), n-heptyle, 3-heptyle, 4-heptyle, n-octyle, -(CH₂)-(CH) (C₂H₅) - (CH₂)- (CH₂) - (CH₂) - (CH₃), vinyle, 1-propényle, 2-propényle, 1-butényle, 2-butényle, 3-butényle, 1-propynyle, 2-propynyle, 1-butynyle, 2-butynyle et 3-butynyle, où le radical peut être substitué à chaque fois par 1, 2, 3, 4 ou 5 substituants choisis indépendamment l'un de l'autre dans le groupe constitué par F, Cl, Br, I, -CN, -NO₂, -OH, -SH, -NH₂, -N(CH₃)₂, -N(C₂H₅)₂ et -N(CH₃) (C₂H₅) ;
- représente un radical (hétéro)cycloaliphatique choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, pipérazinyle, thiomorpholinyle, tétrahydropyrannyle, azépanyle, diazépanyle, dithiolanyle, adamantyle et 7,7-diméthylbicyclo[2,2,1]heptyle, où le radical (hétéro)cycloaliphatique peut à chaque fois être substitué par 1, 2, 3, 4 ou 5 substituants choisis indépendamment l'un de l'autre dans le groupe constitué par oxo (=O), thioxo (=S), F, Cl, Br, I, -CN, -CF₃, -SF₅, -OH, -O-CH₃, -O-C₂H₅, -O-C(CH₃)₃, -NH₂, -NO₂, -O-CF₃, -S-CF₃, -SH, S-CH₃, =S-C₂H₅, -S-C(CH₃)₃, méthyle, éthyle, n-propyle, isopropyle, n-butyle, sec-butyle, isobutyle, tert-butyle, -C(=O)-H, -C(=O)-CH₃, -C(=O)-C₂H₅, -C(=O)-OH, -C(=O)-O-CH₃, -C(=O)-O-C₂H₅, -C (=O) -O-C (CH₃)₃, -(CH₂)-C(=O)-OH, -(CH₂)-C(=O)-O-CH₃, - (CH₂)-C(=O)-O-C₂H₅, -N(CH₃)₂, -N (C₂H₅)₂, -N (H) (CH₃), -N (H) (C₂H₅) -(CH₂)-benzo[b]furannyle, -0-phényle, -O-benzyle, phényle, benzyle, naphtyle et -(CH₂)-naphtyle, la partie cyclique des radicaux -O-phényle, -0-benzyle, phényle, -(CH₂)-benzo[b]furannyle, benzyle, naphtyle et -(CH₂)-naphtyle pouvant être substituée à chaque fois par 1, 2, 3, 4 ou 5 substituants choisis indépendamment l'un de l'autre dans le groupe constitué par F, Cl, Br, -OH, -CF₃, -SF₅, -CN, -NO₂, -O-CH₃, -O-C₂H₅, méthyle, éthyle, n-propyle, isopropyle, n-butyle, sec-butyle, isobutyle, tert-butyle, -O-CF₃, -S-CF₃, phényle et -0-benzyle,
- représente un radical choisi dans le groupe constitué par phényle, naphtyle, thiophényle, furannyle, pyrrolyle, pyrazolyle, pyrazinyle, triazolyle, pyridinyle, imidazolyle, indolyle, iso-indolyle, benzo[b]furannyle, benzo[b]thiophényle, thiazolyle, thiadiazolyle, oxazolyle, isoxazolyle, pyridazinyle, pyrazinyle, pyrimidinyle, indazolyle, quinazolinyle, quinoléinyle, isoquinoléinyle, phénazinyle, phénothiazinyle, oxadiazolyle, benzo[1,2,5]oxadiazolyle, chromanyle et (1,2,3,4)-tétrahydroquinoléinyle, où le radical peut à chaque fois être substitué par 1, 2, 3, 4 ou 5 substituants choisis indépendamment l'un de l'autre dans le groupe constitué par F, Cl, Br, I, -CN, -CF₃, -SF₅, -OH, -O-CH₃, -O-C₂H₅, -O-CH(CH₃)₂, -O-C(CH₃)₃, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, -S-CH(CH₃)₂, -S-C(CH₃)₃, -S-CH₂-CH₂-CH₂-CH₃, méthyle, éthyle, n-propyle, isopropyle, n-butyle, sec-butyle, isobutyle, tert-butyle, (1,1)-diméthylpropyle, -C(=O)-OH, -C(=O)-O-CH3, -C(=O)-O-C₂H₅, -C(=O)-O-C(CH₃)₃, -O-C(=O)-CH₃, -O-C(=O)-C₂H₅, -O-C (=O)-phényle, -(CH₂)-OC(=O)-CH₃, -(CH2)-O-C(=O)-C₂H₅, -(CH₂)-O-C(=O)-phényle, -N(CH₃)₂, -N(C₂H₅)₂, -N(H)(CH₃), -N(H)(C₂H₅), -NH-C (=O) -O-CH₃, -NH-C (=O) -O-C₂H₅, -NH-C(=O)-CH₃, -NH-C (=O) -C₂H₅, -C(=O)-H, -C(=O)-CH₃, -C(=O)-C₂H₅, -C(=O)-CF₃, -C(=O)-NH₂, -C(=O)-NH-CH₃, -C(=O)-NH-C₂H₅, -S(=O)₂-NH₂, -S(=O)₂-NH-CH₃, -S(=O)₂-NH-C₂H₅, -S (=O)₂-NH-phényle, -S(=O)₂-CH₃, -S(=O)₂-C₂H₅, -(CH₂)-benzo[b]furannyle, -O-phényle, -S-phényle, -S-benzyle, -O-benzyle, phényle et benzyle, la partie cyclique des radicaux -O-C(=O)-phényle, -(CH₂)-O-C(=O)-phényle, -S(=O)₂-NH-phényle, -O-phényle, -S-phényle, -S-benzyle, -O-benzyle, phényle, -(CH₂)-benzo[b]furannyle et benzyle pouvant à chaque fois être substituée par 1, 2, 3, 4 ou 5 substituants choisis indépendamment l'un de l'autre dans le groupe constitué par F, Cl, Br, -OH, -CF₃, -SF₅, -CN, -NO₂, -O-CH₃, -O-C₂H₅, -O-CH(CH₃)₂, -O-C(CH₃)₃, méthyle, éthyle, n-propyle, isopropyle, n-butyle, sec-butyle, isobutyle, tert-butyle, -O-CF₃, -S-CF₃, phényle et -O-benzyle,
- ou représente -(CH₂)-R¹⁴, -(CH₂)-(CH₂)-R¹⁴, -(CH₂)-OR¹⁴, -(CH₂)-S-R¹⁴, -(CH₂)-N(CH₃) -R¹⁴, -(CH₂)-(CH₂)-(CH₂)-R¹⁴, -(CH₂)-(CH₂)-O-R¹⁴, -(CH₂)-(CH₂)-S-R¹⁴, -(CH₂)-(CH₂)-NH-R¹⁴, -(CH₂)-(CH₂)-N(CH₃)-R¹⁴, -(CH₂)-(CH₂)-O-(CH₂)-R¹⁴, -(CH₂)-(CH₂)-S-(CH₂)-R¹⁴ ou -(CH₂)-(CH₂) -N(CH₃)-R¹⁴ ;
R⁵ représente un radical choisi dans le groupe constitué par méthyle, éthyle, n-propyle, isopropyle, n-butyle, sec-butyle, isobutyle, tert-butyle, n-pentyle, 2-pentyle, 3-pentyle, -(CH₂)-(C(CH₃)₃), n-hexyle, 3-hexyle, -(CH₂)-(CH₂)-(C(CH₃)₃), n-heptyle, 3-heptyle, 4-heptyle, n-octyle, -(CH₂)-(CH) (C₂H₅)-(CH₂)-(CH₂)-(CH₂)-(CH₃), vinyle, 1-propényle, 2-propényle, 1-butényle, 2-butényle, 3-butényle, 1-propynyle, 2-propynyle, 1-butynyle, 2-butynyle et 3-butynyle, où le radical peut être substitué à chaque fois par 1, 2, 3, 4 ou 5 substituants choisis indépendamment l'un de l'autre dans le groupe constitué par F, Cl, Br, I, -CN, -NO₂, -OH, -SH, -NH₂, -N(CH₃)₂, -N(C₂H₅)₂ et -N(CH₃)(C₂H₅) ;
- représente un radical (hétéro)cycloaliphatique choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, pipérazinyle, thiomorpholinyle, tétrahydropyrannyle, azépanyle, diazépanyle et dithiolanyle, où le radical (hétéro)cycloaliphatique peut à chaque fois être substitué par 1, 2, 3, 4 ou 5 substituants choisis indépendamment l'un de l'autre dans le groupe constitué par oxo (=O), thioxo (=S), F, Cl, Br, I, -CN, -CF₃, -SF₅, -OH, -O-CH₃, -O-C₂H₅, -O-C(CH₃)₃, -NH₂, -NO₂, -O-CF₃, -S-CF₃, -SH, S-CH₃, -S-C₂H₅, -S-C(CH₃)₃, méthyle, éthyle, n-propyle, isopropyle, n-butyle, sec-butyle, isobutyle, tert-butyle, -C(=O)-H, -C(=O)-CH₃, -C (=O) -C₂H₅, -C(=O)-OH, -C(=O)-O-CH₃, -C(=O)-O-C₂H₅, -C (=O) -O-C (CH₃)₃, -(CH₂)-C(=O)-OH, -(CH₂)-C(=O)-O-CH₃, -(CH₂)-C(=O)-O-C₂H₅, -N(CH₃)₂, -N(C₂H₅)₂, -N (H) (CH₃), -N (H) (C₂H₅), -(CH₂)-benzo[b]furannyle, -O-phényle, -0-benzyle, phényle, benzyle, naphtyle et -(CH₂)-naphtyle, la partie cyclique des radicaux -O-phényle, -O-benzyle, phényle, -(CH₂)-benzo[b]furannyle, benzyle, naphtyle et -(CH₂)-naphtyle pouvant à chaque fois être substituée par 1, 2, 3, 4 ou 5 substituants choisis indépendamment l'un de l'autre dans le groupe constitué par F, Cl, Br, -OH, -CF₃, -SF₅, -CN, -NO₂, -O-CH₃, -O-C₂H₅, méthyle, éthyle, n-propyle, isopropyle, n-butyle, sec-butyle, isobutyle, tert-butyle, -O-CF₃, -S-CF₃, phényle et -0-benzyle,
- représente un radical choisi dans le groupe constitué par phényle, naphtyle, thiophényle, furannyle, pyrrolyle, pyrazolyle, pyrazinyle, triazolyle, pyridinyle, imidazolyle, indolyle, iso-indolyle, benzo[b]furannyle, benzo[b]thiophényle, thiazolyle, thiadiazolyle, oxazolyle, isoxazolyle, pyridazinyle, pyrazinyle, pyrimidinyle, indazolyle, quinazolinyle, quinoléinyle, isoquinoléinyle, phénazinyle, phénothiazinyle, oxadiazolyle, benzo[1,2,5]oxadiazolyle et chromanyle, où le radical peut à chaque fois être substitué par 1, 2, 3, 4 ou 5 substituants choisis indépendamment l'un de l'autre dans le groupe constitué par F, Cl, Br, I, -CN, -CF₃, -SF₅, -OH, -O-CH₃, -O-C₂H₅, -O-CH(CH₃)₂, -O-C(CH₃)₃, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, -S-CH(CH₃)₂, -S-C(CH₃)₃, -S-CH₂-CH₂-CH₂-CH₃, méthyle, éthyle, n-propyle, isopropyle, n-butyle, sec-butyle, isobutyle, tert-butyle, (1,1)-diméthylpropyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-phényle, -(CH₂)-O-C(=O)-CH₃, -(CH₂)-O-C(=O)-C₂H₅, - (CH₂) -O-C (=O) -phényle, -N (CH₃)₂, -N (C₂H₅)₂, -N (H) (CH₃), -N (H) (C₂H₅), -NH-C (=O) -O-CH₃, -NH-C (=O)-O-C₂H₅, -NH-C(=O)-CH₃, -NH-C(=O)-C₂H₅, -C(=O)-H, -C (=O) -CH₃, -C(=O)-C₂H₅, -C(=O)-CF₃, -C(=O)-NH₂, -C(=O)-NH-CH₃, -C(=O)-NH-C₂H₅, -S(=O)₂-NH₂, -S(=O)₂-NH-CH₃, -S (=O)₂-NH-C₂H₅, -S(=O)₂-NH-phényle, -S(=O)₂-CH₃, -S(=O)₂-C₂H₅, -(CH₂)-benzo[b]furannyle, -O-phényle, -S-phényle, -S-benzyle, -O-benzyle, phényle et benzyle, la partie cyclique des radicaux -O-C(=O)-phényle, -(CH₂)-O-C(=O) -phényle, -S(=O)₂-NH-phényle, -O-phényle, -S-phényle, -S-benzyle, -O-benzyle, phényle, -(CH₂)-benzo[b]furannyle et benzyle pouvant à chaque fois être substituée par 1, 2, 3, 4 ou 5 substituants choisis indépendamment l'un de l'autre dans le groupe constitué par F, Cl, Br, -OH, -CF₃, -SF₅, -CN, -NO₂, -O-CH₃, -O-C₂H₅, -O-CH(CH₃)₂, -O-C(CH₃)₃, méthyle, éthyle, n-propyle, isopropyle, n-butyle, sec-butyle, isobutyle, tert-butyle, -O-CF₃, -S-CF₃, phényle et -0-benzyle,
- représente -(CHR¹⁵)-R¹⁶, -(CHR¹⁵) -(CH₂)-R¹⁶, -(CHR¹⁵)-O-R¹⁶, -(CHR¹⁵)-S-R¹⁶, -(CHR¹⁵)-N(CH₃)-R¹⁶, -(CHR¹⁵)-(CH₂)-(CH₂)-R¹⁶, -(CHR¹⁵)-(CH₂)-O-R¹⁶, -(CHR¹⁵)-(CH₂)-S-R¹⁶, -(CHR¹⁵)-(CH₂)-NH-R¹⁶, -(CHR¹⁵)-(CH₂)-N(CH₃)-R¹⁶, -(CHR¹⁵)-(CH₂)-O-(CH₂)-R¹⁶, -(CHR¹⁵)- CH₂)-S-(CH₂)-R¹⁶ ou -(CHR¹⁵)-(CH₂)-N(CH₃)-R¹⁶ ;
- représente un radical -C(=O)-OR⁸ ;
- ou représente un radical -(CHR¹⁷)-C(=O)-OR⁹ ou un radical -(CHR¹⁶)-(CH₂)-C(=O)-OR⁹ ;
R⁶ représente un radical (hétéro)cycloaliphatique, choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, pipérazinyle, thiomorpholinyle, tétrahydropyrannyle, azépanyle, diazépanyle et dithiolanyle, où le radical (hétéro)cycloaliphatique peut à chaque fois être substitué par 1, 2, 3, 4 ou 5 substituants choisis indépendamment l'un de l'autre dans le groupe constitué par oxo (=O), thioxo (=S), F, Cl, Br, I, -CN, -CF₃, -SF₅, -OH, -O-CH₃, -O-C₂H₅, -O-C(CH₃)₃, -NH₂, -NO₂, -O-CF₃, -S-CF₃, -SH, S-CH₃, -S-C₂H₅, -S-C(CH₃)₃, méthyle, éthyle, n-propyle, isopropyle, n-butyle, sec-butyle, isobutyle, tert-butyle, -C(=O)-H, -C(=O)-CH₃, -C(=O)-C₂H₅, -C(=O)-OH, -C(=O)-O-CH₃, -C(=O)-O-C₂H₅, -C (=O) -O-C(CH₃)₃, -(CH₂)-C(=O)-OH, -(CH₂)-C(=O)-O-CH₃, -(CH₂)-C(=O)-OC₂H₅, -N(CH₃)₂, -N(C₂H₅)₂, -N (H) (CH₃), -N (H) (C₂H₅), -(CH₂)-benzo[b]furannyle, -O-phényle, -O-benzyle, phényle, benzyle, naphtyle et -(CH₂)-naphtyle, la partie cyclique des radicaux -O-phényle, -O-benzyle, phényle, -(CH₂)-benzo[b] furannyle, benzyle, naphtyle et -(CH₂)-naphtyle pouvant à chaque fois être substituée par 1, 2, 3, 4 ou 5 substituants choisis indépendamment l'un de l'autre dans le groupe constitué par F, Cl, Br, -OH, -CF₃, -SF₅, -CN, -NO₂, -O-CH₃, -O-C₂H₅, méthyle, éthyle, n-propyle, isopropyle, n-butyle, sec-butyle, isobutyle, tert-butyle, -O-CF₃, -S-CF₃, phényle et -0-benzyle,
- représente un radical choisi dans le groupe constitué par phényle, naphtyle, thiophényle, furannyle, pyrrolyle, pyrazolyle, pyrazinyle, triazolyle, pyridinyle, imidazolyle, indolyle, iso-indolyle, benzo[b]furannyle, benzo[b]thiophényle, thiazolyle, thiadiazolyle, oxazolyle, isoxazolyle, pyridazinyle, pyrazinyle, pyrimidinyle, indazolyle, quinazolinyle, quinoléinyle, isoquinoléinyle, phénazinyle, phénothiazinyle, oxadiazolyle, benzo[1,2,5]oxadiazolyle et chromanyle, où le radical peut être à chaque fois substitué par 1, 2, 3, 4 ou 5 substituants choisis indépendamment l'un de l'autre dans le groupe constitué par F, Cl, Br, I, -CN, -CF₃, -SF₅, -OH, -O-CH₃, -O-C₂H₅, -O-CH(CH₃)₂, -O-C(CH₃)₃, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, -S-CH(CH₃)₂, -S-C(CH₃)₃, -S-CH₂-CH₂-CH₂-CH₃, méthyle, éthyle, n-propyle, isopropyle, n-butyle, sec-butyle, isobutyle, tert-butyle, (1,1)-diméthylpropyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-phényle, -(CH₂)-O-C(=O)-CH₃, -(CH₂)-O-C(=O)-C₂H₅, - (CH₂) -O-C (=O) -phényle, -N (CH₃)₂, -N (C₂H₅)₂, -N (H) (CH₃), -N (H) (C₂H₅), -NH-C (=O) -O-CH₃, -NH-C (=O) - O-C₂H₅, -NH-C (=O)-CH₃, -NH-C(=O)-C₂H₅, -C(=O)-H, -C(=O)-CH₃, -C(=O)-C₂H₅, -C(=O)-CF₃, -C(=O)-NH₂, -C(=O)-NH-CH₃, -C(=O)-NH-C₂H₅, -S(=O)₂-NH₂, -S(=O)₂-NH-CH₃, -S (=O)₂-NH-C₂H₅, -S (=O)₂-NH-phényle, -S(=O)₂-CH₃, -S(=O)₂-C₂H₅, -(CH₂)-benzo[b]furannyle, -O-phényle, -S-phényle, -S-benzyle, -O-benzyle, phényle et benzyle, la partie cyclique des radicaux -O-C(=O)-phényle, -(CH₂)-O-C(=O)-phényle, -S(=O)₂-NH-phényle, -0-phényle, -S-phényle, -S-benzyle, -0-benzyle, phényle, -(CH₂)-benzo[b]furannyle et benzyle pouvant à chaque fois être substituée par 1, 2, 3, 4 ou 5 substituants choisis indépendamment l'un de l'autre dans le groupe constitué par F, Cl, Br, -OH, -CF₃, -SF₅, -CN, -NO₂, -O-CH₃, -O-C₂H₅, -O-CH (CH₃)₂, -O-C (CH₃)₃, méthyle, éthyle, n-propyle, isopropyle, n-butyle, sec-butyle, isobutyle, tert-butyle, -O-CF₃, -S-CF₃, phényle et -O-benzyle,
- ou représente -(CH₂)-R¹⁸, -(CH₂)-(CH₂)-R¹⁸, -(CH₂)-O-R¹⁸, -(CH₂)-S-R¹⁸, -(CH₂)-N(CH₃)-R¹⁸, -(CH₂)-(CH₂)-(CH₂)-R¹⁸, -(CH₂)-(CH₂)-O-R¹⁸, -(CH₂)-(CH₂)-S-R¹⁸, -(CH₂)-(CH₂)-NH-R¹⁸, -(CH₂)-(CH₂)-N(CH₃)-R¹⁸, -(CH₂)-(CH₂)-O-(CH₂)-R¹⁸, -(CH₂)-(CH₂)-S-(CH₂)-R¹⁸ ou -(CH₂)-(CH₂)-N(CH₃)-R¹⁸ ;
⁷, R⁸, R⁹, R¹⁰ et R¹¹ R indépendamment l'un de l'autre, à chaque fois
- représentent un radical choisi dans le groupe constitué par méthyle, éthyle, n-propyle, isopropyle, n-butyle, sec-butyle, isobutyle, tert-butyle, n-pentyle, 2-pentyle, 3-pentyle, -(CH₂)-(C(CH₃)₃), n-hexyle, 3-hexyle, -(CH₂)-(CH₂)-(C(CH₃)₃), n-heptyle, 3-heptyle, 4-heptyle, n-octyle, -(CH₂)-(CH) (C₂H₅)-(CH₂)-(CH₂)-(CH₂)-(CH₃), vinyle, 1-propényle, 2-propényle, 1-butényle, 2-butényle, 3-butényle, 1-propynyle, 2-propynyle, 1-butynyle, 2-butynyle et 3-butynyle, où le radical peut à chaque fois être substitué par 1, 2, 3, 4 ou 5 substituants choisis, indépendamment l'un de l'autre dans le groupe constitué par F, Cl, Br, I, -CN, -NO₂, -OH, -SH, -NH₂, -N(CH₃)₂, -N(C₂H₅)₂ et -N(CH₃)(C₂H₅) ;
R¹² et R¹³ indépendamment l'un de l'autre, à chaque fois
- représentent un radical choisi dans le groupe constitué par méthyle, éthyle, n-propyle, isopropyle, n-butyle, sec-butyle, isobutyle, tert-butyle, n-pentyle, 2-pentyle, 3-pentyle, -(CH₂)-(C(CH₃)₃), n-hexyle, 3-hexyle, -(CH₂)- CH₂)-(C(CH₃)₃), n-heptyle, 3-heptyle, 4-heptyle, n-octyle, -(CH₂)-(CH)(C₂H₅)-(CH₂)-(CH₂)-(CH₂)-(CH₃), vinyle, 1-propényle, 2-propényle, 1-butényle, 2-butényle, 3-butényle, 1-propynyle, 2-propynyle, 1-butynyle, 2-butynyle et 3-butynyle, où le radical peut à chaque fois être substitué par 1, 2, 3, 4 ou 5 substituants choisis indépendamment l'un de l'autre dans le groupe constitué par F, Cl, Br, I, -CN, -NO₂, -OH, -SH, -NH₂, -N(CH₃)₂, -N (C₂H₅)₂ et -N(CH₃)(C₂H₅) ;
- ou représentent un radical choisi dans le groupe constitué par phényle, naphtyle, thiophényle, furannyle, pyrrolyle, pyrazolyle, pyrazinyle, triazolyle, pyridinyle, imidazolyle, indolyle, iso-indolyle, benzo[b]furannyle, benzo[b]thiophényle, thiazolyle, thiadiazolyle, oxazolyle, isoxazolyle, pyridazinyle, pyrazinyle, pyrimidinyle, indazolyle, quinazolinyle, quinoléinyle, isoquinoléinyle, phénazinyle, phénothiazinyle, oxadiazolyle, benzo[1,2,5]oxadiazolyle et chromanyle, où le radical peut être substitué à chaque fois par 1, 2, 3, 4 ou 5 substituants choisis indépendamment l'un de l'autre dans le groupe constitué par F, Cl, Br, I, -CN, -CF₃, -SF₅, -OH, -O-CH₃, -O-C₂H₅, -O-CH(CH₃)₂, -O-C(CH₃)₃, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, -S-CH(CH₃)₂, -S-C(CH₃)₃, -S-CH₂-CH₂-CH₂-CH₃, méthyle, éthyle, n-propyle, isopropyle, n-butyle, sec-butyle, isobutyle, tert-butyle, (1,1)-diméthylpropyle, -C(=O)-OH, -C(=O)-O-CH₃, -C(=O)-O-C₂H₅, -C(=O)-OC(CH₃)₃, -O-C(=O)-CH₃, -O-C(=O)-C₂H₅, -O-C(=O)-phényle, -(CH₂)-O-C(=O)-CH₃, -(CH₂)-O-C(=O)-C₂H₅, -(CH₂)-O-C(=O)-phényle, -N(CH₃)₂, -N(C₂H₅)₂, -N(H)(CH₃), -N(H)(C₂H₅)_{,} -NH-C(=O)-O-CH₃, -NH-C(=O)-O-C₂H₅, -NH-C(=O)-CH₃, -NH-C(=O)-C₂H₅, -C(=O)-H, -C(=O)-CH₃, -C(=O)-C₂H₅, -C(=O)-CF₃, -C(=O)-NH₂, -C(=O)-NH-CH₃, -C(=O)-NH-C₂H₅, -S(=O)₂-NH₂, -S(=O)₂-NH-CH₃, -S(=O)₂-NH-C₂H₅, -S(=O)₂-NH-phényle, -S(=O)₂-CH₃, -S(=O)₂-C₂H₅, -(CH₂)-benzo[b]furannyle, -O-phényle, -S-phényle, -S-benzyle, -O-benzyle, phényle et benzyle, la partie cyclique des radicaux -O-C(=O)-phényle, -(CH₂)-O-C(=O)-phényle, -S(=O)₂-NH-phényle, -O-phényle, -S-phényle, -S-benzyle, -O-benzyle, phényle, -(CH₂)-benzo[b]furannyle et benzyle pouvant à chaque fois être substituée par 1, 2, 3, 4 ou 5 substituants choisis indépendamment l'un de l'autre dans le groupe constitué par F, Cl, Br, -OH, -CF₃, -SF₅, -CN, -NO₂, -O-CH₃, -O-C₂H₅, -O-CH(CH₃) ₂, -O-C(CH₃)₃, méthyle, éthyle, n-propyle, isopropyle, n-butyle, sec-butyle, isobutyle, tert-butyle, -O-CF₃, -S-CF₃, phényle et -O-benzyle,
R¹⁴ représente un radical choisi dans le groupe constitué par méthyle, éthyle, n-propyle, isopropyle, n-butyle, sec-butyle, isobutyle, tert-butyle, n-pentyle, 2-pentyle, 3-pentyle, -(CH₂)-(C(CH₃)₃), n-hexyle, . 3-hexyle, -(CH₂)-(CH₂)-(C(CH₃)₃), n-heptyle, 3-heptyle, 4-heptyle, n-octyle, -(CH₂)-(CH)(C₂H₅)-(CH₂)-(CH₂)-(CH₂)-(CH₃), vinyle, 1-propényle, 2-propényle, 1-butényle, 2-butényle, 3-butényle, 1-propynyle, 2-propynyle, 1-butynyle, 2-butynyle et 3-butynyle, où le radical peut à chaque fois être substitué par 1, 2, 3, 4 ou 5 substituants choisis indépendamment l'un de l'autre dans le groupe constitué par F, Cl, Br, I, -CN, -NO₂, -OH, -SH, -NH₂, -N(CH₃)₂,-N(C₂H₅)₂ et -N(CH₃)(C₂H₅) ;
- représente un radical (hétéro)cycloaliphatique choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, pipérazinyle, thiomorpholinyle, tétrahydropyrannyle, azépanyle, diazépanyle, dithiolanyle, adamantyle et 7,7-diméthylbicyclo[2,2,1]heptyle, où le radical (hétéro)cycloaliphatique peut à chaque fois être substitué par 1, 2, 3, 4 ou 5 substituants choisis indépendamment l'un de l'autre dans le groupe constitué par oxo (=O), thioxo (=S), F, Cl, Br, I, -CN, -CF₃, -SF₅, -OH, -O-CH₃, -O-C₂H₅, -O-C(CH₃)₃, -NH₂, -NO₂, -O-CF₃, -S-CF₃, -SH, S-CH₃, -S-C₂H₅, -S-C(CH₃)₃, méthyle, éthyle, n-propyle, isopropyle, n-butyle, sec-butyle, isobutyle, tert-butyle, -C(=O)-H, -C(=O)-CH₃, -C(=O)-C₂H₅, -C(=O)-OH, -C(=O)-O-CH₃, -C(=O)-O-C₂H₅, -C(=O)-O-C(CH₃)₃, -(CH₂)-C(=O)-OH, -(CH₂)-C(=O)-O-CH₃, -(CH₂)-C(=O)-O-C₂H₅, -N(CH₃)₂, -N(C₂H₅)₂, -N(H)(CH₃), -N(H)(C₂H₅), -(CH₂)-benzo[b]furannyle, -O-phényle, -O-benzyle, phényle, benzyle, naphtyle et -(CH₂)-naphtyle, la partie cyclique des radicaux -0-phényle, -0-benzyle, phényle, -(CH₂)-benzo[b]furannyle, benzyle, naphtyle et -(CH₂)-naphtyle pouvant à chaque fois être substituée par 1, 2, 3, 4 ou 5 substituants choisis indépendamment l'un de l'autre dans le groupe constitué par F, Cl, Br, -OH, -CF₃, -SF₅, -CN, -NO₂, -O-CH₃, -O-C₂H₅, méthyle, éthyle, n-propyle, isopropyle, n-butyle, sec-butyle, isobutyle, tert-butyle, -O-CF₃, -S-CF₃, phényle et -O-benzyle,
- ou représente un radical choisi dans le groupe constitué par phényle, naphtyle, thiophényle, furannyle, pyrrolyle, pyrazolyle, pyrazinyle, triazolyle, pyridinyle, imidazolyle, indolyle, iso-indolyle, benzo[b]furannyle, benzo[b]thiophényle, thiazolyle, thiadiazolyle, oxazolyle, isoxazolyle, pyridazinyle, pyrazinyle, pyrimidinyle, indazolyle, quinazolinyle, quinoléinyle, isoquinoléinyle, phénazinyle, phénothiazinyle, oxadiazolyle, benzo[1,2,5]oxadiazolyle, chromanyle et (1,2,3,4)-tétrahydroquinoléinyle, où le radical peut à chaque fois être substitué par 1, 2, 3, 4 ou 5 substituants choisis indépendamment l'un de l'autre dans le groupe constitué par F, Cl, Br, I, -CN, -CF₃, -SF₅, -OH, -O-CH₃, -O-C₂H₅, -O-CH(CH₃)₂, -O-C(CH₃)₃, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, -S-CH(CH₃)₂, -S-C(CH₃)₃, -S-CH₂-CH₂-CH₂-CH₃, méthyle, éthyle, n-propyle, isopropyle, n-butyle, sec-butyle, isobutyle, tert-butyle, (1,1)-diméthylpropyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-phényle, -(CH₂)-O-C(=O)-CH₃, -(CH₂)-O-C(=O)-C₂H₅, -(CH₂)-O-C(=O)-phényle, -N(CH₃)₂, -N(C₂H₅)₂,-N(H)(CH₃), -N(H)(C₂H₅), NH-C(=O)-O-CH₃, -NH-C(=O)-O-C₂H₅, -NH-C(=O)-CH₃, -NH-C(=O)-C₂H₅, -C(=O)-H, -C(=O)-CH₃, -C(=O)-C₂H₅, -C(=O)-CF₃, -C(=O)-NH₂, -C(=O)-NH-CH₃, -C(=O)-NH-C₂H₅, -S(=O)₂-NH₂, -S(=O)₂-NH-CH₃, -S(=O)₂-NH-C₂H₅, -S(=O)₂-NH-phényle, -S(=O)₂-CH₃, -S(=O)₂-C₂H₅, -(CH₂)-benzo[b]furannyle, -O-phényle, -S-phényle, -S-benzyle, -O-benzyle, phényle et benzyle, la partie cyclique des radicaux -O-C(=O)-phényle, -(CH₂)-O-C(=O)-phényle, -S(=O)₂-NH-phényle, -O-phényle, -S-phényle, -S-benzyle, -O-benzyle, phényle, -(CH₂)-benzo[b]furannyle et benzyle pouvant à chaque fois être substituée par 1, 2, 3, 4 ou 5 substituants choisis indépendamment l'un de l'autre dans le groupe constitué par F, Cl, Br, -OH, -CF₃, -SF₅, -CN, -NO₂, -O-CH₃, -O-C₂H₅, -O-CH(CH₃)₂, -O-C(CH₃)₃, méthyle, éthyle, n-propyle, isopropyle, n-butyle, sec-butyle, isobutyle, tert-butyle, -O-CF₃, -S-CF₃, phényle et -O-benzyle,
R¹⁵ représente un radical hydrogène ;
- ou représente un radical choisi dans le groupe constitué par méthyle, éthyle, n-propyle, isopropyle, n-butyle, sec-butyle, isobutyle, tert-butyle, n-pentyle, 2-pentyle, 3-pentyle, -(CH₂)-(C(CH₃)₃), n-hexyle, 3-hexyle, -(CH₂)-(CH₂)-(C(CH₃)₃), n-heptyle, 3-heptyle, 4-heptyle, n-octyle, -(CH₂)-(CH)(C₂H₅)-(CH₂)-(CH₂)-(CH₂)-(CH₃), vinyle, 1-propényle, 2-propényle, 1-butényle, 2-butényle, 3-butényle, 1-propynyle, 2-propynyle, 1-butynyle, 2-butynyle et 3-butynyle, où le radical peut à chaque fois être substitué par 1, 2, 3, 4 ou 5 substituants choisis indépendamment l'un de l'autre dans le groupe constitué par F, Cl, Br, I, -CN, -NO₂, -OH, -SH, -NH₂, -N(CH₃)₂, -N(C₂H₅)₂ et -N (CH₃) (C₂H₅) ;
R¹⁶ représente un radical choisi dans le groupe constitué par méthyle, éthyle, n-propyle, isopropyle, n-butyle, sec-butyle, isobutyle, tert-butyle, n-pentyle, 2-pentyle, 3-pentyle, -(CH₂)-(C(CH₃)₃), n-hexyle, 3-hexyle, -(CH₂)-(CH₂)-(C(CH₃)₃), n-heptyle, 3-heptyle, 4-heptyle, n-octyle, -(CH₂)-(CH)(C₂H₅)-(CH₂)-(CH₂)-(CH₂)-(CH₃), Vinyle, 1-propényle, 2-propényle, 1-butényle, 2-butényle, 3-butényle, 1-propynyle, 2-propynyle, 1-butynyle, 2-butynyle et 3-butynyle, où le radical peut à chaque fois être substitué par 1, 2, 3, 4 ou 5 substituants choisis indépendamment l'un de l'autre dans le groupe constitué par F, Cl, Br, I, -CN, -NO₂, -OH, -SH, -NH₂, -N(CH₃)₂, -N(C₂H₅)₂ et -N(CH₃)(C₂H₅) ;
- représente un radical (hétéro)cycloaliphatique choisi dans le groupe constitué par cyclopropyle, cyclobutyle, cyclopentyle, cyclohexyle, cycloheptyle, cyclopentényle, cyclohexényle, cycloheptényle, imidazolidihyle, tétrahydrofurannyle, tétrahydrothiophényle, pyrrolidinyle, pipéridinyle, morpholinyle, pipérazinyle, thiomorpholinyle, tétrahydropyrannyle, azépanyle, diazépanyle et dithiolanyle, où le radical (hétéro)cycloaliphatique peut à chaque fois être substitué par 1, 2, 3, 4 ou 5 substituants choisis indépendamment l'un de l'autre dans le groupe constitué par oxo (=O), thioxo (=S), F, Cl, Br, I, -CN, -CF₃, -SF₅, -OH, -O-CH₃, -O-C₂H₅, -O-C(CH₃)₃, -NH₂, -NO₂, -O-CF₃, -S-CF₃, -SH, S-CH₃, -S-C₂H₅, -S-C(CH₃)₃, méthyle, éthyle, n-propyle, isopropyle, n-butyle, sec-butyle, isobutyle, tert-butyle, -C(=O)-H, -C (=O) -CH₃, -C (=O) -C₂H₅, -C (=O) -OH, -C(=O)-O-CH₃, -C(=O)-O-C₂H₅, -C(=O)-O-C(CH₃)₃, -(CH₂)-C(=O)-OH, -(CH₂)-C(=O)-O-CH₃, -(CH₂)-C(=O)-O-C₂H₅, -N(CH₃)₂, -N(C₂H₅)₂, -N(H)(CH₃), -N(H)(C₂H₅), -(CH₂)-benzo[b]furannyle, -O-phényle, -O-benzyle, phényle, benzyle, naphtyle et - (CH₂)-naphtyle, la partie cyclique des radicaux -O-phényle, -0-benzyle, phényle, -(CH₂)-benzo[b]furannyle, benzyle, naphtyle et -(CH₂)-naphtyle pouvant à chaque fois être substituée par 1, 2, 3, 4 ou 5 substituants choisis indépendamment l'un de l'autre dans le groupe constitué par F, Cl, Br, -OH, -CF₃, -SF₅, -CN, -NO₂, -O-CH₃, -O-C₂H₅, méthyle, éthyle, n-propyle, isopropyle, n-butyle, sec-butyle, isobutyle, tert-butyle, -O-CF₃, -S-CF₃, phényle et -O-benzyle,
- ou représente un radical choisi dans le groupe constitué par phényle, naphtyle, thiophényle, furannyle, pyrrolyle, pyrazolyle, pyrazinyle, triazolyle, pyridinyle, imidazolyle, indolyle, iso-indolyle, benzo[b]furannyle, benzo[b]thiophényle, thiazolyle, thiadiazolyle, oxazolyle, isoxazolyle, pyridazinyle, pyrazinyle, pyrimidinyle, indazolyle, quinazolinyle, quinoléinyle, isoquinoléinyle, phénazinyle, phénothiazinyle, oxadiazolyle, benzo[1,2,5]oxadiazolyle, chromanyle et (1,2,3,4)-tétrahydroquinoléinyle, où le radical peut à chaque fois être substitué par 1, 2, 3, 4 ou 5 substituants choisis indépendamment l'un de l'autre dans le groupe constitué par F, Cl, Br, I, -CN, -CF₃, -SF₅, -OH, -O-CH₃, -O-C₂H₅, -O-CH (CH₃) ₂, -O-C (CH₃) ₃, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, -S-CH (CH₃) ₂, -S-C(CH₃)₃, -S-CH₂-CH₂-CH₂-CH₃, méthyle, éthyle, n-propyle, isopropyle, n-butyle, sec-butyle, isobutyle, tert-butyle, (1,1)-diméthylpropyle, -C(=O)-OH, -C(=O)-O-CH₃, -C(=O)-O-C₂H₅, -C(-O)-O-C(CH₃)₃, -O-C(=O)-CH₃, -O-C(=O)-C₂H₅, -O-C(=O)-phényle, -(CH₂)-OC (=O) -CH₃, -(CH₂)-O-C(=O)-C₂H₅, -(CH₂)-O-C(-O)-phényle, -N(CH₃)₂, -N(C₂H₅)₂, -N(H)(CH₃), -N(H)(C₂H₅), -NH-C(=O)-O-CH₃, -NH-C(=O)-O-C₂H₅, -NH-C(=O)-CH₃, -NH-C(=O)-C₂H₅, -C(=O)-H, -C(=O)-CH₃, -C(-O)-C₂H₅, -C(=O)-CF₃, -C(=O)-NH₂, -C(=O)-NH-CH₃, -C(=O)-NH-C₂H₅, -S(=O)₂-NH₂, -S(=O)₂-NH-CH₃, -S(=O)₂-NH-C₂H₅, -S(=O)₂-NH-phényle, -S(=O)₂-CH₃, -S(=O)₂-C₂H₅, -(CH₂)-benzo[b]furannyle, -O-phényle, -S-phényle, -S-benzyle, -O-benzyle, phényle et benzyle, la partie cyclique des radicaux -O-C(=O)-phényle, -(CH₂)-O-C(=O)-phényle, -S(=O)₂-NH-phényle, -O-phényle, -S-phényle, -S-benzyle, -O-benzyle, phényle, -(CH₂)-benzo[b]furannyle et benzyle pouvant à chaque fois être substituée par 1, 2, 3, 4 ou 5 substituants choisis indépendamment l'un de l'autre dans le groupe constitué par F, Cl, Br, -OH, -CF₃, -SF₅, -CN, -NO₂, -O-CH₃, -O-C₂H₅, -O-CH (CH₃) ₂, -O-C (CH₃) ₃, méthyle, éthyle, n-propyle, isopropyle, n-butyle, sec-butyle, isobutyle, tert-butyle, -O-CF₃, -S-CF₃, phényle et -O-benzyle,
R¹⁷ représente un radical choisi dans le groupe constitué par méthyle, éthyle, n-propyle, isopropyle, n-butyle, sec-butyle, isobutyle, tert-butyle, n-pentyle, 2-pentyle, 3-pentyle, -(CH₂)-(C(CH₃)₃), n-hexyle, 3-hexyle, -(CH₂)-(CH₂)-(C(CH₃)₃), n-heptyle, 3-heptyle, 4-heptyle, n-octyle, -(CH₂)-(CH)(C₂H₅)-(CH₂)-(CH₂)-(CH₂)-(CH₃), vinyle, 1-propényle, 2-propényle, 1-butényle, 2-butényle, 3-butényle, 1-propynyle, 2-propynyle, 1-butynyle, 2-butynyle et 3-butynyle, où le radical peut à chaque fois être substitué par 1, 2, 3, 4 ou 5 substituants choisis indépendamment l'un de l'autre dans le groupe constitué par F, Cl, Br, I, -CN, -NO₂, -OH, -SH, -NH₂, -N(CH₃)₂, -N(C₂H₅)₂ et -N(CH₃)(C₂H₅) ;
et
R¹⁸ représente un radical (hétéro)cycloaliphatique choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, pipérazinyle, thiomorpholinyle, tétrahydropyrannyle, azépanyle, diazépanyle et dithiolanyle, où le radical (hétéro)cycloaliphatique peut à chaque fois être substitué par 1, 2, 3, 4 ou 5 substituants choisis indépendamment l'un de l'autre dans le groupe constitué par oxo (=O), thioxo (=S), F, Cl, Br, I, -CN, -CF₃, -SF₅, -OH, -O-CH₃, -O-C₂H₅, -O-C(CH₃)₃, -NH₂, -NO₂, -O-CF₃, -S-CF₃, -SH, S-CH₃, -S-C₂H₅, -S-C(CH₃)₃, méthyle, éthyle, n-propyle, isopropyle, n-butyle, sec-butyle, isobutyle, tert-butyle, -C(=O)-H, -C(=O)-CH₃, -C(=O)-C₂H₅, -C(=O)-OH, -C(=O)-O-CH₃, -C(=O)-O-C₂H₅, -C(=O)-O-C(CH₃)₃, -(CH₂)-C(=O)-OH, -(CH₂)-C(=O)-O-CH₃, -(CH₂)-C(=O)-O-C₂H₅, -N(CH₃)₂, -N(C₂H₅)₂, -N(H)(CH₃), -N(H)(C₂H₅), -(CH₂)-benzo[b]furannyle, -O-phényle, -O-benzyle, phényle, benzyle, naphtyle et -(CH₂)-naphtyle, la partie cyclique des radicaux -O-phényle, -0-benzyle, phényle, -(CH₂)-benzo[b]furannyle, benzyle, naphtyle et -(CH₂)-naphtyle pouvant à chaque fois être substituée par 1, 2, 3, 4 ou 5 substituants choisis indépendamment l'un de l'autre dans le groupe constitué par F, Cl, Br, -OH, -CF₃, -SF₅, -CN, -NO₂, -O-CH₃, -O-C₂H₅, méthyle, éthyle, n-propyle, isopropyle, n-butyle, sec-butyle, isobutyle, tert-butyle, -O-CF₃, -S-CF₃, phényle et -O-benzyle,
- ou représente un radical choisi dans le groupe constitué par phényle, naphtyle, thiophényle, furannyle, pyrrolyle, pyrazolyle, pyrazinyle, triazolyle, pyridinyle, imidazolyle, indolyle, iso-indolyle, benzo[b]furannyle, benzo[b]thiophényle, thiazolyle, thiadiazolyle, oxazolyle, isoxazolyle, pyridazinyle, pyrazinyle, pyrimidinyle, indazolyle, quinazolinyle, quinoléinyle, isoquinoléinyle, phénazinyle, phénothiazinyle, oxadiazolyle, benzo[1,2,5]oxadiazolyle, chromanyle et (1,2,3,4)-tétrahydroquinoléinyle, où le radical peut à chaque fois être substitué par 1, 2, 3, 4 ou 5 substituants choisis indépendamment l'un de l'autre dans le groupe constitué par F, Cl, Br, I, -CN, -CF₃, -SF₅, -OH, -O-CH₃, -O-C₂H₅, -O-CH (CH₃) ₂, -O-C(CH₃)₃, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, -S-CH(CH₃)₂, -S-C(CH₃)₃, -S-CH₂-CH₂-CH₂-CH₃, méthyle, éthyle, n-propyle, isopropyle, n-butyle, sec-butyle, isobutyle, tert-butyle, (1,1)-diméthylpropyle, -C(=O)-OH, -C(=O)-O-CH₃, -C (=O) -O-C₂H₅, -C (=O) -O-C (CH₃) ₃, -O-C(=O)-CH₃, -O-C(=O)-C₂H₅, -O-C(=O)-phényle, -(CH₂)-O-C(=O)-CH₃, -(CH₂)-O-C(=O)-C₂H₅, -(CH₂)-O-C(=O)-phényle, -N(CH₃)₂, -N(C₂H₅)₂, -N(H)(CH₃), -N(H)(C₂H₅), -NH-C(=O)-O-CH₃, -NH-C(=O)-O-C₂H₅, -NH-C(=O)-CH₃, -NH-C(=O)-C₂H₅, -C(=O)-H, -C(=O)-CH₃, -C(=O)-C₂H₅, -C(=O)-CF₃, -C(=O)-NH₂, -C(=O)-NH-CH₃, -C(=O)-NH-C₂H₅, -S(=O)₂-NH₂, -S(=O)₂-NH-CH₃, -S(=O)₂-NH-C₂H₅, -S(=O)₂-NH-phényle, -S(=O)₂-CH₃, -S(=O)₂-C₂H₅, -(CH₂)-benzo[b]furannyle, -O-phényle, -S-phényle, -S-benzyle, -O-benzyle, phényle et benzyle, la partie cyclique des radicaux -O-C(=O)-phényle, -(CH₂)-O-C(=O)-phényle, -S(=O)₂-NH-phényle, -O-phényle, -S-phényle, -S-benzyle, -O-benzyle, phényle, -(CH₂)-benzo[b]furannyle et benzyle pouvant à chaque fois être substituée par 1, 2, 3, 4 ou 5 substituants choisis indépendamment l'un de l'autre dans le groupe constitué par F, Cl, Br, -OH, -CF₃, -SF₅, -CN, -NO₂, -O-CH₃, -O-C₂H₅, -O-CH(CH₃)₂, -O-C(CH₃)₃, méthyle, éthyle, n-propyle, isopropyle, n-butyle, sec-butyle, isobutyle, tert-butyle, -O-CF₃, -S-CF₃, phényle et -0-benzyle.

11. Composés selon la revendication 9 ou 10, **caractérisés**
**en ce que**
R¹ représente un radical hydrogène ;
- représente un radical choisi dans le groupe constitué par méthyle, éthyle, n-propyle, isopropyle, n-butyle, sec-butyle, isobutyle, tert-butyle, n-pentyle, 1-propényle, 2-propényle, 1-propynyle et 2-propynyle ;
- représente -(CH₂)-C(=O)-O-R⁷;
- ou représente -(CH₂)-R¹², -(CH₂)-(CH₂)-R¹², - (CH₂)-O-R¹², -(CH₂)-S-R¹², -(CH₂)-N(CH₃)-R¹², -(CH₂)-(CH₂)-(CH₂)-R¹² ou -(CH₂)-(CH₂)-O-R¹² ;
R² représente un radical hydrogène ;
- représente un radical choisi dans le groupe constitué par méthyle, éthyle, n-propyle, isopropyle, n-butyle, sec-butyle, isobutyle, tert-butyle et n-pentyle ;
- ou représente -(CH₂)-R¹³, -(CH₂)-(CH₂)-R¹³, -(CH₂)-O-R¹³, -(CH₂)-S-R¹³, -(CH₂) -N(CH₃)-R¹³, -(CH₂) -(CH₂)-S-R¹³ ou -(CH₂)-(CH₂)-O-R¹³ ;
R³ représente un groupe -S(=O)₂-R₄, un groupe -C(=S)-NH-R⁵ ou un groupe -C(=O)-NH-R⁶
R⁴ représente un radical -NR¹⁰R¹¹ ;
- représente un radical choisi dans le groupe constitué par méthyle, éthyle, n-propyle, isopropyle, n-butyle, sec-butyle, isobutyle, tert-butyle, n-pentyle, 2-pentyle, 3-pentyle, -(CH₂)-(C(CH₃)₃) et n-hexyle ;
- représente un radical choisi dans le groupe constitué par phényle, naphtyle, thiophényle, furannyle et (1,2,3,4)-tétrahydroquinoléinyle, où le radical peut à chaque fois être substitué par 1, 2, 3, 4 ou 5 substituants choisis indépendamment l'un de l'autre dans le groupe constitué par F, Cl, Br, -CF₃, -O-CH₃, -O-C₂H₅, -O-CH (CH₃) 2, -O-C(CH₃)₃, -O-CH₂-CH₂-CH₂-CH₃, -NO₂, -O-CF₃, -O-CHF₂, -O-CH₂F, -S-CF₃, -S-CHF₂, -S-CH₂F, méthyle, éthyle, n-propyle, isopropyle, n-butyle, sec-butyle, isobutyle, tert-butyle, (1,1)-diméthylpropyle, -C(=O)-O-CH₃, -C(=O)-O-C₂H₅, -C(=O)-CH₃, -C(=O)-C₂H₅, -C(=O)-CF₃, -S(=O)₂-CH₃ et -S(=O)₂-C₂H₅,
- ou représente -(CH₂)-R¹⁴, -(CH₂)-(CH₂)-R¹⁴ et -(CH₂)-(CH₂)-(CH₂)-R¹⁴
R⁵ représente un radical choisi dans le groupe constitué par méthyle, éthyle, n-propyle, isopropyle, 1-propényle et 2-propényle ;
- représente un radical phényle, où le radical phényle peut à chaque fois être substitué par 1, 2, 3, 4 ou 5 substituants choisis indépendamment l'un de l'autre dans le groupe constitué par F, Cl, Br, -CN, -CF₃, -O-CH₃, -O-C₂H₅, -O-CH(CH₃)₂, -OC (CH₃) ₃, -O-CH₂-CH₂-CH₂-CH₃, -O-CF₃, -O-CHF₂, -O-CH₂F, -S-CF₃, -S-CHF₂, -S-CH₂F, -SH, méthyle, éthyle, n-propyle, isopropyle, n-butyle, sec-butyle, isobutyle, tert-butyle, (1,1)-diméthylpropyle, -C(=O)-O-CH₃, -C(=O)-O-C₂H₅ et -C(=O)-CH₃, -C(=O)-C₂H₅,
- représente -(CHR¹⁵)-R¹⁶, -(CHR¹⁵)-(CH₂)-R¹⁶, -(CHR¹⁵)-(CH₂)-(CH₂)-R¹⁶, -(CHR¹⁵) (CH₂)-O-R¹⁶ ou -(CHR¹⁵) (CH₂)-S-R¹⁶ ;
- représente un radical -C(=O)-OR⁸ ;
- représente un radical - (CHR¹⁷)-C(=O)-OR⁹ ou un radical -(CHR¹⁷)-(CH₂)-C(=O)-OR⁹ ;
R⁶ représente un radical cycloaliphatique choisi dans le groupe constitué par cyclopentyle, cyclohexyle et cycloheptyle ;
- représente un radical phényle, qui peut être substitué par 1, 2, 3, 4 ou 5 substituants choisis indépendamment l'un de l'autre dans le groupe constitué par F, Cl, Br, I, -CN, -CF₃, -O-CH₃, -O-C₂H₅, -O-CH(CH₃)₂, -O-C(CH₃)₃, -O-CF₃, -O-CHF₂, -O-CH₂F, -C(=O)-O-CH₃, -C(=O)-O-C₂H₅, -C(=O)-CH₃ et -C(=O)-C₂H₅,
- ou représente -(CH₂)-R¹⁸, -(CH₂)-(CH₂)-R¹⁸ ou -(CH₂)-(CH₂)-(CH₂)-R¹⁸ ;
⁷, R⁸, R⁹, R¹⁰ et R¹¹ R indépendamment l'un de l'autre, à chaque fois
- représentent un radical méthyle ou éthyle ;
R¹² représente un radical phényle ou naphtyle, où le radical peut à chaque fois être substitué par 1, 2, 3, 4 ou 5 substituants choisis indépendamment l'un de l'autre dans le groupe constitué par F, Cl, Br, -CN, -CF₃, -NO₂, méthyle, éthyle, n-propyle, isopropyle, n-butyle, sec-butyle, isobutyle, tert-butyle, -C(=O)-O-CH₃, -C(=O)-O-C₂H₅, -C(=O)-CH₃ et -C(=O)-C₂H₅,
R¹³ représente un radical méthyle ou éthyle ;
- ou représente un radical phényle, qui peut être substitué par 1, 2, 3, 4 ou 5 substituants choisis indépendamment l'un de l'autre dans le groupe constitué par F, Cl, Br et -CN ;
R¹⁴ représente un radical 7,7-diméthyl-2-oxobicyclo[2,2,1]heptyle ou phényle ;
R¹⁵ représente un radical hydrogène ou un radical méthyle ou éthyle ;
R¹⁶ représente un radical méthyle ou éthyle ;
- représente un radical (hétéro)cycloaliphatique choisi dans le groupe constitué par cyclopentyle, cyclohexyle, cycloheptyle et tétrahydrofurannyle ;
- ou représente un radical phényle, qui peut être substitué par 1, 2, 3, 4 ou 5 substituants choisis indépendamment l'un de l'autre dans le groupe constitué par F, Cl, Br, -CN, -CF₃, -O-CH₃, -O-C₂H₅ et -O-CH(CH₃)₂;
R¹⁷ représente un radical méthyle ou éthyle ;
et
R¹⁸ représente un radical (hétéro)cycloaliphatique choisi dans le groupe constitué par cyclopentyle, cyclohexyle et cycloheptyle ;
- ou représente un radical phényle, qui peut être substitué par 1, 2, 3, 4 ou 5 substituants choisis indépendamment l'un de l'autre dans le groupe constitué par F, Cl et Br.

12. Composés selon l'une ou plusieurs des revendications 1 à 11 choisis dans le groupe constitué par
1. la 3-isopropyl-8-(4-nitrobenzènesulfonyl)-1,4,8-triazaspiro[4,5]décan-2-one,
2. la 8-(2-chlorobenzènesulfonyl)-3-isobutyl-1,4,8-triazaspiro[4,5]décan-2-one,
3. la 3-benzyl-8-[2-(2,2,2-trifluoroacétyl)-1,2,3,4-tétrahydro-isoquinoléine-7-sulfonyl]-1,4,8-triazaspiro[4,5]décan-2-one,
4. la 3-benzyl-8-(4-méthoxy-2,3,6-triméthylbenzènesulfonyl)-1,4,8-triazaspiro[4,5]décan-2-one,
5. le 2-[8-(4-butoxybenzènesulfonyl)-2-oxo-1,4,8-triazaspiro[4,5]déc-1-ylméthyl]-benzonitrile,
6. le 2-{8-[4-(1,1-diméthylpropyl)-benzènesulfonyl]-2-oxo-1,4,8-triazaspiro[4,5]déc-1-ylméthyl}-benzonitrile,
7. la 3-benzyl-8-(2-fluorobenzènesulfonyl)-1,4,8-triazaspiro[4,5]décan-2-one,
8. la 8-(2-chlorobenzènesulfonyl)-3-isopropyl-1,4,8-triazaspiro[4,5]décan-2-one,
9. le diméthylamide de l'acide 1-benzyl-2-oxo-1,4,8-triazaspiro[4,5]décane-8-sulfonique,
10. le 3-[8-(4-acétylbenzènesulfonyl)-3-(2-méthylsulfanyléthyl)-2-oxo-1,4,8-triazaspiro[4,5]déc-1-ylméthyl]-benzonitrile,
11. la 1-(2-fluorobenzyl)-3-isopropyl-8-(4-méthoxy-2,3,6-triméthylbenzènesulfonyl)-1,4,8-triazaspiro[4,5]décan-2-one,
12. la 1-butyl-8-(5-chlorothiophène-2-sulfonyl)-3-(2-méthylsulfanyléthyl)-1,4,8-triazaspiro[4,5]décan-2-one,
13. la 8-(4-acétylbenzènesulfonyl)-1-butyl-3-(2-méthylsulfanyléthyl)-1,4,8-triazaspiro[4,5]décan-2-one,
14. la 1-benzyl-3-isopropyl-8-(2,4,6-triméthylbenzènesulfonyl)-1,4,8-triazaspiro[4,5]décan-2-one,
15. l'ester méthylique de l'acide 2-(1-butyl-3-isopropyl-2-oxo-1,4,8-triazaspiro[4,5]décane-8-sulfonyl)-benzoïque,
16. la 1,3-dibenzyl-8-(thiophène-2-sulfonyl)-1,4,8-triazaspiro[4,5]décan-2-one,
17. la 8-(3-chloro-4-fluorobenzènesulfonyl)-3-isopropyl-1-(2-phénoxyéthyl)-1,4,8-triazaspiro[4,5]décan-2-one,
18. la 1-butyl-3-(2-méthylsulfanyléthyl)-8-(toluène-3-sulfonyl)-1,4,8-triazaspiro[4,5]décan-2-one,
19. la 1-butyl-8-(2,4-difluorobenzènesulfonyl)-3-isopropyl-1,4,8-triazaspiro[4,5]décan-2-one,
20. la 1,3-dibenzyl-8-(2,5-dichlorothiophène-3-sulfonyl)-1,4,8-triazaspiro[4,5]décan-2-one,
21. la 3-isopropyl-1-(2-phénoxyéthyl)-8-(toluène-4-sulfonyl)-1,4_{;}8-triazaspiro[4,5]décan-2-one,
22. la 3-benzyl-1-butyl-8-(2-trifluorométhylbenzènesulfonyl)-1,4,8-triazaspiro[4,5]décan-2-one,
23. l'ester méthylique de l'acide 2-[1-(4-fluorobenzyl)-3-(2-méthylsulfanyléthyl)-2-oxo-1,4,8-triazaspiro[4,5]décane-8-sulfonyl]-benzoïque,
24. la 1,3-dibenzyl-8-(2,3,5,6-tétraméthylbenzènesulfonyl)-1,4,8-triazaspiro[4,5]décan-2-one,
25. la 1-(4-fluorobenzyl)-3-(2-méthylsulfanyléthyl)-8-(2-trifluorométhylbenzènesulfonyl)-1,4,8-triazaspiro[4,5]décan-2-one,
26. la 8-(4-chloro-2,5-diméthylbenzènesulfonyl)-1-(4-fluorobenzyl)-3-(2-méthylsulfanyléthyl)-1,4,8-triazaspiro[4,5]décan-2-one,
27. la 1-benzyl-8-(4-chloro-2,5-diméthylbenzènesulfonyl)-3-isobutyl-1,4,8-triazaspiro[4,5]décan-2-one,
28. la 3-benzyl-1-butyl-8-[4-(1,1-diméthylpropyl)-benzènesulfonyl]-1,4,8-triazaspiro[4,5]décan-2-one,
29. la 1,3-dibenzyl-8-(3,4-diméthoxybenzènesulfonyl)-1,4,8-triazaspiro[4,5]décan-2-one,
30. la 1-butyl-8-(3,4-diméthoxybenzènesulfonyl)-3-(2-méthylsulfanyléthyl)-1,4,8-triazaspiro[4,5]décan-2-one,
31. la 1,3-dibenzyl-8-(4-méthoxy-2,3,6-triméthylbenzènesulfonyl)-1,4,8-triazaspiro[4,5]décan-2-one,
32. la 1-benzyl-8-éthanesulfonyl-3-(2-méthylsulfanyléthyl)-1,4,8-triazaspiro[4,5]décan-2-one,
33. la 3-benzyl-1-butyl-8-(4-éthylbenzènesulfonyl)-1,4,8-triazaspiro[4,5]décan-2-one,
34. la 3-(2-méthylsulfanyléthyl)-1-prop-2-ynyl-8-(3-trifluorométhylbenzènesulfonyl)-1,4,8-triazaspiro[4,5]décan-2-one,
35. la 1,3-dibenzyl-8-(4-éthylbenzènesulfonyl)-1,4,8-triazaspiro[4,5]décan-2-one,
36. la 3-benzyl-1-butyl-8-(3-chlorobenzènesulfonyl)-1,4,8-triazaspiro[4,5]décan-2-one,
37. la 1,3-dibenzyl-8-(2-fluorobenzènesulfonyl)-1,4,8-triazaspiro[4,5]décan-2-one,
38. l'ester méthylique de l'acide 2-[1-(2-cyano-benzyl)-3-(2-méthylsulfanyléthyl)-2-oxo-1,4,8-triazaspiro[4,5]décane-8-sulfonyl]-benzoïque,
39. la 3-benzyl-1-prop-2-ynyl-8-(2-trifluorométhylbenzènesulfonyl)-1,4,8-triazaspiro[4,5]décan-2-one,
40. la 3-benzyl-8-(3-chlorobenzènesulfonyl)-1-méthyl-1,4,8-triazaspiro[4,5]décan-2-one,
41. la 1-benzyl-8-[4-(l,l-diméthylpropyl)-benzènesulfonyl]-3-(2-méthylsulfanyléthyl)-1,4,8-triazaspiro[4,5]décan-2-oné,
42. la 8-(2,4-difluorobenzènesulfonyl)-1-(3,5-diméthylbenzyl)-3-(2-méthylsulfanyléthyl)-1,4,8-triazaspiro[4,5]décan-2-one,
43. la 1,3-dibenzyl-8-(4-nitrobenzènesulfonyl)-1,4,8-triazaspiro[4,5]décan-2-one,
44. la 1-(4-fluorobenzyl)-3-(2-méthylsulfanyléthyl)-8-(toluène-4-sulfonyl)-1,4,8-triazaspiro[4,5]décan-2-one,
45. la 1,3-dibenzyl-8-(toluène-4-sulfonyl)-1,4,8-triazaspiro[4,5]décan-2-one,
46. l'ester éthylique de l'acide [3-benzyl-8-(2-méthyl-5-nitrobenzènesulfonyl)-2-oxo-1,4,8-triazaspiro[4,5]déc-1-yl]-acétique,
47. l'ester éthylique de l'acide [3-benzyl-8-(2-méthanesulfonylbenzènesulfonyl)-2-oxo-1,4,8-triazaspiro[4,5]déc-1-yl]-acétique,
48. l'ester éthylique de l'acide [3-benzyl-2-oxo-8-(thiophène-2-sulfonyl)-1,4,8-triazaspiro[4,5]déc-1-yl]-acétique,
49. l'ester méthylique de l'acide 4-(8-éthanesulfonyl-2-oxo-1,4,8-triazaspiro[4,5]déc-1-ylméthyl)-benzoïque,
50. l'ester éthylique de l'acide [3-benzyl-8-(4-méthoxybenzènesulfonyl)-2-oxo-1,4,8-triazaspiro[4,5]déc-1-yl]-acétique,
51. l'ester méthylique de l'acide 4-(2-oxo-8-benzèneméthanesulfonyl-1,4,8-triazaspiro[4,5]déc-1-ylméthyl)-benzoïque,
52. le 3-[3-isopropyl-8-(4-nitrobenzènesulfonyl)-2-oxo-1,4,8-triazaspiro[4,5]déc-1-ylméthyl]-benzonitrile,
53. l'ester méthylique de l'acide 4-[8-(butane-1-sulfonyl)-2-oxo-1,4,8-triazaspiro[4,5]déc-1-ylméthyl]-benzoïque,
54. la 1-allyl-8-(2-méthanesulfonylbenzènesulfonyl)-3-(2-méthylsulfanyléthyl)-1,4,8-triazaspiro[4,5]décan-2-one,
55. le diméthylamide de l'acide 1-(2-fluorobenzyl)-2-oxo-1,4,8-triazaspiro[4,5]décane-8-sulfonique,
56. la 8-(7,7-diméthyl-2-oxobicyclo[2,2,1]hept-1-ylméthanesulfonyl)-1-(2-fluorobenzyl)-1,4,8-triazaspiro[4,5]décan-2-one,
57. l'ester éthylique de l'acide [3-(2-méthylsulfanyléthyl)-2-oxo-8-benzèneméthanesulfonyl-1,4,8-triazaspiro[4,5]déc-1-yl]-acétique,
58. la 1-allyl-3-(2-méthylsulfanyléthyl)-8-benzèneméthanesulfonyl-1,4,8-triazaspiro[4,5]décan-2-one,
59. le 3-[2-oxo-8-(2,4,6-triméthylbenzènesulfonyl)-1,4,8-triazaspiro[4,5]déc-1-ylméthyl]-benzonitrile,
60. l'ester méthylique de l'acide 4-[2-oxo-8-(2,4,6-triméthylbenzènesulfonyl)-1,4,8-triazaspiro[4,5]déc-1-ylméthyl]-benzoïque,
61. l'ester éthylique de l'acide [3-benzyl-8-(5-chlorothiophène-2-sulfonyl)-2-oxo-1,4,8-triazaspiro[4,5]déc-1-yl]-acétique,
62. l'ester éthylique de l'acide [3-benzyl-8-(butane-1-sulfonyl)-2-oxo-1,4,8-triazaspiro[4,5]déc-1-yl]-acétique,
63. l'ester éthylique de l'acide [3-benzyl-2-oxo-8-(toluène-3-sulfonyl)-1,4,8-triazaspiro[4,5]déc-1-yl]-acétique,
64. la 1-(2-fluorobenzyl)-8-(4-méthoxybenzènesulfonyl)-1,4,8-triazaspiro[4,5]décan-2-one,
65. l'ester éthylique de l'acide [3-benzyl-2-oxo-8-(2-trifluorométhylbenzènesulfonyl)-1,4,8-triazaspiro[4,5]déc-1-yl]-acétique,
66. l'ester éthylique de l'acide (8-benzènesulfonyl-3-benzyl-2-oxo-1,4,8-triazaspiro[4,5]déc-1-yl)-acétique,
67. l'ester éthylique de l'acide [3-benzyl-2-oxo-8-(4-propylbenzènesulfonyl)-1,4,8-triazaspiro[4,5]déc-1-yl]-acétique,
68. l'ester éthylique de l'acide (3-benzyl-2-oxo-8-benzèneméthanesulfonyl-1,4,8-triazaspiro[4,5]déc-1-yl)-acétique,
69. l'ester éthylique de l'acide [3-benzyl-8-(3-chloro-4-fluorobenzènesulfonyl)-2-oxo-1,4,8-triazaspiro[4,5]déc-1-yl]-acétique,
70. la 1-allyl-3-isopropyl-8-(2,4,6-triméthylbenzènesulfonyl)-1,4,8-triazaspiro[4,5]décan-2-one,
71. la 8-benzènesulfonyl-3-benzyl-1-(2-fluorobenzyl)-1,4,8-triazaspiro[4,5]décan-2-one,
72. l'ester éthylique de l'acide [3-benzyl-2-oxo-8-(2,4,6-triméthylbenzènesulfonyl)-1,4,8-triazaspiro[4,5]déc-1-yl]-acétique,
73. l'ester éthylique de l'acide [3-benzyl-8-(4-butoxybenzènesulfonyl)-2-oxo-1,4,8-triazaspiro[4,5]déc-1-yl]-acétique,
74. l'ester éthylique de l'acide [3-benzyl-8-(2,5-diméthoxybenzènesulfonyl)-2-oxo-1,4;8-triazaspiro[4,5]déc-1-yl]-acétique,
75. le diméthylamide de l'acide 3-(2-méthylsulfanyléthyl)-1-naphtalén-2-ylméthyl-2-oxo-1,4,8-triazaspiro[4,5]décane-8-sulfonique,
76. le diméthylamide de l'acide 1-(3-cyanobenzyl)-3-isopropyl-2-oxo-1,4,8-triazaspiro[4,5]décane-8-sulfonique,
77. le 3-{3-isopropyl-2-oxo-8-[2-(2,2,2-trifluoroacétyl)-1,2,3,4-tétrahydro-isoquinoléine-7-sulfonyl]-1,4,8-triazaspiro[4,5]déc-1-ylméthyl}-benzonitrile,
78. la 1-allyl-3-(2-méthylsulfanyléthyl)-8-[2-(2,2,2-trifluoroacétyl)-1,2,3,4-tétrahydro-isoquinoléine-7-sulfonyl]-1,4,8-triazaspiro[4,5]décan-2-one,
79. la 1-allyl-8-(4-méthoxy-2,3,6-triméthylbenzènesulfonyl)-3-(2-méthylsulfanyléthyl)-1,4,8-triazaspiro[4,5]décan-2-one,
80. l'ester méthylique de l'acide 2-(3-benzyl-1-éthoxycarbonylméthyl-2-oxo-1,4,8-triazaspiro[4,5]décane-8-sulfonyl)-benzoïque,
81. la 3-benzyl-1-(2-fluorobenzyl)-8-(2-trifluorométhylbenzènesulfonyl)-1,4,8-triazaspiro[4,5]décan-2-one,
82. la 8-(2-chlorobenzènesulfonyl)-3-(2-méthylsulfanyléthyl)-1-(2-nitrobenzyl)-1,4,8-triazaspiro[4,5]décan-2-one,
83. la 1-allyl-3-(2-méthylsulfanyléthyl)-8-(toluène-3-sulfonyl)-1,4,8-triazaspiro[4,5]décan-2-one,
84. l'ester éthylique de l'acide [8-(2-chlorobenzènesulfonyl)-3-(2-méthylsulfanyléthyl)-2-oxo-1,4,8-triazaspiro[4,5]déc-1-yl]-acétique,
85. la 3-benzyl-1-(2-fluorobenzyl)-8-(toluène-4-sulfonyl)-1,4,8-triazaspiro[4,5]décan-2-one,
86. l'ester méthylique de l'acide 4-[8-(2,5-dichlorothiophène-3-sulfonyl)-2-oxo-1,4,8-triazaspiro[4,5]déc-1-ylméthyl]-benzoïque,
87. le 3-[8-(4-fluorobenzènesulfonyl)-3-isopropyl-2-oxo-1,4,8-triazaspiro[4,5]déc-1-ylméthyl]-benzonitrile,
88. la 8-(2,5-diméthoxybenzènesulfonyl)-3-(2-méthylsulfanyléthyl)-1-(2-nitrobenzyl)-1,4,8-triazaspiro[4,5]décan-2-one,
89. la 8-(3-chloro-4-fluorobenzènesulfonyl)-1-(2-fluorobenzyl)-1,4,8-triazaspiro[4,5]décan-2-one,
90. la 1-(2-fluorobenzyl)-8-(toluène-3-sulfonyl)-1,4,8-triazaspiro[4,5]décan-2-one,
91. la 1-(2,6-dichlorobenzyl)-8-(4,5-dichlorothiophène-2-sulfonyl)-3-isobutyl-1,4,8-triazaspiro[4,5]décan-2-one,
92. la 8-(5-chlorothiophène-2-sulfonyl)-1-(2-fluorobenzyl)-1,4,8-triazaspiro[4,5]décan-2-one,
93. la 3-benzyl-1-(2-fluorobenzyl)-8-(4-propylbenzènesulfonyl)-1,4,8-triazaspiro[4,5]décan-2-one,
94. le 3-[8-(butane-1-sulfonyl)-3-isopropyl-2-oxo-1,4,8-triazaspiro[4,5]déc-1-ylméthyl]-benzonitrile,
95. le 3-[8-(2-méthanesulfonylbenzènesulfonyl)-2-oxo-1,4,8-triazaspiro[4,5]déc-1-ylméthyl]-benzonitrile,
96. la 8-(2-fluorobenzènesulfonyl)-1-(2-fluorobenzyl)-1,4,8-triazaspiro[4,5]décan-2-one,
97. l'ester méthylique de l'acide 2-[1-(3-cyanobenzyl)-3-isopropyl-2-oxo-1,4,8-triazaspiro[4,5]décane-8-sulfonyl]-benzoïque,
98. la 3-benzyl-1-(2-fluorobenzyl)-8-(5-fluoro-2-méthylbenzènesulfonyl)-1,4,8-triazaspiro[4,5]décan-2-one,
99. la 1-allyl-8-(4-méthoxybenzènesulfonyl)-3-(2-méthylsulfanyléthyl)-1,4,8-triazaspiro[4,5]décan-2-one,
100. la 1-(3,5-diméthylbenzyl)-3-isobutyl-8-(thiophène-2-sulfonyl)-1,4,8-triazaspiro[4,5]décan-2-one,
101. la 3-(2-méthylsulfanyléthyl)-8-(4-nitrobenzènesulfonyl)-1-(2-nitrobenzyl)-1,4,8-triazaspiro[4,5]décan-2-one,
102. l'o-toluylamide de l'acide 2-isobutyl-3-oxo-1,4,8-triazaspiro[4,5]décane-8-thiocarboxylique,
103. l'isopropylamide de l'acide 2-benzyl-3-oxo-1,4,8-triazaspiro[4,5]décane-8-thiocarboxylique,
104. le (tétrahydrofurann-2-ylméthyl)-amide de l'acide 1-butyl-3-isopropyl-2-oxo-1,4,8-triazaspiro[4,5]décane-8-thiocarboxylique,
105. l'ester éthylique de l'acide 3-{[1-méthyl-3-(2-méthylsulfanyléthyl)-2-oxo-1,4,8-triazaspiro[4,5]décane-8-carbothioyl]-amino}butyrique,
106. l'isopropylamide de l'acide 1-allyl-3-isopropyl-2-oxo-1,4,8-triazaspiro[4,5]décane-8-thiocarboxylique,
107. l'isopropylamide de l'acide 1-butyl-3-isopropyl-2-oxo-1,4,8-triazaspiro[4,5]décane-8-thiocarboxylique,
108. le (2-méthoxybenzène)-amide de l'acide 3-benzyl-1-(2-fluorobenzyl)-2-oxo-1,4,8-triazaspiro[4,5]décane-8-thiocarboxylique,
109. l'ester éthylique de l'acide [8-isopropylthiocarbamoyl-3-(2-méthylsulfanyléthyl)-2-oxo-1,4,8-triazaspiro[4,5]déc-1-yl]-acétique,
110. le m-toluylamide de l'acide 1-méthyl-3-(2-méthylsulfanyléthyl)-2-oxo-1,4,8-triazaspiro[4,5]décane-8-thiocarboxylique,
111. le 4-méthoxybenzylamide de l'acide 1-méthyl-3-(2-méthylsulfanyléthyl)-2-oxo-1,4,8-triazaspiro[4,5]décane-8-thiocarboxylique,
112. le (4-fluorobenzène)-amide de l'acide 2-isobutyl-3-oxo-1,4,8-triazaspiro[4,5]décane-8-thiocarboxylique,
113. l'ester éthylique de l'acide [8-éthoxycarbonylaminocarbothioyl-3-(2-méthylsulfanyléthyl)-2-oxo-1,4,8-triazaspiro[4,5]déc-1-yl]-acétique,
114. l'ester éthylique de l'acide 3-{ [1-éthoxycarbonylméthyl-3-(2-méthylsulfanyléthyl)-2-oxo-1,4,8-triazaspiro[4,5]décane-8-carbothioyl]-amino}-butyrique,
115. le (2-méthoxybenzène)-amide de l'acide 1-allyl-3-isopropyl-2-oxo-1,4,8-triazaspiro[4,5]décane-8-thiocarboxylique,
116. le benzénamide de l'acide 1-butyl-3-isopropyl-2-oxo-1,4,8-triazaspiro[4,5]décane-8-thiocarboxylique,
117. le (2,5-dichlorobenzène)-amide de l'acide 1-butyl-3-isopropyl-2-oxo-1,4,8-triazaspiro[4,5]décane-8-thiocarboxylique,
118. le (2,5-difluorobenzène)-amide de l'acide 3-benzyl-1-méthyl-2-oxo-1,4,8-triazaspiro[4,5]décane-8-thiocarboxylique,
119. le (2-bromobenzène)-amide de l'acide 1-méthyl-3-(2-méthylsulfanyléthyl)-2-oxo-1,4,8-triazaspiro[4,5]décane-8-thiocarboxylique,
120. le (2,5-dichlorobenzène)-amide de l'acide 1-butyl-3-(2-méthylsulfanyléthyl)-2-oxo-1,4,8-triazaspiro[4,5]décane-8-thiocarboxylique,
121. le (2-méthoxyéthyl)-amide de l'acide 3-(2-méthylsulfanyléthyl)-1-naphtalén-2-ylméthyl-2-oxo-1,4,8-triazaspiro[4,5]décane-8-thiocarboxylique,
122. le (2-fluorobenzène)-amide de l'acide 1-butyl-3-isopropyl-2-oxo-1,4,8-triazaspiro[4,5]décane-8-thiocarboxylique,
123. l'allylamide de l'acide 3-isobutyl-2-oxo-1-prop-2-ynyl-1,4,8-triazaspiro[4,5]décane-8-thiocarboxylique,
124. le m-toluylamide de l'acide 1-butyl-3-isopropyl-2-oxo-1,4,8-triazaspiro[4,5]décane-8-thiocarboxylique,
125. le (4-fluorobenzène)-amide de l'acide 1-allyl-3-isopropyl-2-oxo-1,4,8-triazaspiro[4,5]décane-8-thiocarboxylique,
126. l'ester méthylique de l'acide 4-(8-allylthiocarbamoyl-3-isopropyl-2-oxo-1,4,8-triazaspiro[4,5]déc-1-ylméthyl)-benzoïque,
127. le (2-méthoxybenzène)-amide de l'acide 1-butyl-3-(2-méthylsulfanyléthyl)-2-oxo-1,4,8-triazaspiro[4,5]décane-8-thiocarboxylique,
128. le (2,5-difluorobenzène)-amide de l'acide 1-allyl-3-isopropyl-2-oxo-1,4,8-triazaspiro[4,5]décane-8-thiocarboxylique,
129. l'allylamide de l'acide 1-(2-cyanobenzyl)-3-(2-méthylsulfanyléthyl)-2-oxo-1,4,8-triazaspiro[4,5]décane-8-thiocarboxylique,
130. l'allylamide de l'acide 1-(3-cyanobenzyl)-3-(2-méthylsulfanyléthyl)-2-oxo-1,4,8-triazaspiro[4,5]décane-8-thiocarboxylique,
131. l'ester éthylique de l'acide 3-[(3-benzyl-1-méthyl-2-oxo-1,4,8-triazaspiro[4,5]décane-8-carbothioyl)-amino]-butyrique,
132. le cyclohexylméthylamide de l'acide 1-butyl-3-(2-méthylsulfanyléthyl)-2-oxo-1,4,8-triazaspiro[4,5]décane-8-thiocarboxylique,
133. le (4-chlorobenzène)-amide de l'acide 1-méthyl-3-(2-méthylsulfanyléthyl)-2-oxo-1,4,8-triazaspiro[4,5]décane-8-thiocarboxylique,
134. le 4-fluorobenzylamide de l'acide 1-méthyl-3-(2-méthylsulfanyléthyl)-2-oxo-1,4,8-triazaspiro[4,5]décane-8-thiocarboxylique,
135. l'ester méthylique de l'acide 4-[8-(2,6-dichlorobenzènethiocarbamoyl)-3-isopropyl-2-oxo-1,4,8-triazaspiro[4,5]déc-1-ylméthyl]-benzoïque,
136. le (2,5-dichlorobenzène)-amide de l'acide 1-benzyl-3-isopropyl-2-oxo-1,4,8-triazaspiro[4,5]décane-8-thiocarboxylique,
137. l'ester méthylique de l'acide 4-{3-isopropyl-2-oxo-8-[(tétrahydrofurann-2-ylméthyl)-thiocarbamoyl]-1,4,8-triazaspiro[4,5]déc-1-ylméthyl}-benzoïque,
138. le (2,5-difluorobenzène)-amide de l'acide 1-méthyl-3-(2-méthylsulfanyléthyl)-2-oxo-1,4,8-triazaspiro[4,5]décane-8-thiocarboxylique,
139. le (4-isopropylbenzène)-amide de l'acide 1-benzyl-3-(2-méthylsulfanyléthyl)-2-oxo-1,4,8-triazaspiro[4,5]décane-8-thiocarboxylique,
140. le (2,5-difluorobenzène)-amide de l'acide 1-benzyl-3-(2-méthylsulfanyléthyl)-2-oxo-1,4,8-triazaspiro[4,5]décane-8-thiocarboxylique,
141. le (3-acétylbenzène)-amide de l'acide 1-(2-cyanobenzyl)-3-isobutyl-2-oxo-1,4,8-triazaspiro[4,5]décane-8-thiocarboxylique,
142. le (3-chlorobenzène)-amide de l'acide 1-butyl-3-isopropyl-2-oxo-1,4,8-triazaspiro[4,5]décane-8-thiocarboxylique,
143. l'ester méthylique de l'acide 2-[(1-butyl-3-isopropyl-2-oxo-1,4,8-triazaspiro[4,5]décane-8-carbothioyl)-amino]-benzoïque,
144. l'ester éthylique de l'acide (3-benzyl-8-éthoxycarbonylaminocarbothioyl-2-oxo-1,4,8-triazaspiro[4,5]déc-1-yl)-acétique,
145. l'ester méthylique de l'acide 2-[(3-benzyl-1-éthoxycarbonylméthyl-2-oxo-1,4,8-triazaspiro[4,5]décane-8-carbothioyl)-amino]-benzoïque,
146. l'ester éthylique de l'acide [1-(2-cyanobenzyl)-3-(2-méthylsulfanyléthyl)-2-oxo-1,4,8-triazaspiro[4,5]décane-8-carbothioyl]-carbamique,
147. le (2-chloro-5-trifluorométhylbenzène)-amide de l'acide 1-(2-cyanobenzyl)-3-(2-méthylsulfanyléthyl)-2-oxo-1,4,8-triazaspiro[4,5]décane-8-thiocarboxylique,
148. le (1-benzénéthyl)-amide de l'acide 3-(2-méthylsulfanyléthyl)-1-naphtalén-2-ylméthyl-2-oxo-1,4,8-triazaspiro[4,5]décane-8-thiocarboxylique,
149. le pentafluorobenzénamide de l'acide 1-allyl-3-isopropyl-2-oxo-1,4,8-triazaspiro[4,5]décane-8-thiocarboxylique,
150. le 4-chlorobenzylamide de l'acide 1-butyl-3-isopropyl-2-oxo-1,4,8-triazaspiro[4,5]décane-8-thiocarboxylique,
151. l'o-toluylamide de l'acide 3-(2-méthylsulfanyléthyl)-1-naphtalén-2-ylméthyl-2-oxo-1,4,8-triazaspiro[4,5]décane-8-thiocarboxylique,
152. le pentafluorobenzénamide de l'acide 1-butyl-3-isopropyl-2-oxo-1,4,8-triazaspiro[4,5]décane-8-thiocarboxylique,
153. l'ester méthylique de l'acide 2-{[1-(2-cyanobenzyl)-3-(2-méthylsulfanyléthyl)-2-oxo-1,4,8-triazaspiro[4,5]décane-8-carbothioyl]-aminol-benzoïque,
154. le pentafluorobenzénamide de l'acide 1-benzyl-3-isopropyl-2-oxo-1,4,8-triazaspiro[4,5]décane-8-thiocarboxylique,
155. le (2-méthoxybenzène)-amide de l'acide 1-benzyl-3-isopropyl-2-oxo-1,4,8-triazaspiro[4,5]décane-8-thiocarboxylique,
156. le benzénamide de l'acide 1-benzyl-3-isopropyl-2-oxo-1,4,8-triazaspiro[4,5]décane-8-thiocarboxylique,
157. le (2,5-difluorobenzène)-amide de l'acide 1-benzyl-3-isopropyl-2-oxo-1,4,8-triazaspiro[4,5]décane-8-thiocarboxylique,
158. le (4-éthoxybenzène)-amide de l'acide 1-butyl-3-(2-méthylsulfanyléthyl)-2-oxo-1,4,8-triazaspiro[4,5]décane-8-thiocarboxylique,
159. l'ester éthylique de l'acide [3-benzyl-8-(cyclohexylméthylthiocarbamoyl)-2-oxo-1,4,8-triazaspiro[4,5]déc-1-yl]-acétique,
160. le benzylamide de l'acide 1-butyl-3-(2-méthylsulfanyléthyl)-2-oxo-1,4,8-triazaspiro[4,5]décane-8-thiocarboxylique,
161. le m-toluylamide de l'acide 1-(2-cyanobenzyl)-3-(2-méthylsulfanyléthyl)-2-oxo-1,4,8-triazaspiro[4,5]décane-8-thiocarboxylique,
162. le 4-fluorobenzylamide de l'acide 1-allyl-3-isopropyl-2-oxo-1,4,8-triazaspiro[4,5]décane-8-thiocarboxylique,
163. le (3-acétylbenzène)-amide de l'acide 3-isobutyl-2-oxo-1-prop-2-ynyl-1,4,8-triazaspiro[4,5]décane-8-thiocarboxylique,
164. l'ester méthylique de l'acide 4-(3-isopropyl-2-oxo-8-pentafluorobenzènethiocarbamoyl-1,4,8-triazaspiro[4,5]déc-1-ylméthyl)-benzoïque,
165. le (3-acétylbenzène)-amide de l'acide 3-(2-méthylsulfanyléthyl)-1-naphtalén-2-ylméthyl-2-oxo-1,4,8-triazaspiro[4,5]décane-8-thiocarboxylique,
166. le m-toluylamide de l'acide 3-benzyl-1-(2-fluorobenzyl)-2-oxo-1,4,8-triazaspiro[4,5]décane-8-thiocarboxylique,
167. l'ester méthylique de l'acide 4-(8-éthoxycarbonylaminocarbothioyl-3-isopropyl-2-oxo-1,4,8-triazaspiro[4,5]déc-1-ylméthyl)-benzoïque,
168. le (tétrahydrofurann-2-ylméthyl)-amide de l'acide 1-(4-fluorobenzyl)-3-(2-méthylsulfanyléthyl)-2-oxo-1,4,8-triazaspiro[4,5]décane-8-thiocarboxylique,
169. le (3,5-bis-trifluorométhylbenzène)-amide de l'acide 1-(2-cyanobenzyl)-3-isobutyl-2-oxo-1,4,8-triazaspiro[4,5]décane-8-thiocarboxylique,
170. le benzylamide de l'acide 1-(2-cyanobenzyl)-3-isobutyl-2-oxo-1,4,8-triazaspiro[4,5]décane-8-thiocarboxylique,
171. le cyclohexylméthylamide de l'acide 3-benzyl-1-(2-fluorobenzyl)-2-oxo-1,4,8-triazaspiro[4,5]décane-8-thiocarboxylique,
172. le (4-chlorobenzène)-amide de l'acide 1-(3-cyanobenzyl)-3-(2-méthylsulfanyléthyl)-2-oxo-1,4,8-triazaspiro[4,5]décane-8-thiocarboxylique,
173. le pentafluorobenzénamide de l'acide 1-butyl-3-(2-méthylsulfanyléthyl)-2-oxo-1,4,8-triazaspiro[4,5]décane-8-thiocarboxylique,
174. l'ester éthylique de l'acide [3-benzyl-1-(2-fluorobenzyl)-2-oxo-1,4,8-triazaspiro[4,5]décane-8-carbothioyl]-carbamique,
175. le 4-chlorobenzylamide de l'acide 3-benzyl-1-méthyl-2-oxo-1,4,8-triazaspiro[4,5]décane-8-thiocarboxylique,
176. l'isopropylamide de l'acide 1-(4-fluorobenzyl)-3-(2-méthylsulfanyléthyl)-2-oxo-1,4,8-triazaspiro[4,5]décane-8-thiocarboxylique,
177. le (3,5-dichlorobenzène)-amide de l'acide 1-benzyl-3-isopropyl-2-oxo-1,4,8-triazaspiro[4,5]décane-8-thiocarboxylique,
178. l'ester éthylique de l'acide [1-(3-cyanobenzyl)-3-(2-méthylsulfanyléthyl)-2-oxo-1,4,8-triazaspiro[4,5]décane-8-carbothioyl]-carbamique,
179. le 4-fluorobenzylamide de l'acide 1-benzyl-3-isopropyl-2-oxo-1,4,8-triazaspiro[4,5]décane-8-thiocarboxylique,
180. le (3,5-dichlorobenzène)-amide de l'acide 1-allyl-3-isopropyl-2-oxo-1,4,8-triazaspiro[4,5]décane-8-thiocarboxylique,
181. le (1-benzénéthyl)-amide de l'acide 1-(3-cyanobenzyl)-3-(2-méthylsulfanyléthyl)-2-oxo-1,4,8-triazaspiro[4,5]décane-8-thiocarboxylique,
182. le 4-fluorobenzylamide de l'acide 1-(4-fluorobenzyl)-3-(2-méthylsulfanyléthyl)-2-oxo-1,4,8-triazaspiro[4,5]décane-8-thiocarboxylique,
183. le (2-méthoxybenzène)-amide de l'acide 1-(2-cyanobenzyl)-3-(2-méthylsulfanyléthyl)-2-oxo-1,4,8-triazaspiro[4,5]décane-8-thiocarboxylique,
184. le (2-méthoxybenzène)-amide de l'acide 1-(3-cyanobenzyl)-3-(2-méthylsulfanyléthyl)-2-oxo-1,4,8-triazaspiro[4,5]décane-8-thiocarboxylique,
185. le 4-chlorobenzylamide de l'acide 1-(3-cyanobenzyl)-3-(2-méthylsulfanyléthyl)-2-oxo-1,4,8-triazaspiro[4,5]décane-8-thiocarboxylique,
186. l'o-toluylamide de l'acide 1-(4-fluorobenzyl)-3-(2-méthylsulfanyléthyl)-2-oxo-1,4,8-triazaspiro[4,5]décane-8-thiocarboxylique,
187. le (2,6-dichlorobenzène)-amide de l'acide 3-benzyl-1-(2-fluorobenzyl)-2-oxo-1,4,8-triazaspiro[4,5]décane-8-thiocarboxylique,
188. le (2-chloro-5-trifluorométhylbenzène)-amide de l'acide 3-benzyl-1-(2-fluorobenzyl)-2-oxo-1,4,8-triazaspiro[4,5]décane-8-thiocarboxylique,
189. le (4-fluorobenzène)-amide de l'acide 1-butyl-3-(2-méthylsulfanyléthyl)-2-oxo-1,4,8-triazaspiro[4,5]décane-8-thiocarboxylique,
190. le (2,6-dichlorobenzène)-amide de l'acide 3-isobutyl-2-oxo-1-prop-2-ynyl-1,4,8-triazaspiro[4,5]décane-8-thiocarboxylique,
191. l'ester méthylique de l'acide 4-[8-(4-chlorobenzylthiocarbamoyl)-3-isopropyl-2-oxo-1,4,8-triazaspiro[4,5]déc-1-ylméthyl]-benzoïque,
192. le (3-cyanobenzène)-amide de l'acide 1-(4-fluorobenzyl)-3-(2-méthylsulfanyléthyl)-2-oxo-1,4,8-triazaspiro[4,5]décane-8-thiocarboxylique,
193. le 4-chlorobenzylamide de l'acide 1-(2-cyanobenzyl)-3-(2-méthylsulfanyléthyl)-2-oxo-1,4,8-triazaspiro[4,5]décane-8-thiocarboxylique,
194. le (3-trifluorométhylbenzène)-amide de l'acide 1-méthyl-3-(2-méthylsulfanyléthyl)-2-oxo-1,4,8-triazaspiro[4,5]décane-8-thiocarboxylique,
195. le (3,5-dichlorobenzène)-amide de l'acide 3-isobutyl-2-oxo-1-prop-2-ynyl-1,4,8-triazaspiro[4,5]décane-8-thiocarboxylique,
196. le (1-benzénéthyl)-amide de l'acide 1-(4-fluorobenzyl)-3-(2-méthylsulfanyléthyl)-2-oxo-1,4,8-triazaspiro[4,5]décane-8-thiocarboxylique,
197. le 4-chlorobenzylamide de l'acide 3-benzyl-1-(2-fluorobenzyl)-2-oxo-1,4,8-triazaspiro[4,5]décane-8-thiocarboxylique,
198. le (4-fluorobenzène)-amide de l'acide 2-isopropyl-3-oxo-1,4,8-triazaspiro[4,5]décane-8-thiocarboxylique,
199. le benzylamide de l'acide 1-benzyl-3-isopropyl-2-oxo-1,4,8-triazaspiro[4,5]décane-8-thiocarboxylique,
200. le (4-éthoxybenzène)-amide de l'acide 1-(4-fluorobenzyl)-3-(2-méthylsulfanyléthyl)-2-oxo-1,4,8-triazaspiro[4,5]décane-8-thiocarboxylique,
201. le (3-acétylbenzène)-amide de l'acide 3-benzyl-1-(2-fluorobenzyl)-2-oxo-1,4,8-triazaspiro[4,5]décane-8-thiocarboxylique,
202. le cyclohexylamide de l'acide 3-benzyl-1-méthyl-2-oxo-1,4,8-triazaspiro[4,5]décane-8-carboxylique,
203. l'ester éthylique de l'acide [3-(2-méthylsulfanyléthyl)-2-oxo-8-benzènecarbamoyl-1,4,8-triazaspiro[4,5]déc-1-yl]-acétique,
204. le (2,5-diméthoxybenzène)-amide de l'acide 1-allyl-3-isopropyl-2-oxo-1,4,8-triazaspiro[4,5]décane-8-carboxylique,
205. le (3-fluorobenzène)-amide de l'acide 1-méthyl-3-(2-méthylsulfanyléthyl)-2-oxo-1,4,8-triazaspiro[4,5]décane-8-carboxylique,
206. l'ester méthylique de l'acide 4-(8-cyclohexylcarbamoyl-3-isopropyl-2-oxo-1,4,8-triazaspiro[4,5]déc-1-ylméthyl)-benzoïque,
207. le benzénamide de l'acide 1-butyl-3-(2-méthylsulfanyléthyl)-2-oxo-1,4,8-triazaspiro[4,5]décane-8-carboxylique,
208. le (3-cyanobenzène)-amide de l'acide 1-benzyl-3-(2-méthylsulfanyléthyl)-2-oxo-1,4,8-triazaspiro[4,5]décane-8-carboxylique,
209. le (2,5-difluorobenzène)-amide de l'acide 1-allyl-3-isopropyl-2-oxo-1,4,8-triazaspiro[4,5]décane-8-carboxylique,
210. le (3,4,5-triméthoxybenzène)-amide de l'acide 1-butyl-3-(2-méthylsulfanyléthyl)-2-oxo-1,4,8-triazaspiro[4,5]décane-8-carboxylique,
211. le (4-chloro-3-trifluorométhylbenzène)-amide de l'acide 1-benzyl-3-(2-méthylsulfanyléthyl)-2-oxo-1,4,8-triazaspiro[4,5]décane-8-carboxylique,
212. le (5-chloro-2-méthoxybenzène)-amide de l'acide 1-méthyl-3-(2-méthylsulfanyléthyl)-2-oxo-1,4,8-triazaspiro[4,5]décane-8-carboxylique,
213. le (4-éthoxybenzène)-amide de l'acide 1-benzyl-3-(2-méthylsulfanyléthyl)-2-oxo-1,4,8-triazaspiro[4,5]décane-8-carboxylique,
214. le (3-acétylbenzène)-amide de l'acide 1-benzyl-3-(2-méthylsulfanyléthyl)-2-oxo-1,4,8-triazaspiro[4,5]décane-8-carboxylique,
215. l'ester éthylique de l'acide 3-[(1-butyl-3-isopropyl-2-oxo-1,4,8-triazaspiro[4,5]décane-8-carbonyl)-amino]-benzoïque,
216. le (2,5-diméthoxybenzène)-amide de l'acide 1-butyl-3-isopropyl-2-oxo-1,4,8-triazaspiro[4,5]décane-8-carboxylique,
217. le 3,4-dichlorobenzylamide de l'acide 1-(2-cyanobenzyl)-3-isobutyl-2-oxo-1,4,8-triazaspiro[4,5]décane-8-carboxylique,
218. le (2,5-difluorobenzène)-amide de l'acide 1-(2-cyanobenzyl)-3-isobutyl-2-oxo-1,4,8-triazaspiro[4,5]décane-8-carboxylique,
219. le 3,4-dichlorobenzylamide de l'acide 1-butyl-3-(2-méthylsulfanyléthyl)-2-oxo-1,4,8-triazaspiro[4,5]décarie-8-carboxylique,
220. le (4-trifluorométhoxybenzène)-amide de l'acide 1-allyl-3-isopropyl-2-oxo-1,4,8-triazaspiro[4,5]décane-8-carboxylique,
221. le (4-éthoxybenzène)-amide de l'acide 1-butyl-3-(2-méthylsulfanyléthyl)-2-oxo-1,4,8-triazaspiro[4,5]décane-8-carboxylique,
222. le (4-trifluorométhoxybenzène)-amide de l'acide 1-méthyl-3-(2-méthylsulfanyléthyl)-2-oxo-1,4,8-triazaspiro[4,5]décane-8-carboxylique,
223. l'ester méthylique de l'acide 4-[3-isopropyl-8-(3-méthoxybenzènecarbamoyl)-2-oxo-1,4,8-triazaspiro[4,5]déc-1-ylméthyl]-benzoïque,
224. le benzénamide de l'acide 1-(2-cyanobenzyl)-3-(2-méthylsulfanyléthyl)-2-oxo-1,4,8-triazaspiro[4,5]décane-8-carboxylique,
225. l'ester éthylique de l'acide [3-benzyl-8-(3,4-dichlorobenzylcarbamoyl)-2-oxo-1,4,8-triazaspiro[4,5]déc-1-yl]-acétique,
226. le (3-fluorobenzène)-amide de l'acide 1-(2-cyanobenzyl)-3-(2-méthylsulfanyléthyl)-2-oxo-1,4,8-triazaspiro[4,5]décane-8-carboxylique,
227. le (4-chloro-3-trifluorométhylbenzène)-amide de l'acide 1-butyl-3-(2-méthylsulfanyléthyl)-2-oxo-1,4,8-triazaspiro[4,5]décane-8-carboxylique,
228. le 3,4-dichlorobenzylamide de l'acide 1-(4-fluorobenzyl)-3-(2-méthylsulfanyléthyl)-2-oxo-1,4,8-triazaspiro[4,5]décane-8-carboxylique,
229. le 3,4-dichlorobenzylamide de l'acide 1-(2-cyanobenzyl)-3-(2-méthylsulfanyléthyl)-2-oxo-1,4,8-triazaspiro[4,5]décane-8-carboxylique,
230. le (2,5-difluorobenzène)-amide de l'acide 3-benzyl-1-(2-fluorobenzyl)-2-oxo-1,4,8-triazaspiro[4,5]décane-8-carboxylique,
231. le (4-éthoxybenzène)-amide de l'acide 3-(2-méthylsulfanyléthyl)-1-naphtalén-2-ylméthyl-2-oxo-1,4,8-triazaspiro[4,5]décane-8-carboxylique,
232. l'ester méthylique de l'acide 4-[8-(3,4-dichlorobenzylcarbamoyl)-3-isopropyl-2-oxo-1,4,8-triazaspiro[4,5]déc-1-ylméthyl]-benzoïque,
233. le 3,4-dichlorobenzylamide de l'acide 3-benzyl-1-(2-fluorobenzyl)-2-oxo-1,4,8-triazaspiro[4,5]décane-8-carboxylique,
234. le (3-acétylbenzène)-amide de l'acide 1-(2-cyanobenzyl)-3-(2-méthylsulfanyléthyl)-2-oxo-1,4,8-triazaspiro[4,5]décane-8-carboxylique,
235. l'ester éthylique de l'acide 4-{[3-benzyl-1-(2-fluorobenzyl)-2-oxo-1,4,8-triazaspiro[4,5]décane-8-carbonyl]-amino}-benzoïque,
236. le (3-méthoxybenzène)-amide de l'acide, 1-(3-cyanobenzyl)-3-(2-méthylsulfanyléthyl)-2-oxo-1,4,8-triazaspiro[4,5]décane-8-carboxylique,
237. le (4-trifluorométhoxybenzène)-amide de l'acide 1-(4-fluorobenzyl)-3-(2-méthylsulfanyléthyl)-2-oxo-1,4,8-triazaspiro[4,5]décane-8-carboxylique,
238. le cyclohexylamide de l'acide 3-benzyl-1-(2-fluorobenzyl)-2-oxo-1,4,8-triazaspiro[4,5]décane-8-carboxylique,
239. le phénéthylamide de l'acide 1-(4-fluorobenzyl)-3-(2-méthylsulfanyléthyl)-2-oxo-1,4,8-triazaspiro[4,5]décane-8-carboxylique,
240. le (3-fluorobenzène)-amide de l'acide 3-(2-méthylsulfanyléthyl)-1-naphtalén-2-ylméthyl-2-oxo-1,4,8-triazaspiro[4,5]décane-8-carboxylique,
241. l'ester méthylique de l'acide 4-[8-(4-chloro-3-trifluorométhylbenzènecarbamoyl)-3-isopropyl-2-oxo-1,4,8-triazaspiro[4,5]déc-1-ylméthyl]-benzoïque,
242. le (2,5-diméthoxybenzène)-amide de l'acide 1-(3-cyanobenzyl)-3-(2-méthylsulfanyléthyl)-2-oxo-1,4,8-triazaspiro[4,5]décane-8-carboxylique,
243. le (4-éthoxybenzène)-amide de l'acide 1-(4-fluorobenzyl)-3-(2-méthylsulfanyléthyl)-2-oxo-1,4,8-triazaspiro[4,5]décane-8-carboxylique,
244. le (3-cyanobenzène)-amide de l'acide 1-(4-fluorobenzyl)-3-(2-méthylsulfanyléthyl)-2-oxo-1,4,8-triazaspiro[4,5]décane-8-carboxylique,
245. l'ester éthylique de l'acide [3-benzyl-8-(3-fluorobenzènecarbamoyl)-2-oxo-1,4,8-triazaspiro[4,5]déc-1-yl]-acétique,
246. la 1,3-dibenzyl-8-méthanesulfonyl-1,4,8-triazaspiro[4,5]décan-2-one
247. la 8-benzènesulfonyl-1,3-dibenzyl-1,4,8-triazaspiro[4,5]décan-2-one,
248. la 1,3-dibenzyl-8-(4-chlorobenzènesulfonyl)-1,4,8-triazaspiro[4,5]décan-2-one,
249. la 1,3-dibenzyl-8-(4-méthoxybenzènesulfonyl)-1,4,8-triazaspiro[4,5]décan-2-one,
250. le phénylamide de l'acide 1,3-dibenzyl-2-oxo-1,4,8-triazaspiro[4,5]décane-8-carboxylique,
251. le (4-méthoxyphényl)-amide de l'acide 1,3-dibenzyl-2-oxo-1,4,8-triazaspiro[4,5]décane-8-carboxylique,
252. le phénylamide de l'acide 1,3-dibenzyl-2-oxo-1,4,8-triazaspiro[4,5]décane-8-thiocarboxylique,
253. le (2,4-difluorophényl)-amide de l'acide 1,3-dibenzyl-2-oxo-1,4,8-triazaspiro[4,5]décane-8-thiocarboxylique,
le cas échéant à chaque fois sous forme d'un de leurs stéréo-isomères purs, en particulier leurs énantiomères ou diastéréo-isomères, leurs racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

13. Procédé pour la préparation de composés substitués de la 1,4,8-triazaspiro[4,5]décan-2-one selon l'une ou plusieurs des revendications 1 à 12, **caractérisé**
**en ce qu'**on transforme une pipéridin-4-one protégée de formule générale II, 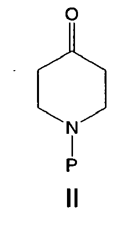 où P représente un groupe de protection, par transformation avec au moins un composé de formule générale III, 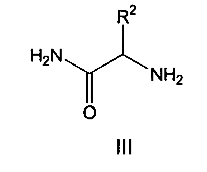 où R² a la signification selon l'une ou plusieurs des revendications 1 à 12, en au moins un composé de formule générale IV, 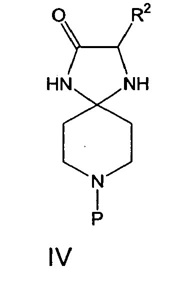 où P et R² ont la signification susmentionnée, qui est le cas échéant purifié et/ou le cas échéant isolé, et qui est transformé le cas échéant par transformation avec au moins un composé de formule générale R¹-X¹, où R¹ a la signification selon l'une ou plusieurs des revendications 1 à 12 et X¹ représente un groupe partant approprié, de préférence un radical halogène, le cas échéant en présence d'au moins une base, en au moins un composé de formule générale V, 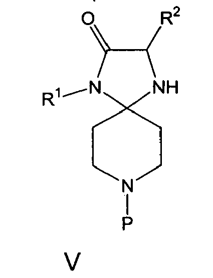 où R¹, R² et P ont la signification susmentionnée et qui est le cas échéant purifié et/ou le cas échéant isolé et le cas échéant transformé, par dissociation du groupe de protection P en au moins un composé de formule générale VI, 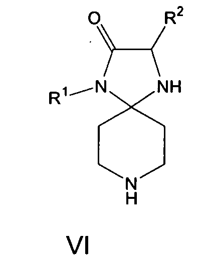 et celui-ci est le cas échéant purifié et/ou le cas échéant isolé et au moins un composé de formule générale IV, V ou VI est transformé par transformation avec un composé sulfonyle de formule générale R⁹-SO₂-X², un thiocyanate de formule générale R⁵-NCS ou un isocyanate de formule générale R⁶-NCO, où les radicaux R⁴, R⁵ et R⁶ présentent à chaque fois la signification selon l'une ou plusieurs des revendications 1 à 12 et X² représente un groupe partant approprié, de préférence un radical halogène, en au moins un composé de formule générale I selon la revendication 1, où les radicaux R¹-R³ présentent à chaque fois la signification selon l'une ou plusieurs des revendications 1 à 12, et ce composé est le cas échéant purifié et/ou le cas échéant isolé.

14. Médicament contenant au moins un composé substitué de 1,4,8-triazaspiro[4,5]décan-2-one selon l'une ou plusieurs des revendications 1 à 12 et le cas échéant un ou plusieurs adjuvants pharmaceutiquement acceptables.

15. Médicament selon la revendication 14 destiné à la régulation, en particulier l'inhibition, de la résorption de la noradrénaline (Noradrenalin-Uptake), destiné à la régulation, en particulier l'inhibition de la résorption du 5-hydroxytryptophane (5-HT-Uptake) et/ou destiné à la régulation du récepteur de la batrachotoxine (BTX) et/ou à la régulation du récepteur de CB2.

16. Médicament selon la revendication 14 ou 15 destiné à la prophylaxie et/ou au traitement de dépressions.

17. Médicament selon la revendication 14 ou 15 destiné à la prophylaxie et/ou au traitement de douleurs, de préférence au traitement et/ou à la prophylaxie de douleurs aiguës, de douleurs chroniques, de douleurs neuropathiques et/ou d'algies vasculaires de la face.

18. Médicament selon la revendication 14 ou 15 destiné à la prophylaxie et/ou au traitement combinés de dépression et de douleurs, de préférence au traitement combiné de dépressions et de douleurs choisies dans le groupe constitué par les douleurs aiguës, les douleurs chroniques, les douleurs neuropathiques et les algies vasculaires de la face.

19. Médicament selon la revendication 14 ou 15 destiné à la prophylaxie et/ou au traitement de l'abus d'alcool et/ou de drogues et/ou de médicaments, à la prophylaxie et/ou au traitement de la dépendance à l'alcool et/ou aux drogues et/ou aux médicaments, à la prophylaxie et/ou au traitement d'inflammations, à la prophylaxie et/ou au traitement du manque d'entrain, à la prophylaxie et/ou au traitement de troubles de l'absorption alimentaire, de préférence choisis dans le groupe constitué par la boulimie, l'anorexie, l'obésité et la cachexie, à la prophylaxie et/ou au traitement de la catalepsie, à l'augmentation de la vigilance, à l'augmentation de la libido ou à l'anxiolyse, à la prophylaxie et/ou au traitement de maladies de neurodégénérescence, de préférence d'une ou de plusieurs maladies de neurodégénérescence choisies dans le groupe constitué par la maladie de Parkinson, de Huntington, d'Alzheimer et de la sclérose en plaques, à la prophylaxie et/ou au traitement de l'ischémie et/ou à l'anesthésie locale.

20. Médicament selon la revendication 14 ou 15 pour le traitement et/ou la prophylaxie d'une ou de plusieurs maladies choisies dans le groupe constitué par les maladies allergiques non aiguës, de préférence la dermatite allergique ; l'asthme ; la rhinite ; la conjonctivite ; les maladies qui sont provoquées par le 2-arachidoneglycérol et/ou les éthers correspondants tels que l'Hematol ; la septicémie ; le cancer, en particulier le cancer du sang et/ou les tumeurs au cerveau ; les troubles de la circulation ; le glaucome ; les inflammations, en particulier les maladies inflammatoires provoquées par voie immunologique; de manière particulièrement préférée la polyarthrite rhumatoïde, le lupus érythémateux, le psoriasis et la thyroïdite ; le diabète ; l'empoisonnement du sang ; l'épilepsie ; le syndrome de Gilles de la Tourette ; l'ostéoporose ; la maladie de Bechterew ; la goutte ; l'arthrite goutteuse ; l'ostéoarthrite ; les troubles d'irrigation sanguine ; les ischémies, de préférence l'ischémie rénale, et/ou à la régulation du système immunitaire, de préférence à la suppression du système immunitaire.

21. Utilisation d'au moins un composé substitué de 1,4,8-triazaspiro[4,5]décan-2-one selon l'une quelconque des revendications 1 à 12 pour la préparation d'un médicament destiné à la régulation de la résorption de la noradrénaline (Noradrenalin-Uptake), de préférence à l'inhibition de la résorption de la noradrénaline (Noradrenalin-Uptake).

22. Utilisation d'au moins un composé substitué de 1,4,8-triazaspiro[4,5]décan-2-one selon l'une quelconque des revendications 1 à 12 pour la préparation d'un médicament destiné à la régulation de la résorption du 5-hydroxytryptophane (5-HAT-Uptake), de préférence à l'inhibition du 5-hydroxytryptophane (5-HT-Uptake).

23. Utilisation d'au moins un composé substitué de 1,4,8-triazaspiro[4,5]décan-2-one selon l'une quelconque des revendications 1 à 12 pour la préparation d'un médicament destiné à la régulation du récepteur de la batrachotoxine-(BTX).

24. Utilisation d'au moins un composé substitué de 1,4,8-triazaspiro[4,5]décan-2-one selon l'une quelconque des revendications 1 à 12 pour la préparation d'un médicament destiné à la régulation du récepteur de CB2.

25. Utilisation d'au moins un composé substitué de 1,4,8-triazaspiro[4,5]décan-2-one selon l'une quelconque des revendications 1 à 12 pour la préparation d'un médicament destiné à la prophylaxie et/ou au traitement de dépressions.

26. Utilisation d'au moins un composé substitué de 1,4,8-triazaspiro[4,5]décan-2-one selon l'une quelconque des revendications 1 à 12 pour la préparation d'un médicament destiné à la prophylaxie et/ou au traitement de douleurs, de préférence au traitement et/ou à la prophylaxie de douleurs aiguës, de douleurs chroniques, de douleurs neuropathiques et/ou d'algies vasculaires de la face.

27. Utilisation d'au moins un composé substitué de 1,4,8-triazaspiro[4,5]décan-2-one selon l'une quelconque des revendications 1 à 12 pour la préparation d'un médicament destiné à la prophylaxie et/ou au traitement combinés de dépressions et de douleurs, de préférence à la prophylaxie et/au au traitement combinés de dépressions et de douleurs choisies dans le groupe constitué par les douleurs aiguës, les douleurs chroniques, les douleurs neuropathiques et les algies vasculaires de la face.

28. Utilisation d'au moins un composé substitué de 1,4,8-triazaspiro[4,5]décan-2-one selon l'une quelconque des revendications 1 à 12 pour la préparation d'un médicament destiné à la prophylaxie et/ou au traitement de l'abus d'alcool et/ou de drogues et/ou de médicaments, à la prophylaxie et/ou au traitement de la dépendance à l'alcool et/ou aux drogues et/ou aux médicaments, à la prophylaxie et/ou au traitement d'inflammations, à la prophylaxie et/ou au traitement du manque d'entrain, à la prophylaxie et/ou au traitement de troubles de l'absorption alimentaire, de préférence choisis dans le groupe constitué par la boulimie, l'anorexie, l'obésité et la cachexie, à la prophylaxie et/ou au traitement de la catalepsie, à l'augmentation de la vigilance, à l'augmentation de la libido ou à l'anxiolyse, à la prophylaxie et/ou au traitement de maladies de neurodégénérescence, de préférence d'une ou de plusieurs maladies de neurodégénérescence choisies dans le groupe constitué par la maladie de Parkinson, de Huntington, d'Alzheimer et de la sclérose en plaques, à la prophylaxie et/ou au traitement de l'ischémie et/ou à l'anesthésie locale.

29. Utilisation d'au moins un composé substitué de 1,4,8-triazaspiro[4,5]décan-2-one selon l'une quelconque des revendications 1 à 12 pour la préparation d'un médicament destiné au traitement et/ou à la prophylaxie d'une ou de plusieurs maladies choisies dans le groupe constitué par les maladies allergiques non aiguës, de préférence la dermatite allergique ; l'asthme ; la rhinite ; la conjonctivite ; les maladies qui sont provoquées par le 2-arachidoneglycérol et/ou les éthers correspondants tels que l'Hematol ; la septicémie ; le cancer, en particulier le cancer du sang et/ou les tumeurs au cerveau ; les troubles de la circulation ; le glaucome ; les inflammations, en particulier les maladies inflammatoires provoquées par voie immunologique, de manière particulièrement préférée la polyarthrite rhumatoïde, le lupus érythémateux, le psoriasis et la thyroïdite ; le diabète ; l'empoisonnement du sang ; l'épilepsie ; le syndrome de Gilles de la Tourette ; l'ostéoporose ; la maladie de Bechterew ; la goutte ; l'arthrite goutteuse ; l'ostéoarthrite ; les troubles d'irrigation sanguine ; les ischémies, de préférence l'ischémie rénale, et/ou à la régulation du système immunitaire, de préférence à la suppression du système immunitaire.
